# EUROPEAN PATENT SPECIFICATION

(11) **EP 4 305 037 B1**
(45) Date of publication and mention of the grant of the patent: **26.02.2025**
(21) Application number: 22714316.1
(22) Date of filing: 14.03.2022
(51) Int. Cl.: C07D 487/08, A61K 31/551, A61P 35/00

(54) **KRAS INHIBITORS**
KRAS INHIBITOREN
INHIBITEURS DE KRAS

(30) Priority: 12.03.2021 US 202163160436 P; 13.12.2021 US 202163288965 P
(43) Date of publication of application: 17.01.2024
(73) Proprietor: Bristol-Myers Squibb Company, Princeton, NJ 08543 (US)
(72) Inventor: FINK, Brian Edward, Princeton, New Jersey 08543 (US); CHERNEY, Robert Joseph, Princeton, New Jersey 08543 (US); NGU, Khehyong, Princeton, New Jersey 08543 (US); VELAPARTHI, Upender, Princeton, New Jersey 08543 (US); VACCARO, Wayne David, Princeton, New Jersey 08543 (US); RUAN, Zheming, Princeton, New Jersey 08543 (US); QIN, Lan-Ying, Princeton, New Jersey 08543 (US); SHIRUDE, Pravin S., Princeton, New Jersey 08543 (US); RAHAMAN, Hasibur, Bangalore 560 099 (IN)
(74) Representative: Mewburn Ellis LLP
(86) International application number: PCT/US2022/020230
(87) International publication number: WO 2022/192790

(56) References cited:
- WO-A1-2017/172979
- WO-A1-2021/031952

## Description

### FIELD

The present disclosure provides KRAS inhibitors. Also provided are the inhibitors for use in methods of treating cancers.

### BACKGROUND

The KRAS oncogene is a member of the Ras family of GTPases that are involved in numerous cellular signaling processes. KRAS mutations are gain-of-function mutations that are present in up to 30% of all tumors, including as many as 90% of pancreatic cancers. KRAS G 12D mutation is present in 28% of all pancreatic ductal adenocarcinoma patients, 13% of all colorectal carcinoma patients, 4% of all non-small cell lung carcinoma patients and 3% of all gastric carcinoma patients (e.g., see https://www.mycancergenome.org/content/alteration/kras-g12d/). Due to the clinical significance of this protein, many attempts have been made to develop Ras inhibitors, but such attempts have been mostly unsuccessful. This is largely due to the difficulty in outcompeting GTP for the KRAS binding pocket in cells, and the lack of known allosteric regulatory sites. Accordingly, agents that inhibit KRAS G12D are desired. WO 2021/031952 discloses six-membered cyclopyrimidine compounds, as a selective inhibitor of KRAS gene mutation, for use in the treatment of cancer.

### SUMMARY

In a first aspect, the present disclosure provides a compound of formula (I): 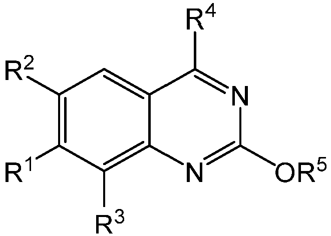 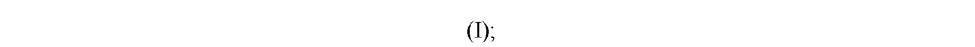 or a pharmaceutically acceptable salt thereof, wherein:
R¹ is aryl or heteroaryl, wherein the aryl and the heteroaryl are optionally substituted with one, two, three, four, or five substituents independently selected from C₁-C₃alkyl, C₂-C₄alkenyl, C₂-C₄alkynyl, amino, aminoC₁-C₃alkyl, C₃-C₄cycloalkyl, halo, haloC₁-C₃alkoxy, haloC₁-C₃alkyl, hydroxy, and hydroxyC₁-C₃alkyl;
R² and R³ are independently selected from hydrogen, C₁-C₃alkoxy, C₁-C₃alkyl, cyano, halo, haloC₁-C₃alkyl, and hydroxy;
R⁴ is selected from: 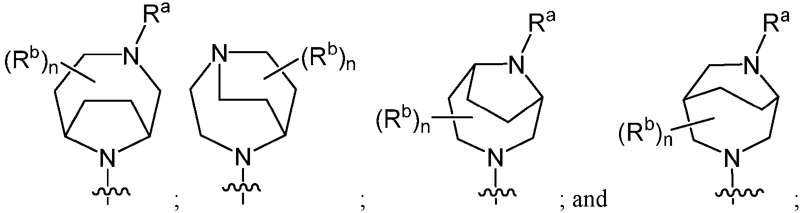 wherein
R^{a} is hydrogen or C₁-C₃alkyl;
n is 0, 1, 2, 3, or 4;
each R^{b} is independently selected from C₁-C₃alkyl, C₃-C₆cycloalkyl, halo, and hydroxy; or, alternatively, two geminal R^{b} groups, together with the carbon atom to which they are attached, can form a 3- to 6-membered cycloalkyl ring; and
^{a} denotes the point of attachment to the parent molecular moiety;
R⁵ is -(C₁-C₃alkyl)-R⁶ or -(C₁-C₆alkyl)NR^{C}R^{d}, wherein R⁶ is selected from:
a C₃-C₆cycloalkyl substituted with NR^{c}R^{d}(C₁-C₃alkyl); and
a five- to ten-membered monocyclic, bicyclic, or tricyclic ring containing one nitrogen atom and optionally a second heteroatom selected from oxygen or nitrogen, wherein the ring contains zero to three double bonds and wherein the ring is optionally substituted with one, two, or three groups independently selected from C₁-C₃alkoxy, C₁-C₃alkyl, halo, haloC₁-C₃alkoxy, haloC₁-C₃alkyl, and hydroxy; wherein
R^{C} and R^{d}, together with the nitrogen atom to which they are attached, form a five-to ten-membered ring monocyclic or bicyclic ring optionally containing one additional heteroatom selected from nitrogen, oxygen, and sulfur, wherein the ring is optionally substituted with one, two, or three groups independently selected from C₁-C₃alkoxy, C₁-C₃alkoxyC₁-C₃alkyl, C₁-C₃alkyl, benzyl, halo, haloC₁-C₃alkyl, hydroxy, hydroxyC₁-C₃alkyl, and oxo; or
one of R^{c} and R^{d} is selected from hydrogen and C₁-C₃alkyl and the other is selected from hydrogen, C₁-C₃alkyl, C₁-C₃alkoxycarbonyl, and C₁-C₃alkylcarbonyl.

In certain aspects, n is zero. In other aspects, n is 1, 2, 3 or 4. In one aspect, n is 1. In another aspect, n is 2. In another aspect, n is 3. And in another aspect, n is 4.

In some aspects, R⁴ is selected from:

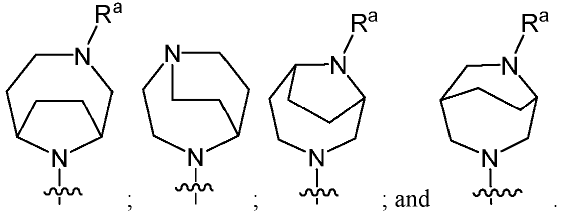

In some aspects, R⁴ is selected from: 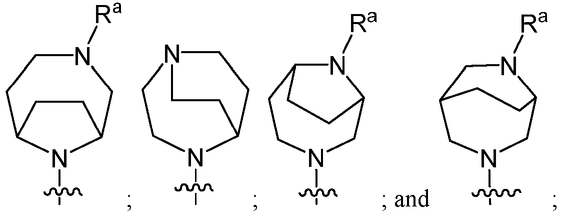 and
R⁶ is a five- to ten-membered monocyclic, bicyclic, or tricyclic ring containing one nitrogen atom and optionally a second heteroatom selected from oxygen or nitrogen, wherein the ring contains zero to three double bonds and wherein the ring is optionally substituted with one, two, or three groups independently selected from C₁-C₃alkoxy, C₁-C₃alkyl, halo, haloC₁-C₃alkoxy, haloC₁-C₃alkyl, and hydroxy.

In some aspects, R² and R³ are each halo. In some aspects, R² is chloro and R³ is fluoro. In some aspects, R² is hydrogen and R³ is fluoro.

In some aspects, R⁴ is 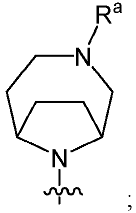 wherein R^{a} is hydrogen or C₁-C₃alkyl.

In some aspects, R⁴ is 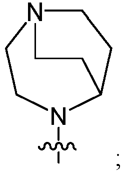 wherein R^{a} is hydrogen or C₁-C₃alkyl.

In some aspects, R⁴ is 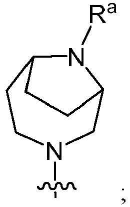 wherein R^{a} is hydrogen or C₁-C₃alkyl.

In some aspects, R⁴ is 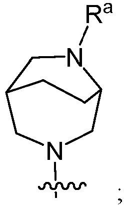 wherein R^{a} is hydrogen or C₁-C₃alkyl.

In some aspects, R⁵ is -(C₁-C₃alkyl)-R⁶.

In some aspects, R⁵ is selected from: 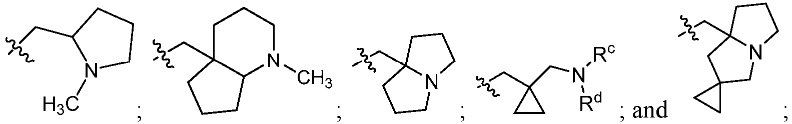 wherein each ring is optionally substituted with 1 or 2 groups independently selected from C₁-C₃alkoxy, C₁-C₃alkyl, halo, haloC₁-C₃alkyl, and hydroxy.

In some aspects, R⁵ is 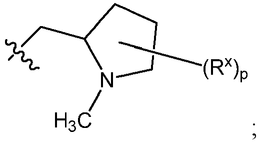 wherein p is 0, 1, or 2; and wherein each R^{x} is independently selected from C₁-C₃alkoxy, C₁-C₃alkyl, halo, haloC₁-C₃alkyl, and hydroxy.

In some aspects, R⁵ is 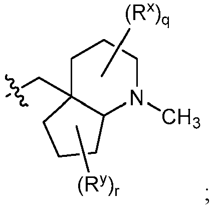 wherein q and r are each independenty 0 or 1; and wherein R^{x} and R^{y} are independently selected from C₁-C₃alkoxy, C₁-C₃alkyl, halo, haloC₁-C₃alkyl, and hydroxy.

In some aspects, R⁵ is

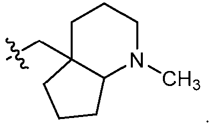

In some aspects, R⁵ is 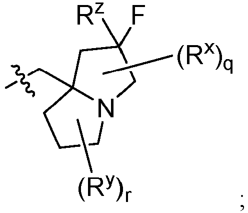 wherein q and r are each independenty 0 or 1; wherein R^{x} and R^{y} are independently selected from C₁-C₃alkoxy, C₁-C₃alkyl, halo, haloC₁-C₃alkyl, and hydroxy; and wherein R^{z} is hydrogen or fluoro.

In some aspects, R⁵ is 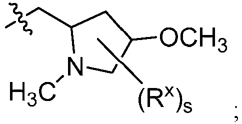 wherein s is 0 or 1; and wherein R^{x} is selected from C₁-C₃alkoxy, C₁-C₃alkyl, halo, haloC₁-C₃alkyl, and hydroxy.

In some aspects, R⁵ is

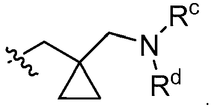

In some aspects, R⁵ is 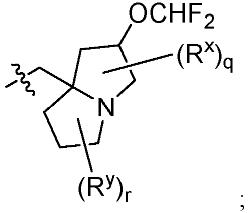 wherein q and r are each independenty 0 or 1; and wherein R^{x} and R^{y} are independently selected from C₁-C₃alkoxy, C₁-C₃alkyl, halo, haloC₁-C₃alkyl, and hydroxy.

In some aspects, R⁵ is 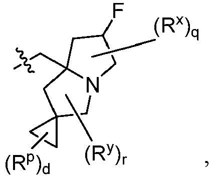 wherein q, r, and d are each independenty 0 or 1; and wherein R^{x}, R^{y}, and R^{p} are independently selected from C₁-C₃alkoxy, C₁-C₃alkyl, halo, haloC₁-C₃alkyl, and hydroxy.

In some aspects, R⁵ is

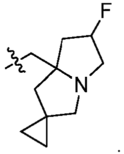

In some aspects, R¹ is naphthyl, wherein the naphthyl is substituted with a hydroxy group and is optionally further substituted with one or two additional groups selected from C₁-C₃alkyl, C₂-C₄alkynyl, and halo.

In some aspects, R¹ is

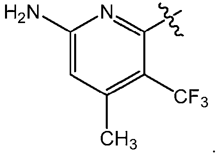

In some aspects, R¹ is

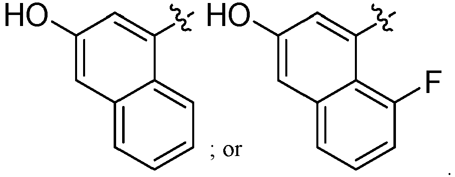

In some aspects, R¹ is

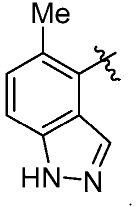

In some aspects, R¹ is

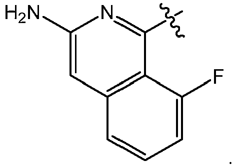

In some aspects, the present disclosure provides a compound of formula (I), or a pharmaceutically acceptable salt thereof, wherein:
R² is hydrogen;
R³ is fluoro;
R¹ is selected from 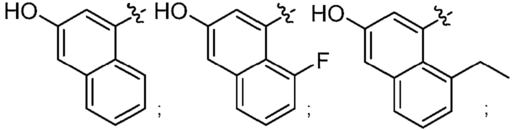 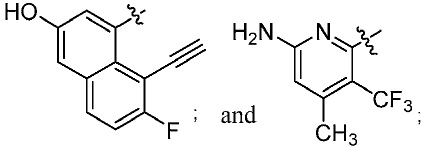 and
R⁵ is selected from 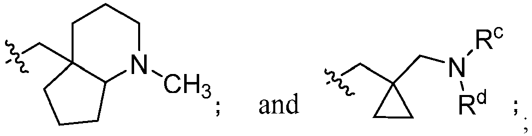 wherein
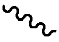 denotes the point of attachment to the parent molecular moiety.

In some aspects, the present disclosure provides a compound of formula (I), or a pharmaceutically acceptable salt thereof, wherein:
R² is chloro;
R³ is fluoro;
R¹ is selected from 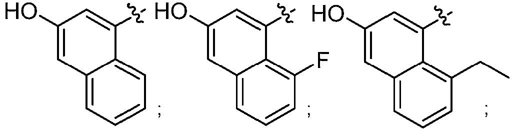 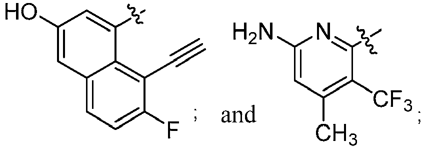 and
R⁵ is selected from 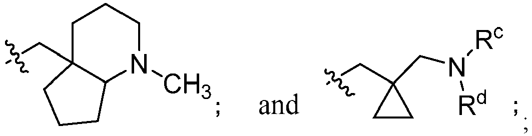 wherein
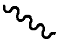 denotes the point of attachment to the parent molecular moiety.

In some aspects, the present disclosure provides a compound of formula (II): 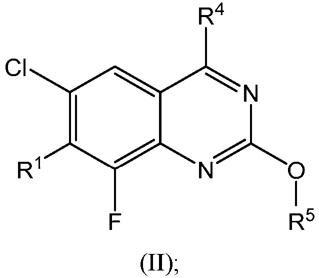 or a pharmaceutically acceptable salt thereof, wherein:

R¹ is selected from 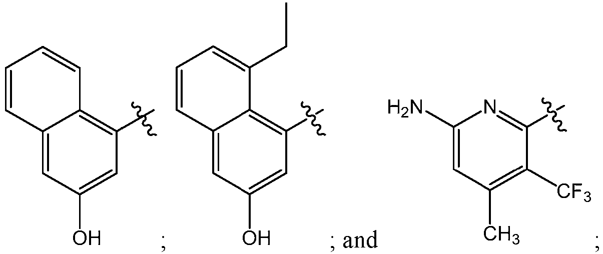 wherein 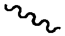 denotes the point of attachment to the parent molecular moiety;

R⁴ is selected from: 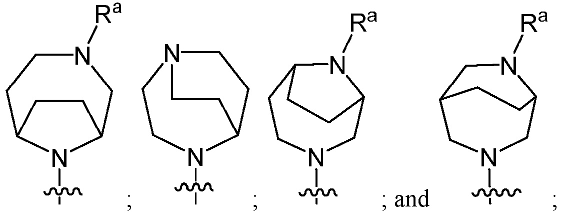 wherein
R^{a} is hydrogen or C₁-C₃alkyl; and
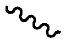 denotes the point of attachment to the parent molecular moiety; and
R⁵ is selected from: 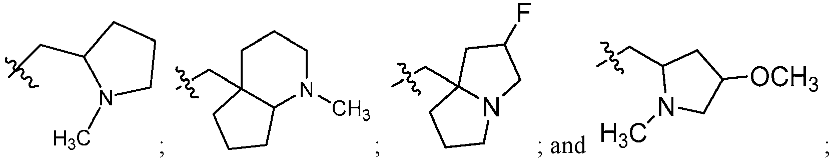 wherein
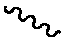 denotes the point of attachment to the parent molecular moiety

In certain aspects, the present disclosure provides a compound of formula (IIa): 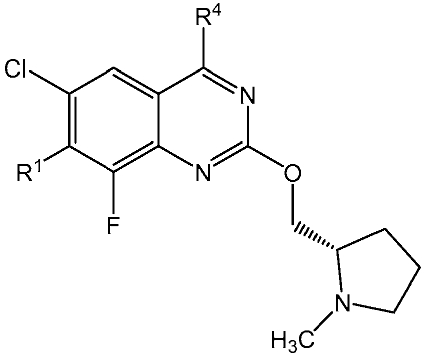 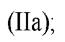 or a pharmaceutically acceptable salt thereof, wherein:
R¹ is selected from 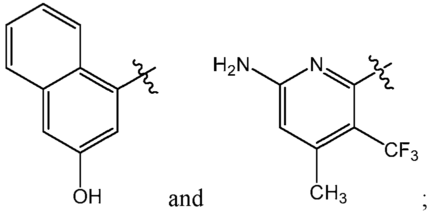
wherein 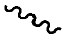 denotes the point of attachment to the parent molecular moiety; and
R⁴ is selected from: 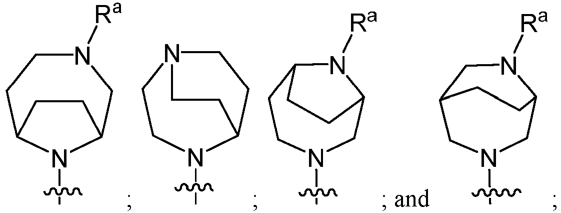 wherein
R^{a} is hydrogen or C₁-C₃alkyl; and
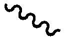 denotes the point of attachment to the parent molecular moiety.

In some aspects, R⁴ is 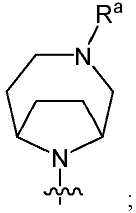 wherein R^{a} is hydrogen or C₁-C₃alkyl.

In some aspects, R⁴ is 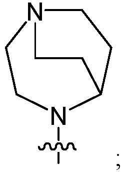 wherein R^{a} is hydrogen or C₁-C₃alkyl.

In some aspects, R⁴ is 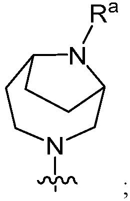 wherein R^{a} is hydrogen or C₁-C₃alkyl.

In some aspects, R⁴ is 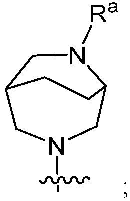 wherein R^{a} is hydrogen or C₁-C₃alkyl.

In some aspects, the present disclosure provides an atropisomer of a compound of any of the prior aspects. In certain embodiments, the compound is a stable atropisomer as described herein.

In some aspects, the present disclosure provides a pharmaceutical composition comprising a compound of formula (I), (II), or (IIa), or a pharmaceutically acceptable salt thereof, and one or more pharmaceutically acceptable excipients.

In some aspects, the present disclosure provides an oral dosage form comprising a compound of formula (I), (II) or (IIa), or a pharmaceutically acceptable salt thereof, and one or more pharmaceutically acceptable excipients.

In certain aspects, the present disclosure provides a compound of formula (I) for use in a method of treating cancer expressing KRAS G12D mutation: 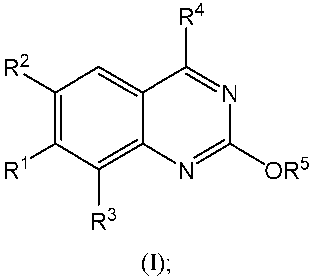 or a pharmaceutically acceptable salt thereof, wherein:
R¹ is aryl or heteroaryl, wherein the aryl and the heteroaryl are optionally substituted with one, two, three, four, or five substituents independently selected from C₁-C₃alkyl, C₂-C₄alkenyl, C₂-C₄alkynyl, amino, aminoC₁-C₃alkyl, C₃-C₄cycloalkyl, halo, haloC₁-C₃alkoxy, haloC₁-C₃alkyl, hydroxy, and hydroxyC₁-C₃alkyl;
R² and R³ are independently selected from hydrogen, C₁-C₃alkoxy, C₁-C₃alkyl, cyano, halo, haloC₁-C₃alkyl, and hydroxy;
R⁴ is selected from: 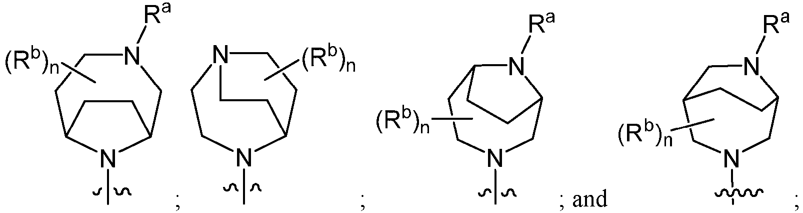 wherein
R^{a} is hydrogen or C₁-C₃alkyl;
n is 0, 1, 2, 3, or 4;
each R^{b} is independently selected from C₁-C₃alkyl, C₃-C₆cycloalkyl, halo, and hydroxy; or, alternatively, two geminal R^{b} groups, together with the carbon atom to which they are attached, can form a 3- to 6-membered cycloalkyl ring; and
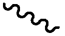 denotes the point of attachment to the parent molecular moiety;
R⁵ is -(C₁-C₃alkyl)-R⁶ or -(C₁-C₆alkyl)NR^{c}R^{d}, wherein R⁶ is selected from:
a C₃-C₆cycloalkyl substituted with NR^{c}R^{d}(C₁-C₃alkyl); and
a five- to ten-membered monocyclic, bicyclic, or tricyclic ring containing one nitrogen atom and optionally a second heteroatom selected from oxygen or nitrogen, wherein the ring contains zero to three double bonds and wherein the ring is optionally substituted with one, two, or three groups independently selected from C₁-C₃alkoxy, C₁-C₃alkyl, halo, haloC₁-C₃alkoxy, haloC₁-C₃alkyl, and hydroxy; wherein
R^{c} and R^{d}, together with the nitrogen atom to which they are attached, form a five-to ten-membered ring monocyclic or bicyclic ring optionally containing one additional heteroatom selected from nitrogen, oxygen, and sulfur, wherein the ring is optionally substituted with one, two, or three groups independently selected from C₁-C₃alkoxy, C₁-C₃alkoxyC₁-C₃alkyl, C₁-C₃alkyl, benzyl, halo, haloC₁-C₃alkyl, hydroxy, hydroxyC₁-C₃alkyl, and oxo; or
one of R^{c} and R^{d} is selected from hydrogen and C₁-C₃alkyl and the other is selected from hydrogen, C₁-C₃alkyl, C₁-C₃alkoxycarbonyl, and C₁-C₃alkylcarbonyl. In certain aspects, n is zero. In other aspects, n is 1, 2, 3 or 4. In one aspect, n is 1. In another aspect, n is 2. In another aspect, n is 3. And in another aspect, n is 4.

In some aspects of the method, R⁴ is selected from

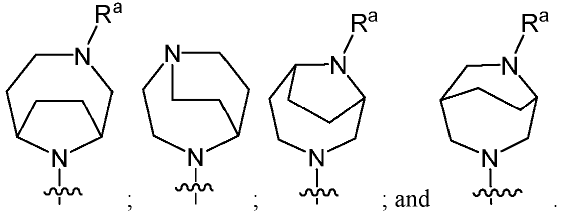

In some aspects of the method, R⁴ is selected from: 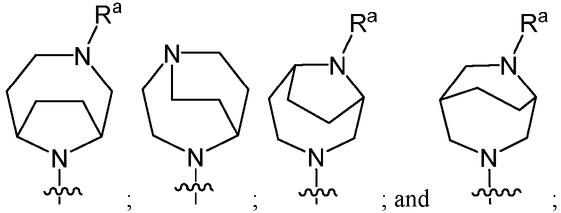 and
R⁶ is a five- to ten-membered monocyclic, bicyclic, or tricyclic ring containing one nitrogen atom and optionally a second heteroatom selected from oxygen or nitrogen, wherein the ring contains zero to three double bonds and wherein the ring is optionally substituted with one, two, or three groups independently selected from C₁-C₃alkoxy, C₁-C₃alkyl, halo, haloC₁-C₃alkoxy, haloC₁-C₃alkyl, and hydroxy.

In some aspects of the method, R² and R³ are each halo. In some aspects, R² is chloro and R³ is fluoro. In some aspects of the method, R² is hydrogen and R³ is fluoro.

In some aspects of the method, R⁴ is 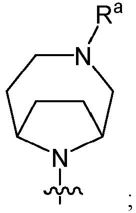 wherein R^{a} is hydrogen or C₁-C₃alkyl.

In some aspects of the method, R⁴ is 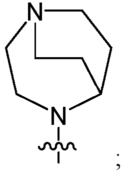 wherein R^{a} is hydrogen or C₁-C₃alkyl.

In some aspects of the method, R⁴ is 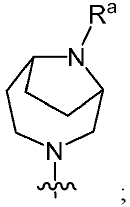 wherein R^{a} is hydrogen or C₁-C₃alkyl.

In some aspects of the method, R⁴ is 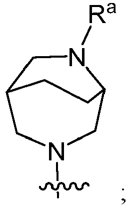 wherein R^{a} is hydrogen or C₁-C₃alkyl.

In some aspects of the method, R⁵ is -(C₁-C₃alkyl)-R⁶.

In some aspects of the method, R⁵ is selected from: 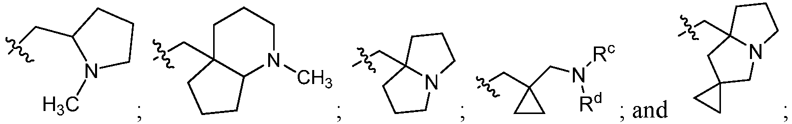 wherein each ring is optionally substituted with 1 or 2 groups independently selected from C₁-C₃alkoxy, C₁-C₃alkyl, halo, haloC₁-C₃alkyl, and hydroxy.

In some aspects of the method, R⁵ is 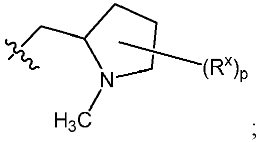 wherein p is 0, 1, or 2; and wherein each R^{x} is independently selected from C₁-C₃alkoxy, C₁-C₃alkyl, halo, haloC₁-C₃alkyl, and hydroxy.

In some aspects of the method, R⁵ is 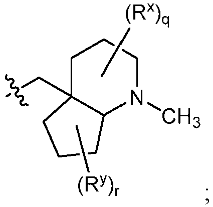 wherein q and r are each independenty 0 or 1; and wherein R^{x} and R^{y} are independently selected from C₁-C₃alkoxy, C₁-C₃alkyl, halo, haloC₁-C₃alkyl, and hydroxy.

In some aspects of the method, R⁵ is

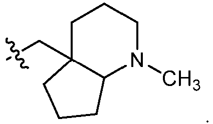

In some aspects of the method, R⁵ is 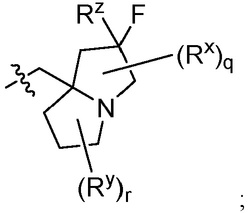 wherein q and r are each independenty 0 or 1; wherein R^{x} and R^{y} are independently selected from C₁-C₃alkoxy, C₁-C₃alkyl, halo, haloC₁-C₃alkyl, and hydroxy; and wherein R^{z} is hydrogen or fluoro.

In some aspects of the method, R⁵ is 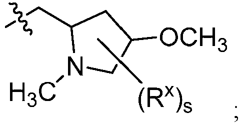 wherein s is 0 or 1; and wherein R^{x} is selected from C₁-C₃alkoxy, C₁-C₃alkyl, halo, haloC₁-C₃alkyl, and hydroxy.

In some aspects of the method, R⁵ is

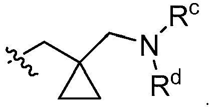

In some aspects of the method, R⁵ is 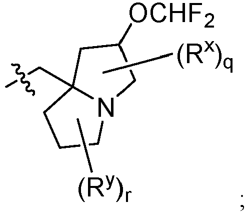 wherein q and r are each independenty 0 or 1; and wherein R^{x} and R^{y} are independently selected from C₁-C₃alkoxy, C₁-C₃alkyl, halo, haloC₁-C₃alkyl, and hydroxy.

In some aspects of the method, R⁵ is 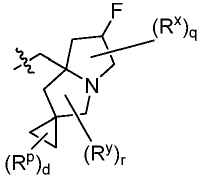 wherein q, r, and d are each independenty 0 or 1; and wherein R^{x}, R^{y}, and R^{p} are independently selected from C₁-C₃alkoxy, C₁-C₃alkyl, halo, haloC₁-C₃alkyl, and hydroxy.

In some aspects of the method, R⁵ is

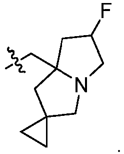

In some aspects of the method, R¹ is naphthyl, wherein the naphthyl is substituted with a hydroxy group and is optionally further substituted with one or two additional groups selected from C₁-C₃alkyl, C₂-C₄alkynyl, and halo.

In some aspects of the method, R¹ is

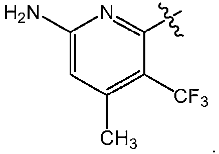

In some aspects of the method, R¹ is

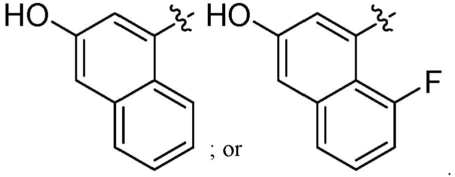

In some aspects of the method, R¹ is

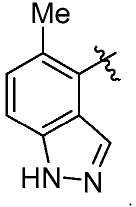

In some aspects of the method, R¹ is

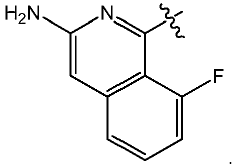

In some aspects of the method, R² is hydrogen; R³ is fluoro; R¹ is selected from 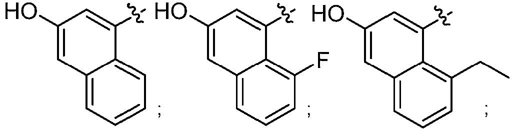 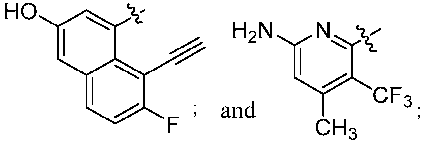 and

R⁵ is selected from 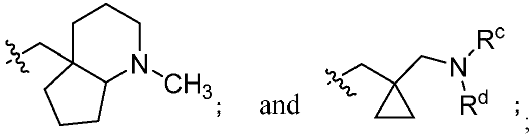 wherein
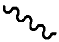 denotes the point of attachment to the parent molecular moiety.

In some aspects of the method, R² is chloro; R³ is fluoro; R¹ is selected from 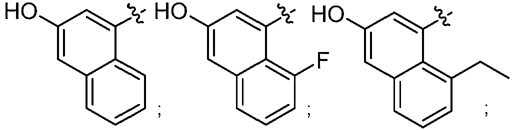 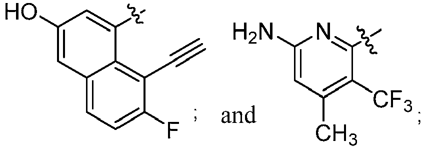 and
R⁵ is selected from 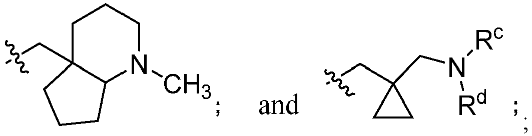 wherein
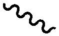 denotes the point of attachment to the parent molecular moiety.

In some aspects of the method, the compound is an atropisomer of a compound of any of the prior aspects. In certain embodiments, the compound is a stable atropisomer as described herein.

In another aspect, the present disclosure provides a method for inhibiting KRAS Gl2D activity in a in a cell, comprising contacting the cell with a compound of formula (I), formula (II), or formula (IIa), or a pharmaceutically acceptable salt thereof, or a pharmaceutical composition thereof as defined herein. In one aspect, the contacting is *in vitro.* In one embodiment, the contacting is *in vivo.*

In another aspect, the present disclosure provides a method of inhibiting cell proliferation, *in vitro* or *in vivo,* the method comprising contacting a cell with an effective amount of a compound of formula (I), formula (II), or formula (IIa), or a pharmaceutically acceptable salt thereof, or a pharmaceutical composition thereof as defined herein.

In another aspect, the present disclosure provides a compound of formula (I), formula (II), or formula (IIa), or a pharmaceutically acceptable salt thereof, or a pharmaceutical composition thereof as defined herein, for use in a method of treating a KRAS Gl2D-associated disease or disorder.

In some aspects, the present disclosure provides a compound selected from: 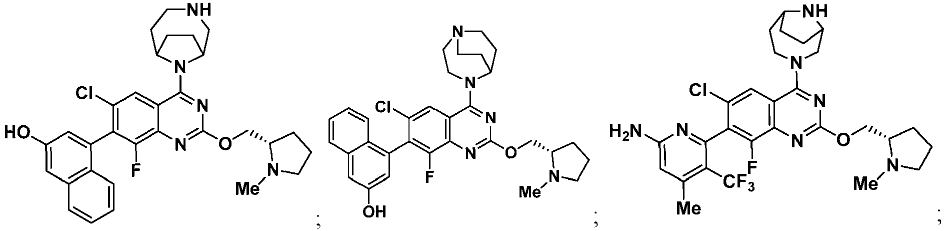 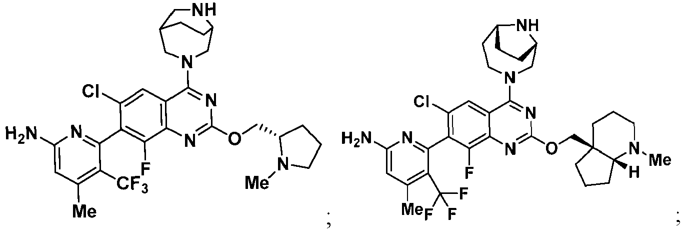 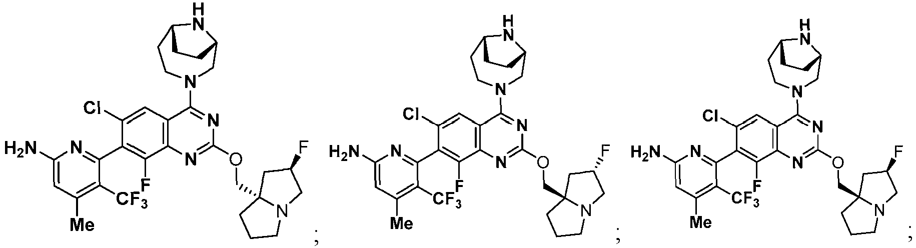 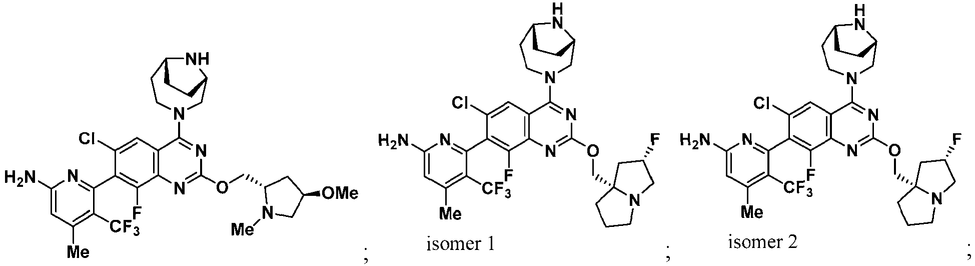 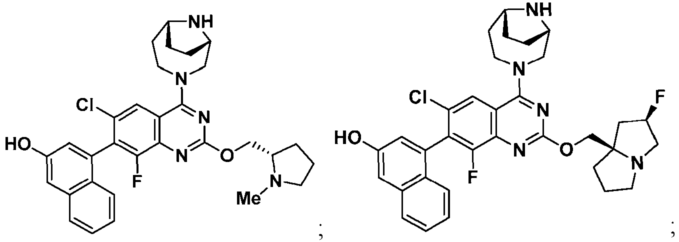 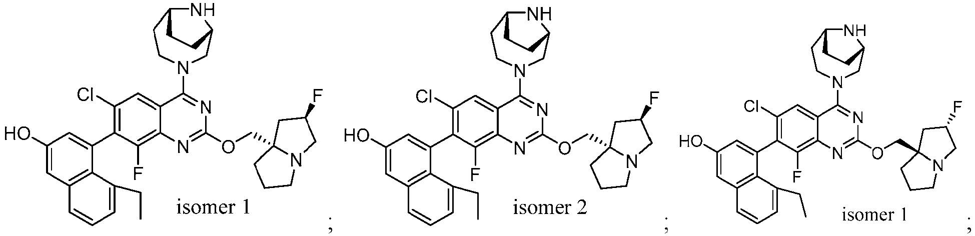 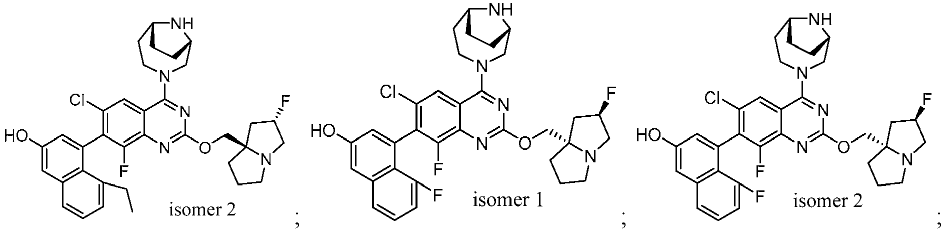 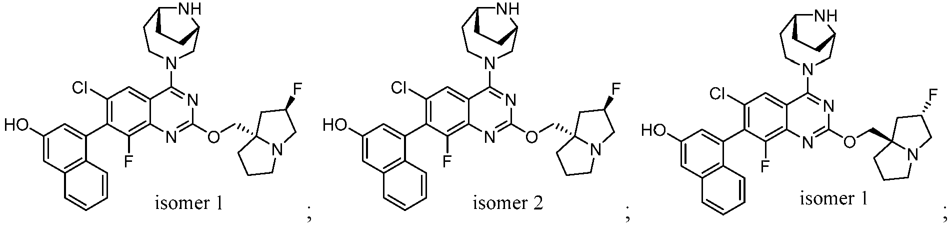 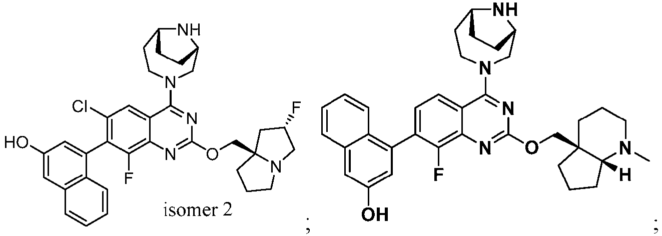 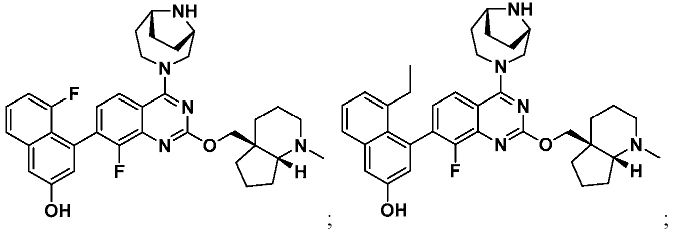 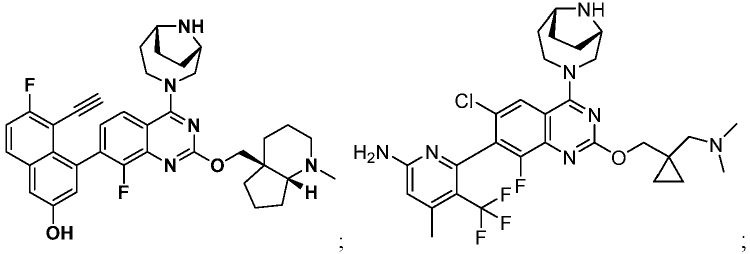 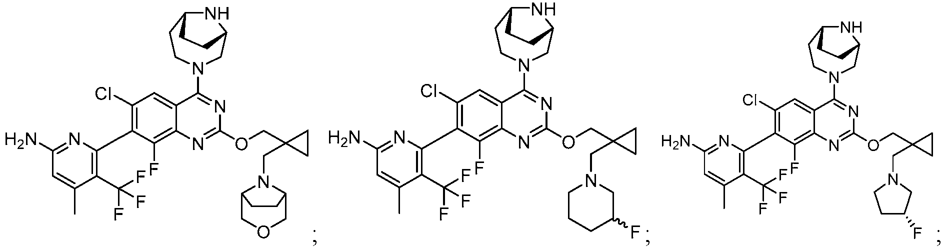 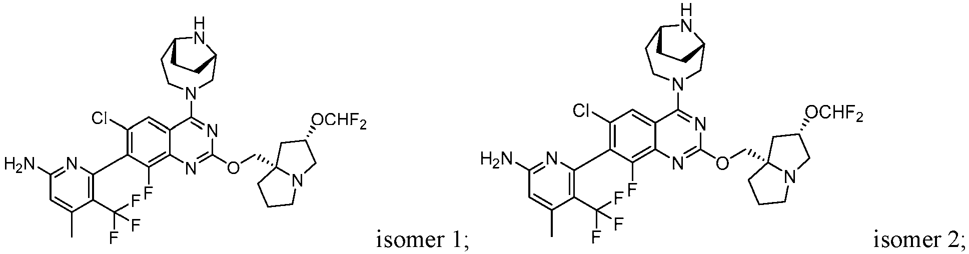 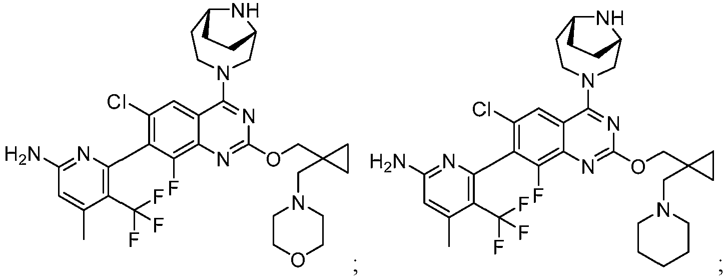 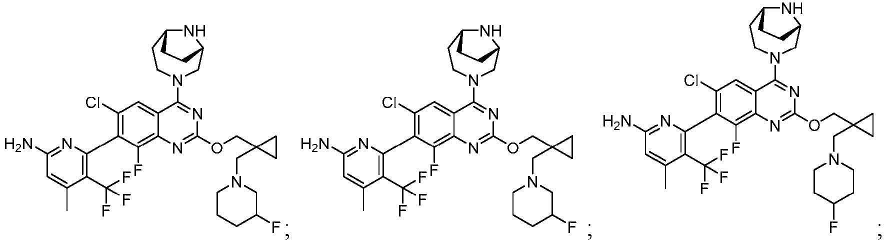 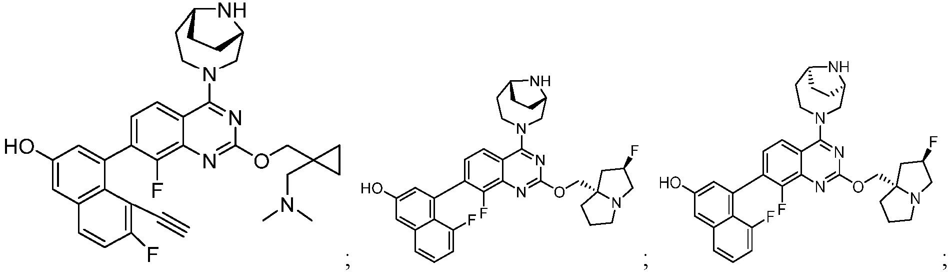 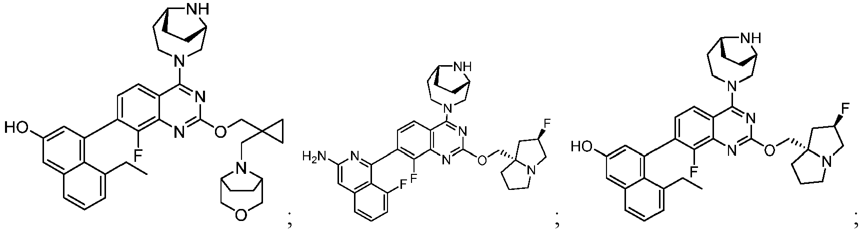 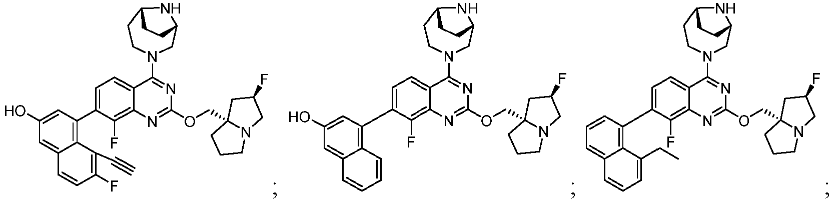 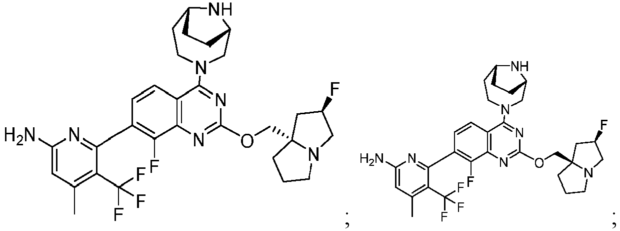 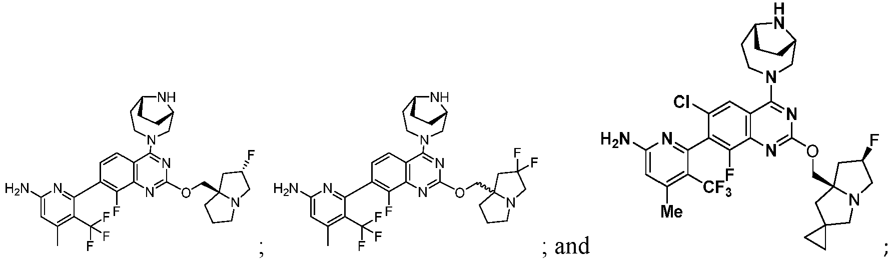 or a pharmaceutically acceptable salt thereof.

In some aspects, the present disclosure provides a compound selected from
4-(6-chloro-4-{3,9-diazabicyclo[4.2.1]nonan-9-yl}-8-fluoro-2-{[(2S)-1-methylpyrrolidin-2-yl]methoxy}quinazolin-7-yl)naphthalen-2-ol;
4-(6-chloro-4-{1,4-diazabicyclo[3.2.2]nonan-4-yl}-8-fluoro-2-{[(2S)-1-methylpyrrolidin-2-yl]methoxy}quinazolin-7-yl)naphthalen-2-ol;
6-(6-chloro-4-{3,9-diazabicyclo[4.2.1]nonan-3-yl}-8-fluoro-2-{[(2S)-1-methylpyrrolidin-2-yl]methoxy}quinazolin-7-yl)-4-methyl-5-(trifluoromethyl)pyridin-2-amine;
6-(6-chloro-4-{3,6-diazabicyclo[3.2.2]nonan-3-yl}-8-fluoro-2-{[(2S)-1-methylpyrrolidin-2-yl]methoxy}quinazolin-7-yl)-4-methyl-5-(trifluoromethyl)pyridin-2-amine;
6-(2- f [(4aS,7aR)-1-methyl-octahydro-1H-cyclopenta[b]pyridin-4a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amine;
6-(2-{[(2R,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amine;
6-(2- f [(2S,7aR)-2-fluoro-hexahydro-1H-pyrrolizin-7a-yl]methoxy}-6-chloro-4-[(1 S,6R)-3,9-diazabicyclo[4.2.1]nonan-3-yl]-8-fluoroquinazolin-7-yl)-4-methyl-5-(trifluoromethyl)pyridin-2-amine;
6-(2- f [(2R,7aR)-2-fluoro-hexahydro-1H-pyrrolizin-7a-yl]methoxy}-6-chloro-4-[(1 S,6R)-3,9-diazabicyclo[4.2.1]nonan-3-yl]-8-fluoroquinazolin-7-yl)-4-methyl-5-(trifluoromethyl)pyridin-2-amine;
6-{6-chloro-4-[(1S,6R)-3,9-diazabicyclo[4.2.1]nonan-3-yl]-8-fluoro-2-{[(2S,4R)-4-methoxy-1-methylpyrrolidin-2-yl]methoxy}quinazolin-7-yl}-4-methyl-5-(trifluoromethyl)pyridin-2-amine;
6-(2-{[(2S,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amine;
6-(2- {[(2S,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amine, Isomer 1;
6-(2-{[(2S,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amine, Isomer 2;
4-{6-chloro-4-[(1S,6R)-3,9-diazabicyclo[4.2.1]nonan-3-yl]-8-fluoro-2-{[(2S)-1-methylpyrrolidin-2-yl]methoxy}quinazolin-7-yl}naphthalen-2-ol;
4-(2-{[(2R,7aR)-2-fluoro-hexahydro-1H-pyrrolizin-7a-yl]methoxy}-6-chloro-4-[(1 S,6R)-3,9-diazabicyclo[4.2.1]nonan-3-yl]-8-fluoroquinazolin-7-yl)naphthalen-2-ol;
4-(2-{[(2R,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-5-ethylnaphthalen-2-ol;
4-(2-{[(2R,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-5-ethylnaphthalen-2-ol, Isomer 1;
4-(2-{[(2R,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-5-ethylnaphthalen-2-ol, Isomer 2;
4-(2- f [(2S,7aR)-2-fluoro-hexahydro-1H-pyrrolizin-7a-yl]methoxy}-6-chloro-4-[(1 S,6R)-3,9-diazabicyclo[4.2.1]nonan-3-yl]-8-fluoroquinazolin-7-yl)-5-ethylnaphthalen-2-ol;
4-(2-{[(2S,7aR)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-5-ethylnaphthalen-2-ol, Isomer 1;
4-(2-{[(2S,7aR)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-5-ethylnaphthalen-2-ol, Isomer 2;
4-(2-{[(2R,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-5-fluoronaphthalen-2-ol;
4-(2-{[(2R,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-5-fluoronaphthalen-2-ol, Isomer 1;
4-(2-{[(2R,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-5-fluoronaphthalen-2-ol, Isomer 2;
4-(2-{[(2R,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)naphthalen-2-ol;
4-(2-{[(2R,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)naphthalen-2-ol, Isomer 1;
4-(2-{[(2R,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)naphthalen-2-ol, Isomer 2;
4-(2-{[(2S,7aR)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)naphthalen-2-ol;
4-(2-{[(2S,7aR)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)naphthalen-2-ol, Isomer 1;
4-(2-{[(2S,7aR)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)naphthalen-2-ol, Isomer 2;
4-(2-{[(4aS,7aR)-1-methyl-octahydro-1H-cyclopenta[b]pyridin-4a-yl]methoxy}-4-[(1S,6R)-3,9-diazabicyclo[4.2.1]nonan-3-yl]-8-fluoroquinazolin-7-yl)naphthalen-2-ol;
4-(2-{[(4aS,7aR)-1-methyl-octahydro-1H-cyclopenta[b]pyridin-4a-yl]methoxy}-4-[(1S,6R)-3,9-diazabicyclo[4.2.1]nonan-3-yl]-8-fluoroquinazolin-7-yl)-5-fluoronaphthalen-2-ol-,
4-(2-{[(4aS,7aR)-1-methyl-octahydro-1H-cyclopenta[b]pyridin-4a-yl]methoxy}-4-[(1S,6R)-3,9-diazabicyclo[4.2.1]nonan-3-yl]-8-fluoroquinazolin-7-yl)-5-ethylnaphthalen-2-ol;
4-(2-{[(4aS,7aR)-1-methyl-octahydro-1H-cyclopenta[b]pyridin-4a-yl]methoxy}-4-[(1S,6R)-3,9-diazabicyclo[4.2.1]nonan-3-yl]-8-fluoroquinazolin-7-yl)-5-ethynyl-6-fluoronaphthalen-2-ol;
6-{6-chloro-4-[(1S,6R)-3,9-diazabicyclo[4.2.1]nonan-3-yl]-2-({1-[(dimethylamino)methyl]cyclopropyl}methoxy)-8-fluoroquinazolin-7-yl}-4-methyl-5-(trifluoromethyl)pyridin-2-amine;
6-{6-chloro-4-[(1S,6R)-3,9-diazabicyclo[4.2.1]nonan-3-yl]-8-fluoro-2-{[1-({3-oxa-8-azabicyclo[3.2.1]octan-8-yl}methyl)cyclopropyl]methoxy}quinazolin-7-yl}-4-methyl-5-(trifluoromethyl)pyridin-2-amine;
6-{6-chloro-4-[(1S,6R)-3,9-diazabicyclo[4.2.1]nonan-3-yl]-8-fluoro-2-({1-[(3-fluoropiperidin-1-yl)methyl]cyclopropyl}methoxy)quinazolin-7-yl}-4-methyl-5-(trifluoromethyl)pyridin-2-amine;
6-{6-chloro-4-[(1S,6R)-3,9-diazabicyclo[4.2.1]nonan-3-yl]-8-fluoro-2-[(1-{[(3R)-3-fluoropyrrolidin-1-yl]methyl}cyclopropyl)methoxy]quinazolin-7-yl}-4-methyl-5-(trifluoromethyl)pyridin-2-amine;
6-(2-{[(2S,7aS)-2-(difluoromethoxy)-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amine isomer 1;
6-(2-{[(2S,7aS)-2-(difluoromethoxy)-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amine isomer 2;
6-{6-chloro-4-[(1S,6R)-3,9-diazabicyclo[4.2.1]nonan-3-yl]-8-fluoro-2-({1-[(morpholin-4-yl)methyl]cyclopropyl}methoxy)quinazolin-7-yl}-4-methyl-5-(trifluoromethyl)pyridin-2-amine;
6-{6-chloro-4-[(1S,6R)-3,9-diazabicyclo[4.2.1]nonan-3-yl]-8-fluoro-2-({1-[(piperidin-1-yl)methyl]cyclopropyl}methoxy)quinazolin-7-yl}-4-methyl-5-(trifluoromethyl)pyridin-2-amine;
6-{6-chloro-4-[(1S,6R)-3,9-diazabicyclo[4.2.1]nonan-3-yl]-8-fluoro-2-({1-[(3-fluoropiperidin-1-yl)methyl]cyclopropyl}methoxy)quinazolin-7-yl}-4-methyl-5-(trifluoromethyl)pyridin-2-amine;
6-{6-chloro-4-[(1S,6R)-3,9-diazabicyclo[4.2.1]nonan-3-yl]-8-fluoro-2-({1-[(3-fluoropiperidin-1-yl)methyl]cyclopropyl}methoxy)quinazolin-7-yl}-4-methyl-5-(trifluoromethyl)pyridin-2-amine;
6-{6-chloro-4-[(1S,6R)-3,9-diazabicyclo[4.2.1]nonan-3-yl]-8-fluoro-2-({1-[(4-fluoropiperidin-1-yl)methyl]cyclopropyl}methoxy)quinazolin-7-yl}-4-methyl-5-(trifluoromethyl)pyridin-2-amine;
4-{4-[(1S,6R)-3,9-diazabicyclo[4.2.1]nonan-3-yl]-2-({1-[(dimethylamino)methyl]cyclopropyl}methoxy)-8-fluoroquinazolin-7-yl}-5-ethynyl-6-fluoronaphthalen-2-ol;
4-(2-{[(2R,7aS)-2-fluoro-hexahydro-1H-pyrrolizin-7a-yl]methoxy}-4-[(1S,6R)-3,9-diazabicyclo[4.2.1]nonan-3-yl]-8-fluoroquinazolin-7-yl)-5-fluoronaphthalen-2-ol;
4-(2-{[(2R,7aS)-2-fluoro-hexahydro-1H-pyrrolizin-7a-yl]methoxy}-4-[(1R,6S)-3,9-diazabicyclo[4.2.1]nonan-3-yl]-8-fluoroquinazolin-7-yl)-5-fluoronaphthalen-2-ol;
4-{4-[(1S,6R)-3,9-diazabicyclo[4.2.1]nonan-3-yl]-8-fluoro-2-{[1-({3-oxa-8-azabicyclo[3.2.1]octan-8-ylymethyl)cyclopropylmethoxy}quinazolin-7-yl}-5-ethylnaphthalen-2-ol;
1-(2-{ [(2R,7aS)-2-fluoro-hexahydro-1H-pyrrolizin-7a-yl]methoxy}-4-[(1S,6R)-3,9-diazabicyclo[4.2.1]nonan-3-yl]-8-fluoroquinazolin-7-yl)-8-fluoroisoquinolin-3-amine;
4-(2-{[(2R,7aS)-2-fluoro-hexahydro-1H-pyrrolizin-7a-ylmethoxy}-4-[(1S,6R)-3,9-diazabicyclo[4.2.1]nonan-3-yl]-8-fluoroquinazolin-7-yl)-5-ethylnaphthalen-2-ol;
4-(2-{[(2R,7aS)-2-fluoro-hexahydro-1H-pyrrolizin-7a-yl]methoxy}-4-[(1S,6R)-3,9-diazabicyclo[4.2.1]nonan-3-yl]-8-fluoroquinazolin-7-yl)-5-ethynyl-6-fluoronaphthalen-2-ol;
4-(2-{[(2R,7aS)-2-fluoro-hexahydro-1H-pyrrolizin-7a-yl]methoxy}-4-[(1S,6R)-3,9-diazabicyclo[4.2.1]nonan-3-yl]-8-fluoroquinazolin-7-yl)naphthalen-2-ol;
2-{[(2R,7aS)-2-fluoro-hexahydro-lH-pyrrolizin-7a-yl]methoxy}-4-[(1S,6R)-3,9-diazabicyclo[4.2.1]nonan-3-yl]-7-(8-ethylnaphthalen-1-yl)-8-fluoroquinazoline;
4-(2-{[(2R,7aS)-2-fluoro-hexahydro-1H-pyrrolizin-7a-yl]methoxy}-4-[(1S,6R)-3,9-diazabicyclo[4.2.1]nonan-3-yl]-8-fluoroquinazolin-7-yl)-5-fluoronaphthalen-2-ol;
6-(2-{[(2R,7aR)-2-fluoro-hexahydro-1H-pyrrolizin-7a-yl]methoxy}-4-[(1S,6R)-3,9-diazabicyclo[4.2.1]nonan-3-yl]-8-fluoroquinazolin-7-yl)-4-methyl-5-(trifluoromethyl)pyridin-2-amine;
6-(2-{[(2S,7aR)-2-fluoro-hexahydro-1H-pyrrolizin-7a-yl]methoxy}-4-[(1S,6R)-3,9-diazabicyclo[4.2.1]nonan-3-yl]-8-fluoroquinazolin-7-yl)-4-methyl-5-(trifluoromethyl)pyridin-2-amine;
6-{4-[(1S,6R)-3,9-diazabicyclo[4.2.1]nonan-3-yl]-2-[(2,2-difluoro-hexahydro-1H-pyrrolizin-7a-yl)methoxy]-8-fluoroquinazolin-7-yl}-4-methyl-5-(trifluoromethyl)pyridin-2-amine;and
6-(2- f [(6'R,7'aR)-6'-fluoro-hexahydrospiro[cyclopropane-1,2'-pyrrolizine]-7'a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amine;
or a pharmaceutically acceptable salt thereof.

In some aspects, the present disclosure provides a pharmaceutical composition comprising a compound as described in any of the above aspects, or a pharmaceutically acceptable salt thereof, and one or more pharmaceutically acceptable excipients.

In another aspect, the present disclosure provides a compound or pharmaceutical composition of any of the above aspects, or a pharmaceutically acceptable salt thereof for use in a method for treating cancer.

In another aspect, the present disclosure provides a compound or pharmaceutical composition of any of the above aspects, or a pharmaceutically acceptable salt thereof for use in a method for treating a cancer susceptible to KRAS G12D inhibition.

In another aspect, the present disclosure provides a compound or pharmaceutical composition of any of the above aspects, or a pharmaceutically acceptable salt thereof for use in a method for treating a cancer expressing KRAS G12D inhibition.

### DETAILED DESCRIPTION

Unless otherwise indicated, any atom with unsatisfied valences is assumed to have hydrogen atoms sufficient to satisfy the valences.

The singular forms "a," "an," and "the" include plural referents unless the context dictates otherwise.

As used herein, the term "or" is a logical disjunction (i.e., and/or) and does not indicate an exclusive disjunction unless expressly indicated such as with the terms "either," "unless," "alternatively," and words of similar effect.

As used herein, the phrase "or a pharmaceutically acceptable salt thereof" refers to at least one compound, or at least one salt of the compound, or a combination thereof. For example, "a compound of Formula (I) or a pharmaceutically acceptable salt thereof" includes, but is not limited to, a compound of Formula (I), two compounds of Formula (I), a pharmaceutically acceptable salt of a compound of Formula (I), a compound of Formula (I) and one or more pharmaceutically acceptable salts of the compound of Formula (I), and two or more pharmaceutically acceptable salts of a compound of Formula (I).

The term "C₂-C₄alkenyl", as used herein, refers to a group derived from a straight or branched chain hydrocarbon containing from two to four carbon atoms and one double bond.

The term "C₁-C₃alkoxy", as used herein, refers to a C₁-C₃alkyl group attached to the parent molecular moiety through an oxygen atom.

The term "C₁-C₃alkoxycarbonyl", as used herein, refers to a C₁-C₃alkoxy group attached to the parent molecular moiety through a carbonyl group.

The term "C₁-C₃alkyl", as used herein, refers to a group derived from a straight or branched chain saturated hydrocarbon containing from one to three carbon atoms.

The term "C₁-C₆alkyl", as used herein, refers to a group derived from a straight or branched chain saturated hydrocarbon containing from one to three carbon atoms.

The term "C₁-C₃alkylcarbonyl", as used herein, refers to a C₁-C₃alkyl group attached to the parent molecular moiety through a carbonyl group.

The term "C₂-C₄alkynyl", as used herein, refers to a group derived from a straight or branched chain hydrocarbon containing from two to four carbon atoms and one triple bond.

The term "amino," as used herein, refers to -NH₂.

The term "aminoC₁-C₃alkyl," as used herein, refers to an amino group attached to the parent molecular moiety through a C₁-C₃alkyl group.

The term "aryl," as used herein, refers to a phenyl group, or a bicyclic fused ring system wherein one or both of the rings is a phenyl group. Bicyclic fused ring systems consist of a phenyl group fused to a four- to six-membered aromatic or non-aromatic carbocyclic ring. The aryl groups of the present disclosure can be attached to the parent molecular moiety through any substitutable carbon atom in the group. Representative examples of aryl groups include, but are not limited to, indanyl, indenyl, naphthyl, phenyl, and tetrahydronaphthyl.

The term "cyano", as used herein, refers to -CN.

The term "C₃-C₄cycloalkyl", as used herein, refers to a saturated monocyclic hydrocarbon ring system having three or four carbon atoms and zero heteroatoms..

The terms "halo" and "halogen", as used herein, refer to F, Cl, Br, or I.

The term "haloC₁-C₃alkoxy," as used herein, refers to a haloC₁-C₃alkyl group attached to the parent molecular moiety through an oxygen atom.

The term "haloC₁-C₃alkyl," as used herein, refers to a C₁-C₃alkyl group substituted with one, two, or three halogen atoms.

The term "heteroaryl," as used herein, refers to an aromatic five- or six-membered ring where at least one atom is selected from N, O, and S, and the remaining atoms are carbon. The term "heteroaryl" also includes bicyclic systems where a heteroaryl ring is fused to a four- to six-membered aromatic or non-aromatic ring containing zero, one, or two additional heteroatoms selected from N, O, and S; and tricyclic systems where a bicyclic system is fused to a four- to six-membered aromatic or non-aromatic ring containing zero, one, or two additional heteroatoms selected from N, O, and S. The heteroaryl groups are attached to the parent molecular moiety through any substitutable carbon or nitrogen atom in the group. Representative examples of heteroaryl groups include, but are not limited to, alloxazine, benzo[1,2-d:4,5-d']bisthiazole, benzoxadiazolyl, benzoxazolyl, benzofuranyl, benzothienyl, furanyl, imidazolyl, indazolyl, indolyl, isoxazolyl, isoquinolinyl, isothiazolyl, naphthyridinyl, oxadiazolyl, oxazolyl, purine, pyridinyl, pyridazinyl, pyrimidinyl, pyrazinyl, pyrazolyl, pyrrolyl, quinolinyl, thiazolyl, thienopyridinyl, thienyl, triazolyl, thiadiazolyl, and triazinyl.

The term "hydroxy," as used herein, refers to OH.

The term "hydroxyC₁-C₃alkyl," as used herein, refers to a hydroxy group attached to the parent molecular moiety through a C₁-C₃alkyl group.

An additional aspect of the disclosure is the use of the disclosed compounds as radiolabeled ligands for development of ligand binding assays or for monitoring of *in vivo* adsorption, metabolism, distribution, receptor binding or occupancy, or compound disposition. For example, a compound described herein can be prepared using a radioactive isotope and the resulting radiolabeled compound can be used to develop a binding assay or for metabolism studies. Alternatively, and for the same purpose, a compound described herein can be converted to a radiolabeled form by catalytic tritiation using methods known to those skilled in the art.

Certain compounds of the present disclosure exist as atropisomers. The term "atropisomers" refers to conformational stereoisomers which occur when rotation about a single bond in the molecule is prevented, or greatly slowed, as a result of steric interactions with other parts of the molecule and the substituents at both ends of the single bond are asymmetrical (i.e., optical activity arises without requiring an asymmetric carbon center or stereocenter). Where the rotational barrier about the single bond is high enough, and interconversion between conformations is slow enough, separation and isolation of the isomeric species may be permitted. Atropisomers are enantiomers (or epimers) without a single asymmetric atom.

The atropisomers can be considered stable if the barrier to interconversion is high enough to permit the atropisomers to undergo little or no interconversion at room temperature for at least a week. In some aspects the atropisomers undergo little or no interconversion at room temperature for at least a year. In some aspects, an atropisomeric compound of the disclosure does not undergo more than about 5% interconversion to its opposite atropisomer at room temperature during one week when the atropisomeric compound is in substantially pure form, which is generally a solid state. In some aspects, an atropisomeric compound of the disclosure does not undergo more than about 5% interconversion to its opposite atropisomer at room temperature (approximately 25 °C) during one year. In some aspects, the atropisomeric compounds of the disclosure are stable enough to undergo no more than about 5% interconversion in an aqueous pharmaceutical formulation held at 0 °C for at least one week. The present chemical entities, pharmaceutical compositions and methods are meant to include all such possible atropisomers, including racemic mixtures, diastereomeric mixtures, epimeric mixtures, optically pure forms of single atropisomers, and intermediate mixtures.

The energy barrier to thermal racemization of atropisomers may be determined by the steric hindrance to free rotation of one or more bonds forming a chiral axis. Certain biaryl compounds exhibit atropisomerism where rotation around an interannular bond lacking C2 symmetry is restricted. The free energy barrier for isomerization (enantiomerization) is a measure of the stability of the interannular bond with respect to rotation. Optical and thermal excitation can promote racemization of such isomers, dependent on electronic and steric factors.

Ortho-substituted biaryl compounds may exhibit this type of conformational, rotational isomerism. Such biaryls are enantiomeric, chiral atropisomers where the sp²-sp² carbon-carbon, interannular bond between the aryl rings has a sufficiently high energy barrier to prevent free rotation, and where substituents W¹ ≠ W² and W³ ≠ W⁴ render the molecule asymmetric.

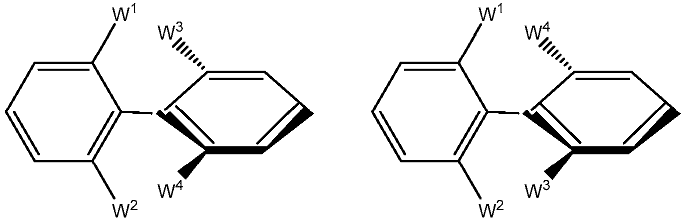

The steric interaction between W¹:W³, W¹:W⁴, and/or W²:W⁴, W²:W³ is large enough to make the planar conformation an energy maximum. Two non-planar, axially chiral enantiomers then exist as atropisomers when their interconversion is slow enough such that they can be isolated free of each other. Bold lines and dashed lines in the figures shown above indicate those moieties, or portions of the molecule, which are sterically restricted due to a rotational energy barrier. Balded moieties exist orthogonally above the plane of the page, and dashed moieties exist orthogonally below the plane of the page. The 'flat' part of the molecule (the left ring in each of the two depicted biaryls) is in the plane of the page.

The pharmaceutical compositions of the disclosure can include one or more pharmaceutically acceptable salts. A "pharmaceutically acceptable salt" refers to a salt that retains the desired biological activity of the parent compound and does not impart any undesired toxicological effects (see e.g., Berge, S.M. et al., J. Pharm. Sci., 66:1-19 (1977)). The salts can be obtained during the final isolation and purification of the compounds described herein, or separately be reacting a free base function of the compound with a suitable acid or by reacting an acidic group of the compound with a suitable base. Acid addition salts include those derived from nontoxic inorganic acids, such as hydrochloric, nitric, phosphoric, sulfuric, hydrobromic, hydroiodic, phosphorous and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.

### Pharmaceutical Compositions

In another aspect, the present disclosure provides a composition, e.g., a pharmaceutical composition, containing one or a combination of the compounds described within the present disclosure, formulated together with a pharmaceutically acceptable carrier. Pharmaceutical compositions of the disclosure also can be administered in combination therapy, *i.e.,* combined with other agents, as described herein.

As used herein, "pharmaceutically acceptable carrier" includes any and all solvents, dispersion media, coatings, antibacterial and antifungal agents, isotonic and absorption delaying agents, and the like that are physiologically compatible. In some aspects, the carrier is suitable for intravenous, intramuscular, subcutaneous, parenteral, spinal or epidermal administration (e.g., by injection or infusion). Depending on the route of administration, the active compound can be coated in a material to protect the compound from the action of acids and other natural conditions that can inactivate the compound.

The pharmaceutical compositions of the present disclosure can be administered *via* one or more routes of administration using one or more of a variety of methods known in the art. As will be appreciated by the skilled artisan, the route and/or mode of administration will vary depending upon the desired results. In some aspects, the routes of administration for compounds of the disclosure include intravenous, intramuscular, intradermal, intraperitoneal, subcutaneous, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

Sterile injectable solutions can be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, some methods of preparation are reduced pressure drying and freeze-drying (lyophilization) that yield a powder of the active ingredient plus any additional desired ingredient from a previously sterile-filtered solution thereof.

Examples of suitable aqueous and non-aqueous carriers that can be employed in the pharmaceutical compositions of the disclosure include water, ethanol, polyols (such as glycerol, propylene glycol, polyethylene glycol, and the like), and suitable mixtures thereof, vegetable oils, and injectable organic esters. Proper fluidity can be maintained, for example, by the use of coating materials, such as lecithin, by the maintenance of the required particle size in the case of dispersions, and by the use of surfactants.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the disclosure is contemplated. Supplementary active compounds can also be incorporated into the compositions.

Therapeutic compositions typically must be sterile and stable under the conditions of manufacture and storage. The composition can be formulated as a solution or as a liquid with ordered structure suitable to high drug concentration.. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be desirable to include isotonic agents, for example, sugars, polyalcohols such as mannitol, sorbitol, or sodium chloride in the composition. Prolonged absorption of the injectable compositions can be brought about by including in the composition an agent that delays absorption, for example, monostearate salts and gelatin.

Alternatively, the compounds of the disclosure can be administered via a non-parenteral route, such as a topical, epidermal or mucosal route of administration, for example, intranasally, orally, vaginally, rectally, sublingually or topically.

Any pharmaceutical composition contemplated herein can, for example, be delivered orally via any acceptable and suitable oral preparation. Exemplary oral preparations include, but are not limited to, for example, tablets, troches, lozenges, aqueous and oily suspensions, dispersible powders or granules, emulsions, hard and soft capsules, liquid capsules, syrups, and elixirs. Pharmaceutical compositions intended for oral administration can be prepared according to any methods known in the art for manufacturing pharmaceutical compositions intended for oral administration. In order to provide pharmaceutically palatable preparations, a pharmaceutical composition in accordance with the disclosure can contain at least one agent selected from sweetening agents, flavoring agents, coloring agents, demulcents, antioxidants, and preserving agents.

A tablet can, for example, be prepared by admixing at least one compound of Formula (I) and/or at least one pharmaceutically acceptable salt thereof with at least one non-toxic pharmaceutically acceptable excipient suitable for the manufacture of tablets.

An aqueous suspension can be prepared, for example, by admixing at least one compound of Formula (I) and/or at least one pharmaceutically acceptable salt thereof with at least one excipient suitable for the manufacture of an aqueous suspension, including, but are not limited to, for example, suspending agents, such as, for example, sodium carboxymethylcellulose, methylcellulose, hydroxypropylmethyl-cellulose, sodium alginate, alginic acid, polyvinylpyrrolidone, gum tragacanth, and gum acacia; dispersing or wetting agents, such as, for example, a naturally-occurring phosphatide, e.g., lecithin; condensation products of alkylene oxide with fatty acids, such as, for example, polyoxyethylene stearate; condensation products of ethylene oxide with long chain aliphatic alcohols, such as, for example, heptadecathylene-oxycetanol; condensation products of ethylene oxide with partial esters derived from fatty acids and hexitol, such as, for example, polyoxyethylene sorbitol monooleate; and condensation products of ethylene oxide with partial esters derived from fatty acids and hexitol anhydrides, such as, for example, polyethylene sorbitan monooleate. An aqueous suspension can also contain at least one preservative, such as, for example, ethyl and n-propyl p-hydroxybenzoate; at least one coloring agent; at least one flavoring agent; and/or at least one sweetening agent, including but not limited to, for example, sucrose, saccharin, and aspartame.

Oily suspensions can, for example, be prepared by suspending at least one compound of Formula (I) and/or at least one pharmaceutically acceptable salt thereof in either a vegetable oil, such as, for example, arachis oil, sesame oil, and coconut oil; or in mineral oil, such as, for example, liquid paraffin. An oily suspension can also contain at least one thickening agent, such as, for example, beeswax, hard paraffin, and cetyl alcohol. In order to provide a palatable oily suspension, at least one of the sweetening agents already described herein above, and/or at least one flavoring agent can be added to the oily suspension. An oily suspension can further contain at least one preservative, including, but not limited to, for example, an anti-oxidant, such as, for example, butylated hydroxyanisol, and alpha-tocopherol.

Dispersible powders and granules can, for example, be prepared by admixing at least one compound of Formula (I) and/or at least one pharmaceutically acceptable salt thereof with at least one dispersing and/or wetting agent, at least one suspending agent, and/or at least one preservative. Suitable dispersing agents, wetting agents, and suspending agents are already described above. Exemplary preservatives include, but are not limited to, for example, antioxidants, e.g., ascorbic acid. In addition, dispersible powders and granules can also contain at least one excipient, including, but not limited to, for example, sweetening agents, flavoring agents, and coloring agents.

The active compounds can be prepared with carriers that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. See, *e.g.,* Robinson, J.R., ed., Sustained and Controlled Release Drug Delivery Systems, Marcel Dekker, Inc., New York (1978).

Therapeutic compositions can be administered with medical devices known in the art. For example, in one aspect, a therapeutic composition of the disclosure can be administered with a needleless hypodermic injection device, such as the devices disclosed in U.S. Patent Nos. 5,399,163, 5,383,851, 5,312,335, 5,064,413, 4,941,880, 4,790,824, or 4,596,556. Examples of well-known implants and modules useful in the present disclosure include: U.S. Patent No. 4,487,603, which discloses an implantable micro-infusion pump for dispensing medication at a controlled rate; U.S. Patent No. 4,486,194, which discloses a therapeutic device for administering medication through the skin; U.S. Patent No. 4,447,233, which discloses a medication infusion pump for delivering medication at a precise infusion rate; U.S. Patent No. 4,447,224, which discloses a variable flow implantable infusion apparatus for continuous drug delivery; U.S. Patent No. 4,439,196, which discloses an osmotic drug delivery system having multi-chamber compartments; and U.S. Patent No. 4,475,196, which discloses an osmotic drug delivery system. Many other such implants, delivery systems, and modules are known to those skilled in the art.

In certain aspects, the compounds of the present disclosure can be administered parenterally, i.e., by injection, including, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and/or infusion.

In some aspects, the compounds of the present disclosure can be administered orally, i.e, via a gelatin capsule, tablet, hard or soft capsule, or a liquid capsule.

### Use of KRAS Inhibitors/Methods of Treating

Any reference to methods for treatment of the human or animal body herein only form part of the claimed invention insofar as the claimed compounds are for use in those methods. Administration of a therapeutic agent described herein may include administration of a therapeutically effective amount of therapeutic agent. The term "therapeutically effective amount" as used herein refers, without limitation, to an amount of a therapeutic agent to treat a condition treatable by administration of a composition comprising the KRAS inhibitors described herein. That amount is the amount sufficient to exhibit a detectable therapeutic or ameliorative effect. The effect can include, for example and without limitation, treatment of the conditions listed herein. The precise effective amount for a subject will depend upon the subject's size and health, the nature and extent of the condition being treated, recommendations of the treating physician, and therapeutics or combination of therapeutics selected for administration.

For administration of the compounds described herein, the dosage ranges from about 0.0001 to 100 mg/kg, and more usually 0.01 to 40 mg/kg, of the host body weight. An exemplary treatment regime entails administration once per day, bi-weekly, tri-weekly, weekly, once every two weeks, once every three weeks, once every four weeks, once a month, once every 3 months or once every three to 6 months.

The disclosed compounds strongly inhibit anchorage-independent cell growth and therefore have the potential to inhibit tumor metastasis. Accordingly, in another aspect the disclosure provides a method for inhibiting tumor metastasis, the method comprising administering an effective amount a pharmaceutical composition of comprising any of the compounds disclosed herein and a pharmaceutically acceptable carrier to a subject in need thereof.

Ras mutations including but not limited to KRAS mutations have also been identified in hematological malignancies (e.g., cancers that affect blood, bone marrow and/or lymph nodes). Accordingly, certain aspects are directed to administration of a disclosed compounds (e.g., in the form of a pharmaceutical composition) to a patient in need of treatment of a hematological malignancy. Such malignancies include, but are not limited to leukemias and lymphomas. For example, the presently disclosed compounds can be used for treatment of diseases such as Acute lymphoblastic leukemia (ALL), Acute myelogenous leukemia (AML), Chronic lymphocytic leukemia (CLL), small lymphocytic lymphoma (SLL), Chronic myelogenous leukemia (CML), Acute monocytic leukemia (AMoL) and/ or other leukemias. In other aspects, the compounds are useful for treatment of lymphomas such as all subtypes of Hodgkins lymphoma or non-Hodgkins lymphoma.

Determining whether a tumor or cancer comprises a KRAS mutation can be undertaken by assessing the nucleotide sequence encoding the KRAS protein, by assessing the amino acid sequence of KRAS protein, or by assessing the characteristics of a putative KRAS mutant protein. The sequence of wild-type human KRAS proteins is known in the art.

Methods for detecting a KRAS mutation are known by those of skill in the art. These methods include, but are not limited to, polymerase chain reaction-restriction fragment length polymorphism (PCR-RFLP) assays, polymerase chain reaction-single strand conformation polymorphism (PCR-SSCP) assays, real-time PCR assays, PCR sequencing, mutant allele-specific PCR amplification (MASA) assays, direct sequencing, primer extension reactions, electrophoresis, oligonucleotide ligation assays, hybridization assays, TaqMan assays, SNP genotyping assays, high resolution melting assays and microarray analyses. In some aspects, samples are evaluated for KRAS mutations including by real-time PCR. In real-time PCR, fluorescent probes specific for the KRAS mutation are used. When a mutation is present, the probe binds and fluorescence is detected. In some aspects, the KRAS mutation is identified using a direct sequencing method of specific regions (e.g., exon 2 and/or exon 3) in the KRAS gene, for example. This technique will identify all possible mutations in the region sequenced.

Methods for detecting a mutation in a KRAS protein are known by those of skill in the art. These methods include, but are not limited to, detection of a KRAS mutant using a binding agent (e.g., an antibody) specific for the mutant protein, protein electrophoresis and Western blotting, and direct peptide sequencing.

Methods for determining whether a tumor or cancer comprises a KRAS mutation can use a variety of samples. In some aspects, the sample is taken from a subject having a tumor or cancer. In some aspects, the sample is taken from a subject having a cancer or tumor. In some aspects, the sample is a fresh tumor/cancer sample. In some aspects, the sample is a frozen tumor/cancer sample. In some aspects, the sample is a formalin-fixed paraffin-embedded sample. In some aspects, the sample is processed to a cell lysate. In some aspects, the sample is processed to DNA or RNA. he disclosure also relates to a method of treating a hyperproliferative disorder in a mammal that comprises administering to said mammal a therapeutically effective amount of a compound of the present disclosure, or a pharmaceutically acceptable salt, solvate, hydrate thereof. In some aspects, said method relates to the treatmentof cancer such as acute myeloid leukemia, cancer in adolescents, adrenocortical carcinoma childhood, AIDS-related cancers (e.g. Lymphoma and Kaposi's Sarcoma), anal cancer, appendix cancer, astrocytomas, atypical teratoid, basal cell carcinoma, bile duct cancer, bladder cancer, bone cancer, brain stem glioma, brain tumor, breast cancer, bronchial tumors, burkitt lymphoma, carcinoid tumor, atypical teratoid, embryonal tumors, germ cell tumor, primary lymphoma, cervical cancer, childhood cancers, chordoma, cardiac tumors, chronic lymphocytic leukemia (CLL), chronic myelogenous leukemia (CML), chronic myleoproliferative disorders, colon cancer, colorectal cancer, craniopharyngioma, cutaneous T-cell lymphoma, extrahepatic ductal carcinoma in situ (DCIS), embryonal tumors, CNS cancer, endometrial cancer, ependymoma, esophageal cancer, esthesioneuroblastoma, ewing sarcoma, extracranial germ cell tumor, extragonadal germ cell tumor, eye cancer, fibrous histiocytoma of bone, gall bladder cancer, gastric cancer, gastrointestinal carcinoid tumor, gastrointestinal stromal tumors (GIST), germ cell tumor, gestational trophoblastic tumor, hairy cell leukemia, head and neck cancer, heart cancer, liver cancer, hodgkin lymphoma, hypopharyngeal cancer, intraocular melanoma, isletcell tumors, pancreatic neuroendocrine tumors, kidney cancer, laryngeal cancer, lip and oral cavity cancer, liver cancer, lobular carcinoma in situ (LCIS), lung cancer, lymphoma, metastatic squamous neck cancer with occult primary, midline tract carcinoma, mouth cancer, multiple endocrine neoplasia syndromes, multiple myeloma/plasma cell neoplasm, mycosis fungoides, myelodysplastic syndromes, myelodysplastic/myeloproliferative neoplasms, multiple myeloma, merkel cell carcinoma, malignant mesothelioma, malignant fibrous histiocytoma of bone and osteosarcoma, nasal cavity and paranasal sinus cancer, nasopharyngeal cancer, neuroblastoma, non-hodgkin lymphoma, non-small cell lung cancer (NSCLC), oral cancer, lip and oral cavity cancer, oropharyngeal cancer, ovarian cancer, pancreatic cancer, papillomatosis, paraganglioma, paranasal sinus and nasal cavity cancer, parathyroid cancer, penile cancer, pharyngeal cancer, pleuropulmonary blastoma, primary central nervous system (CNS) lymphoma, prostate cancer, rectal cancer, transitional cell cancer, retinoblastoma, rhabdomyosarcoma, salivary gland cancer, skin cancer, stomach (gastric) cancer, small cell lung cancer, small intestine cancer, soft tissue sarcoma, T-Cell lymphoma, testicular cancer, throat cancer, thymoma and thymic carcinoma, thyroid cancer, transitional cell cancer of the renal pelvis and ureter, trophoblastic tumor, unusual cancers of childhood, urethral cancer, uterine sarcoma, vaginal cancer, vulvar cancer, or Viral-Induced cancer. In some aspects, said method relates to the treatment of a non-cancerous hyperproliferative disorder such as benign hyperplasia of the skin (e.g., psoriasis), restenosis, or prostate (e.g., benign prostatic hypertrophy (BPH)).

In certain aspects, the disclosure relates to methods for treatment of lung cancers, the methods comprise administering an effective amount of any of the above-described compound (or a pharmaceutical composition comprising the same) to a subject in need thereof. In certain aspects the lung cancer is a non-small cell lung carcinoma (NSCLC), for example adenocarcinoma, squamous-cell lung carcinoma or large-cell lung carcinoma. In other aspects, the lung cancer is a small cell lung carcinoma. Other lung cancers treatable with the disclosed compounds include, but are not limited to, glandular tumors, carcinoid tumors and undifferentiated carcinomas. Subjects that can be treated with compounds of the disclosure, or pharmaceutically acceptable salt, solvate, tautomer, hydrate of said compounds, according to the methods of this disclosure include, for example, subjects that have been diagnosed as having acute myeloid leukemia, acute myeloid leukemia, cancer in adolescents, adrenocortical carcinoma childhood, AIDS-related cancers (e.g. Lymphoma and Kaposi's Sarcoma), anal cancer, appendix cancer, astrocytomas, atypical teratoid, basal cell carcinoma, bile duct cancer, bladder cancer, bone cancer, brain stem glioma, brain tumor, breast cancer, bronchial tumors, burkitt lymphoma, carcinoid tumor, atypical teratoid, embryonal tumors, germ cell tumor, primary lymphoma, cervical cancer, childhood cancers, chordoma, cardiac tumors, chronic lymphocytic leukemia (CLL), chronic myelogenous leukemia (CML), chronic myleoproliferative disorders, colon cancer, colorectal cancer, craniopharyngioma, cutaneous T-cell lymphoma, extrahepatic ductal carcinoma in situ (DCIS), embryonal tumors, CNS cancer, endometrial cancer, ependymoma, esophageal cancer, esthesioneuroblastoma, ewing sarcoma, extracranial germ cell tumor, extragonadal germ cell tumor, eye cancer, fibrous histiocytoma of bone, gall bladder cancer, gastric cancer, gastrointestinal carcinoid tumor, gastrointestinal stromal tumors (GIST), germ cell tumor, gestational trophoblastic tumor, hairy cell leukemia, head and neck cancer, heart cancer, liver cancer, hodgkin lymphoma, hypopharyngeal cancer, intraocular melanoma, islet cell tumors, pancreatic neuroendocrine tumors, kidney cancer, laryngeal cancer, lip and oral cavity cancer, liver cancer,lobular carcinoma in situ (LCIS), lung cancer, lymphoma, metastatic squamous neck cancer with occult primary, midline tract carcinoma, mouth cancer, multiple endocrine neoplasia syndromes, multiple myeloma/plasma cell neoplasm, mycosis fungoides, myelodysplastic syndromes, myelodysplastic/myeloproliferative neoplasms, multiple myeloma, merkel cell carcinoma, malignant mesothelioma, malignant fibrous histiocytoma of bone and osteosarcoma, nasal cavity and paranasal sinus cancer, nasopharyngeal cancer, neuroblastoma, non-hodgkin lymphoma, non-small cell lung cancer (NSCLC), oral cancer, lip and oral cavity cancer, oropharyngeal cancer, ovarian cancer, pancreatic cancer, papillomatosis, paraganglioma, paranasal sinus and nasalcavity cancer, parathyroid cancer, penile cancer, pharyngeal cancer, pleuropulmonary blastoma, primary central nervous system (CNS) lymphoma, prostate cancer, rectal cancer, transitional cell cancer, retinoblastoma, rhabdomyosarcoma, salivary gland cancer, skin cancer, stomach (gastric) cancer, small cell lung cancer, small intestine cancer, soft tissue sarcoma, T-Cell lymphoma, testicular cancer, throat cancer, thymoma and thymic carcinoma, thyroid cancer, transitional cell cancer of the renal pelvis and ureter, trophoblastic tumor, unusual cancers of childhood, urethral cancer, uterine sarcoma, vaginal cancer, vulvar cancer, or Viral-Induced cancer. In some aspects subjects that are treated with the compounds of the disclosure include subjects that have been diagnosed as having a non-cancerous hyperproliferative disorder such as benign hyperplasia of the skin (e. g., psoriasis), restenosis, or prostate (e. g., benign pro static hypertrophy (BPH)). The disclosure further provides methods of modulating a mutant KRAS protein activity by contacting the protein with an effective amount of a compound of the disclosure. Modulation can be inhibiting or activating protein activity. In some aspects, the disclosure provides methods of inhibiting protein activity by contacting the mutant KRAS protein with an effective amount of a compound of the disclosure in solution. In some aspects, the disclosure provides methods of inhibiting the mutant KRAS protein activity by contacting a cell, tissue, organ that express the protein of interest. In some aspects, the disclosure provides methods of inhibiting protein activity in a subject including but not limited to rodents and mammal (e.g., human) by administering into the subject an effective amount of a compound of the disclosure. In some aspects, the percentage modulation exceeds 25%, 30%, 40%, 50%, 60%, 70%, 80%, or 90%. In some aspects, the percentage of inhibiting exceeds 25%, 30%, 40%, 50%, 60%, 70%, 80%, or 90%.In some aspects, the disclosure provides methods of inhibiting KRAS activity in a cell by contacting said cell with an amount of a compound of the disclosure sufficient to inhibit the activity of a KRAS mutant in said cell. In some aspects, the disclosure provides methods of inhibiting mutant KRAS in a tissue by contacting said tissue with an amount of a compound of the disclosure sufficient to inhibit the activity of mutant KRAS in said tissue. In some aspects, the disclosure provides methods of inhibiting KRAS in an organism by contacting said organism with an amount of a compound of the disclosure sufficient to inhibit the activity of KRAS in said organism. In some aspects, the disclosure provides methods of inhibiting KRAS activity in an animal by contacting said animal with an amount of a compound of the disclosure sufficient to inhibit the activity of KRAS in said animal. In some aspects, the disclosure provides methods of inhibiting KRAS including in a mammal by contacting said mammal with an amount of a compound of the disclosure sufficient to inhibit the activity of KRAS in said mammal. In some aspects, the disclosure provides methods of inhibiting KRAS activity in a human by contacting said human with an amount of a compond of the disclosure sufficient to inhibit the activity of KRAS in said human. The present disclosure provides methods of treating a disease mediated by KRAS activity in a subject in need of such treatment. The present disclosure also provides methods for combination therapies in which an agent known to modulate other pathways, or other components of the same pathway, or even overlapping sets of target enzymes are used in combination with a compound of the present disclosure, or a pharmaceutically acceptable salt, ester, prodrug, solvate, tautomer, hydrate or derivative thereof. In one aspect, such therapy includes but is not limited to the combination of one or more compounds of the disclosure with chemotherapeutic agents, therapeutic antibodies, and radiation treatment.

Many chemotherapeutics are presently known in the art and can be used in combination with the compounds of the disclosure. In some aspects, the chemotherapeutic is selected from the group consisting of mitotic inhibitors, alkylating agents, anti-metabolites, intercalating antibiotics, growth factor inhibitors, cell cycle inhibitors, enzymes, topoisomerase inhibitors, biological response modifiers, anti-hormones, angiogenesis inhibitors, and antiandrogens. In some aspects, the chemotherapeutic agent is an immunooncology (IO) agent that can enhance, stimulate, or upregulate the immune system.

The compounds described herein can be used in combination with the agents disclosed herein or other suitable agents, depending on the condition being treated. Hence, in some aspects the one or more compounds of the disclosure will be co-administered with other agents as described above. When used in combination therapy, the compounds described herein are administered with the second agent simultaneously or separately. This administration in combination can include simultaneous administration of the two agents in the same dosage form, simultaneous administration in separate dosage forms, and separate administration. That is, a compound described herein and any of the agents described above can be formulated together in the same dosage form and administered simultaneously. Alternatively, a compound of the disclosure and any of the agents described above can be simultaneously administered, wherein both the agents are present in separate formulations. In another alternative, a compound of the present disclosure can be administered just followed by and any of the agents described above, or vice versa. In some aspects of the separate administration protocol, a compound of the disclosure and any of the agents described above are administered a few minutes apart, or a few hours apart, or a few days apart.

The compounds can be made by methods known in the art including those described below Some reagents and intermediates are known in the art. Other reagents and intermediates can be made by methods known in the art using readily available materials. Any variables (e.g. numbered "R" substituents) used to describe the synthesis of the compounds are intended only to illustrate how to make the compounds and are not to be confused with variables used in the claims or in other sections of the specification. The following methods are for illustrative purposes.

### Synthesis

### General Schemes

The compounds described herein can be prepared as shown below and as described in Methods 1-4.

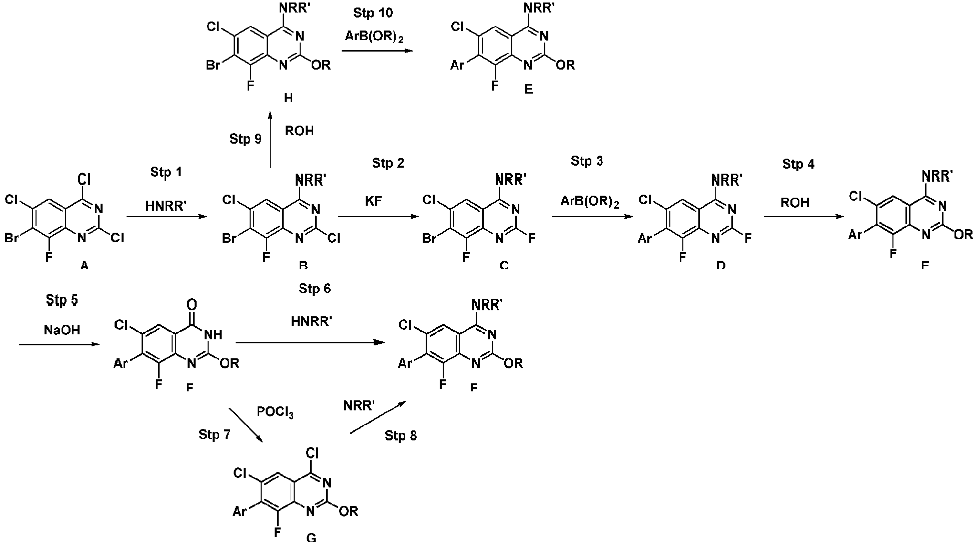

Method 1: In Step 1, known compound A (CAS 1698028-11-3) is reacted with an amine in a suitable solvent such as THF with a base such as diisopropylethylamine to provide compound B. In Step 2, treatment of compound B with potassium fluoride in a solvent such as dimethylacetamide provides compound C. In Step 3, compound C is coupled with an arylboronic acid or ester under Suzuki conditions to provide compound D. In Step 4, compound D is treated with ROH in the presence of a base in a solvent such as THF to provide compound E.

Method 2: The amino group of compound E may be switched to a different amino group by the following sequence. In Step 5, base hydrolysis provides compound F. In step 6, introduction of an amine substituent is accomplished with using a coupling agent such as BOP in the presence of base in solvent such as dichloromethane to provide compound E.

Method 3: In Step 7, treatment of compound H with POCl₃ in the presence of a base provides compound G. Treatment of compound G with an appropriate amine in the presence of a base in a solvent such as dimethylacetamide provides compound E.

Method 4: In Step 9, treatment of compound B with an alcohol of formula R-OH in the presence of base provides compound H. In Step 10, compound H is coupled with an arylboronic acid or ester under Suzuki conditions to provide compound E. Protecting groups such as Boc, PMB, MOM, etc. may be introduced and removed as required by one skilled in the art and are described in the Examples. Functionalization and elaboration of the Aryl, NRR', and OR groups to prepare compounds of general structure E are described in the Examples.

### EXAMPLES

The invention is further defined in the following Examples.

### Abbreviations

The following abbreviations are used in the example section below and elsewhere herein:

| *Abbreviation* | *Full Name* |
|---|---|
| Ac | acetyl |
| ACN | acetonitrile |
| aq. | aqueous |
| BOP | (benzotriazol- 1 -yloxytris(dimethylamino)phosphonium hexafluorophosphate) |
| DCE | 1,2-dichloroethane |
| DCM | dichloromethane |
| DIPEA | diisopropylethylamine |
| DMA | N,N-dimethylacetamide |
| DMF | N,N-dimethylformamide |
| DMSO | dimethylsulfoxide |
| DMOCP | 2-chloro-5,5-dimethyl-1,3,2-dioxaphosphorinane 2-oxide |
| dppf | 1,1'-bis(diphenylphosphino)ferrocene |
| EtOAc | ethyl acetate |
| Et₃N or TEA | triethylamine |
| EtOH | ethanol |
| h | hours |
| HPLC | high-performance liquid chromatography |
| iPr | isopropyl |
| LiHMDS | lithium hexamethyldisilazide |
| MeOH | methanol |
| min | minutes |
| NIS | N-iodosuccinimide |
| RT | room temperature |
| sat., satd. or sat'd | saturated |
| TBAF | tetrabutylammonia fluoride |
| TBS | t-butyldimethylsilyl |
| TFA | trifluoroacetic acid |
| TosOH | p-toluenesulfonic acid |
| tR | retention time |

### Example 1-1

### 4-(6-chloro-4-{3,9-diazabicyclo[4.2.1][nonan-9-yl}-8-fluoro-2-{[(2S)-1-methylpyrrolidin-2-yl]methoxy}quinazolin-7-yl)naphthalen-2-ol

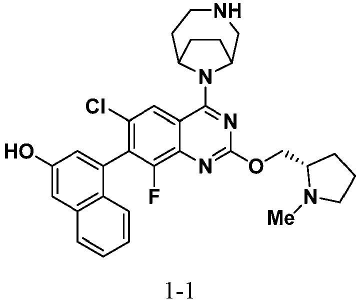

### Preparation of Intermediate 1A: tert-butyl 4-(7-bromo-2, 6-dichloro-8-fluoroquinazolin-4-yl)piperazine-1-carboxylate

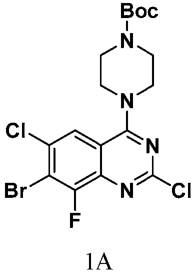

To a solution of 7-bromo-2,4,6-trichloro-8-fluoroquinazoline (1 g, 3.03 mmol) and DIPEA (1.32 mL, 7.57 mmol) in THF (15 mL) was added tert-butyl piperazine-1-carboxylate (0.56 g, 3.03 mmol) under N₂. The reaction was stirred at 25 °C for 2 h. The mixture was then concentrated. The residue was diluted with ethyl acetate (60 mL) and washed with water (30 mLx2) and brine (50 mL). The organic phase was dried over anhydrous sodium sulphate, filtered and concentrated under reduced pressure. The residue was purified by silica gel column chromatography (silica gel, petroleum ether: ethyl acetate = 10: 1 to 4: 1) to afford tert-butyl 4-(7-bromo-2,6-dichloro-8-fluoroquinazolin-4-yl)piperazine-1-carboxylate (1.33 g, 2.77 mmol, 91.5% yield) as a yellow solid. MS (ESI) *m*/*z* 481.0 [M+1]⁺. ¹H NMR (400 MHz, CDCl₃) δ 7.77 (d, *J*= 1.6 Hz, 1H), 3.93 - 3.84 (m, 4H), 3.72 - 3.61 (m, 4H), 1.50 (s, 9H).

### Preparation of Intermediate IE: tert-butyl (S)-4-(7-bromo-6-chloro-8-fluoro-2-((1-methylpyrrolidin-2-yl)methoxy)quinazolin-4-yl)piperazine-1-carboxylate

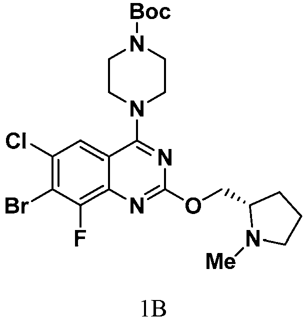

To a solution of tert-butyl 4-(7-bromo-2,6-dichloro-8-fluoroquinazolin-4-yl)piperazine-1-carboxylate (540 mg, 1.125 mmol) in DMSO (6 mL) was added cesium fluoride (342 mg, 2.249 mmol) and (S)-(1-methylpyrrolidin-2-yl)methanol (324 mg, 2.81 mmol). The mixture was heated to 100 °C for 2 h. After the mixture was cooled to room temperature, sat. NaHCO₃ (50 mL) was added. The inorganic phase was extracted with DCM (50 mL x2). The combined DCM extracts were washed with brine and dried (Na₂SO₄), filtered and then concentrated. The residue was purified on a 40 g silica column, eluting with 0-10% MeOH/DCM (w/0.5% TEA) to provide tert-butyl (S)-4-(7-bromo-6-chloro-8-fluoro-2-((1-methylpyrrolidin-2-yl)methoxy)quinazolin-4-yl)piperazine-1-carboxylate (0.43 g, 0.769 mmol, 68.4 % yield) as a yellow solid. MS (ESI) *m*/*z* 558.2/560.2 [M+1]⁺.

### Preparation of Intermediate 1C: tert-butyl 4-(6-chloro-8-fluoro-7-(3-hydroxynaphthalen-1-yl)-2-(((S)-1-methylpyrrolidin-2-yl)methoxy)quinazolin-4-yl)piperazine-1-carboxylate

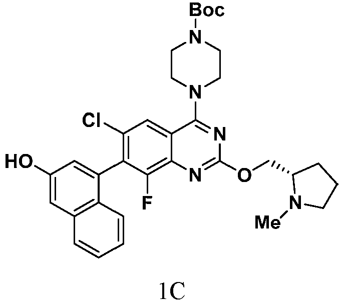

A suspension of tert-butyl (S)-4-(7-bromo-6-chloro-8-fluoro-2-((1-methylpyrrolidin-2-yl)methoxy)quinazolin-4-yl)piperazine-1-carboxylate (430 mg, 0.769 mmol), 4-(4,4,5,5-tetramethyl-1,3,2-dioxaborolan-2-yl)naphthalen-2-ol (249 mg, 0.923 mmol), Na₂CO₃ (245 mg, 2.308 mmol) in 1,4-dioxane (10 mL) and water (2 mL) was degassed with N₂ for 5 min, then tetrakis(triphenylphosphine)palladium(0)(178 mg, 0.154 mmol) was added in one portion. The resulting mixture was degassed with N₂, then heated in a microwave at 95 °C for 1 h. The reaction was cooled to RT, filtered and the filter cake was washed with dioxane (2 mL x3). The filtrate and washes were combined and concentrated. The residue was purified on 24 g of silica, eluting with 0-10% MeOH/DCM (w 0. 5% TEA) to provide tert-butyl 4-(6-chloro-8-fluoro-7-(3-hydroxynaphthalen-1-yl)-2-(((S)-1-methylpyrrolidin-2-yl)methoxy)quinazolin-4-yl)piperazine-1-carboxylate (174 mg, 0.280 mmol, 36.4 % yield) as a yellow solid. MS (ESI) *m*/*z* 622.4 [M+1]⁺.

### Preparation of Intermediate 1D: 6-chloro-8-fluoro-7-(3-hydroxynaphthalen-1-yl)-2-(((S)-1-methylpyrrolidin-2-yl)methoxy)quinazolin-4(3H)-one

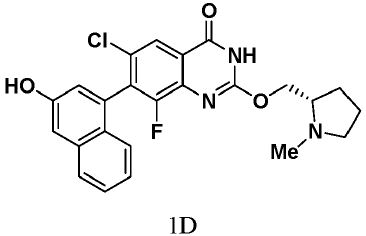

A solution of tert-butyl 4-(6-chloro-8-fluoro-7-(3-hydroxynaphthalen-1-yl)-2-(((S)-1-methylpyrrolidin-2-yl)methoxy)quinazolin-4-yl)piperazine-1-carboxylate (400 mg, 0.643 mmol) in EtOH (5 mL) and water (0.5 mL) was treated with sodium hydroxide (1.286 mL, 1.286 mmol). The reaction was stirred at 50 °C for 16 h. The mixture was concentrated under vacuum. The residue was purified by flash chromatography on silica gel eluting with 0-10% MeOH/DCM (w 0.5% TEA) to afford the desired compound (174 mg, 0.383 mmol, 59.6 % yield) as a white solid. MS (ESI) *m*/*z:* 454.1 [M+H]⁺.

### Preparation of Intermediate IE: tert-butyl 9-(6-chloro-8-fluoro-7-(3-hydroxynaphthalen-1-yl)-2-(((S)-1-methylpyrrolidin-2-yl)methoxy)quinazolin-4-yl)-3,9-diazabicyclo[4.2.1]nonane-3-carboxylate

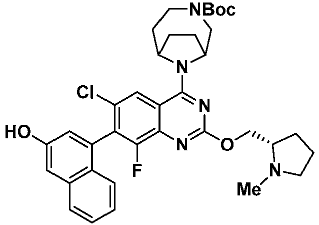

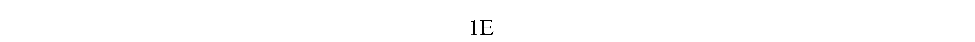

To a mixture of 6-chloro-8-fluoro-7-(3-hydroxynaphthalen-1-yl)-2-(((S)-1-methylpyrrolidin-2-yl)methoxy)quinazolin-4-ol (10 mg, 0.022 mmol), tert-butyl 3,9-diazabicyclo[4.2.1]nonane-3-carboxylate (5.98 mg, 0.026 mmol) and DIEA (11.54 µL, 0.066 mmol) in DCM (2 mL) was added BOP (12.18 mg, 0.028 mmol) and the reaction was stirred for 16 h at room temperature. The reaction was quenched with 4 mL of NaHCO₃ aq, and extracted with DCM (5 mLx3). The combined organic layers were washed with brine, dried over Na₂SO₄ filtered and concentrated under reduced pressure. The residue was dissolved in 2 mL of THF and TBAF (88 µL, 0.088 mmol) was added. The mixture was stirred at room temperature for 15 min. The reaction mixture was concentrated and the residue was purified on 4 g of silica eluting with 0-10% MeOH/DCM (w 0.5% TEA) to provide 1E (12 mg, 0.018 mmol, 82 % yield). MS (ESI) *m*/*z:* 662.7[M+H]⁺.

### Preparation of Example 1-1: 4-(4-(3,9-diazabicyclo[4.2. 1]nonan-9-yl)-6-chloro-8-fluoro-2-(((S)-1-methylpyrrolidin-2-yl)methoxy)quinazolin-7-yl)naphthalen-2-ol

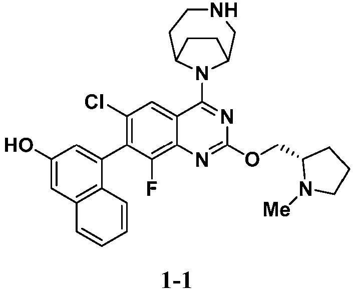

tert-Butyl 9-(6-chloro-8-fluoro-7-(3-hydroxynaphthalen-1-yl)-2-(((S)-1-methylpyrrolidin-2-yl)methoxy)quinazolin-4-yl)-3,9-diazabicyclo[4.2.1]nonane-3-carboxylate (12 mg, 0.018 mmol) was treated with 1 mL of 30% TFA/DCM for 30 min at room temperatue. Then the reaction mixture was concentrated. The crude material was purified via preparative HPLC with the following conditions: Column: XBridge C18, 200 mm x 19 mm, 5-µm particles; Mobile Phase A: 5:95 acetonitrile: water with ammonium acetate; Mobile Phase B: 95:5 acetonitrile: water with ammonium acetate; Gradient: a 0-minute hold at 14% B, 14-54% B over 20 minutes, then a 0-minute hold at 100% B; Flow Rate: 20 mL/min; Column Temperature: 25 C. Fractions containing the desired product were combined and dried via centrifugal evaporation. The material was further purified via preparative HPLC with the following conditions: Column: XBridge C18, 200 mm x 19 mm, 5-µm particles; Mobile Phase A: 5:95 acetonitrile: water with 0.05% trifluoroacetic acid; Mobile Phase B: 95:5 acetonitrile: water with 0.05% trifluoroacetic acid; Gradient: a 0-minute hold at 2% B, 2-42% B over 20 minutes, then a 0-minute hold at 100% B; Flow Rate: 20 mL/min; Column Temperature: 25 C. Fractions containing the desired product were combined and dried via centrifugal evaporation to provide Example 1-1 (1.3 mg, 1.6 µmol, 9.1% yield). MS (ESI) *m*/*z* 562.2 [M+1]⁺; ¹H NMR (500 MHz, DMSO-d₆) δ 8.28 - 7.93 (m, 1H), 7.83 - 7.61 (m, 1H), 7.54 - 7.35 (m, 1H), 7.27 - 7.18 (m, 2H), 7.11 - 6.93 (m, 2H), 5.49 - 5.11 (m, 1H), 4.79 - 4.47 (m, 2H), 4.29 - 4.06 (m, 1H), 3.23 - 3.09 (m, 1H), 2.98 - 2.76 (m, 2H), 2.72 (s, 3H), 2.65 - 2.56 (m, 1H), 2.33 - 2.08 (m, 2H), 2.01 - 0.67 (m, 11H).

Examples in Table 1 were prepared according to procedures described for Example 1-1 from appropriate starting materials.

**Table 1**

| Example Number | Structure | Name | **MS** m/z: [M+H]+ | ¹H NMR |
|---|---|---|---|---|
| 1-2 | 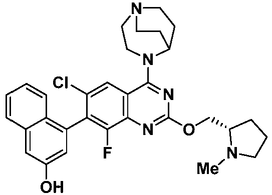 | 4-(6-chloro-4-{1,4-diazabicyclo[3.2.2]nonan-4-yl}-8-fluoro-2-{[(2S)-1-methylpyrrolidin-2-yl]methoxy}quinazolin-7-yl)naphthalen-2-ol | 561.93 | ¹H NMR (500 MHz, DMSO-d₆) δ 7.99 (s, 1H), 7.82 (br d, J=8.2 Hz, 1H), 7.45 (br t, J=7.2 Hz, 1H), 7.30 (s, 1H), 7.27 - 7.14 (m, 2H), 7.07 (d, J=2.1 Hz, 1H), 4.74 (br s, 1H), 4.43 (m, 1H), 4.36 - 4.21 (m, 1H), 4.19 - 4.01 (m, 2H), 3.20 - 2.98 (m, 5H), 2.55 (s, 3H), 2.38 (m, 2H), 2.26 (m, 2H), 2.10 - 1.94 (m, 4H), 1.84 - 1.62 (m, 4H) |

### Example 2-1

### 6-(6-chloro-4-{3,9-diazabicyclo[4.2.1]nonan-3-yl}-8-fluoro-2-{[(2S)-1-methylpyrrolidin-2-yl]methoxy}quinazolin-7-yl)-4-methyl-5-(trifluoromethyl)pyridin-2-amine

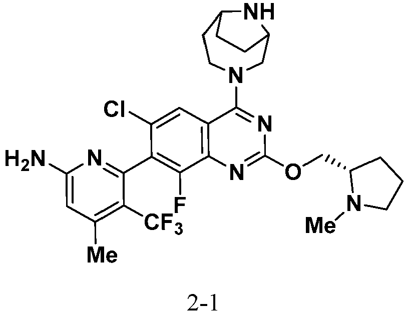

### Preparation of Intermediate 2A: 6-bromo-N,N-bis(4-methoxybenzyl)-4-methylpyridin-2-amine

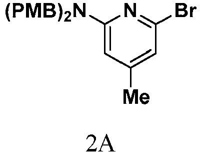

To a solution of 6-bromo-4-methylpyridin-2-amine (1 g, 5.35 mmol) in DMF (20 mL) was added NaH (0.64 g, 16 mmol, 60%) at 0 °C. The mixture was stirred at 0 °C for 0.5 h. Then 1-(chloromethyl)-4-methoxybenzene (2.1 g, 13.4 mmol) was added. The mixture was stirred at 0 °C for 1.5 h. The reaction was quenched with saturated NH₄Cl (20 mL) and extracted with ethyl acetate (20 mLx3), The combined organic layers were washed with brine (50 mL) and dried over anhydrous Na₂SO₄. The mixture was filtered. The filtrate was concentrated in vacuo. The residue was purified by column chromatography (silica gel, petroleum ether:ethyl acetate = 5: 1) to afford 6-bromo-N,N-bis(4-methoxybenzyl)-4-methylpyridin-2-amine (2 g, 4.68 mmol, 87.5% yield) as a colorless oil. ¹H NMR (400 MHz, CDCl₃) δ 7.16 (d, *J =* 8.8 Hz, 4H), 6.88 - 6.84 (m, 4H), 6.60 (s, 1H), 6.16 (s, 1H), 4.64 (s, 4H), 3.80 (s, 6H), 2.13 (s, 3H).

### Preparation of Intermediate 2B: (6-(bis(4-methoxybenzyl)amino)-4-methylpyridin-2-yl)boronic acid

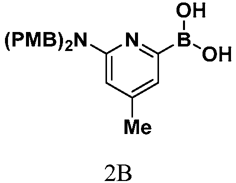

To a solution of 6-bromo-N,N-bis(4-methoxybenzyl)-4-methylpyridin-2-amine (1000 mg, 2.34 mmol), bis(pinacolato)diboron (832.5 mg, 3.28 mmol), and (1,1'-bis(diphenylphosphino)ferrocene)palladium(II) dichloride (171 mg, 0.23 mmol) in 1,4-dioxane (20 mL) was added KOAc (459.32 mg, 4.68 mmol). The mixture was stirred at 90°C for 5 h under N₂. The reaction mixture was filtered. The filtrate containing the crude product (6-(bis(4-methoxybenzyl)amino)-4-methylpyridin-2-yl)boronic acid (918 mg, 2.34 mmol, crude) in 1,4-dioxane (20 mL)) was used in the next step without purification. MS (ESI) *m*/*z* 393.3 [M+1]⁺.

### Preparation of Intermediate 2C: tert-butyl 4-(7-bromo-2,6-dichloro-8-fluoroquinazolin-4-yl)piperazine-1-carboxylate

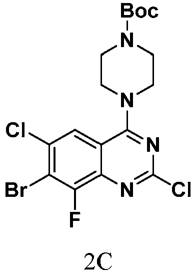

To a solution of 7-bromo-2,4,6-trichloro-8-fluoroquinazoline (1 g, 3.03 mmol) and DIPEA (1.32 mL, 7.57 mmol) in THF (15 mL) was added tert-butyl piperazine-1-carboxylate (0.56 g, 3.03 mmol) under N₂. The reaction mixture was stirred at 25 °C for 2 h. The mixture was then concentrated. The residue was diluted with ethyl acetate (60 mL),washed with water (30 mLx2) and brine (50 mL). The organic phase was dried over anhydrous sodium sulfate, filtered and concentrated under reduced pressure. The residue was purified by silica gel column chromatography (silica gel, petroleum ether:ethyl acetate = 10: 1 to 4: 1) to afford tert-butyl 4-(7-bromo-2,6-dichloro-8-fluoroquinazolin-4-yl)piperazine-1-carboxylate (1.33 g, 2.77 mmol, 91.5% yield) as a yellow solid. MS (ESI) *m*/*z* 481.0 [M+3]⁺. ¹H NMR (400 MHz, CDCl₃) δ 7.77 (d, *J*= 1.6 Hz, 1H), 3.93 - 3.84 (m, 4H), 3.72 - 3.61 (m, 4H), 1.50 (s, 9H).

### Preparation of Intermediate 2D: tert-butyl 4-(7-bromo-6-chloro-2,8-difluoroquinazolin-4-yl)piperazine-1-carboxylate

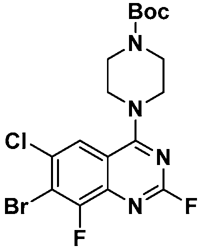

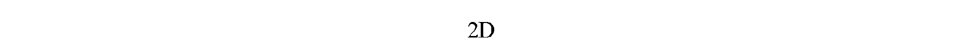

A solution of *tert-butyl* 4-(7-bromo-2,6-dichloro-8-fluoroquinazolin-4-yl)piperazine-1-carboxylate (1000 mg, 2.08 mmol) and potassium fluoride (2420 mg, 41.65 mmol) in DMA (10 mL) was stirred at 110°C for 12 h under N₂. The reaction mixture was quenched with water (30 mL) and extracted with EtOAc (30 mLx3). The combined organic layers were washed with brine (30 mLx3) and dried over anhydrous Na₂SO₄. The mixture was filtered, and the filtrate was concentrated under reduced pressure. The residue was purified by silica gel column chromatography (silica gel, petroleum ether: ethyl acetate = 20: 1 to 3: 1) to provide tert-butyl 4-(7-bromo-6-chloro-2,8-difluoroquinazolin-4-yl)piperazine-1-carboxylate (730 mg, 1.57 mmol, 75.6% yield) as a yellow solid. MS (ESI) *m*/*z* 463.1 [M+1]⁺.

### Preparation of Intermediate 2E: tert-butyl 4-(7-(6-(bis(4-methoxybenzyl)amino)-4-methylpyridin-2-yl)-6-chloro-2,8-difluoroquinazolin-4-yl)piperazine-1-carboxylate

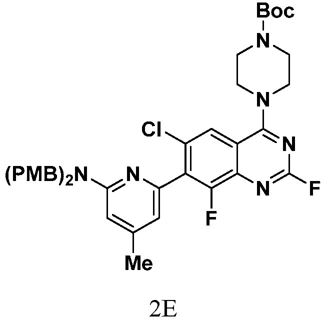

A solution of *tert-butyl* 4-(7-bromo-6-chloro-2,8-difluoroquinazolin-4-yl)piperazine-1-carboxylate (600 mg, 1.29 mmol), (6-(bis(4-methoxybenzyl)amino)-4-methylpyridin-2-yl)boronic acid (756 mg, 1.93 mmol), (1,1'-bis(diphenylphosphino)ferrocene)palladium(II) dichloride (104 mg, 0.14 mmol) and potassium phosphate (548 mg, 2.59 mmol) in 1,4-dioxane (20 mL) and water (2 mL) was stirred at 60 °C for 12h under N₂. The mixture was filtered. The filtrate was concentrated in vacuo. The residue was purified by silica gel column chromatography (silica gel, petroleum ether:ethyl acetate = 10: 1 to 3: 1) to provide tert-butyl 4-(7-(4-(bis(4-methoxybenzyl)amino)-6-methylpyridin-2-yl)-6-chloro-2,8-difluoroquinazolin-4-yl)piperazine-1-carboxylate (600 mg, 0.82 mmol, 63.4% yield) as a yellow oil. MS (ESI) *m*/*z* 731.4 [M+1]⁺; ¹H NMR (400 MHz, CDCl₃) δ 7.76 (d, *J=* 1.2 Hz, 1 H), 7.18 (d, *J= 8.8* Hz, 4 H), 6.85 (d, *J =* 8.8 Hz, 4H), 6.59 (s, 1H), 6.37 (s, 1H), 4.69 (s, 4H), 3.98 - 3.87 (m, 4H), 3.80 (s, 6H), 3.69 - 3.65 (m, 4H), 2.27 (s, 3H), 1.51 (s, 9H).

### Preparation of Intermediate 2F: tert-butyl 4-(7-(6-(bis(4-methoxybenzyl)amino)-3-iodo-4-methylpyridin-2-yl)-6-chloro-2,8-difluoroquinazolin-4-yl)piperazine-1-carboxylate

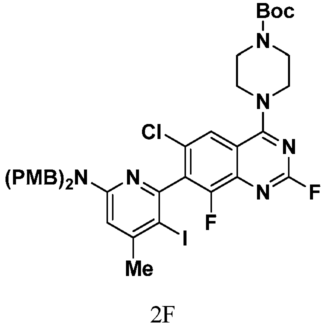

A solution of *tert*-butyl 4-(7-(4-(bis(4-methoxybenzyl)amino)-6-methylpyridin-2-yl)-6-chloro-2,8-difluoroquinazolin-4-yl)piperazine-1-carboxylate (800 mg, 1.09 mmol), TosOH (5 mg, 0.05 mmol) and NIS (1200 mg, 5.33 mmol) in DMF (10 mL) was stirred at 25 °C for 12 h. The reaction mixture was diluted with water (15 mL) and EtOAc (15 mL). The mixture was extracted with EtOAc (30 mLx3). The combined organic layers were washed with brine (30 mLx3), dried over anhydrous sodium sulfate, filtered and concentrated under reduced pressure. The residue was purified by column chromatography (silica gel, petroleum ether: ethyl acetate = 10: 1 to 3: 1) to afford tert-butyl 4-(7-(6-(bis(4-methoxybenzyl)amino)-3-iodo-4-methylpyridin-2-yl)-6-chloro-2,8-difluoroquinazolin-4-yl)piperazine-1-carboxylate (400 mg, 0.467 mmol, 42.7% yield) as a yellow solid. MS (ESI) *m*/*z* 857.2 [M+1]⁺; ¹H NMR (400 MHz, CDCl₃) δ 7.82 (d, *J =* 1.2 Hz, 1H), 7.17 (d, *J* = 8.4 Hz, 4H), 6.86 (d, *J* = 8.4 Hz, 4H), 6.48 (s, 1H), 4.76 - 4.65 (m, 2H), 4.62 - 4.50 (m, 2H), 4.01 - 3.92 (m, 4H), 3.82 (s, 6H), 3.72 - 3.63 (m, 4H), 2.38 (s, 3H), 1.52 (s, 9H).

### Preparation of Intermediate 2G: tert-butyl 4-(7-(6-(bis(4-methoxybenzyl)amino)-4-methyl-3-(trifluoromethyl)pyridin-2-yl)-6-chloro-2,8-difluoroquinazolin-4-yl)piperazine-1-carboxylate

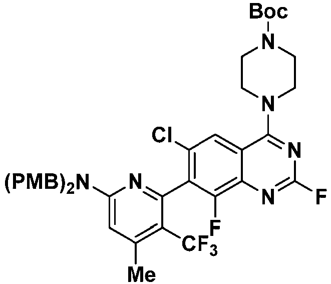

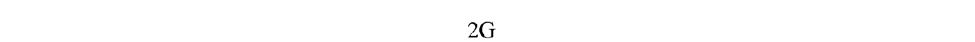

A mixture of *tert-butyl* 4-(7-(6-(bis(4-methoxybenzyl)amino)-3-iodo-4-methylpyridin-2-yl)-6-chloro-2,8-difluoroquinazolin-4-yl)piperazine-1-carboxylate (400 mg, 0.4700 mmol), methyl 2,2-difluoro-2-fluorosulfonyl-acetate (1345 mg, 7 mmol) and CuI (267 mg, 1.4 mmol) in DMA (10 mL) was stirred at 80 °C for 5 h under N₂. The reaction mixture was then cooled to room temperature and additional CuI (267 mg, 1.4 mmol) and methyl 2,2-difluoro-2-fluorosulfonyl-acetate (1345 mg, 7 mmol) were added to the mixture. The reaction mixture was stirred at 80 °C for another 12 h under N₂. The mixture was diluted with EtOAc (50 mL) and filtered. The filtrate was washed with brine (30 mLx3) and dried over anhydrous Na₂SO₄ and filtered. The filtrate was concentrated in vacuo. The residue was purified by column chromatography (silica gel, petroleum ether:ethyl acetate = 10: 1 to 3: 1) to afford tert-butyl 4-(7-(6-(bis(4-methoxybenzyl)amino)-4-methyl-3-(trifluoromethyl)pyridin-2-yl)-6-chloro-2,8-difluoroquinazolin-4-yl)piperazine-1-carboxylate (270 mg, 0.34 mmol, 72.4% yield) as a yellow solid. MS (ESI) *m*/*z* 799.0 [M+1]⁺.

### Preparation of Intermediate 2H: tert-butyl 4-(7-(6-(bis(4-methoxybenzyl)amino)-4-methyl-3-(trifluoromethyl)pyridin-2-yl)-6-chloro-2,8-difluoroquinazolin-4-yl)piperazine-1-carboxylate

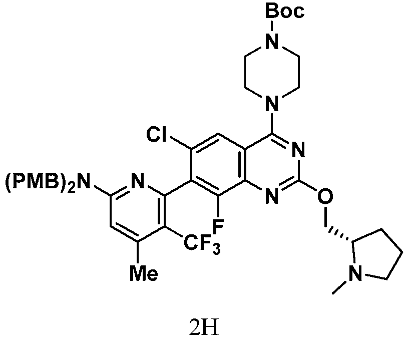

To a solution of (2S)-1-methylpyrrolidin-2-yl methanol (97.6 mg, 0.85 mmol) in THF (10 mL) was added NaH (81 mg, 2.03 mmol, 60%) at 0 °C. The mixture was stirred at 0 °C for 0.5 h. tert-Butyl 4-(7-(6-(bis(4-methoxybenzyl)amino)-4-methyl-3-(trifluoromethyl)pyridin-2-yl)-6-chloro-2,8-difluoroquinazolin-4-yl)piperazine-1-carboxylate (270 mg, 0.34 mmol) in THF (5 mL) was added. The mixture was stirred at 0 °C for 1 h. The reaction mixture was then quenched with saturated NH₄Cl (20 mL) and extracted with EtOAc (20 mLx3). The combined organic layers were washed with brine (30 mL) and dried over anhydrous Na₂SO₄. The mixture was filtered and the filtrate was concentrated under reduced pressure. The residue was purified by column chromatography (silica gel, DCM:MeOH = 10: 1) to provide *tert*-butyl 4-(7-(6-(bis(4-methoxybenzyl)amino)-4-methyl-3-(trifluoromethyl)pyridin-2-yl)-6-chloro-2,8-difluoroquinazolin-4-yl)piperazine-1-carboxylate (250 mg, 0.28 mmol, 82.7% yield) as a white solid. MS (ESI) *m*/*z* 894.5 [M+1]⁺.

### Preparation of Intermediate 2I: 7-(6-(bis(4-methoxybenzyl)amino)-4-methyl-3-(trifluoromethyl)pyridin-2-yl)-6-chloro-8-fluoro-2-(((S)-1-methylpyrrolidin-2-yl)methoxy)quinazolin-4(3H)-one

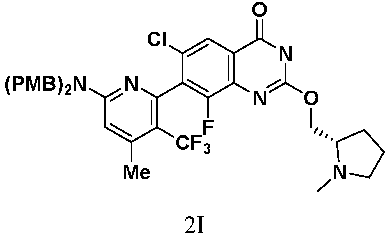

A mixture of *tert*-butyl 4-(7-(6-(bis(4-methoxybenzyl)amino)-4-methyl-3-(trifluoromethyl)pyridin-2-yl)-6-chloro-2,8-difluoroquinazolin-4-yl)piperazine-1-carboxylate (250 mg, 0.28 mmol) and NaOH (224 mg, 5.59 mmol) in ethanol (30 mL) and water (10 mL) was stirred at 45 °C for 3 d. The mixture was quenched with 2N HCl to pH= 6~7. The mixture was concentrated in vacuo to remove the EtOH. The residue was extracted with DCM (20 mLx3). The combined organic layers were washed with brine (30 mL) and dried over anhydrous Na₂SO₄. The mixture was filtered and the filtrate was concentrated under reduced pressure to afford 7-(6-(bis(4-methoxybenzyl)amino)-4-methyl-3-(trifluoromethyl)pyridin-2-yl)-6-chloro-8-fluoro-2-(((S)-1-methylpyrrolidin-2-yl)methoxy)quinazolin-4(3*H*)-one (200 mg, 0.28 mmol, 98.5% yield) as a light yellow solid. MS (ESI) *m*/*z* 726.3 [M+1]⁺; ¹H NMR (400 MHz, CDCl₃) δ 8.07 (s, 1H), 7.14 (d, *J* = 8.4 Hz, 4H), 6.85 (d, *J = 8.4* Hz, 4H), 6.41 (s, 1H), 4.98 - 4.65 (m, 4H), 4.59 - 4.49 (m, 2H), 3.80 (s, 6H), 3.55 - 3.38 (m, 1H), 2.90 (d, *J =* 8.0 Hz, 3H), 2.41 (s, 3H), 2.31 - 2.21 (m, 2H), 2.14 - 2.03 (m, 2H), 1.37 - 1.19 (m, 2H).

### Preparation of Intermediate 2J: tert-butyl 3-(7-(6-(bis(4-methoxybenzyl)amino)-4-methyl-3-(trifluoromethyl)pyridin-2-yl)-6-chloro-8-fluoro-2-(((S)-1-methylpyrrolidin-2-yl)methoxy)quinazolin-4-yl)-3,9-diazabicyclo[4.2. 1]nonane-9-carboxylate

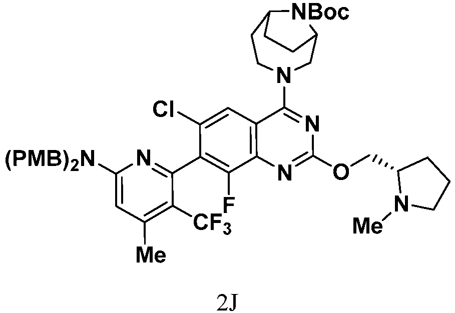

To a solution of 7-(6-(bis(4-methoxybenzyl)amino)-4-methyl-3-(trifluoromethyl)pyridin-2-yl)-6-chloro-8-fluoro-2-(((S)-1-methylpyrrolidin-2-yl)methoxy)quinazolin-4(3*H*)-one (50 mg, 0.0700 mmol) and *tert-butyl* 3,9-diazabicyclo[4.2.1]nonane-9-carboxylate (75 mg, 0.3300 mmol) in DCM (1 mL) were added DIEA (0.07 mL, 0.7800 mmol) and BOP (105 mg, 0.4100 mmol). The mixture was stirred at 25 °C for 12 hours. The reaction mixture was diluted with water (20 mL), then extracted with dichloromethane (15 mL x 3). The combined organic layers were washed with brine (30 mL), dried over anhydrous sodium sulfate, filtered and concentrated under reduced pressure. The residue was purified by preparative HPLC (TFA as additive, Instrument: ACSWH-GX-N; Column: Phenomenex Synergi C18 150 x 25mm x10um; Mobile phase: A for H₂O (0.1%TFA) and B for Acetonitrile; Gradient: B 56%-86% in 10min linearly; Flow rate: 25mL/min; Column temperature: R.T.; Wavelength: 220nm.254nm) to afford the desired product *tert*-butyl 3-(7-(6-(bis(4-methoxybenzyl)amino)-4-methyl-3-(trifluoromethyl)pyridin-2-yl)-6-chloro-8-fluoro-2-(((S)-1-methylpyrrolidin-2-yl)methoxy)quinazolin-4-yl)-3,9-diazabicyclo[4.2.1]nonane-9-carboxylate (40 mg, 0.043 mmol) as a yellow solid. MS (ESI) *m*/*z:* 934.7 [M+H]⁺.

### Example 2-1

### 6-(6-chloro-4-{3,9-diazabicyclo[4.2.1]nonan-3-yl}-8-fluoro-2-{[(2S)-1-methylpyrrolidin-2-yl]methoxy}quinazolin-7-yl)-4-methyl-5-(trifluoromethyl)pyridin-2-amine

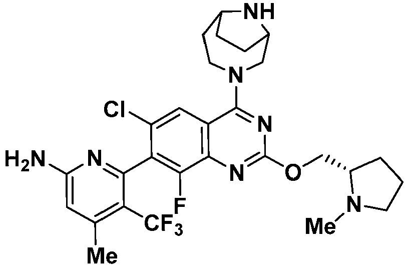

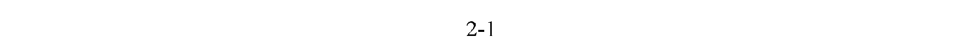

A solution of *tert*-butyl 3-(7-(6-(bis(4-methoxybenzyl)amino)-4-methyl-3-(trifluoromethyl)pyridin-2-yl)-6-chloro-8-fluoro-2-(((*S*)-1-methylpyrrolidin-2-yl)methoxy)quinazolin-4-yl)-3,6-diazabicyclo[3.2.2]nonane-6-carboxylate (45 mg, 0.05 mmol) in TFA (4. mL, 52.24 mmol) was stirred at 50 °C for 4 hours. The reaction mixture was concentrated under reduced pressure. The residue was purified by preparative HPLC (formic acid as additive, Instrument: GX-c; Column: Phenomenex luna C18 150x25mm, 10um; Mobile phase: A for H₂O (0.225%FA) and B for Acetonitrile; Gradient: B 3%-33% in 10min linearly; Flow rate: 25mL/m; Column temperature: R.T.; Wavelength: 220nm.254nm) to afford the desired product 6-(4-(3,6-diazabicyclo[3.2.2]nonan-3-yl)-6-chloro-8-fluoro-2-(((*S*)-1-methylpyrrolidin-2-yl)methoxy)quinazolin-7-yl)-4-methyl-5-(trifluoromethyl)pyridin-2-amine (17.98 mg, 0.028 mmol) as a yellow solid. MS (ESI) *m*/*z:* 594.2 [M+H]⁺. ¹H NMR (400 MHz, CD₃OD) δ 8.51 (s, 2H), 7.93 (s, 1H), 6.62 (s, 1H), 4.76 - 4.69 (m, 1H), 4.66 - 4.58 (m, 1H), 4.54 - 4.42 (m, 1H), 4.13 - 3.98 (m, 2H), 3.97 - 3.88 (m, 1H), 3.65 - 3.50 (m, 4H), 3.49 - 3.43 (m, 1H), 3.05 - 2.97 (m, 1H), 2.92 (s, 3H), 2.55 - 2.48 (m, 1H), 2.45 (s, 3H), 2.36 - 2.27 (m, 1H), 2.13 - 2.05 (m, 3H), 2.04 - 1.97 (m, 2H), 1.94 - 1.81 (m, 2H).

Examples in Table 2 were prepared according to procedures described for Example 2-1 from appropriate starting materials.

**Table 2**

| Example Number | Structure | Name | **MS** m/z: [M+H]+ | ¹H NMR |
|---|---|---|---|---|
| 2-2 | 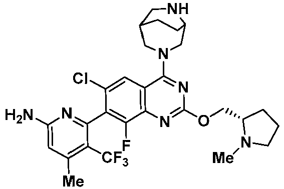 | 6-(6-chloro-4-{3,6-diazabicyclo[3.2.2]nonan-3-yl}-8-fluoro-2-{[(2S)-1-methylpyrrolidin-2-yl]methoxy}quinazolin-7-yl)-4-methyl-5-(trifluoromethyl)pyridin-2-amine | 594.2 | (400 MHz, CD₃OD) δ 8.51 (s, 2H), 7.93 (s, 1H), 6.62 (s, 1H), 4.76 - 4.69 (m, 1H), 4.66 - 4.58 (m, 1H), 4.54 - 4.42 (m, 1H), 4.13 - 3.98 (m, 2H), 3.97 - 3.88 (m, 1H), 3.65 - 3.50 (m, 4H), 3.49 - 3.43 (m, 1H), 3.05 - 2.97 (m, 1H), 2.92 (s, 3H), 2.55 - 2.48 (m, |
| | | | | 1H), 2.45 (s, 3H), 2.36 - 2.27 (m, 1H), 2.13 - 2.05 (m, 3H), 2.04 - 1.97 (m, 2H), 1.94 - 1.81 (m, 2H) |

### Example 3

### 6-(2-{[(4aS, 7aR)-1-methyl-octahydro-1H-cyclopenta[b]pyridin-4a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amine

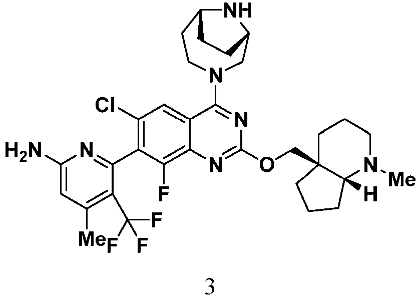

### Preparation of Intermediate 3A: ethyl octahydro-4aH-cyclopenta[b]pyridine-4a-carboxylate

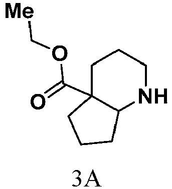

Intermediate 3A was prepared according to the procedure in Molecules 2017, 22, 827, compound 25a.

### Preparation of Intermediate 3B: 1-benzyl 4a-ethyl (4aS,7aR)-hexahydro-1H-cyclopenta[b]pyridine-1,4a(2H)-dicarboxylate

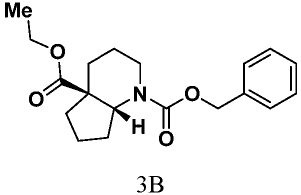

To a solution of ethyl octahydro-4aH-cyclopenta[b]pyridine-4a-carboxylate (1.7 g, 8.62 mmol) and TEA (2.40 mL, 17.23 mmol) in THF (10 mL) was added N-(benzyloxycarbonyl)succinimide (1.718 g, 6.89 mmol), the mixture was stirred at room temperature for 18 hours. The mixture was then diluted with EtOAc (15 mL) and was washed with a solution of aqueous saturated sodium bicarbonate (2 x 15 mL). The ethyl acetate layer was dried over sodium sulfate, filtered and concentrated. The crude product was subjected to ISCO flash chromatography (silica gel/DCM-20% MeOH/DCM 100:0 to 50:50 gradient) to afford 1-benzyl 4a-ethyl hexahydro-1H-cyclopenta[b]pyridine-1,4a(2H)-dicarboxylate (2.20 g, 6.64 mmol, 77 % yield). The 1-benzyl 4a-ethyl hexahydro-1H-cyclopenta[b]pyridine-1,4a(2H)-dicarboxylate (2.20 g) was subjected to SFC chiral separation [column: Cellulose-4 (5×25cm, 5µm) method = CO₂/IPA:heptane (1:3) with 0.1% ammonia hydroxide. 320 mL/min] to afford 1-benzyl 4a-ethyl (4aS,7aR)-hexahydro-1H-cyclopenta[b]pyridine-1,4a(2H)-dicarboxylate (640 mg, 1.835 mmol, 21.29 % yield). LCMS (ESI) m/z: 332.3 [M+H]+ LC retention time: 1.05 min (Waters Acquity UPLC BEH C18, 2.1 x 50 mm, 1.7 □m particles; Mobile Phase A: water with 0.05% TFA; Mobile Phase B: ACN with 0.05% TFA; Gradient: 2-98% B over 1 minute, then a 0.5 minute hold at 98% B; Flow: 0.8 mL/min; Detection: MS and UV (220 nm)). ¹H NMR (499 MHz, CHLOROFORM-d) δ 7.41 - 7.28 (m, 5H), 5.17 (br s, 2H), 4.13 (br d, J=6.4 Hz, 2H), 2.86 (br s, 1H), 2.15 (br d, J=10.8 Hz, 1H), 2.03 - 1.90 (m, 1H), 1.90 - 1.74 (m, 4H), 1.73 - 1.63 (m, 1H), 1.58 - 1.42 (m, 3H), 1.27 - 1.14 (m, 4H), 0.98 - 0.68 (m, 1H)

### Preparation of Intermediate 8C: ethyl (4aS, 7aR)-octahydro-4aH-cyclopenta[b]pyridine-4a-carboxylate

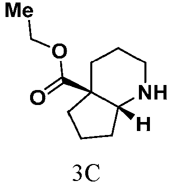

A mixture of 1-benzyl 4a-ethyl (4aS,7aR)-hexahydro-1H-cyclopenta[b]pyridine-1,4a(2H)-dicarboxylate (640 mg, 1.931 mmol) and 10 % Pd-C (103 mg, 0.097 mmol) in MeOH (10 mL) was hydrogenated under 1 atm of hydrogen for 18 hours. The Pd/C was filtered off and the filtrate was concentrated to provide crude ethyl (4aS,7aR)-octahydro-4aH-cyclopenta[b]pyridine-4a-carboxylate (385 mg, 1.854 mmol, 96 % yield) as clear oil. ¹H NMR (499 MHz, CDCl₃) δ 4.17 (dtt, *J*=10.6, 7.1, 3.6 Hz, 2H), 3.57 (t, *J*=6.1 Hz, 1H), 2.90 (ddd, *J*=13.0, 7.7, 3.7 Hz, 1H), 2.71 (ddd, *J*=13.0, 7.0, 3.6 Hz, 1H), 2.01 - 1.92 (m, 2H), 1.84 - 1.62 (m, 7H), 1.60 - 1.40 (m, 2H), 1.28 (t, *J*=7.1 Hz, 3H).

### Preparation of Intermediate 3D: ethyl (4aS,7aR)-1-methyloctahydro-4aH-cyclopenta[b]pyridine-4a-carboxylate

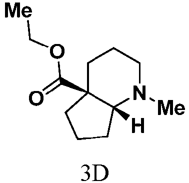

To a solution of ethyl (4aS,7aR)-octahydro-4aH-cyclopenta[b]pyridine-4a-carboxylate (385 mg, 1.952 mmol) and formaldehyde solution, 37 wt. % in H₂O (176 mg, 5.85 mmol) in MeOH (5.0 mL) was added sodium cyanoborohydride (123 mg, 1.952 mmol) and the mixture was stirred at room temperature for 18 hours. The mixture was then concentrated. The residue was diluted with EtOAc (5 mL) and was washed with a solution of aqueous saturated sodium carbonate (2 x 5 mL). The ethyl acetate layer was dried over sodium sulfate, filtered and concentrated to afford crude ethyl (4aS,7aR)-1-methyloctahydro-4aH-cyclopenta[b]pyridine-4a-carboxylate (380 mg, 1.798 mmol, 92 % yield). ¹H NMR (499 MHz, CDCl₃) δ 4.24 - 4.12 (m, 2H), 3.29 (t, *J*=6.4 Hz, 1H), 2.60 - 2.51 (m, 1H), 2.36 - 2.28 (m, 3H), 2.00 - 1.88 (m, 2H), 1.83 - 1.60 (m, 8H), 1.56 - 1.40 (m, 1H), 1.32 - 1.26 (m, 3H).

### Preparation of Intermediate 3E: ((4aS, 7aR)-1-methyloctahydro-4aH-cyclopenta[b]pyridin-4a-yl)methanol

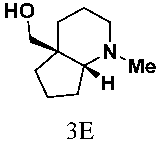

To a solution of ethyl (4aS,7aR)-1-methyloctahydro-4aH-cyclopenta[b]pyridine-4a-carboxylate (380 mg, 1.798 mmol) in anhydrous THF (2.0 mL) was added a solution of lithium aluminum hydride (1.0 M in THF, 4496 µL, 4.50 mmol) and the mixture was stirred at room temperature for 18 hours. Brine (0.3 mL) was added dropwise to the mixture. EtOAc (5.0 mL) was then added to the mixture. The reaction mixture was filtered and the filtrate was concentrated to afford crude ((4aS,7aR)-1-methyloctahydro-4aH-cyclopenta[b]pyridin-4a-yl)methanol (327 mg, 1.739 mmol, 97 % yield). ¹H NMR (499 MHz, CHLOROFORM-d) δ 3.69 - 3.62 (m, 2H), 2.87 (t, *J*=7.6 Hz, 1H), 2.51 (td, *J*=11.1, 3.4 Hz, 1H), 2.43 - 2.35 (m, 1H), 2.30 (s, 3H), 2.01 - 1.85 (m, 2H), 1.82 - 1.74 (m, 1H), 1.68 - 1.52 (m, 6H), 1.47 - 1.42 (m, 1H), 1.39 - 1.33 (m, 1H).

### Preparation of Intermediate 3F: tert-butyl 3-(7-bromo-2,6-dichloro-8-fluoroquinazolin-4-yl)-3,8-diazabicyclo[3.2.1 joctane-8-carboxylate

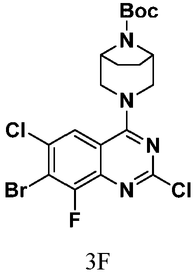

To a solution of 7-bromo-2,4,6-trichloro-8-fluoroquinazoline (300 mg, 3.03 mmol) in dioxane (8 mL) was added DIPEA (0.476 mL, 2.72 mmol) and tert-butyl 3,8-diazabicyclo[3.2.1]octane-8-carboxylate (193 mg, 0.908 mmol). The resulting mixture was stirred at 25 °C for 2 hours. The mixture was then concentrated. The residue was diluted with ethyl acetate (50 mL) and washed with water (30 mLx2) and brine (50 mL). The organic phase was dried over anhydrous sodium sulfate, filtered and concentrated under reduced pressure. The residue was purified by silica gel column chromatography (12 g ISCO column, MeOH/DCM, 0-5%, 20 min.) to afford tert-butyl 3-(7-bromo-2,6-dichloro-8-fluoroquinazolin-4-yl)-3,8-diazabicyclo[3.2.1]octane-8-carboxylate (415 mg, 0.82 mmol, 90% yield) as a white solid. MS (ESI) *m*/*z* 507.0 [M+1]⁺. ¹H NMR (499 MHz, DMSO-d₆) δ 8.10 (d, J=1.9 Hz, 1H), 4.38 (br d, J=10.6 Hz, 2H), 4.25 (br s, 2H), 3.66 (m, 2H) 1.79 (m, 2H), 1.62 (m, 2H), 1.47 s, 9H).

### Preparation of Intermediate 3G: tert-butyl 3-(7-bromo-6-chloro-2, 8-difluoroquinazolin-4-yl)-3, 8-diazabicyclo[3. 2.1 Joctane-8-carboxylate

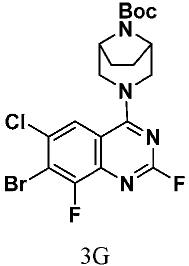

To a degassed solution of *tert*-butyl 3-(7-bromo-2,6-dichloro-8-fluoroquinazolin-4-yl)-3,8-diazabicyclo[3.2.1]octane-8-carboxylate (1 g, 3.03 mmol) in DMA (80 mL) was added cesium fluoride (5.25 g, 34.6 mmol). The mixture was degassed with nitrogen for 10 min and was heated at 88 °C for 5 h in a sealed tube. Water (200 mL) and ethyl acetate (150 mL) were added and this mixture was stirred for 15 min. The separated aqueous layer was extracted with ethyl acetate (2 X 100 mL) and the combined organic layers were dried over anhydrous Na₂SO₄, filtered, and concentrated. The resulting residue was purified by flash column chromatography using 15-25% ethyl acetate in petroleum ether as an eluent to provide tert-butyl-3-(7-bromo-6-chloro-2,8-difluoroquinazolin-4-yl)-3,8-diazabicyclo[3.2.1]octane-8-carboxylate (4.7 g, 8.77 mmol, 63.4 % yield) as a pale yellow solid. MS (ESI) *m*/*z* 489.0 [M+1]⁺.

### Preparation of Intermediate 3H: tert-butyl 3-(7-(6-(bis(4-methoxybenzyl)amino)-4-methylpyridin-2-yl)-6-chloro-2,8-difluoroquinazolin-4-yl)-3,8-diazabicyclo[3.2.1]octane-8-carboxylate

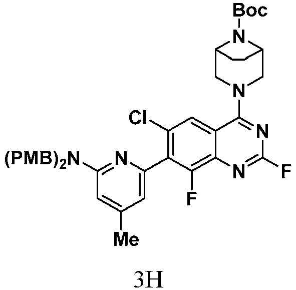

To a degassed solution of Example 3G (2.00 g, 4.08 mmol) in anhydrous 1,4-dioxane (20 mL) were added potassium phosphate (1.73 g, 8.17 mmol), N,N-bis(4-methoxybenzyl)-4-methyl-6-(4,4,5,5-tetramethyl-1,3,2-dioxaborolan-2-yl)pyridin-2-amine (5.8 g, 12.25 mmol) and PdCl₂(dppf) (149 mg, 0.204 mmol). The mixture was degassed again and heated at 80 °C for 48 h. The reaction vessel was then allowed to cool to ambient temperature, diluted with ethyl acetate (40 mL), filtered through a bed of Celite^{®} and concentrated in vacuo to afford the crude product. The residue was purified by silica gel column chromatography using 30% ethyl acetate in petroleum ether to obtain tert-butyl 3-(7-(6-(bis(4-methoxybenzyl)amino)-4-methylpyridin-2-yl)-6-chloro-2,8-difluoroquinazolin-4-yl)-3,8-diazabicyclo[3.2.1]octane-8-carboxylate (1.5 g, 1.74 mmol, 42% yield). ¹H NMR (400 MHz, CDCl₃): *δ* 7.76 (d, *J* = 1.6 Hz, 1H), 7.20-7.18 (d, J = 8.8 Hz, 4H), 6.86 (dt, *J* = 9.6 Hz, 4H), 6.60 (s, 1H), 6.38 (s, 1H), 4.60 (s, 3H), 4.39-4.21 (m, 4H), 3.63 (s, 6H), 2.29 (s, 3H), 1.98-1.96 (m, 6H), 1.76-1.63 (m, 2H), 1.49 (s, 9H) ppm. LCMS (ESI) *m*/*z:* 757.2 [M+H]⁺.

### Preparation of Intermediate 3I: tert-butyl 3-(7-(6-(bis(4-methoxybenzyl)amino)-3-iodo-4-methylpyridin-2-yl)-6-chloro-2,8-difluoroquinazolin-4-yl)-3.8 diazabicyclo[3.2.1]octane-8-carboxylate

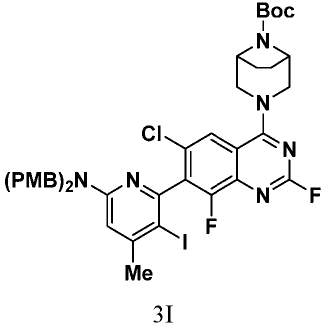

To a stirred solution of tert-butyl 3-(7-(6-(bis(4-methoxybenzyl)amino)-4-methylpyridin-2-yl)-6-chloro-2,8-difluoroquinazolin-4-yl)-3,8-diazabicyclo[3.2.1]octane-8-carboxylate (1.40 g, 1.849 mmol) in anhydrous acetonitrile (15 mL) under nitrogen at 0 °C were added N-iodosuccinimide (0.42 g, 1.849 mmol) and trifluoroacetic acid (0.028 mL, 0.370 mmol). The reaction mixture was allowed to reach room temperature over one hour. The reaction mixture was then quenched with saturated aq. sodium thiosulfate (5 mL) and saturated aq. sodium bicarbonate (4 mL). The mixture was extracted with ethyl acetate (3x20 mL). The combined organic layers were dried over anhydrous sodium sulphate, filtered and concentrated under reduced pressure to obtain a crude residue. The crude residue was purified by silica gel column chromatography using 30% ethyl acetate in petroleum ether to obtain tert-butyl 3-(7-(6-(bis(4-methoxybenzyl)amino)-3-iodo-4-methylpyridin-2-yl)-6-chloro-2,8-difluoroquinazolin-4-yl)-3,8 diazabicyclo[3.2.1]octane-8-carboxylate (1.42 g, 1.560 mmol, 84% yield) as pale yellow solid. LCMS (ESI) *m*/*z:* 883.3 [M+H]⁺.

### Preparation of Intermediate 3J: tert-butyl 3-(7-(6-(bis(4-methoxybenzyl)amino)-4-methyl-3-(trifluoromethyl)pyridin-2-yl)-6-chloro-2,8-difluoroquinazolin-4-yl)-3,8-diazabicyclo[3.2.1]octane-8-carboxylate

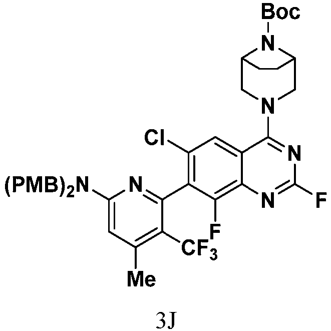

To a stirred solution of tert-butyl 3-(7-(6-(bis(4-methoxybenzyl)amino)-3-iodo-4-methylpyridin-2-yl)-6-chloro-2,8-difluoroquinazolin-4-yl)-3,8-diazabicyclo[3.2.1]octane-8-carboxylate (1.40 g, 1.585 mmol) in anhydrous DMA (10 mL) in a sealed tube under a nitrogen atmosphere was added copper(I) iodide (0.60 g, 3.17 mmol). The reaction mixture was degassed for 10 minutes before the addition of methyl 2,2-difluoro-2-(fluorosulfonyl)acetate (0.91 g, 4.76 mmol) and reaction mixture was heated to 90 °C for 12 h. The reaction mixture was diluted with diethyl ether (20 mL) and water (10 mL). The layers were separated and the aqueous layer was extracted with diethyl ether (3x20 mL). The combined organic layers were dried over anhydrous sodium sulfate, filtered and concentrated under reduced pressure to obtain the crude residue. The crude material was purified by silica gel column chromatography using 30% ethyl acetate in petroleum ether to obtain tert-butyl 3-(7-(6-(bis(4-methoxybenzyl)amino)-4-methyl-3-(trifluoromethyl)pyridin-2-yl)-6-chloro-2,8-difluoroquinazolin-4-yl)-3,8-diazabicyclo[3.2.1]octane-8-carboxylate (0.85 g, 0.630 mmol, 40% yield) as a pale yellow solid. ¹H NMR (400MHz, CDCl₃): *δ* 7.77 (s, 1H), 7.16 (d, *J=* 8.8 Hz, 4H), 6.87 (dt, *J=* 9.6 and 2.8 Hz, 4H), 6.43 (s, 1H), 4.76-4.72 (m, 2H), 4.59-4.55 (m, 2H), 3.81 (s, 6H), 2.43 (s, 3H), 1.97-1.82 (m, 4H), 1.97-1.82 (m, 4H), 1.53 (s, 9H) ppm. LCMS (ESI) *m*/*z:* 825.2 [M+H]⁺.

### Intermediates 3K and 3L: tert-butyl 3-(7-(6-(bis(4-methoxybenzyl)amino)-4-methyl-3-(trifluoromethyl)pyridin-2-yl)-6-chloro-2,8-difluoroquinazolin-4-yl)-3,8-diazabicyclo[3. 2.1 joctane-8-carboxylate

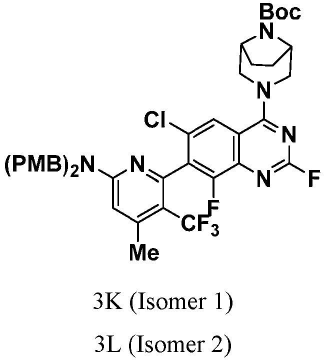

Example 3J (5.0 g, 6.06 mmol) separated into single atropisomers by SFC (Column: Chiralpak IH (250mm x 4.6 x 5u), mobile phase- 0.25% Isopropanol), where Peak-1 eluted at retention time = 5.85 minutes (2.4 g, 2.90 mmol, 40% yield) and Peak-2 at retention time = 9.53 minutes (2.4 g, 2.90 mmol, 40% yield).

Peak-1 (3K): ¹H NMR (400MHz, CDCl₃): *δ* 7.78 (s, 1H), 7.16 (d, *J* = 8.8 Hz, 4H), 6.87 (dt, *J=* 9.6 and 2.8 Hz, 4H), 6.43 (s, 1H), 4.76-4.72 (m, 2H), 4.59-4.55 (m, 2H), 3.81 (s, 6H), 2.43 (s, 3H), 1.97-1.82 (m, 4H), 1.97-1.82 (m, 4H), 1.53 (s, 9H) ppm. LCMS (ESI) *m*/*z:* 825.2 [M+H]⁺. LCMS (ESI) m/z: 825.2 [M+H]⁺. [α]^{23.5} (MeOH = 0.10) = +96.00

Peak-2: (3L ) ¹H NMR (400MHz, CDCl₃): *δ* 7.78 (s, 1H), 7.16 (d, *J* = 8.8 Hz, 4H), 6.87 (dt, *J=* 9.6 and 2.8 Hz, 4H), 6.43 (s, 1H), 4.76-4.72 (m, 2H), 4.59-4.55 (m, 2H), 3.81 (s, 6H), 2.43 (s, 3H), 1.97-1.82 (m, 4H), 1.97-1.82 (m, 4H), 1.53 (s, 9H) ppm. LCMS (ESI) m/z: 825.2 [M+H]⁺. [α]^{23 3} (MeOH = 0.10) = -110.00.

### Preparation of Intermediate 3M: tert-butyl 3-(7-(6-(bis(4-methoxybenzyl)amino)-4-methyl-3-(trifluoromethyl)pyridin-2-yl)-6-chloro-8-fluoro-2-(((4aS,7aR)-1-methyloctahydro-4aH-cyclopenta[b]pyridin-4a-yl)methoxy)quinazolin-4-yl)-3,8-diazabicyclo[3.2.1]octane-8-carboxylate

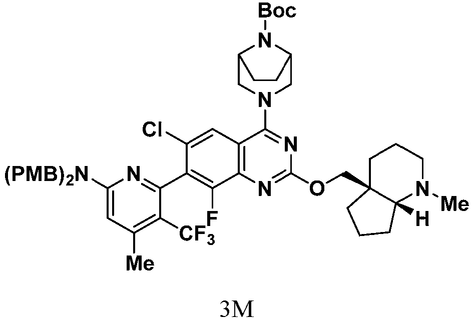

To a solution of Intermediate 3L and ((4aS,7aR)-1-methyloctahydro-4aH-cyclopenta[b]pyridin-4a-yl)methanol (41.0 mg, 0.242 mmol) in anhydrous THF (1.0 mL) at room temperature under nitrogen was added a solution of 1.0 M LiHMDS in THF (364 µl, 0.364 mmol) and the mixture was stirred at room temperature for 18 hours. The mixture was diluted with DMF (1 mL) and the crude product was purified by prep-HPLC (Phenomenex, Luna 5 micron 30 x 250 mm, flow rate = 30 mL/min., gradient = 20% A to 100%B in 30 min., A =H₂O/ ACN/TFA(90:10:0.1), B = H₂O/ ACN/TFA(10:90:0.1) to afford tert-butyl 3-(7-(6-(bis(4-methoxybenzyl)amino)-4-methyl-3-(trifluoromethyl)pyridin-2-yl)-6-chloro-8-fluoro-2-(((4aS,7aR)-1-methyloctahydro-4aH-cyclopenta[b]pyridin-4a-yl)methoxy)quinazolin-4-yl)-3,8-diazabicyclo[3.2.1]octane-8-carboxylate (267 mg, 0.233 mmol, 96 % yield). LCMS (ESI) m/z: 974.5 [M+H]⁺LC retention time: 1.13 min (Waters Acquity UPLC BEH C18, 2.1 x 50 mm, 1.7 □m particles; Mobile Phase A: water with 0.05% TFA; Mobile Phase B: ACN with 0.05% TFA; Gradient: 2-98% B over 1 minute, then a 0.5 minute hold at 98% B; Flow: 0.8 mL/min; Detection: MS and UV (220 nm)).

### Preparation of Intermediate 3N: 7-(6-(bis(4-methoxybenzyl)amino)-4-methyl-3-(trifluoromethyl)pyridin-2-yl)-6-chloro-8-fluoro-2-(((4aS,7aR)-1-methyloctahydro-4aH-cyclopenta[b]pyridin-4a-yl)methoxy)quinazolin-4-ol

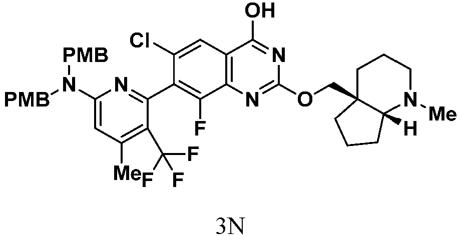

A mixture of 1.0 M sodium hydroxide (2453 µl, 2.453 mmol) and tert-butyl 3-(7-(6-(bis(4-methoxybenzyl)amino)-4-methyl-3-(trifluoromethyl)pyridin-2-yl)-6-chloro-8-fluoro-2-(((4aS,7aR)-1-methyloctahydro-4aH-cyclopenta[b]pyridin-4a-yl)methoxy)quinazolin-4-yl)-3,8-diazabicyclo[3.2.1]octane-8-carboxylate (267 mg, 0.245 mmol) in EtOH (20 mL) was stirred at 65 °C for 3 days. The mixture was then concentrated to remove the EtOH. The mixture was extracted with EtOAc (2 x 15 mL) and the ethyl acetate layers were dried over sodium sulfate filtered and concentrated. The crude product was purified by prep-HPLC (Phenomenex, Luna 5 micron 30 x 250 mm, flow rate = 30 mL/min., gradient = 20% A to 100% B in 30 min., A =H₂O/ ACN/TFA(90:10:0.1), B = H₂O/ ACN/TFA(10:90:0.1)). The pure fractions were combined. and concentrated. The pure product was then diluted with EtOAc (15 mL) and was washed with a solution of aqueous saturated sodium carbonate (2 x 15 mL). The ethyl acetate layer was dried over sodium sulfate, filtered and concentrated to afford 7-(6-(bis(4-methoxybenzyl)amino)-4-methyl-3-(trifluoromethyl)pyridin-2-yl)-6-chloro-8-fluoro-2-(((4aS,7aR)-1-methyloctahydro-4aH-cyclopenta[b]pyridin-4a-yl)methoxy)quinazolin-4-ol (60 mg, 0.077 mmol, 31.4 % yield) as a tan solid. LCMS (ESI) m/z: 780.3 [M+H]⁺LC retention time: 1.04 min ((Waters Acquity UPLC BEH C18, 2.1 x 50 mm, 1.7 □m particles; Mobile Phase A: water with 0.05% TFA; Mobile Phase B: ACN with 0.05% TFA; Gradient: 2-98% B over 1 minute, then a 0.5 minute hold at 98% B; Flow: 0.8 mL/min; Detection: MS and UV (220 nm)).

### Preparation of Example 3: 6-(2-{[(4aS, 7aR)-1-methyl-octahydro-1H-cyclopenta[b]pyridin-4a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amine

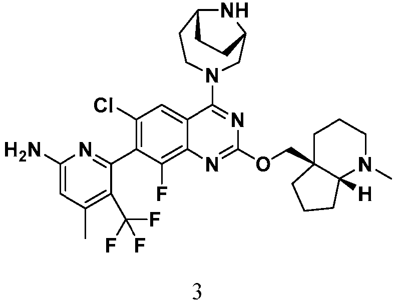

To a solution of 7-(6-(bis(4-methoxybenzyl)amino)-4-methyl-3-(trifluoromethyl)pyridin-2-yl)-6-chloro-8-fluoro-2-(((4aS,7aR)-1-methyloctahydro-4aH-cyclopenta[b]pyridin-4a-yl)methoxy)quinazolin-4-ol (25 mg, 0.032 mmol), tert-butyl (1S,6R)-3,9-diazabicyclo[4.2.1]nonane-9-carboxylate (10.88 mg, 0.048 mmol) and BOP (21.26 mg, 0.048 mmol) in DCM (1.0 mL) was added DIEA (16.79 µl, 0.096 mmol) and the mixture was stirred at room temperature for 18 hours. The mixture was then concentrated. The crude product was purified by prep-HPLC (Phenomenex, Luna 5 micron 30 x 250 mm, flow rate = 30 mL/min., gradient = 20% A to 100% B in 30 min., A =H₂O/ ACN/TFA(90:10:0.1), B = H₂O/ ACN/TFA(10:90:0.1)) to provide tert-butyl (1S,6R)-3-(7-(6-amino-4-methyl-3-(trifluoromethyl)pyridin-2-yl)-6-chloro-8-fluoro-2-(((4aS,7aR)-1-methyloctahydro-4aH-cyclopenta[b]pyridin-4a-yl)methoxy)quinazolin-4-yl)-3,9-diazabicyclo[4.2.1]nonane-9-carboxylate. A mixture of tert-butyl (1S,6R)-3-(7-(6-amino-4-methyl-3-(trifluoromethyl)pyridin-2-yl)-6-chloro-8-fluoro-2-(((4aS,7aR)-1-methyloctahydro-4aH-cyclopenta[b]pyridin-4a-yl)methoxy)quinazolin-4-yl)-3,9-diazabicyclo[4.2.1]nonane-9-carboxylate in water (1 drop), triethylsilane (1 drop) and TFA (1.5 mL) was stirred at 40 °C for 18 hours. The mixture was then concentrated. The crude product was purified by prep-HPLC (Phenomenex, Luna 5 micron 30 x 250 mm, flow rate = 30 mL/min., gradient = 20% A to 100%B in 30 min., A =H₂O/ ACN/TFA(90:10:0.1), B = H₂O/ ACN/TFA(10:90:0.1)). The pure fractions were loaded onto an Oasis MCX cation mixed-mode polymer cartridge (150 mg), and the cartridge was washed with methanol (30 mL) and the product was eluted with 0.1 N ammonia in methanol (5.0 mL). The ammonia eluent was concentrated. The resulting product was lyophilized with ACN/H₂O (1:1, 5 mL) to afford 4 6-(4-((1S,6R)-3,9-diazabicyclo[4.2.1]nonan-3-yl)-6-chloro-8-fluoro-2-(((4aS,7aR)-1-methyloctahydro-4aH-cyclopenta[b]pyridin-4a-yl)methoxy)quinazolin-7-yl)-4-methyl-5-(trifluoromethyl)pyridin-2-amine (10.96 mg, 0.016 mmol, 50.1 % yield) as a white powder. LCMS (ESI) m/z: 648.2 [M+H]+ LC retention time: 1.27 min ((Waters Acquity UPLC BEH C18, 2.1 x 50 mm, 1.7 □m particles; Mobile Phase A: water with 0.05% TFA; Mobile Phase B: ACN with 0.05% TFA; Gradient: 2-98% B over 1 minute, then a 0.5 minute hold at 98% B; Flow: 0.8 mL/min; Detection: MS and UV (220 nm)). ¹H NMR (499 MHz, METHANOL-d₄) δ 7.96 (d, *J=1.5* Hz, 1H), 6.62 (s, 1H), 4.55 - 4.47 (m, 2H), 4.27 (d, *J*=10.7 Hz, 1H), 4.05 - 3.91 (m, 2H), 3.84 - 3.77 (m, 2H), 3.68 (dd, *J*=13.5, 3.9 Hz, 1H), 2.87 (br s, 1H), 2.73 - 2.64 (m, 1H), 2.47 (d, *J*=1.2 Hz, 3H), 2.44 - 2.35 (m, 1H), 2.33 (s, 3H), 2.29 - 2.18 (m, 1H), 2.13 - 1.92 (m, 4H), 1.91 - 1.82 (m, 2H), 1.81 - 1.64 (m, 8H), 1.63 - 1.54 (m, 1H).

### Example 4-1

### 6-(2-{[(2R, 7aS)-2-fluoro-hexahydro-1H-pyrrolizin-7a-yl]methoxy)-6-chloro-4-[(]S, 6R)-3,9-diazabicyclo[4.2.1]nonan-3-yl]-8-fluoroquinazolin-7-yl)-4-methyl-5-(trifluoromethyl)pyridin-2-amine

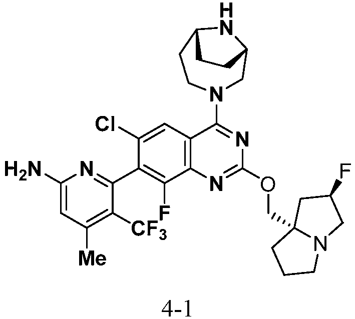

### Preparation of intermediate 4A: tert-butyl-3-(7-(6-(bis(4-methoxybenzyl)amino)-4-methyl-3-(trifluoromethyl)pyridin-2-yl)-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate

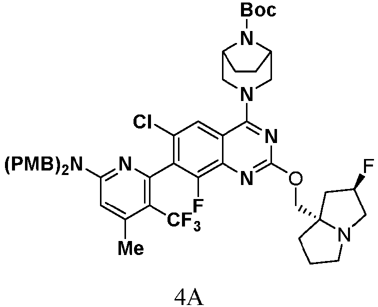

To a stirred solution of ((2*R*,7a*S*)-2-fluorotetrahydro-1H-pyrrolizin-7a(5H)-yl)methanol and HCl (81 mg, 0.509 mmol) in DCM was added sodium carbonate (231 mg, 2.181 mmol) and the reaction mixture was sonicated for 10 min and then stirred at room temperature for 30 min. The solvent was decanted and concentrated under reduced pressure to obtain ((2R,7aS)-2-fluorotetrahydro-1H-pyrrolizin-7a(5H)-yl) methanol as a free base. The obtained crude residue was dissolved in anhydrous THF (5 mL) and cooled to 0 °C, and then sodium hydride (60% in mineral oil, 12.21 mg, 0.509 mmol) was added. The reaction mixture was stirred at the same temperature for 30 min. before adding Intermediate 3L (280 mg, 0.339 mmol) in THF (1 mL) dropwise. The reaction mixture was stirred overnight at room temperature. The reaction mixture was then cooled using ice, quenched with saturated aq. ammonium chloride solution (10 mL) and extracted with ethyl acetate (2x20 mL). The combined organic layers were washed with water, and then brine solution, dried over anhydrous Na₂SO₄, filtered and concentrated under reduced pressure. The crude product obtained was purified by column chromatography (Grace REVELERIS^{®}, 50 g snap, dry pack) over neutral alumina using 50-100% ethyl acetate in petroleum ether. The desired fractions were pooled and concentrated under reduced pressure to obtain tert-butyl 3-(7-(6-(bis(4-methoxybenzyl)amino)-4-methyl-3-(trifluoromethyl)pyridin-2-yl)-6-chloro-8-fluoro-2-(((2*S*,7a*R*)-2-fluorotetrahydro-1H-pyrrolizin-7a(5H)-yl)methoxy)quinazolin-4-yl)-3,8-diazabicyclo[3.2.1]octane-8-carboxylate (240 mg, 0.241 mmol, 71% yield). LCMS (ESI) m/z: 965.8 [M+H]⁺.

### Preparation of intermediate 4B: 7-(5-(bis(4-methoxybenzyl)amino)-3-methyl-2-(trifluoromethyl)phenyl)-6-chloro-8-fluoro-2-(((2R,7aS)-2-fluorotetrahydro-1H-pyrrolizin-7a(5H)-yl)methoxy)quinazolin-4-ol.

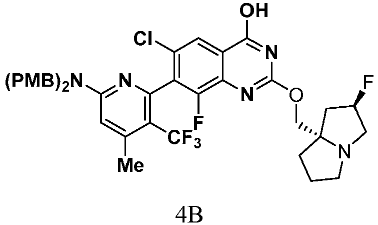

To a stirred solution of tert-butyl-3-(7-(5-(bis(4-methoxybenzyl)amino)-3-methyl-2-(trifluoromethyl)phenyl)-6-chloro-8-fluoro-2-(((2*R*,7a*S*)-2-fluorotetrahydro-1H-pyrrolizin-7a(5H)-yl)methoxy)quinazolin-4-yl)-3,8-diazabicyclo[3.2.1]octane-8-carboxylate (240 mg, 0.249 mmol) in ethanol (5 mL) was added NaOH (1M aq. solution, 199 mg, 0.498 mmol) and the mixture was heated at 70 °C for 48h. The reaction mixture was then concentrated under reduced pressure to obtain a crude residue which was purified by column chromatography (Biotage, Alumina-neutral) using 30-40% of ethyl acetate in petroleum ether to obtain 7-(5-(bis(4-methoxybenzyl)amino)-3-methyl-2-(trifluoromethyl)phenyl)-6-chloro-8-fluoro-2-(((2R,7aS)-2-fluorotetrahydro-1H-pyrrolizin-7a(5H)-yl)methoxy)quinazolin-4-ol (60 mg, 0.077 mmol, 31% yield) as an off white solid. LCMS (ESI) m/z: 771.2 [M+H]⁺.

### Preparation of intermediate 4C: tert-butyl (1S,6R)-3-(4-methoxybenzyl)-3,9-diazabicyclo[4. 2.1]nonane-9-carboxylate

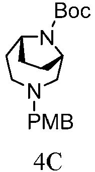

To a stirred solution of tert-butyl 3,9-diazabicyclo[4.2.1]nonane-9-carboxylate (4.5 g, 19.88 mmol) and 4-methoxybenzaldehyde (3.25 g, 23.86 mmol) in DCE (50 mL) was added DIPEA (17.36 mL, 99 mmol) and the reaction mixture was stirred for 15 min at 25 °C before cooling the reaction to 0 °C. After 15 min, sodium triacetoxyborohydride (12.64 g, 59.7 mmol) was added portion-wise to the reaction while maintaining the temperature below 5 °C. After the addition, the reaction mixture was gradually warmed to 25 °C and stirred overnight.. The reaction mixture was then quenched with saturated aq. ammonium chloride solution (10 mL) and extracted with dichloromethane (3x20 mL). The combined organic layers were washed with water and saturated brine solution, then dried over anhydrous Na₂SO₄, filtered and concentrated under reduced pressure to afford the crude product. The crude compound was purified by silica gel (60-120 mesh) chromatography eluting with 10-15% of ethylacetate/petroleum ether to obtain the *tert-*butyl-3-(4-methoxybenzyl)-3,9-diazabicyclo[4.2.1]nonane-9-carboxylate. The pure fractions were subjected to chiral SFC separation [column: Chiralpak ADH (250X4.6)mm 5µ method = 0.5% Isopropylamine in MeOH_10, run time = 5.00 min.] to obtain *tert*-butyl (1*R*,6*S*)-3-(4-methoxybenzyl)-3,9-diazabicyclo[4.2.1]nonane-9- carboxylate (1.9 g, 5.48 mmol, 28% yield) as light brown oil and *tert*-butyl (1*S*,6*R*)-3-(4-methoxybenzyl)-3,9- diazabicyclo[4.2.1]nonane-9-carboxylate (2 g, 5.77 mmol, 29% yield) as light brown oil. LCMS (ESI) m/z: 347.2 [M+H]⁺. ¹H NMR (400 MHz, DMSO-d₆, 298 K) δ 7.31 - 7.19 (m, 2H), 6.88 (d, *J* = 8.6 Hz, 2H), 5.78 - 5.71 (m, 1H), 4.15 - 3.89 (m, 2H), 3.73 (s, 3H), 3.49 (s, 2H), 2.78 - 2.60 (m, 1H), 2.48 - 2.42 (m, 1H), 2.40 (d, *J =* 2.8 Hz, 1H), 2.20 - 1.99 (m, 2H), 1.96 - 1.55 (m, 4H), 1.40 (d, *J =* 1.6 Hz, 9H) ppm.

### Preparation of intermediate 4D: tert-Butyl (1S,6R)-3,9-diazabicyclo[4.2.1]nonane-9-carboxylate

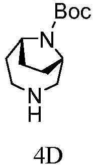

To a stirred solution of *tert-butyl* (1*S*,6*R*)-3-(4-methoxybenzyl)-3,9-diazabicyclo[4.2.1]nonane-9-carboxylate (1500 mg, 4.33 mmol) in EtOH (20 mL) was added Pd/C (921 mg, 8.66 mmol) at 25 °C. The resulting mixture was stirred for 3 h at 25 °C under a hydrogen atmosphere (1 atm).. The reaction mixture was then filtered through a Celite pad and washed with ethanol, and the filtrate was concentrated under reduced pressure to obtain a crude compound. The crude residue was purified by column chromatography (Biotage, Alumina-neutral) using MeOH in dichloromethane (10%) to obtain tert-butyl (1*S*,6*R*)-3,9-diazabicyclo[4.2.1]nonane-9-carboxylate (700 mg and 71% ). LCMS (ESI) m/z: 227.1 [M+H]⁺.

### Preparation of intermediate 4E: tert-butyl (1S,6R)-3-(7-(5-(bis(4-methoxybenzyl)amino)-3-methyl-2-(trifluoromethyl)phenyl)-6-chloro-8-fluoro-2-(((2R, 7aS)-2-fluorotetrahydro-1H-pyrrolizin-7a(5H)-yl)methoxy)quinazolin-5-yl)-3,9-diazabicyclo[4.2.1]nonane-9-carboxylate.

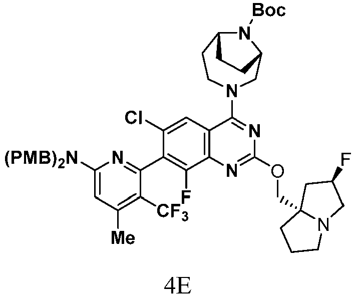

To a stirred solution of 7-(5-(bis(4-methoxybenzyl)amino)-3-methyl-2-(trifluoromethyl)phenyl)-6-chloro-8-fluoro-2-(((2*R*,7a*S*)-2-fluorotetrahydro-1H-pyrrolizin-7a(5H)-yl)methoxy)quinazolin-4-ol (55 mg, 0.072 mmol) in acetonitrile (2 mL) were added BOP (47.4 mg, 0.107 mmol) and TEA (0.015 mL, 0.107 mmol). After stirring for 5 min, tert-butyl (1*S*,6*R*)-3,9-diazabicyclo[4.2.1]nonane-9-carboxylate (20.23 mg, 0.089 mmol) was added and the mixture was stirred at 40 °C for 16h. The reaction mixture was quenched with water (10 mL) and extracted with ethyl acetate (2x15 mL). The combined organic layers were washed with brine (10 mL), dried over anhydrous sodium sulfate, filtered, and the filtrate was concentrated under reduced pressure to afford a residue which was purified by column chromatography (Biotage, Alumina-neutral) using 30-40% of ethyl acetate in petroleum ether to provide tert-butyl (1*S*,6*R*)-3-(7-(5-(bis(4-methoxybenzyl)amino)-3-methyl-2-(trifluoromethyl)phenyl)-6-chloro-8-fluoro-2-(((2*R*,7a*S*)-2-fluorotetrahydro-1H-pyrrolizin-7a(5H)-yl)methoxy)quinazolin-5-yl)-3,9-diazabicyclo[4.2.1]nonane-9-carboxylate (55 mg, 0.052 mmol, 73% yield). LCMS (ESI) m/z: 979.51 [M+H]⁺.

### Example 4-1

### 6-(2-{[(2R, 7aS)-2-fluoro-hexahydro-1H-pyrrolizin-7a-yl]methoxy)-6-chloro-4-[(1S, 6R)-3,9-diazabicyclo[4.2.1]nonan-3-yl]-8-fluoroquinazolin-7-yl)-4-methyl-5-(trifluoromethyl)pyridin-2-amine

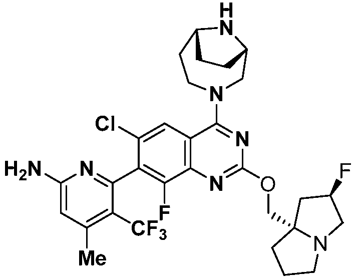

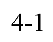

To a stirred solution of tert-butyl (1*S*,6*R*)-3-(7-(6-(bis(4-methoxybenzyl)amino)-4-methyl-3-(trifluoromethyl)pyridin-2-yl)-6-chloro-8-fluoro-2-(((2*R*,7a*S*)-2-fluorotetrahydro-1H-pyrrolizin-7a(5H)-yl)methoxy)quinazolin-4-yl)-3,9-diazabicyclo[4.2.1]nonane-9-carboxylate (55 mg, 0.056 mmol) in TFA (1 mL) was added water (0.01 mL) and triethylsilane (0.898 µL, 5.62 µmol). The reaction was allowed to stir at 40 °C for 16h. The reaction mixture was then concentrated under reduced pressure to obtain the crude residue, which was purified by silica gel column chromatography using 5-7% Methanol in DCM to obtain 6-(4-((1S,6R)-3,9-diazabicyclo[4.2.1]nonan-3-yl)-6-chloro-8-fluoro-2-(((2*R*,7a*S*)-2-fluorotetrahydro-1H-pyrrolizin-7a(5H)-yl)methoxy)quinazolin-7-yl)-4-methyl-5-(trifluoromethyl)pyridin-2-amine (16 mg, 0.023 mmol, 41% yield). LCMS (ESI) m/z: 638.3 [M+H]⁺. ¹H-NMR (400 MHz, MeOD): *δ* 7.97 (s, 1H), 6.62 (s, 1H), 5.33 (d, *J =* 54.00 Hz, 2H), 4.50 (d, *J* = 13.20 Hz, 1H), 4.22-4.48 (m, 2H), 4.01-4.03 (m, 1H), 3.72-3.94 (m, 4H), 3.24-3.33 (m, 2H), 3.04-3.06 (m, 1H), 2.47 (s, 3H), 2.14-2.29 (m, 9H), 1.98-2.02 (m, 1H), 1.70-1.72 (m, 2H) ppm.

Examples in Table 3 were prepared according to procedures described for Example 4-1 from appropriate starting materials.

**Table 3**

| Example Number | Structure | Name | MS m/z: [M+H]+ | ¹H NMR |
|---|---|---|---|---|
| 4-2 | 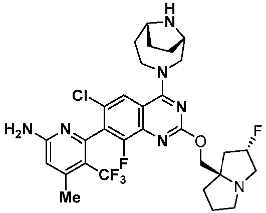 | 6-(2-{ [(2S,7aR)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amine | 638.08 | (400 MHz, MeOD): δ 7.97 (d, *J* = 1.60 Hz, 1H), 6.63 (s, 1H), 5.37 (d, *J = 52.80* Hz, 1H), 4.44-4.48 (m, 3H), 4.03-4.17 (m, 5H), 3.74-3.93 (m, 2H), 3.52-3.57 (m, 3H), 3.32-3.33 (m, 1H), 2.47 (s, 3H), 2.43-2.44 (m, 1H), 2.39-2.42 (m, 2H), 2.05-2.18 (m, 4H), 1.95-2.01 (m, 3H) ppm |
| 4-3 | 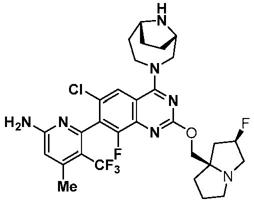 | 6-(2-{[(2R,7aR)-2-fluoro-hexahydro-1H-pyrrolizin-7a-yl]methoxy}-6-chloro-4-[(1S,6R)-3,9-diazabicyclo[4.2. 1]nonan-3-yl]-8-fluoroquinazolin-7-yl)-4-methyl-5-(trifluoromethyl)pyridin-2-amine | 638.4 | (400 MHz, MeOD) *δ* 7.97 (d, *J =* 1.20 Hz, 1H), 6.62 (s, 1H), 5.39 (d, *J* = 84.00 Hz, 1H), 4.52 *(d, J = 13.20* Hz, 1H), 4.42 (d, *J* = 10.40 Hz, 1H), 4.31 (d, *J* = 10.00 Hz, 1H), 3.80-4.12 (m, 2H), 3.69-3.80 (m, 4H), 3.32-3.34 (m, 1H), 2.53-3.10 (m, 3H), 2.51-2.52 (m, 1H), 2.47 (s, 3H), 1.94-2.04 (m, 8H), 1.67-1.69 (m, 1H) ppm |
| 4-4 | 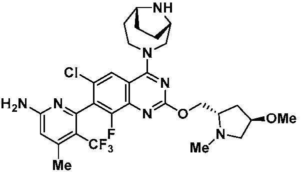 | 6-{6-chloro-4-[(1 S,6R)-3,9-diazabicyclo[4.2.1]nonan-3-yl]-8-fluoro-2-{ [(2S,4R)-4-methoxy-1-methylpyrrolidin-2-yl]methoxy}quinazolin-7-yl}-4-methyl-5-(trifluoromethyl)pyridin-2-amine | 624.3 | (400 MHz, MeOD): *δ* 7.97 (d, *J =* 1.60 Hz, 1H), 6.62 (s, 1H), 4.45-4.52 (m, 3H), 3.98-4.02 (m, 3H), 3.69-3.81 (m, 3H), 3.43-3.50 (m, 1H), 2.96-3.13 (m, 1H), 2.53 (s, 1H), 2.47 (d, *J* = 1.20 Hz, 3H), 2.38-2.42 (m, 4H), 2.11-2.12 (m, 1H), 2.05-2.10 (m, 7H), 1.68 (d, *J* = 7.60 Hz, 2H) ppm. |

### Example 5

### 6-(2-{[(2S,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amine

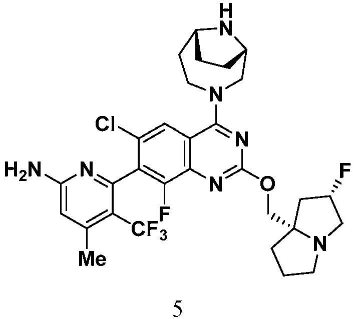

### Preparation of intermediates 5A (isomer 1) and 5B (isomer2): tert-butyl 4-(7-(6-(bis(4-methoxybenzyl)amino)-4- methyl-3-(trifluoromethyl)pyridin-2-yl)-6-chloro-2,8-difluoroquinazolin-4-yl)piperazine-1-carboxylate

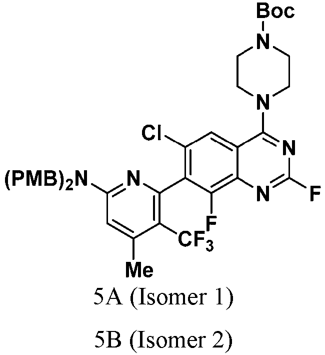

To a stirred solution of tert-butyl 4-(7-(6-(bis(4-methoxybenzyl)amino)-3-iodo-4-methylpyridin-2-yl)-6-chloro-2,8-difluoroquinazolin-4-yl)piperazine-1-carboxylate (21 g, 24.50 mmol) in anhydrous DMA (210 mL) in a sealed tube under nitrogen atmosphere was added copper(I) iodide (21 g, 24.50 mmol). The reaction mixture was degassed for 10 min before the addition of methyl 2,2-difluoro-2-(fluorosulfonyl)acetate (21 g, 24.50 mmol) and the reaction mixture was heated at 90 °C for 12 h. After cooling to room temperature, the reaction mixture was diluted with diethyl ether (200 mL) and water (50 mL). The layers were separated, and the aqueous layer was extracted with diethyl ether (3x50 mL). The combined organic layers were dried over anhydrous sodium sulphate, filtered and concentrated under reduced pressure to obtain the crude residue, which was purified by silica gel column chromatography using 30% ethyl acetate in petroleum ether to obtain *tert*-butyl 4-(7-(6-(bis(4-methoxybenzyl)amino)-4-methyl-3-(trifluoromethyl)pyridin-2-yl)-6-chloro-2,8-difluoroquinazolin-4-yl)piperazine-1-carboxylate (15.1g, 16.44 mmol, 67% yield) as a pale yellow solid. Atropisomers were separated using SFC (Column: Chiralpak IH (250mm x 4.6 x 5µ), mobile phase- 25% Isopropanol), where Peak-1 (Intermediate 5A), eluted at retention time = 6.00 minutes (0.73 g, 0.98 mmol, 37% yield); LCMS (ESI) *m*/*z:* 825.2 [M+H]⁺. and Peak-2 (Intermediate 5B) at retention time = 9.262 minutes (0.77 g, 0.97 mmol, 38%); LCMS (ESI) m/z: 825.2 [M+H]⁺.

### Preparation of intermediate 5C: tert-butyl 4-(7-(6-(bis(4-methoxybenzyl)amino)-4-methyl-3-(trifluoromethyl)pyridin-2yl)-6-chloro-8-fluoro-2-(((2S,7aS)-2-fluorotetrahydro-1Hpyrrolizin-7a(5H)-yl)methoxy)quinazolin-4-yl)piperazine-1-carboxylate

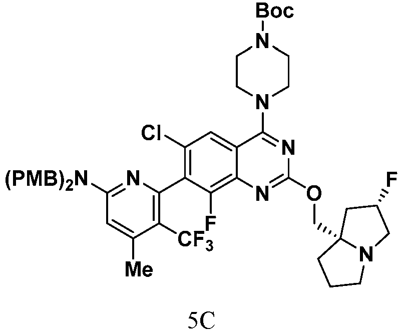

To a stirred solution of ((2*S*,7a*S*)-2-fluorotetrahydro-1H-pyrrolizin-7a(5H)-yl)methanol (100 mg, 0.626 mmol) in THF (5 mL) was added sodium hydride (60% in mineral oil, 25.02 mg, 0.626 mmol) at 0 °C and the reaction mixture was stirred for 30 min. Intermediate 5B (250 mg, 0.313 mmol) in THF (5 mL) was added dropwise to the above reaction mixture at 0 °C followed by stirring at ambient temperature for 2 h. The reaction mixture was quenched with saturated aq. ammonium chloride solution (10 mL) and extracted with ethyl acetate (2x20 mL). The combined organic layers were washed with water and brine and then dried over anhydrous Na₂SO₄, filtered and concentrated under reduced pressure to afford the crude product. The product *tert-butyl* 4-(7-(6-(bis(4-methoxybenzyl)amino)-4-methyl-3-(trifluoromethyl)pyridin-2-yl)-6-chloro-8-fluoro-2-(((2*S*,7a*S*)-2-fluorotetrahydro-1H-pyrrolizin-7a(5H)-yl)methoxy)quinazolin-4-yl)piperazine-1-carboxylate (398 mg, 0.246 mmol, 79% yield) was used in next reaction without any further purification. LCMS (ESI) m/z: 938.3 [M+H]⁺.

### Preparation of intermediate 5D: 7-(6-(bis(4-methoxybenzyl)amino)-4-methyl-3-(trifluoromethyl)pyridin-2yl)-6-chloro-8-fluoro-2-(((2S,7aS)-2-fluorotetrahydro-1H-pyrrolizin-7a(5H)-yl)methoxy)quinazolin-4-ol

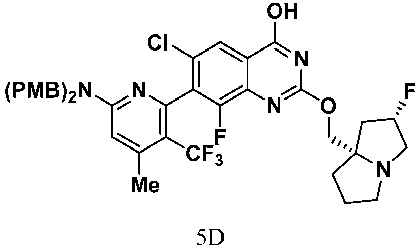

To a stirred solution of *tert*-butyl 4-(7-(6-(bis(4-methoxybenzyl)amino)-4-methyl-3-(trifluoromethyl)pyridin-2-yl)-6-chloro-8-fluoro-2-(((2*S*,7a*S*)-2-fluorotetrahydro-1H-pyrrolizin-7a(5H)-yl)methoxy)quinazolin-4-yl)piperazine-1-carboxylate (398 mg, 0.424 mmol) in EtOH (5 mL) and THF (1 mL) was added NaOH (1M aq. Solution, 1696 mg, 4.98 mmol). After stirring at 70 °C for 48h, the reaction mixture was concentrated under reduced pressure to obtain the crude residue, which was purified by column chromatography (Biotage, Alumina- neutral) using 30-40% ethyl acetate in petroleum ether to obtain 7-(6-(bis(4-methoxybenzyl)amino)-4-methyl-3-(trifluoromethyl)pyridin-2-yl)-6-chloro-8-fluoro-2-(((2*S*,7a*S*)-2-fluorotetrahydro-1H-pyrrolizin-7a(5H) yl)methoxy)quinazolin -4-ol (150 mg, 0.175 mmol, 41% yield) as pale yellow oil. LCMS (ESI) m/z: 770.2 [M+H]⁺.

### Preparation of intermediate 5E: tert-butyl (1S,6R)-3-(7-(6-(bis(4-methoxybenzyl)amino)-4-methyl-3-(trifluoromethyl)pyridin-2-yl)-6-chloro-8-fluoro-2-(((2S,7aS)-2-fluorotetrahydro-1H-pyrrolizin-7a(5H)-yl)methoxy)quinazolin-4-yl)-3,9-diazabicyclo[4.2.1]nonane-9-carboxylate.

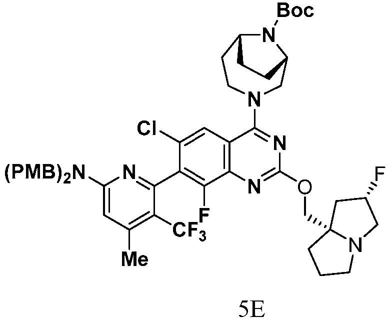

To a stirred solution of 7-(6-(bis(4-methoxybenzyl)amino)-4-methyl-3-(trifluoromethyl)pyridin-2-yl)-6-chloro-8-fluoro-2-(((2S,7aS)-2-fluorotetrahydro-1H-pyrrolizin-7a(5H)-yl)methoxy)quinazolin-4-ol (30 mg, 0.039 mmol) in acetonitrile (1 mL) were added TEA (8.14 µl, 0.058 mmol) and BOP (25.8 mg, 0.058 mmol). After stirring for 5 min. tert-butyl (1S,6R)-3,9-diazabicyclo[4.2.1]nonane-9-carboxylate (11.02 mg, 0.049 mmol) was added to the reaction mixture and the mixture was heated at 40 °C for 24h. The reaction mixture was quenched with water (10 mL) and extracted with ethyl acetate (2 x15 mL). The combined organic layers were washed with brine (10 mL), dried over anhydrous sodium sulfate, filtered, and the filtrate was concentrated under reduced pressure to obtain the crude compound. The crude residue was purified by column chromatography (Biotage, Alumina-neutral) using 45% ethyl acetate in petroleum ether to obtain tert-butyl (1*S*,6*R*)-3-(7-(6-(bis(4-methoxybenzyl)amino)-4-methyl-3-(trifluoromethyl)pyridin-2-yl)-6-chloro-8-fluoro-2-(((2S,7aS)-2-fluorotetrahydro-1H-pyrrolizin-7a(5H)-yl)methoxy)quinazolin-4-yl)-3,9-diazabicyclo[4.2.1]nonane-9-carboxylate (28 mg, 0.027 mmol, 69% yield). LCMS (ESI) m/z: 979.5 [M+H]⁺.

### Example 5

### 6-(2-{[(2S,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amine

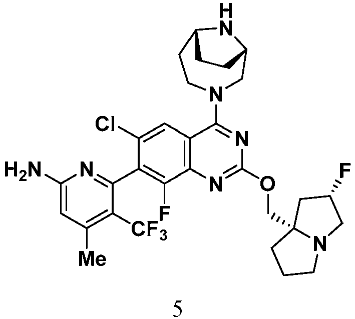

To a stirring solution of tert-butyl (1*S*,6*R*)-3-(7-(6-(bis(4-methoxybenzyl)amino)-4-methyl-3-(trifluoromethyl)pyridin-2-yl)-6-chloro-8-fluoro-2-(((2*S*,7a*S*)-2-fluorotetrahydro-1H-pyrrolizin-7a(5H)-yl)methoxy)quinazolin-4-yl)-3,9-diazabicyclo[4.2.1]nonane-9-carboxylate (28 mg, 0.029 mmol) in TFA (1 mL) was added triethylsilane (0.457 µL, 2.86 µmol) and water (0.05 mL, 2.78 mmol). The reaction was allowed to stir at 40 °C for 16h. The reaction mixture was concentrated under reduced pressure and the residue was purified by column chromatography (Biotage, sfar KP-amino) by eluting with 50-100% ethyl acetate in petroleum ether to obtain 6-(4-((1*S*,6*R*)-3,9-diazabicyclo[4.2.1]nonan-3-yl)-6-chloro-8-fluoro-2-(((2*S*,7a*S*)-2-fluorotetrahydro-1H-pyrrolizin-7a(5H)-yl)methoxy)quinazolin-7-yl)-4-methyl-5-(trifluoromethyl)pyridin-2-amine (11.4 mg, 0.018 mmol, 62% yield). LCMS (ESI) m/z: 638.08 [M+H]⁺ . ¹H-NMR (400 MHz, MeOD) δ 7.97 (d, *J* = 1.60 Hz, 1H), 6.63 (s, 1H), 5.37 (d, *J* = 52.80 Hz, 1H), 4.41-4.89 (m, 3H), 4.02-4.13 (m, 1H), 3.88-3.94 (m, 4H), 3.49-3.50 (m, 2H), 2.88-3.28 (m, 3H), 2.50-2.16 (m, 1H), 0.47 (s, 3H), 2.19-2.47 (m, 1H), 2.12-2.18 (m, 4H), 1.91-2.02 (m, 3H), 1.79-1.88 (m, 2H) ppm.

### Example 6

### 4-{6-chloro-4-[(1S,6R)-3,9-diazabicyclo[4.2.1]nonan-3-yl]-8-fluoro-2-{[(2S)-1-methylpyrrolidin-2-yl]methoxy}quinazolin-7-yl}naphthalen-2-ol

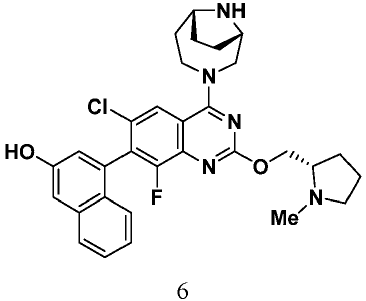

### Preparation of intermediate 6A (Isomer 1) and 6B (Isomer 2): tert-butyl-3-(6-chloro-2,8-difluoro-7-(3-(methoxymethoxy)naphthalen-1-yl)quinazolin-4-yl)-3,8-diazabicyclo[3.2.1]octane-8-carboxylate

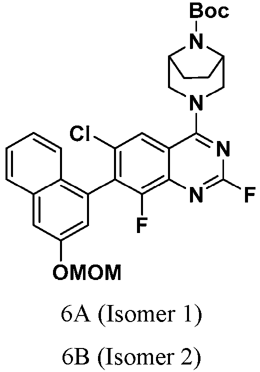

To a degassed solution of *tert*-butyl (1R,5S)-3-(7-bromo-6-chloro-2,8-difluoroquinazolin-4-yl)-3,8-diazabicyclo[3.2.1]octane-8-carboxylate (1.0 g, 2.042 mmol)) in 1,4-dioxane (60 mL), 2-(3-(methoxymethoxy)naphthalen-1-yl)-4,4,5,5-tetramethyl-1,3,2-dioxaborolane (1.28 g, 4.08 mmol), Cs₂CO₃ (1.3 g, 4.08 mmol), Pd₂(dba)₃ (93 mg, 0.102 mmol) and pentaphenyl(di-tert-butylphosphino)ferrocene (0.073 g, 0.102 mmol, QPhos, Cas No: 312959-24-3) were added. The mixture was degassed again and heated in a pressure vial at 70°C for 12 hours. The reaction mixture was concentrated under reduced pressure to obtain the crude residue, which was purified by silica gel column chromatography using 30% ethyl acetate in petroleum ether to obtain *tert*-butyl-3-(6-chloro-2,8-difluoro-7-(3-(methoxymethoxy)naphthalen-1-yl)quinazolin-4-yl)-3,8-diazabicyclo[3.2.1]octane-8-carboxylate (1.0 g, 1.591 mmol, 78%) as pale brown solid. LCMS (ESI) m/z: 597.2 [M+H]⁺. The purified compound was subjected to SFC to separate the atropisomers (Column: Chiralpak ADH (250mm x 4.6 x 5µ), mobile phase- 30% Isopropanol), where Peak-1 (Intermediate 6A) eluted at retention time = 2.72 minutes (230 mg, 46%); LCMS (ESI) m/z: 597.2 [M+H]+ and Peak-2 (Intermediate 6B) at retention time = 5.47 minutes (205 mg, 41%); LCMS (ESI) m/z: 597.2 [M+H]⁺.

### Preparation of intermediate 6C: tert-butyl 3-(6-chloro-8-fluoro-7-(3-(methoxymethoxy)naphthalen-1-yl)-2-(((S)-1-methylpyrrolidin-2-yl)methoxy)quinazolin-4-yl)-3,8-diazabicyclo[3.2.1]octane-8-carboxylate

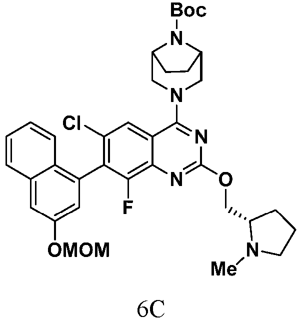

To a stirred solution of (S)-(1-methylpyrrolidin-2-yl)methanol (38.6 mg, 0.335 mmol) in THF (5 mL) at 0 °C was added NaH (60% dispersion in mineral oil, 12.06 mg, 0.301 mmol) and the reaction mixture was stirred for 30 min. and then Intermediate 6A (100 mg, 0.167 mmol) in THF (1 mL) was added dropwise. The reaction mixture was stirred at ambient temperature for 2 h before quenching with saturated aq. ammonium chloride solution (10 mL) followed by extraction with ethyl acetate (2x20 mL). The combined organic layers were washed with water and brine solution, and then dried over anhydrous Na₂SO₄, filtered and concentrated under reduced pressure to afford crude product, which was purified by column chromatography (Grace REVELERIS^{®}, 50 g snap, dry pack) over neutral alumina using 50-100% ethyl acetate in petroleum ether. The desired fractions were pooled and concentrated under reduced pressure to obtain *tert-butyl* 3-(6-chloro-8-fluoro-7-(3-(methoxymethoxy)naphthalen-1-yl)-2-(((*S*)-1-methylpyrrolidin-2-yl)methoxy)quinazolin-4-yl)-3,8-diazabicyclo[3.2.1]octane-8-carboxylate (90 mg, 0.118 mmol, 71% yield) as pale yellow oil. LCMS (ESI) m/z: 691.8 [M+H]⁺.

### Preparation of intermediate 6D: 6-chloro-8-fluoro-7-(3-(methoxymethoxy)naphthalen-1-yl)-2-(((S)-1-methylpyrrolidin-2-yl)methoxy)quinazolin-4-ol

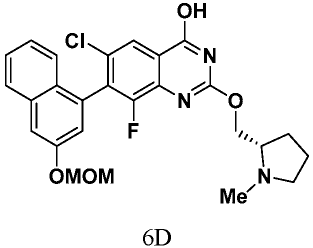

To a stirred solution of *tert-butyl* 3-(6-chloro-8-fluoro-7-(3-(methoxymethoxy)naphthalen-1-yl)-2-(((*S*)-1-methylpyrrolidin-2-yl)methoxy)quinazolin-4-yl)-3,8-diazabicyclo[3.2.1]octane-8-carboxylate (120 mg, 0.173 mmol) in ethanol (5 mL) was added NaOH (1M aq. solution, 199 mg, 0.498 mmol). The reaction was allowed to stir at 70 °C for 48 h. The reaction mixture was then concentrated under reduced pressure to obtain the crude residue which was purified by column chromatography (Biotage, Alumina- neutral) using 30-40% ethyl acetate in petroleum ether to obtain 6-chloro-8-fluoro-7-(3-(methoxymethoxy)naphthalen-1-yl)-2-(((*S*)-1-methylpyrrolidin-2-yl)methoxy)quinazolin-4-ol (35 mg, 0.069 mmol, 40% yield) as an off white solid. LCMS (ESI) m/z: 500.1 [M+H]⁺.

### Preparation of intermediate 6E: tert-butyl (1S,6R)-3-(6-chloro-8-fluoro-7-(3-(methoxymethoxy)naphthalen-1-yl)-2-(((S)-1-methylpyrrolidin-2-yl)methoxy)quinazolin-4-yl)-3,9-diazabicyclo[4.2.1]nonane-9-carboxylate.

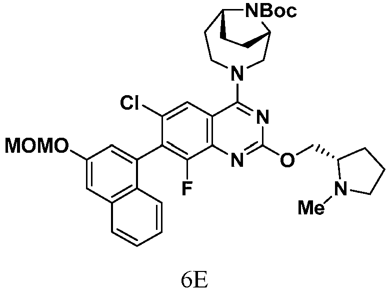

To a stirred solution of 6-chloro-8-fluoro-7-(3-(methoxymethoxy)naphthalen-1-yl)-2-(((S)-1-methylpyrrolidin-2-yl)methoxy)quinazolin-4-ol (35 mg, 0.070 mmol) in acetonitrile (1 mL) was added TEA (0.015 mL, 0.105 mmol) and BOP (46.6 mg, 0.105 mmol). After stirring for 5 min, tert-butyl (1*S*,6*R*)-3,9-diazabicyclo[4.2.1]nonane-9-carboxylate (19.88 mg, 0.088 mmol) was added followed by heating of the reaction mixture at 40 °C for 24h. The reaction mixture was quenched with water (10 mL) and extracted with ethyl acetate (2x15 mL). The combined organic layers were washed with brine (10 mL), dried over anhydrous sodium sulfate, filtered, the filtrate was concentrated under reduced pressure to obtain crude compound. The residue was purified by column chromatography (Biotage, Alumina-neutral) using 40% ethyl acetate in petroleum ether to provide tert-butyl (1*S*,6*R*)-3-(6-chloro-8-fluoro-7-(3-(methoxymethoxy)naphthalen-1-yl)-2-(((*S*)-1-methylpyrrolidin-2-yl)methoxy)quinazolin-4-yl)-3,9-diazabicyclo[4.2.1]nonane-9-carboxylate (30 mg, 0.039 mmol, 56% yield). LCMS (ESI) m/z: 707.2 [M+H]⁺.

### Example 6

### 4-{6-chloro-4-[(1S,6R)-3,9-diazabicyclo[4.2.1]nonan-3-yl]-8-fluoro-2-{[(2S)-1-methylpyrrolidin-2-yl]methoxy}quinazolin-7-yl}naphthalen-2-ol

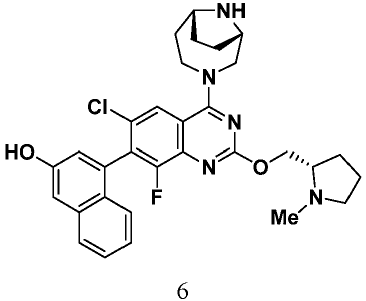

To a stirred solution of tert-butyl (18,6R)-3-(6-chloro-8-fluoro-7-(3-(methoxymethoxy)naphthalen-1-yl)-2-(((*S*)-1-methylpyrrolidin-2-yl)methoxy)quinazolin-4-yl)-3,9-diazabicyclo[4.2.1]nonane-9-carboxylate (30 mg, 0.042 mmol) in methanol (1 mL) was added HCl solution (4.0 M in dioxane, 0.159 mL, 0.637 mmol) at ice cold temperature. The reaction was allowed to warm to room temperature and then stirred for 2 h. The reaction mixture was concentrated under reduced pressure to obtain the crude residue, which was purified by preperative HPLC [Column: Xselect C18 (150 x 19) mm, 5 micron, Mobile phase A: 10mM ammonium acetate in water, Phase B: acetonitrile] to obtain 4-(4-((1S,6R)-3,9-diazabicyclo[4.2.1]nonan-3-yl)-6-chloro-8-fluoro-2-(((*S*)-1-methylpyrrolidin-2-yl)methoxy)quinazolin-7-yl)naphthalen-2-ol (4 mg, 6.67 µmol, 16% yield). LCMS (ESI) m/z: 707.2 [M+H]⁺. ¹H-NMR (400 MHz, MeOD): *δ* 8.11 (d, *J= 1.60* Hz, 1H), 7.78 (d, *J* = 8.40 Hz, 1H), 7.42-7.46 (m, 1H), 7.21-7.29 (m, 4H), 7.05 (s, 1H), 4.52-4.67 (m, 3H), 3.93-4.00 (m, 5H), 3.33-3.34 (m, 1H), 2.83 (s, 4H), 2.18-2.28 (m, 4H), 2.00-2.04 (m, 3H), 1.85-1.96 (m, 2H) ppm.

### Example 7

### 4-(2-{[(2R,7aR)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)naphthalen-2-ol

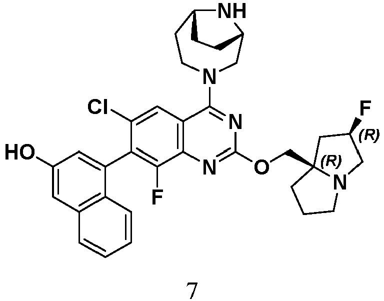

### Preparation of intermediate 7A: tert-butyl-3-(6-chloro-8-fluoro-2-(((2R, 7aR)-2-fluorotetrahydro-1H-pyrrolizin-7a(5H)-yl)methoxy)-7-(3-(methoxymethoxy)naphthalen-1-yl)quinazolin-4-yl)-3,8-diazabicyclo[3.2.1 joctane-8-carboxylate

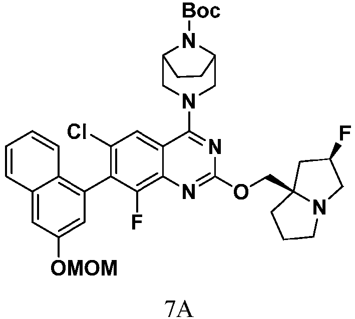

To a stirred solution of ((2*R*,7a*R*)-2-fluorotetrahydro-1H-pyrrolizin-7a(5H)-yl)methanol (53.3 mg, 0.335 mmol) in THF (5 mL) at 0 °C was added NaH (60% dispersion mineral oil, 12.06 mg, 0.301 mmol) and the reaction mixture was stirred for 30 min. Intermediate 6A (100 mg, 0.167 mmol) in THF (1mL) was then added dropwise. The reaction mixture was stirred at ambient temperature for 2 h before quenching with saturated aq. ammonium chloride solution (10 mL) followed by extraction with ethyl acetate (2x20 mL). The combined organic layers were washed with water and brine solution, and then dried over anhydrous Na₂SO₄, filtered and concentrated under reduced pressure to afford the crude product, which was purified by column chromatography (Grace REVELERIS^{®}, 50 g snap, dry pack) over neutral alumina using 50-100% ethyl acetate in petroleum ether. The desired fractions were pooled and concentrated under reduced pressure to obtain tert-butyl-3-(6-chloro-8-fluoro-2-(((2*R*,7a*R*)-2-fluorotetrahydro-1H-pyrrolizin-7a(5H)-yl)methoxy)-7-(3-(methoxymethoxy)naphthalen-1-yl)quinazolin-4-yl)-3,8-diazabicyclo[3.2.1]octane-8-carboxylate (120 mg, 0.150 mmol, 90% yield) as pale yellow oil. LCMS (ESI) m/z: 735.8 [M+H]⁺.

### Preparation of intermediate 7B: 6-chloro-8-fluoro-2-(((2R,7aR)-2-fluorotetrahydro-1H-pyrrolizin-7a(5H)-yl)methoxy)-7-(3-(methoxymethoxy)naphthalen-1-yl)quinazolin-4-ol

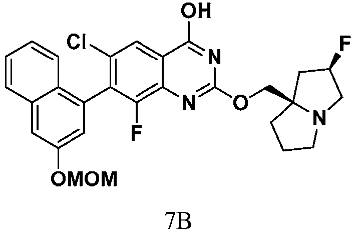

To a stirred solution of tert-butyl 3-(6-chloro-8-fluoro-2-(((2R,7aR)-2-fluorotetrahydro-1H-pyrrolizin-7a(5H)-yl)methoxy)-7-(3-(methoxymethoxy)naphthalen-1-yl)quinazolin-4-yl)-3,8-diazabicyclo[3.2.1]octane-8-carboxylate (230 mg, 0.312 mmol)n ethanol (5 mL) was added NaOH (1M aq. solution, 1249 mg, 3.12 mmol). The reaction was allowed to stir at 70 °C for 48 h. The reaction mixture was then concentrated under reduced pressure to obtain the crude residue which was purified by column chromatography (Biotage, Alumina- neutral) using 30-40% ethyl acetate in petroleum ether to obtain 6-chloro-8-fluoro-2-(((2R,7aR)-2-fluorotetrahydro-1H-pyrrolizin-7a(5H)-yl)methoxy)-7-(3-(methoxymethoxy)naphthalen-1-yl)quinazolin-4-ol (35 mg, 0.061 mmol, 19% yield) off-white solid. LCMS (ESI) m/z: 542.2 [M+H]⁺.

### Preparation of intermediate 7C: To synthesis of tert-butyl (1S,6R)-3-(6-chloro-8-fluoro-2-(((2R, 7aR)-2-fluorotetrahydro-1H-pyrrolizin-7a(5H)-yl)methoxy)-7-(3-(methoxymethoxy)naphthalen-1-yl)quinazolin-4-yl)-3,9-diazabicyclo[4.2.1]nonane-9-carboxylate

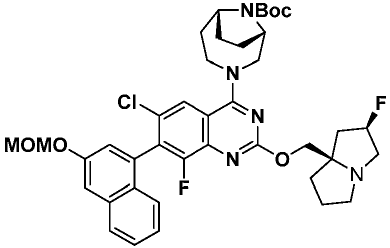

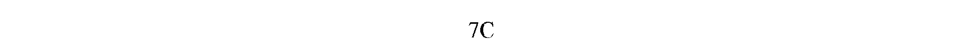

To a stirred solution of 6-chloro-8-fluoro-2-(((2*R*,7a*R*)-2-fluorotetrahydro-1H-pyrrolizin-7a(5H)-yl)methoxy)-7-(3-(methoxymethoxy)naphthalen-1-yl)quinazolin-4-ol (35 mg, 0.065 mmol) in acetonitrile (1 mL) was added TEA (0.014 mL, 0.097 mmol) and BOP (42.8 mg, 0.097 mmol). After stirring for 5 min, tert-butyl (1*S*,6*R*)-3,9-diazabicyclo[4.2.1]nonane-9-carboxylate (18.27 mg, 0.081 mmol) was added and the reaction mixture was heated at 40 °C for 24h. The reaction mixture was quenched with water (10 mL) and extracted with ethyl acetate (2x15 mL). The organic layer was washed with brine (10 mL), dried over anhydrous sodium sulfate, filtered, and the filtrate was concentrated under reduced pressure to obtain the crude compound. The residue was purified by column chromatography (Biotage, Alumina-neutral) using 30-40% ethyl acetate in petroleum ether to provide tert-butyl (1*S*,6*R*)-3-(6-chloro-8-fluoro-2-(((2*R*,7a*R*)-2-fluorotetrahydro-1H-pyrrolizin-7a(5H)-yl)methoxy)-7-(3-(methoxymethoxy)naphthalen-1-yl)quinazolin-4-yl)-3,9-diazabicyclo[4.2.1]nonane-9-carboxylate (38 mg, 0.048 mmol, 75% yield). LCMS (ESI) m/z: 751.2 [M+H]⁺.

### Example 7

### 4-(2-{[(2R,7aR)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)naphthalen-2-ol

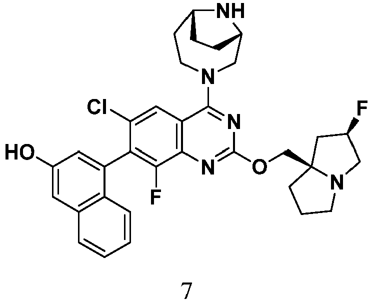

To a stirred solution of tert-butyl (1*S*,6*R*)-3-(6-chloro-8-fluoro-2-(((2*R*,7a*R*)-2-fluorotetrahydro-1*H*-pyrrolizin-7a(5*H*)-yl)methoxy)-7-(3-(methoxymethoxy)naphthalen-1-yl)quinazolin-4-yl)-3,9-diazabicyclo[4.2.1]nonane-9-carboxylate (30 mg, 0.042 mmol) in methanol (1 mL) was added HCl (4.0 M in dioxane, 0.159 mL, 0.637 mmol) at ice cold temperature. The reaction was allowed to warm to room temperature and then stirred for 2 h. The reaction mixture was concentrated under reduced pressure to obtain the crude residue which was purified by preperative HPLC [Column: Xselect C18 (150 x 19) mm, 5 micron, Mobile phase A: 10mM ammonium acetate in water, Phase B: Acetonitrile] to obtain 4-(4-((1S,6R)-3,9-diazabicyclo[4.2.1]nonan-3-yl)-6-chloro-8-fluoro-2-(((2*R*,7a*R*)-2-fluorotetrahydro-1H-pyrrolizin-7a(5H)-yl)methoxy)quinazolin-7-yl)naphthalen-2-ol (7 mg, 0.01 µmol, 35% yield) as an off white solid. LCMS (ESI) m/z: 562.3 [M+H]⁺. ¹H-NMR (400 MHz, MeOD): δ 8.11 (d, *J=* 1.60 Hz, 1H), 7.78 (d, *J* = 8.40 Hz, 1H), 7.42-7.46 (m, 1H), 7.21-7.29 (m, 4H), 7.05 (s, 1H), 4.52-4.67 (m, 3H), 3.93-4.00 (m, 5H), 3.33-3.34 (m, 1H), 2.83 (s, 4H), 2.18-2.28 (m, 4H), 2.00-2.04 (m, 3H), 1.85-1.96 (m, 2H) ppm.

### Examples 8-1 and 8-2

### 4-(2-{[(2R, 7aS)-2-fluoro-hexahydro-1H-pyrrolizin-7a-yl]methoxy)-6-chloro-4-[(]S, 6R)-3,9-diazabicyclo[4.2.1]nonan-3-yl]-8-fluoroquinazolin-7-yl)-5-ethylnaphthalen-2-ol

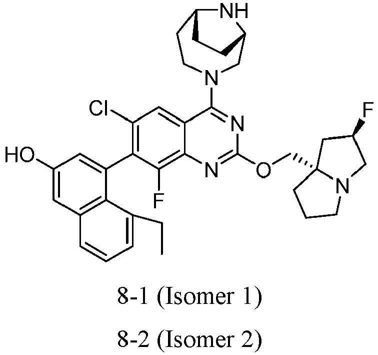

### Preparation of Intermediate 8A: tert-Butyl-3-(7-bromo-6-chloro-8-fluoro-2-(((2R,7aS)-2-fluorotetrahydro-1H-pyrrolizin-7a(5H)-yl)methoxy)quinazolin-4-yl)-3,8-diazabicyclo[3. 2.1 joctane-8-carboxylate

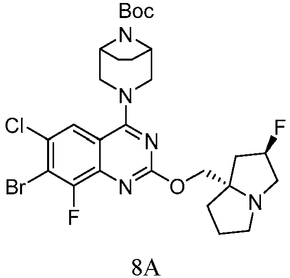

To a stirred solution of ((2*R*,7a*S*)-2-fluorotetrahydro-1H-pyrrolizin-7a(5H)-yl)methanol (195 mg, 1.225 mmol) in THF (8 mL) at 0 °C was added NaH (61.3 mg, 1.531 mmol) and the resulting reaction mixture was stirred for an additional 30 min. Then, a solution of *tert-*butyl-3-(7-bromo-6-chloro-2,8-difluoroquinazolin-4-yl)-3,8-diazabicyclo[3.2.1]octane-8-carboxylate (500 mg, 1.021 mmol) in THF (2 mL) was added and the mixture was gradually warmed up to room temperature over a period of 1 h. The reaction mixture was quenched with saturated aq. ammonium chloride solution and extracted with ethyl acetate. The combined organic layers were washed with water and brine, and then dried over Na₂SO₄ and concentrated to provide the crude residue, which was purified by silica gel column chromatography using a CombiFlash instrument (40 g RediSep^{®} column, 70 to 80% EtOAc-pet. ether) to afford tert-butyl-3-(7-bromo-6-chloro-8-fluoro-2-(((2*R*,7a*S*)-2-fluorotetrahydro-1H-pyrrolizin-7a(5H)-yl)methoxy)quinazolin-4-yl)-3,8-diazabicyclo[3.2.1]octane-8-carboxylate (400 mg, 0.636 mmol, 62.3 % yield). MS(ESI) m/z: 628.2 [M+H]⁺.

### Preparation of Intermediate 8B: tert-butyl 3-(2-{[(2R, 7aS)-2-fluoro-hexahydro-1H-pyrrolizin-7a-yl]methoxy}-6-chloro-7-[8-ethyl-3-(methoxymethoxy)naphthalen-1-yl]-8-fluoroquinazolin-4-yl)-3,8-diazabicyclo[3.2.1]octane-8-carboxylate

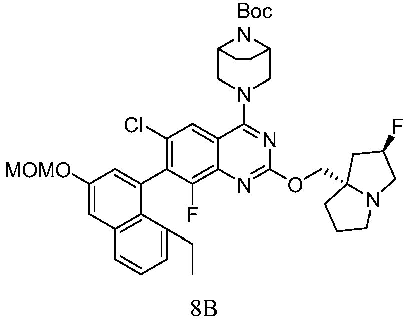

To a stirred solution of tert-butyl-3-(7-bromo-6-chloro-8-fluoro-2-(((2R,7aS)-2-fluorotetrahydro-1H-pyrrolizin-7a(5H)-yl)methoxy)quinazolin-4-yl)-3,8-diazabicyclo[3.2.1]octane-8-carboxylate (700 mg, 1.113 mmol) in 1,4-dioxane (5 mL) at room temperature, were added 2-(8-ethyl-3-(methoxymethoxy)naphthalen-1-yl)-4,4,5,5-tetramethyl-1,3,2-dioxaborolane (457 mg, 1.336 mmol), and tripotassium phosphate (1.5M aq. solution) (1.484 mL, 2.226 mmol). The reaction mixture was purged with nitrogen for 5 minutes and then charged with Pd(Ph₃P)₄ (129 mg, 0.111 mmol). The reaction mixture was again purged with nitrogen for 3 minutes and heated at 85 °C for 16 h. The reaction mixture was cooled, filtered through a Celite pad and the filtrate was concentrated under reduced pressure to afford the crude compound which was purified by silica gel column chromatography using a CombiFlash instrument (24g RediSep^{®} column; pet. ether-ethyl acetate as eluent). The desired product was eluted with 60-70% ethyl acetate in pet. ether. The pure fractions were combined and evaporated to dryness under reduced pressure to afford *tert*-butyl 3-(2-{[(2R,7aS)-2-fluoro-hexahydro-1H- pyrrolizin-7a-yl]methoxy}-6-chloro-7-[8-ethyl-3-(methoxymethoxy)naphthalen-1-yl]-8-fluoroquinazolin-4-yl)-3,8-diazabicyclo[3.2.1]octane-8-carboxylate (350 mg, 0.458 mmol, 41.1 % yield) as a brown solid. MS(ESI) m/z: 764.3 [M+H]⁺.

### Preparation of Intermediate 8C: 2-{[(2R,7aS)-2-fluoro-hexahydro-1H-pyrrolizin-7a-yl]methoxy}-6-chloro-7-[8-ethyl-3-(methoxymethoxy)naphthalen-1-yl]-8-fluoro-3,4-dihydroquinazolin-4-one

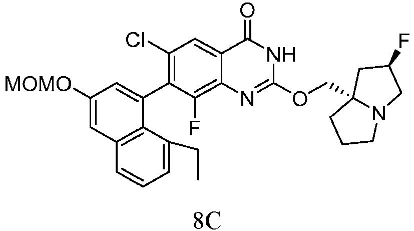

A suspension of tert-butyl 3-(2-{[(2R,7aS)-2-fluoro-hexahydro-1H- pyrrolizin-7a-yl]methoxy}-6-chloro-7-[8-ethyl-3-(methoxymethoxy)naphthalen-1-yl]-8-fluoroquinazolin-4-yl)-3,8-diazabicyclo[3.2.1]octane-8-carboxylate (200 mg, 0.262 mmol) in EtOH (2 mL) was treated with NaOH (1M aq. solution) (2.62 mL, 2.62 mmol). The reaction mixture was stirred at 70 °C for 16 h. The reaction mixture was then concentrated under reduced pressure and the residue was diluted with ethyl acetate and washed with brine, dried over Na₂SO₄ filtered and concentrated to afford the compound 2-{[(2*R*,7a*S*)-2-fluoro-hexahydro-1H-pyrrolizin-7a-yl]methoxy}-6-chloro-7-[8-ethyl-3-methoxymethoxy)naphthalen-1-yl]-8-fluoro-3,4-dihydroquinazolin-4-one (160 mg, crude) as a white solid. MS (ESI) *m*/*z:* 570.2 [M+H]⁺.

### Preparation of Intermediate 8D: tert-butyl (1S,6R)-3-(2-{[(2R,7aS)-2-fluoro-hexahydro-1H-pyrrolizin-7a-yl]methoxy}-6-chloro-7-[8-ethyl-3-(methoxymethoxy)naphthalen-1-yl]-8-fluoroquinazolin-4-yl)-3,9-diazabicyclo[4.2.1]nonane-9-carboxylate

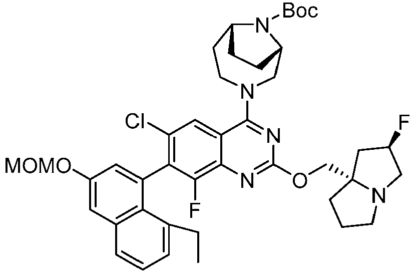

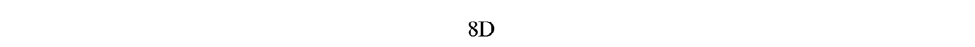

A solution of 2-[[(2*R*,7a*S*)-2-fluoro-hexahydro-1H-pyrrolizin-7a-yl]methoxy}-6-chloro-7-[8-ethyl-3-(methoxymethoxy)naphthalen-1-yl]-8-fluoro-3,4-dihydroquinazolin-4-one (160 mg, 0.281 mmol), *tert*-butyl (1*S*,6*R*)-3,9-diazabicyclo[4.2.1]nonane-9-carboxylate (76 mg, 0.337 mmol) and BOP (186 mg, 0.421 mmol) in acetonitrile (2 mL) was treated with TEA (0.078 mL, 0.561 mmol) and the reaction mixture was stirred at 80 °C for 8 h. The reaction mixture was then concentrated under reduced pressure to afford the crude residue. The crude compound was purified by silica gel column chromatography using a CombiFlash instrument with 0-10% MeOH/DCM (with 0.5% TEA) to afford the desired compound tert-butyl (15,6R)-3-(2-{[(2R,7aS)-2-fluoro-hexahydro-1H-pyrrolizin-7a-yl]methoxy}-6-chloro-7-[8-ethyl-3-(methoxymethoxy)naphthalen-1-yl]-8-fluoroquinazolin-4-yl)-3,9-diazabicyclo[4.2.1]nonane-9-carboxylate (80 mg, 0.103 mmol, 36.6 % yield). MS (ESI) *m*/*z:* 778.3 [M+H]⁺.

### Examples 8-1 and 8-2

### 4-(2-{[(2R,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-5-ethylnaphthalen-2-ol

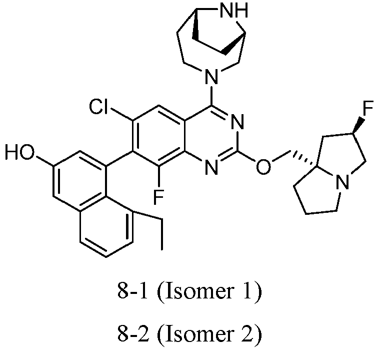

To a solution of *tert*-butyl (1*S*,6R)-3-(2-{[(2*R*,7a*S*)-2-fluoro-hexahydro-1H-pyrrolizin-7a-yl]methoxy}-6-chloro-7-[8-ethyl-3-(methoxymethoxy)naphthalen-1-yl]-8-fluoroquinazolin-4-yl)-3,9-diazabicyclo[4.2.1]nonane-9-carboxylate (80 mg, 0.103 mmol) in MeOH (2 mL) was added HCl (4M in 1,4-dioxane) (2 mL, 65.8 mmol) dropwise at 0 °C and the reaction mixture was then stirred at room temperature for 1 h. The reaction mixture was then concentrated under reduced pressure, co-distilled with toluene (twice), neutralised with DIPEA and re-concentrated under reduced pressure to provide the crude residue. The crude compound was purified by preparative HPLC followed by chiral separation using chiral HPLC to afford atropisomer 1 and 2: 4-(2-{[(2*R*,7a*S*)-2-fluoro-hexahydro-1H-pyrrolizin-7a-yl]methoxy}-6-chloro-4-[(1*S*,6*R*)-3,9-diazabicyclo[4.2.1]nonan-3-yl]-8-fluoroquinazolin-7-yl)-5-ethylnaphthalen-2-ol.

Preparative HPLC Conditions: Column Name: YMC-EXRS (250x21.2 mm), 5µ; Mobile phase A= 10mM Ammonium Bicarbonate in water pH 9.5, Mobile phase B= Acetonitrile: MeOH(1:1), Flow Rate: 20 mL/min; Retention time = 12.6 min.

Preparative Chiral Conditions: Column Name: CELLULOSE-C5 (250x21), 5µ; Mobile Phase: 0.1% DEA in MeOH; Flow Rate: 20 mL/min; Retention time of Peak1 = 5.3 minutes & Retention time of Peak2 = 6.3 minutes.

Example 8-1: MS(ESI) m/z: 634.3, [M+H]⁺. ¹H NMR (400 MHz, DMSO-d₆) δ = 11.0 (bs, 1H), 7.97 (s, 1H), 7.68-7.66 (m, 1H), 7.40-7.33 (m, 1H), 7.28 (d, *J* = 2.6 Hz, 1H), 7.13-7.11 (m, 1H), 6.86 (d, *J* = 2.6 Hz, 1H), 5.39-5.17 (m, 1H), 4.38-4.34 (m, 1H), 4.13 - 4.09 (m, 1H), 4.00 (d, *J=10.4* Hz, 1H), 3.95-3.79 (m, 3H), 3.72-3.64 (m, 3H), 3.12-3.06 (m, 4H), 3.04-2.98 (m, 2H), 2.88-2.78 (m, 2H), 2.36- 2.26 (m, 2H), 2.16-2.10 (m, 1H), 2.01-1.95 (m, 1H), 1.90-1.84 (m, 3H), 1.56-1.30 (m, 3H), 0.87 (t, *J* = 7.4 Hz, 3H).

Example 8-2: MS(ESI) m/z: 634.3, [M+H]⁺.¹H NMR (400 MHz, DMSO-d₆) δ = 7.97 (s, 1H), 7.68-7.66 (m, 1H), 7.38-7.34 (m, 1H), 7.28 (d, *J*=2.6 Hz, 1H), 7.12-7.11 (m, 1H), 6.87 (d, *J*=2.6 Hz, 1H), 5.38 - 5.18 (m, 1H), 4.47-4.43 (m, 1H), 4.11 (d, *J* = 10.4 Hz, 1H), 3.99 (d, *J= 10.4* Hz, 1H), 3.93-3.82 (m, 3H), 3.70 - 3.64 (m, 3H), 3.11-3.05 (m, 4H), 3.09-2.99 (m, 2H), 2.88-2.77 (m, 2H), 2.36 -2.30 (m, 2H), 2.15-2.07 (m, 1H), 2.08-2.00 (m, 1H), 1.91-1.81 (m, 3H), 1.47-1.26 (m, 3H), 0.86 (t, *J=* 7.4 Hz, 3H).

Examples in Table 4 were prepared according to procedures described for Example 8 from appropriate starting materials.

**Table 4**

| Example Number | Structure | Name | **MS** *m*/*z:* [M+H]+ | ¹H NMR |
|---|---|---|---|---|
| 8-3 (Isomer 1) | 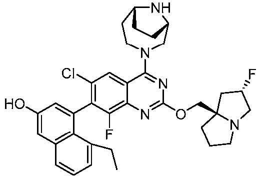 | 4-(2-{[(2*S*,7a*R*)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-5-ethylnaphthalen-2-ol | 634.4 | (400 MHz, DMSO-d₆) *δ* ppm 10.20 (s, 1H), 7.95 (s, 1H), 7.65 (d, *J* = 6 Hz, 1H), 7.34 (t, *J=* 6 Hz, 1H),7.25 (d, *J=* 3 Hz, 1H), 7.09 (d, *J* = 6 Hz, 1H), 6.84 (d, *J* = 3 Hz, 1H), 5.25 (d, *J=* 42 Hz, 1H), 4.42 (d, *J =* 9 Hz, 1H), 4.03 (ABq, *J =* 6 Hz, *J* = 18 Hz, 2H),3.93 - 3.76 (m, 2H), 3.72 - 3.60 (m, 2H), 3.53 - 3.45 (m, 2H), 3.09 - 3.02 (m, 3H), 2.85 - 2.75 (m, 1H), 2.38 - 2.24 (m, 2H), 2.13 - 1.94 (m, 4H), 1.91 - 1.70 (m, 6H), 1.55 - 1.42 (m, 2H), 0.84 (t, *J* = 6 Hz, 3H) |
| 8-4 (Isomer 2) | 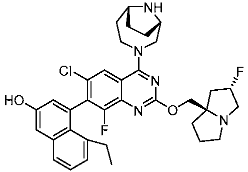 | 4-(2-{[(2*S*,7a*R*)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-5-ethylnaphthalen-2-ol | 634.4 | (400 MHz, DMSO-d₆) *δ* ppm 10.20 (s, 1H), 7.95 (s, 1H), 7.65 (d, *J* = 6 Hz, 1H), 7.34 (t, *J* = 6 Hz, 1H), 7.25 (d, *J = 3* Hz, 1H), 7.09 (d, *J =* 6 Hz, 1H), 6.84 (d, *J* = 3 Hz, 1H), 5.25 (d, *J* = 42 Hz, 1H), 4.42 (d, *J* = 9 Hz, 1H), 4.03 (ABq, J = 6 Hz, *J=* 18 Hz, 2H), 3.92 - 3.78 (m, 2H), 3.71 - 3.60 (m, 2H), 3.51 - 3.39 (m, 2H), 3.12 - 2.95 (m, 3H), 2.87 - 2.75 (m, 1H), 2.39 - 2.24 (m, 2H), 2.14 - 1.92 (m, 4H),1.91 - 1.69 (m, 6H), 1.49 - 1.34 (m, 2H), 0.84 (t, *J* = 6 Hz, 3H). |
| 8-5 (Isomer 1) | 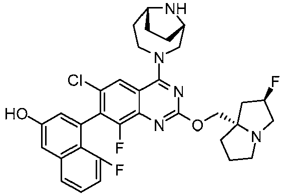 | 4-(2- { [(2R,7aS)-2-fluoro-hexahydro-1H-pyrrolizin-7a-yl]methoxy}-6-chloro-4-[(1S,6R)-3,9-diazabicyclo[4.2. 1]nonan-3-yl]-8-fluoroquinazolin-7-yl)-5-fluoronaphthalen-2-ol | 624.4 | (400 MHz, DMSO-d₆) *δ* ppm 7.93 (s, 1H), 7.65 (d, *J* = 6 Hz, 1H),7.45 - 7.37 (m, 1H), 7.33 (brs, 1H), 7.03 - 6.93 (m, 3H), 5.25 (d, *J* = 40 Hz, 1H), 4.37 (d, *J* = 12 Hz, 1H), 4.03 (ABq,*J* = 6 Hz, *J = 18* Hz, 2H), 4.01-3.91(m, 1H), 3.84 - 3.68 (m, 3H), 3.13 - 3.05 (m, 3H), 3.03 - 2.96 (m, 1H), 2.93 - 2.77 (m, 2H), 2.28 - 1.88 (m, 6H), 1.87 - 1.65 (m, 6H). |
| 8-6 (Isomer2) | 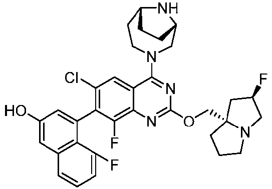 | 4-(2- { [(2R,7aS)-2-fluoro-hexahydro-1H-pyrrolizin-7a-yl]methoxy}-6-chloro-4-[(1S,6R)-3,9-diazabicyclo[4.2. 1]nonan-3-yl]-8-fluoroquinazolin-7-yl)-5-fluoronaphthalen-2-ol | 624.4 | (400 MHz, DMSO-d₆) *δ* ppm 10.24 (s, 1H), 7.93 (s, 1H), 7.65 (d, *J* = 6 Hz, 1H),7.45 - 7.37 (m, 1H), 7.33 (brs, 1H), 7.03 - 6.93 (m, 3H), 5.25 (d, *J* = 40 Hz, 1H), 4.37 (d, *J =* 12 Hz, 1H), 4.03 (ABq, J = 6 Hz, *J =* 18 Hz, 2H), 3.83 - 3.64 (m, 4H), 3.54 - 3.45 (m, 1H), 3.14 - 3.03 (m, 3H), 3.01 - 2.95 (m, 1H), 2.85 - 2.76 (m, 1H), 2.15 - 1.92 (m, 6H), 1.91 - 1.69 (m, 6H). |
| 8-7 (Isomer 1) | 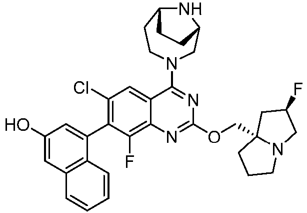 | 4-(2- { [(2R,7aS)-2-fluoro-hexahydro-1H-pyrrolizin-7a-yl]methoxy}-6-chloro-4-[(1S,6R)-3,9-diazabicyclo[4.2. 1]nonan-3-yl]-8-fluoroquinazolin-7-yl)naphthalen-2-ol | 606.5 | (400 MHz, DMSO-d₆) *δ* ppm 9.97 (br s, 1H), 8.07 (s, 1H), 7.86 (d, *J =* 8.4 Hz, 1H), 7.43 - 7.40 (m, 1H), 7.26 - 7.16 (m, 3H), 7.04 (d, *J* = 2.1 Hz, 1H), 5.24 (d, *J*= 40 Hz, 1H), 4.40 (br d, *J* = 12.6 Hz, 1H), 4.10 - 4.08 (m, 1H), 3.98-3.95 (m, 1H), 3.95 - 3.81 (m, 2H), 3.77 - 3.57 (m, 2H), 3.49 - 3.36 (m, 1H), 3.25 - 2.98 (m, 2H), 2.99 - 2.90 (m, 1H), 2.84-2.80 (m, 1H), 2.13 - 1.97 (m, 4H), 1.95 - 1.73 (m, 7H), 1.49 - 1.47 (m, 2H). |
| 8-8 (Isomer 2) | 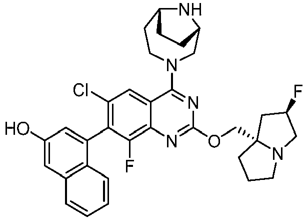 | 4-(2- { [(2R,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)naphthalen-2-ol | 606.5 | (400 MHz, DMSO-d₆) *δ* ppm 9.99 (br s, 1H), 8.01 (s, 1H), 7.80 (d, *J =* 8.4 Hz, 1H), 7.47 - 7.40 (m, 1H), 7.29 - 7.19 (m, 3H), 7.05 (d, *J* = 2.1 Hz, 1H), 5.26 (d, *J* = 40 Hz, 1H), 4.43 (br d, *J* = 12.6 Hz, 1H), 4.16 - 4.05 (m, 1H), 4.03 - 3.95 (m, 1H), 3.95 - 3.81 (m, 2H), 3.77 - 3.57 (m, 2H), 3.49 - 3.45 (m, 1H), 3.10 - 3.00 (m, 2H), 2.99 - 2.90 (m, 1H), 2.84-2.80 (m, 1H), 2.13 - 1.97 (m, 4H), 1.95 - 1.73 (m, 7H), 1.63 - 1.39 (m, 2H). |
| 8-9 (Isomer 1) | 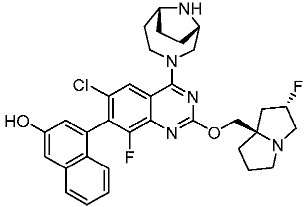 | 4-(2-{[(2*S*,7a*R*)-2-fluoro-hexahydro-1H-pyrrolizin-7a-yl]methoxy}-6-chloro-4-[(1S,6R)-3,9-diazabicyclo[4.2. 1]nonan-3-yl]-8-fluoroquinazolin-7-yl)naphthalen-2-ol | 606.2 | (400 MHz, DMSO-d₆) *δ* ppm 8.02 (s, 1H), 7.80 (d, *J* = 8.3 Hz, 1H), 7.47 - 7.38 (m, 1H), 7.28 (d, *J* = 2.0 Hz, 1H), 7.25 - 7.15 (m, 2H), 7.06 (d, *J*= 2.3 Hz, 1H), 5.38 - 5.07 (m, 1H), 4.42 (br d, *J=* 12.8 Hz, 1H), 4.10 - 3.97 (m, 2H), 3.96 - 3.80 (m, 2H), 3.73 - 3.62 (m, 2H), 3.49 - 3.36 (m, 1H), 3.11 - 3.04 (m, 2H), 3.00 (s, 1H), 2.85 - 2.78 (m, 1H), 2.15 - 1.96 (m, 4H), 1.94 - 1.68 (m, 7H), 1.58 - 1.37 (m, 2H) |
| 8-10 (Isomer 2) | 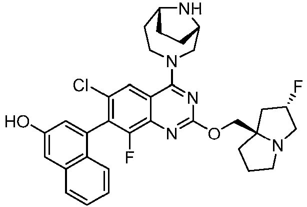 | 4-(2-{[(2*S*,7a*R*)-2-fluoro-hexahydro-1H-pyrrolizin-7a-yl]methoxy}-6-chloro-4-[(1S,6R)-3,9-diazabicyclo[4.2. 1]nonan-3-yl]-8-fluoroquinazolin-7-yl)naphthalen-2-ol | 606.2 | (400 MHz, DMSO-d6) *δ* ppm 8.02 (s, 1H), 7.80 (d, J = 8.5 Hz, 1H), 7.49 - 7.38 (m, 1H), 7.28 (d, *J* = 2.0 Hz, 1H), 7.22 *(br d, J = 4.0* Hz, 2H), 7.07 (d, *J* = 2.3 Hz, 1H), 5.37 - 5.09 (m, 1H), 4.51 - 4.38 (m, 1H), 4.12 - 3.98 (m, 2H), 3.89 (br d, *J=* 4.3 Hz, 2H), 3.73 - 3.61 (m, 2H), 3.48 - 3.45 (m, 1H), 3.12 - 3.04 (m, 2H), 3.00 (s, 1H), 2.87 - 2.76 (m, 1H), 2.12 - 1.97 (m, 4H), 1.94 - 1.71 (m, 7H), 1.49 (br d, *J* = 7.5 Hz, 2H) |

### Example 9-1

### 4-(2-{[(4aS,7aR)-1-methyl-octahydro-1H-cyclopenta[b]pyridin-4a-yl]methoxy)-4-[(1S, 6R)-3,9-diazabicyclo[4.2.1]nonan-3-yl]-8-fluoroquinazolin-7-yl)naphthalen-2-ol

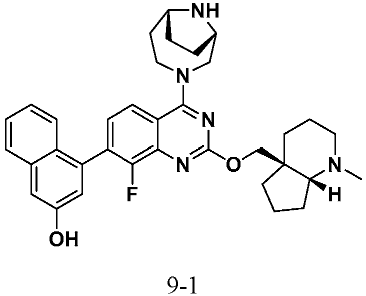

### Preparation of Intermediate 9A: tert-butyl (1S,6R)-3-(7-bromo-2-chloro-8-fluoroquinazolin-4-yl)-3,9-diazabicyclo[4.2. 1]nonane-9-carboxylate

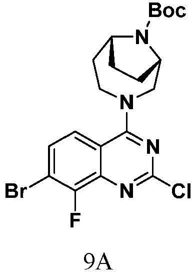

To a solution of 7-bromo-2,4-dichloro-8-fluoroquinazoline (250 mg, 0.845 mmol) and DIEA (148 µl, 0.845 mmol) in THF (30 mL) at 0 °C under nitrogen was added tert-butyl (1S,6R)-3,9-diazabicyclo[4.2.1]nonane-9-carboxylate (191 mg, 0.845 mmol) and the mixture was stirred at 0 °C for 1 hour and room temperature for 18 hours. The mixture was then concentrated. The mixture was then diluted with EtOAc (35 mL) and was washed with a solution of aqueous saturated sodium bicarbonate (2 x 35 mL). The ethyl acetate layer was dried over sodium sulfate, filtered and concentrated. The crude product was subjected to ISCO flash chromatography (silica gel/hexane-EtOAc 100:0 to 60:40 gradient) to yield tert-butyl (1S,6R)-3-(7-bromo-2-chloro-8-fluoroquinazolin-4-yl)-3,9-diazabicyclo[4.2.1]nonane-9-carboxylate (362 mg, 0.708 mmol, 84 % yield) as a white foam. LCMS (ESI) m/z: 486 [M+H]⁺LC retention time: 1.14 min (Waters Acquity UPLC BEH C18, 2.1 x 50 mm, 1.7 □m particles; Mobile Phase A: water with 0.05% TFA; Mobile Phase B: ACN with 0.05% TFA; Gradient: 2-98% B over 1 minute, then a 0.5 minute hold at 98% B; Flow: 0.8 mL/min; Detection: MS and UV (220 nm)).

### Preparation of Intermediate 9B: tert-butyl (1S,6R)-3-(7-bromo-2,8-difluoroquinazolin-4-yl)-3,9-diazabicyclo[4.2.1]nonane-9-carboxylate

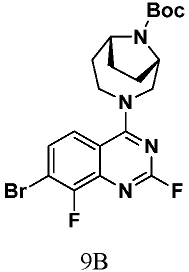

A mixture of tert-butyl (1S,6R)-3-(7-bromo-2-chloro-8-fluoroquinazolin-4-yl)-3,9-diazabicyclo[4.2.1]nonane-9-carboxylate (362 mg, 0.745 mmol) and potassium fluororide (87 mg, 1.490 mmol) in DMSO (5.0 mL) was stirred at 100 °C for 2 days. The mixture was then diluted with EtOAc (25 mL) and was washed with a solution of aqueous saturated sodium bicarbonate (2 x 25 mL). The ethyl acetate layer was dried over sodium sulfate, filtered and concentrated. The crude product was subjected to ISCO flash chromatography (silica gel/hexane-EtOAc 100:0 to 40:60 gradient) to yield tert-butyl (1S,6R)-3-(7-bromo-2,8-difluoroquinazolin-4-yl)-3,9-diazabicyclo[4.2.1]nonane-9-carboxylate (270 mg, 0.547 mmol, 73.3 % yield) as a white foam. LCMS (ESI) m/z: 470 [M+H]⁺LC retention time: 1.09 min (Waters Acquity UPLC BEH C18, 2.1 x 50 mm, 1.7 □m particles; Mobile Phase A: water with 0.05% TFA; Mobile Phase B: ACN with 0.05% TFA; Gradient: 2-98% B over 1 minute, then a 0.5 minute hold at 98% B; Flow: 0.8 mL/min; Detection: MS and UV (220 nm)).

### Preparation of Intermediate 9C: tert-butyl (1S,6R)-3-(7-bromo-8-fluoro-2-(((4aS,7aR)-1-methyloctahydro-4aH-cyclopenta[b]pyridin-4a-yl)methoxy)quinazolin-4-yl)-3,9-diazabicyclo[4. 2.1]nonane-9-carboxylate

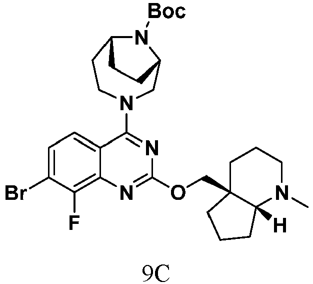

To a solution of ((4aS,7aR)-1-methyloctahydro-4aH-cyclopenta[b]pyridin-4a-yl)methanol (72.9 mg, 0.430 mmol) and tert-butyl (1S,6R)-3-(7-bromo-2,8-difluoroquinazolin-4-yl)-3,9-diazabicyclo[4.2.1]nonane-9-carboxylate (202 mg, 0.430 mmol) in THF (30 mL) at 0 °C under nitrogen was added lithium bis(trimethylsilyl)amide solution in THF (646 µl, 0.646 mmol), the mixture was stirred at 0 °C for 1 hour and room temperature for 18 hours. The mixture was then concentrated. The crude product was purified by chromatography (ISCO C18 100g column, flow rate = 60 mL/min., gradient = 20% A to 100%B in 20 min., A =H₂O/ ACN/TFA (90:10:0.1), B = H₂O/ ACN/TFA(10:90:0.1)). The pure fractions were combined. and concentrated to yield tert-butyl (1S,6R)-3-(7-bromo-8-fluoro-2-(((4aS,7aR)-1-methyloctahydro-4aH-cyclopenta[b]pyridin-4a-yl)methoxy)quinazolin-4-yl)-3,9-diazabicyclo[4.2.1]nonane-9-carboxylate as a white solid. LCMS (ESI) m/z: 619 [M+H]⁺. Retention time: 0.88 min (Waters Acquity UPLC BEH C18, 2.1 x 50 mm, 1.7 □m particles; Mobile Phase A: water with 0.05% TFA; Mobile Phase B: ACN with 0.05% TFA; Gradient: 2-98% B over 1 minute, then a 0.5 minute hold at 98% B; Flow: 0.8 mL/min; Detection: MS and UV (220 nm)).

### Example 9-1

### 4-(2-{[(4aS,7aR)-1-methyl-octahydro-1H-cyclopenta[b]pyridin-4a-yl]methoxy)-4-[(]S, 6R)-3,9-diazabicyclo[4.2. 1]nonan-3-yl]-8-fluoroquinazolin-7-yl)naphthalen-2-ol

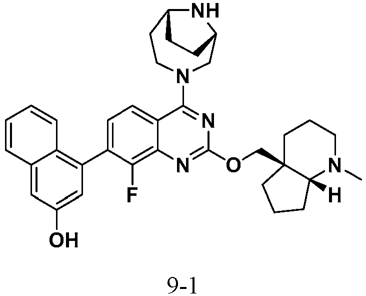

A mixture of tert-butyl (1S,6R)-3-(7-bromo-8-fluoro-2-(((4aS,7aR)-1-methyloctahydro-4aH-cyclopenta[b]pyridin-4a-yl)methoxy)quinazolin-4-yl)-3,9-diazabicyclo[4.2.1]nonane-9-carboxylate (20 mg, 0.032 mmol), 2-(3-(methoxymethoxy)naphthalen-1-yl)-4,4,5,5-tetramethyl-1,3,2-dioxaborolane (10.67 mg, 0.034 mmol), [1,1'-bis(di-tert-butylphosphino)ferrocene]dichloropalladium(II) (1.054 mg, 1.617 µmol) and 2.0 M potassium phosphate tribasic (48.5 µl, 0.097 mmol) in 1,4-dioxane (1 mL) under nitrogen was stirred at 50 °C for 18 hours. To the mixture was then added EtOAc (5 mL) and then the ethyl acetate layer was dried over sodium sulfate, filtered and concentrated to provide crude product. A solution of the crude material from above in DCM (0.6 mL), TES (1 drop) and TFA (0.4 mL) was stirred at room temperature for 30 min. The mixture was then concentrated. The crude product was purified by prep-HPLC (Phenomenex, Luna 5 micron 30 x 250 mm, flow rate = 30 mL/min., gradient = 20% A to 100%B in 12 min., A =H₂O/ ACN/TFA (90:10:0.1), B = H₂O/ ACN/TFA(10:90:0.1)). The combined pure fractions were loaded onto an Oasis MCX cation mixed-mode polymer cartridge (150 mg), the cartridge was washed with methanol (30 mL) and the product was eluted with 0.1 N ammonia in methanol (5.0 mL). The ammonia eluent was concentrated. The pure product was then lyophilized from ACN/H₂O (1:1, 5 mL) to yield the desired (9.15 mg, 0.015 mmol, 47.2 % yield) as a white powder. LCMS (ESI) m/z: 582 [M+H]+ LC retention time: 0.69 min (Waters Acquity UPLC BEH C18, 2.1 x 50 mm, 1.7 □m particles; Mobile Phase A: water with 0.05% TFA; Mobile Phase B: ACN with 0.05% TFA; Gradient: 2-98% B over 1 minute, then a 0.5 minute hold at 98% B; Flow: 0.8 mL/min; Detection: MS and UV (220 nm)). ¹H NMR (499 MHz, METHANOL-d₄) δ 7.99 (d, J=8.7 Hz, 1H), 7.75 (d, J=8.3 Hz, 1H), 7.51 - 7.40 (m, 2H), 7.30 - 7.21 (m, 3H), 7.12 (d, J=2.3 Hz, 1H), 4.77 - 4.61 (m, 1H), 4.50 (br d, J=11.2 Hz, 1H), 4.30 (d, J=10.7 Hz, 1H), 4.13 - 3.94 (m, 2H), 3.86 - 3.78 (m, 2H), 3.72 - 3.58 (m, 1H), 2.90 (br t, J=5.7 Hz, 1H), 2.69 (ddd, J=11.6, 7.4, 4.2 Hz, 1H), 2.41 - 2.32 (m, 4H), 2.26 - 2.08 (m, 3H), 2.07 - 1.93 (m, 2H), 1.90 - 1.69 (m, 10H), 1.67 (br s, 1H), 1.64 - 1.55 (m, 1H).

Examples in Table 5 were prepared according to procedures described for Example 9-1 from appropriate starting materials.

**Table 5**

| Example Number | Structure | Name | **MS** m/z: [M+H]+ | ¹H NMR |
|---|---|---|---|---|
| 9-2 | 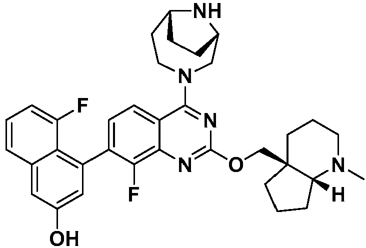 | 4-(2-{[(4aS,7aR)-1-methyl-octahydro-1H-cyclopenta[b]pyridin-4a-yl]methoxy}-4-[(1S,6R)-3,9-diazabicyclo[4.2.1]nona n-3-yl]-8-fluoroquinazolin-7-yl)-5-fluoronaphthalen-2-ol | 600 | ¹H NMR (499 MHz, MeOD) δ 7.93 (d, J=8.8 Hz, 1H), 7.57 (d, J=7.9 Hz, 1H), 7.37 (td, J=8.0, 4.9 Hz, 1H), 7.30 - 7.25 (m, 2H), 7.04 (d, J=2.4 Hz, 1H), 6.92 - 6.87 (m, 1H), 4.77 - 4.61 (m, 1H), 4.48 (dd, J=10.7, 1.0 Hz, 1H), 4.31 (dd, J=10.6, 4.3 Hz, 1H), 4.12 - 3.93 (m, 2H), 3.86 - 3.77 (m, 2H), 3.74 - 3.58 (m, 1H), 2.96 - 2.88 (m, 1H), 2.74 - 2.66 (m, 1H), 2.49 - 2.38 (m, 1H), 2.35 (d, J=4.4 Hz, 3H), 2.25 - 2.08 (m, 3H), 2.06 - 1.94 (m, 2H), 1.91 - 1.82 (m, 2H), 1.81 - 1.67 (m, 8H), 1.65 - 1.55 (m, 1H) |
| 9-3 | 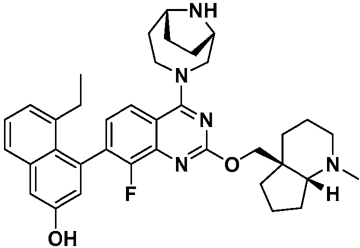 | 4-(2-{[(4aS,7aR)-1-methyl-octahydro-1H-cyclopenta[b]pyridin-4a-yl]methoxy}-4-[(1S,6R)-3,9-diazabicyclo[4.2.1]nona n-3-yl]-8-fluoroquinazolin-7-yl)-5-ethylnaphthalen-2-ol | 610 | ¹H NMR (499 MHz, METHAN OL-d₄) δ 7.97 - 7.85 (m, 1H), 7.66 - 7.57 (m, 1H), 7.39 - 7.30 (m, 1H), 7.28 - 7.09 (m, 3H), 6.97 - 6.88 (m, 1H), 4.77 - 4.55 (m, 1H), 4.53 - 4.44 (m, 1H), 4.34 - 4.22 (m, 1H), 4.14 - 3.92 (m, 2H), 3.87 - 3.75 (m, 2H), 3.73 - 3.54 (m, 1H), 2.97 - 2.81 (m, 1H), 2.74 - 2.62 (m, 1H), 2.48 - 2.36 (m, 3H), 2.35 - 2.31 (m, 3H), 2.25 - 1.92 (m, 6H), 1.90 - 1.54 (m, 10H), 0.94-0.84 (m, 3H) |
| 9-4 | 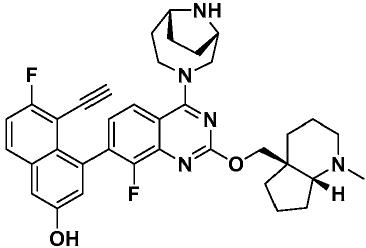 | 4-(2-{[(4aS,7aR)-1-methyl-octahydro-1H-cyclopenta[b]pyridin-4a-yl]methoxy}-4-[(1S,6R)-3,9-diazabicyclo[4.2.1]nona n-3-yl]-8-fluoroquinazolin-7-yl)-5-ethynyl-6-fluoronaphthalen-2-ol | 624 | ¹H NMR (499 MHz, METHAN OL-d₄) δ 7.97 - 7.79 (m, 2H), 7.34 - 7.28 (m, 2H), 7.27 - 7.20 (m, 1H), 7.16 - 7.07 (m, 1H), 4.78 - 4.26 (m, 3H), 4.17 - 3.75 (m, 5H), 3.05 - 2.87 (m, 1H), 2.80 - 2.67 (m, 1H), 2.55 - 2.42 (m, 1H), 2.41 - 2.33 (m, 3H), 2.29 - 1.54 (m, 16H) |

### Example 10-1

### 6-{6-chloro-4-[(1S,6R)-3, 9-diazabicyclo[4.2.1]nonan-3-yl]-2-({1-[(dimethylamino)methyl]cyclopropyl}methoxy)-8-fluoroquinazolin-7-yl}-4-methyl-5-(trifluoromethyl)pyridin-2-amine

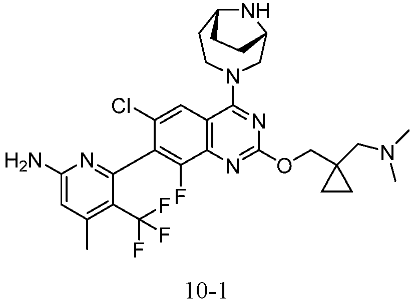

### Preparation of Intermediate 10A: {1-[(dimethylamino)methyl]cyclopropyl}methanol

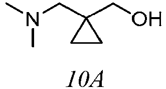

To a solution of methyl 1-(dimethylcarbamoyl)cyclopropane-1-carboxylate (8 g, 46.7 mmol) in THF (150 mL) was added LiAlH₄ (2.4M solution in THF) (38.9 mL, 93 mmol) slowly at 0 °C and the reaction mixture was stirred at room temperature for 4 h. The reaction was cooled and quenched with water (20 mL), 10% NaOH solution (40 mL) and water (40 mL) and extracted with ethyl acetate. The combined organic layers were washed with water, brine, dried over anhydrous Na₂SO₄, filtered and concentrated under reduced pressure to provide {1-[(dimethylamino)methyl]cyclopropyl}methanol (3.7 g, 28.6 mmol, 61.3 % yield) as a yellow liquid. ¹H NMR (300 MHz, CDCl₃) δ ppm 5.30 - 4.15 (m, 1H), 3.55 (s, 2H), 2.41 (s, 2H), 2.31 (s, 6H), 0.54 - 0.47 (m, 2H), 0.39 - 0.32 (m, 2H).

### Preparation of Intermediate 10B: tert-butyl 3-[7-(6-{bis[(4-methoxyphenyl)methyl]amino}-4-methyl-3-(trifluoromethyl)pyridin-2-yl)-6-chloro-2-({1-[(dimethylamino)methyl]cyclopropyl}methoxy)-8-fluoroquinazolin-4-yl]-3,8-diazabicyclo[3.2.1]octane-8-carboxylate

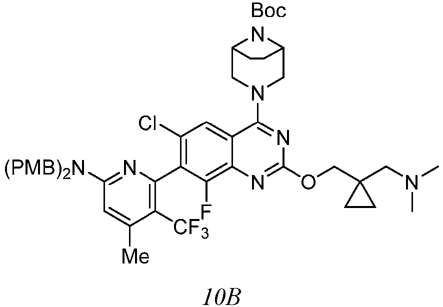

To a solution of {1-[(dimethylamino)methyl]cyclopropyl}methanol in THF (2 mL) at 0 °C was added NaH (29.1 mg, 0.727 mmol) and the mixture was stirred for 30 minutes at the same temperature. Then, Intermediate 3K was added and the mixture was gradually warmed up to room temperature over a period of 2 h. The reaction mixture was then quenched with saturated aq. ammonium chloride solution and extracted with ethyl acetate. The combined organic layers were washed with water, brine, dried over anhydrous Na₂SO₄ filtered and concentrated under reduced pressure to provide a crude residue, which was purified by silica gel column chromatography using a CombiFlash (40 g RediSep^{®} column, 50 to 60% EtOAc - pet. ether) to afford tert-butyl 3-[7-(6-{bis[(4-methoxyphenyl)-methyl]amino}-4-methyl-3-(trifluoromethyl)pyridin-2-yl)-6-chloro-2-({1-[(dimethylamino)methyl cyclopropyl}- methoxy)-8-fluoroquinazolin-4-yl]-3,8-diazabicyclo[3.2.1]octane-8-carboxylate (250 mg, 0.268 mmol, 73.6 % yield) as a pale yellow solid. MS(ESI) m/z: 934.3 (M+H)⁺.

### Preparation of Intermediate 10C: 7-(6-{bis[(4-methoxyphenyl)methyl]amino}-4-methyl-3-(trifluoromethyl)pyridin-2-yl)-6-chloro-2-({1-[(dimethylamino)methyl]cyclopropyl)methoxy)-8-fluoroquinazolin-4-ol

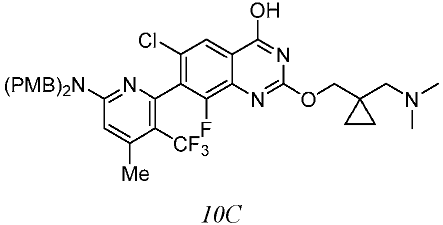

To a solution of tert-butyl 3-[7-(6-{bis[(4-methoxyphenyl)-methyl]amino}-4-methyl-3-(trifluoromethyl)pyridin-2-yl)-6-chloro-2-({1-[(dimethylamino)methyl]cyclopropyl}-methoxy)-8-fluoroquinazolin-4-yl]-3,8-diazabicyclo[3.2.1]octane-8-carboxylate (150 mg, 0.161 mmol) in ethanol (2 mL) and THF (2 mL) was added NaOH (1M aq. soln.) (1.605 mL, 1.605 mmol) and the reaction mixture was stirred at 70 °C for 16 h. The reaction mixture was then concentrated and the crude residue was dissolved in ethyl acetate, washed with brine and concentrated under reduced pressure to provide a crude residue of 7-(6-{bis[(4-methoxyphenyl)methyl]amino}-4-methyl-3-(trifluoromethyl)pyridin-2-yl)-6-chloro-2-({1-[(dimethylamino)methyl]cyclopropyl}methoxy)-8-fluoroquinazolin-4-ol (70 mg, 0.095 mmol, 58.9 % yield). MS(ESI) m/z: 740.2 (M+H)⁺.

### Preparation of Intermediate 10D: tert-butyl (1S,6R)-3-[7-(6-{bis[(4-methoxyphenyl)methyl]amino)-4-methyl-3-(trifluoromethyl)pyridin-2-yl)-6-chloro-2-({1[(dimethylamino)methyl]cyclopropyl}methoxy)-8-fluoroquinazolin-4-yl]-3,9-diazabicyclo[4.2.1]nonane-9-carboxylate

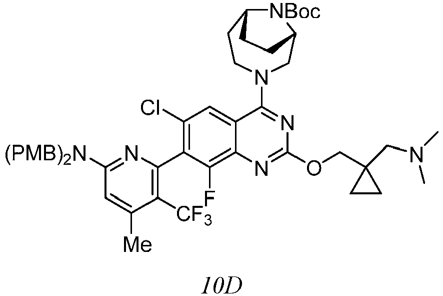

To a solution of 7-(6-{bis[(4-methoxyphenyl)methyl]amino}-4-methyl-3-(trifluoromethyl)pyridin-2-yl)-6-chloro-2-({1-[(dimethylamino)methyl]cyclopropyl}methoxy)-8-fluoroquinazolin-4-ol (70 mg, 0.095 mmol), tert-butyl (1S,6R)-3,9-diazabicyclo[4.2.1]nonane-9-carboxylate (25.7 mg, 0.113 mmol), and BOP (62.7 mg, 0.142 mmol) in ACN (3 mL) was added TEA (0.026 mL, 0.189 mmol) and the reaction mixture was stirred at 80 °C for 8 h. The reaction mixture was then concentrated under reduced pressure. The crude compound was purified by silica gel column chromatography to afford tert-butyl (1S,6R)-3-[7-(6-{bis[(4-methoxyphenyl)methyl]amino}-4-methyl-3-(trifluoromethyl)pyridin-2-yl)-6-chloro-2-({1-[(dimethylamino)methyl]cyclopropyl}methoxy)-8-fluoroquinazolin-4-yl]-3,9-diazabicyclo[4.2.1]nonane-9-carboxylate (70 mg, 0.074 mmol, 78 % yield). MS(ESI) m/z: 948.2 (M+H)⁺.

### Example 10-1

### 6-{6-chloro-4-[(1S,6R)-3,9-diazabicyclo[4.2.1[nonan-3-yl]-2-({1-[(dimethylamino)methyl]cyclopropyl)methoxy)-8-fluoroquinazolin-7-yl)-4-methyl-5-(trifluoromethyl)pyridin-2-amine

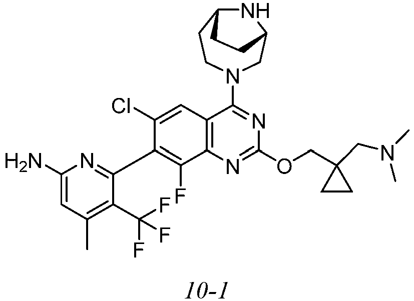

A stirred solution of TFA (1.5 mL, 19.47 mmol) and triethylsilane (0.5 mL, 3.13 mmol) was added to tert-butyl (1S,6R)-3-[7-(6-{bis[(4-methoxyphenyl)methyl]amino}-4-methyl-3-(trifluoromethyl)pyridin-2-yl)-6-chloro-2-({ 1-[(dimethylamino)methyl]cyclopropyl } methoxy)-8-fluoroquinazolin-4-yl]-3,9-diazabicyclo[4.2.1]nonane-9-carboxylate (70 mg, 0.074 mmol) at room temperature and the resulting reaction mixture was heated at 40 °C over a period of 24 h. Then, the reaction mixture was concentrated under reduced pressure, co-distilled with toluene (twice), neutralised with DIPEA and concentrated under reduced pressure to provide the crude residue, which was purified by preparative HPLC (Column/dimensions: Column Name: Waters XBridge C18 (150mm x 19 mm ID, 5µ); Mobile phase A= 10mM Ammonium acetate, Mobile phase B= Acetonitrile; Flow Rate: 20 mL/min; Retention time = 1.748 min) to afford 6-{6-chloro-4-[(1S,6R)-3,9-diazabicyclo[4.2.1]nonan-3-yl]-2-({1-[(dimethylamino)methyl]cyclopropyl}methoxy)-8-fluoroquinazolin-7-yl}-4-methyl-5-(trifluoromethyl)pyridin-2-amine (2.4 mg, 3.68 µmol, 4.98 % yield). LCMS (ESI) m/z: 608.2 [M+H]⁺. ¹H NMR (400 MHz, DMSO-d₆) δ ppm 7.87 (s, 1H), 6.85 (s, 2H), 6.50 (s, 1H), 5.36 - 5.01 (m, 1H), 4.40 - 4.32 (m, 1H), 4.28 - 4.17 (m, 2H), 4.13 - 4.06 (m, 1H), 4.03 - 3.84 (m, 2H), 3.79 - 3.62 (m, 2H), 2.38 (m, 4H), 2.32 - 2.27 (m, 2H), 2.21 (m, 7H), 1.91 (m, 3H), 1.72 - 1.60 (m, 1H), 0.64 (m, 2H), 0.46 - 0.38 (m, 2H).

Examples in Table 6 were prepared according to procedures described for Example 10-1 from appropriate starting materials.

**Table 6**

| Example Number | Structure | Name | MS *m*/*z*: [M+H]+ | ¹H NMR |
|---|---|---|---|---|
| 10-2 | 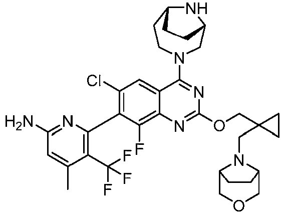 | 6-{6-chloro-4-[(1S,6R)-3,9-diazabicyclo [4.2.1]nona n-3-yl]-8-fluoro-2-{[1-({3-oxa-8-azabicyclo[ 3.2.1]octan-8-yl}methyl)c yclopropyl] methoxy}qu inazolin-7-yl}-4-methyl-5-(trifluorome thyl)pyridin -2-amine | 676.3 | ¹H NMR (500 MHz, DMSO-d₆) δ ppm: 7.88 (s, 1H), 6.86 (s, 2H), 6.49 (s, 1H), 4.41 - 4.29 (m, 3H), 3.90 - 3.75 (m, 2H), 3.68 - 3.62 (m, 2H), 3.49 - 3.38 (m, 5H), 3.10 - 3.03 (m, 2H), 2.40 - 2.34 (m, 4H), 2.31 - 2.27 (m, 2H), 2.07 - 1.98 (m, 1H), 1.94 - 1.83 (m, 2H), 1.82 - 1.71 (m, 3H), 1.70 - 1.63 (m, 2H), 1.50 - 1.35 (m, 2H), 0.61 - 0.53 (m, 2H), 0.46 - 0.39 (m, 2H) |
| 10-3 | 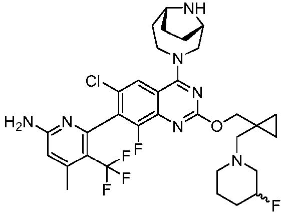 | 6-{6-chloro-4-[(1S,6R)-3,9-diazabicyclo [4.2.1]nona n-3-yl]-8-fluoro-2-({1-[(3-fluoropiperi din-1-yl)methyl]c yclopropyl} methoxy)quinazolin-7-yl}-4-methyl-5-(trifluorome thyl)pyridin -2-amine | 666.3 | ¹H NMR (400 MHz, DMSO-d₆) δ ppm: 7.88 (s, 1H), 6.89 - 6.82 (m, 2H), 6.50 (s, 1H), 5.30 - 4.98 (m, 1H), 4.65 - 4.44 (m, 1H), 4.42 - 4.15 (m, 3H), 3.91 - 3.63 (m, 3H), 3.01 - 2.73 (m, 1H), 2.41 - 2.27 (m, 6H), 2.22 - 2.12 (m, 2H), 2.08 - 1.99 (m, 2H), 1.93 - 1.73 (m, 2H), 1.71 - 1.59 (m, 2H), 1.53 - 1.31 (m, 4H), 1.24 (s, 2H), 0.67 - 0.59 (m, 2H), 0.44 - 0.36 (m, 2H) |
| 10-4 | 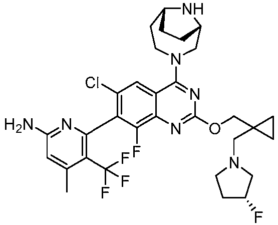 | 6-{6-chloro-4-[(1S,6R)-3,9-diazabicyclo [4.2.1]nona n-3-yl]-8-fluoro-2-[(1-{[(3R)-3-fluoropyrrol idin-1-yl]methyl}c yclopropyl) methoxy]qu inazolin-7-yl}-4-methyl-5-(trifluorome thyl)pyridin -2-amine | 652.3 | ¹H NMR (400 MHz, DMSO-d₆) δ ppm: 7.87 (s, 1H), 6.83 (s, 2H), 6.49 (s, 1H), 5.28 - 5.02 (m, 1H), 4.29 - 4.11 (m, 2H), 2.91 - 2.80 (m, 2H), 2.64 - 2.54 (m, 1H), 2.82 - 2.77 (m, 3H), 2.37 (s, 3H), 2.36 - 2.33 (m, 2H), 2.22 - 1.99 (m, 8H), 1.78 - 1.72 (m, 5H), 0.60 (br s, 2H), 0.42 (s, 2H). |
| 10-5 | 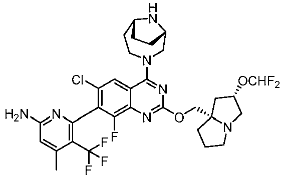 | 6-(2-{[(2S,7aS)-2-(difluoromet hoxy)-hexahydro-1H-pyrrolizin-7a-yl]methoxy} -6-chloro-4-[(1 S,6R)-3,9-diazabicyclo [4.2.1]nona n-3-yl]-8-fluoroquina zolin-7-yl)-4-methyl-5-(trifluorome thyl)pyridin -2-amine | 686.2 | ¹H NMR (400 MHz, DMSO-d₆) δ ppm: 7.88 (d, J = 1.3 Hz, 1H), 6.85 - 6.83 (m, 2H), 6.66 (br t, J = 69.6 Hz, 1H), 6.47 (s, 1H), 4.83 - 4.80 (m, 1H), 4.19 - 4.02 (m, 2H), 3.06 - 2.88 (m, 7H) 2.51 - 2.55 (m, 1H), 2.37 (s, 3H), 2.22 - 2.10 (m, 6H), 2.01 - 1.89 (m, 5H), 1.81 - 1.62 (m, 4H). |
| 10-6 | 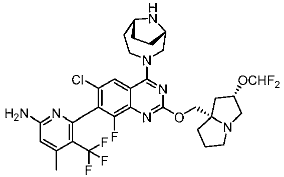 | 6-(2-{[(2S,7aS)-2-(difluoromet hoxy)-hexahydro-1H-pyrrolizin-7a-yl]methoxy} -6-chloro-4-[(1S,6R)-3,9-diazabicyclo [4.2.1]nona n-3-yl]-8-fluoroquina zolin-7-yl)-4-methyl-5-(trifluorome thyl)pyridin -2-amine | 686.2 | ¹H NMR (400 MHz, DMSO-d₆) δ ppm: 7.88 (d, J = 1.3 Hz, 1H), 6.85 - 6.83 (m, 2H), 6.66 (br t, J = 69.6 Hz, 1H), 6.47 (s, 1H), 4.92 - 4.73 (m, 1H), 4.22 - 4.02 (m, 2H), 3.09 - 2.81 (m, 7H), 2.63 - 2.54 (m, 1H), 2.37 (s, 3H), 2.20 - 1.88 (m, 6H), 2.03 - 1.90 (m, 5H),1.88 - 1.60 (m, 4H). |
| 10-7 | 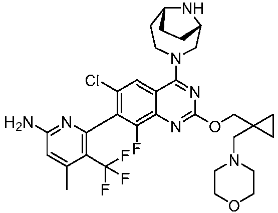 | 6-{6-chloro-4-[(1S,6R)-3,9-diazabicyclo [4.2.1]nona n-3-yl]-8-fluoro-2-({ 1-[(morpholin -4-yl)methyl]c yclopropyl} methoxy)qu inazolin-7-yl}-4-methyl-5-(trifluorome thyl)pyridin -2-amine | 650.3 | ¹H NMR (400 MHz, DMSO-d₆) δ ppm: 7.89 (s, 1H), 6.84 (br s, 2H), 6.50 (s, 1H), 5.36 (br s, 1H), 5.07 - 4.97 (m, 1H), 4.28 - 4.18 (m, 2H), 3.53 - 3.51 (m, 8H), 2.42 - 2.33 (m, 8H), 2.30 (br d, J = 3.0 Hz, 2H), 2.18 - 1.84 (m, 4H), 1.69 - 1.28 (m, 1H), 0.62 (br s, 2H), 0.40 (s, 2H) |
| 10-8 | 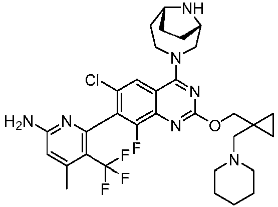 | 6-{6-chloro-4-[(1S,6R)-3,9-diazabicyclo [4.2.1]nona n-3-yl]-8-fluoro-2-({ 1-[(piperidin-1-yl)methyl]c yclopropyl} methoxy)qu inazolin-7-yl}-4-methyl-5-(trifluorome thyl)pyridin -2-amine | 648.1 | ¹H NMR (400 MHz, DMSO-d₆) δ ppm: 7.89 (s, 1H), 6.85 (s, 2H), 6.50 (s, 1H), 5.30 - 4.99 (m, 1H), 4.39 (br d, J = 12.0 Hz, 1H), 4.28 - 4.14 (m, 2H), 3.94 - 3.74 (m, 2H), 3.72 - 3.62 (m, 2H), 3.48 - 3.40 (m, 2H), 2.99 - 2.81 (m, 2H), 2.42 - 2.22 (m, 8H), 2.04 - 1.69 (m, 3H), 1.51 - 1.27 (m, 7H), 0.65 - 0.52 (m, 2H), 0.42 - 0.34 (m, 2H) |
| 10-9 | 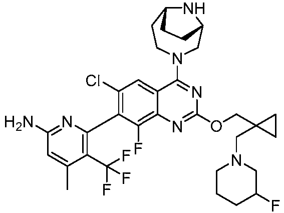 | 6-{6-chloro-4-[(1S,6R)-3,9-diazabicyclo [4.2.1]nona n-3-yl]-8-fluoro-2-({1-[(3-fluoropiperi din-1-yl)methyl]c yclopropyl} methoxy)qu inazolin-7-yl}-4-methyl-5-(trifluorome thyl)pyridin -2-amine | 666.2 | NMR data not available |
| 10-10 | 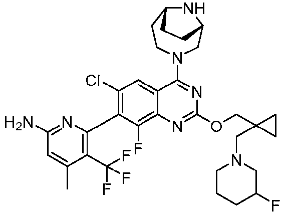 | 6-{6-chloro-4-[(1S,6R)-3,9-diazabicyclo [4.2.1]nona n-3-yl]-8-fluoro-2-({1-[(3-fluoropiperi din-1-yl)methyl]c yclopropyl} methoxy)qu inazolin-7-yl}-4-methyl-5-(trifluorome thyl)pyridin -2-amine | 666.2 | ¹H NMR (400 MHz, DMSO-d₆) δ ppm: 7.87 (s, 1H), 6.84 (s, 2H), 6.49 (s, 1H), 4.65 - 4.44 (m, 1H), 4.37 (br d, J = 12.5 Hz, 1H), 4.29 (d, J = 11.0 Hz, 1H), 4.19 (d, J = 10.8 Hz, 1H), 3.95 - 3.85 (m, 1H), 3.80 - 3.71 (m, 3H), 3.49 (br d, J = 14.3 Hz, 1H), 2.85 - 2.75 (m, 1H), 2.56 - 2.52 (m, 1H), 2.48 - 2.43 (m, 1H), 2.37 (br d, J = 1.3 Hz, 3H), 2.35 - 2.24 (m, 3H), 2.23 - 2.13 (m, 1H), 2.11 - 2.02 (m, 1H), 1.99 - 1.91 (m, 1H), 1.88 - 1.72 (m, 2H), 1.70 - 1.59 (m, 1H), 1.56 - 1.30 (m, 4H), 0.67 - 0.59 (m, 2H), 0.45 - 0.35 (m, 2H) |
| 10-11 | 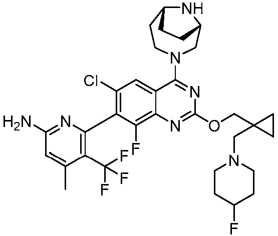 | 6-{6-chloro-4-[(1S,6R)-3,9-diazabicyclo [4.2.1]nona n-3-yl]-8-fluoro-2-({1-[(4-fluoropiperi din-1-yl)methyl]c yclopropyl} methoxy)qu inazolin-7-yl}-4-methyl-5-(trifluorome thyl)pyridin-2-amine | 666.3 | ¹H NMR (400 MHz, DMSO-d₆) δ ppm: 7.89 - 7.86 (m, 1H), 6.84 (s, 2H), 6.49 (s, 1H), 4.71 - 4.50 (m, 1H), 4.42 - 4.34 (m, 1H), 4.25 - 4.23 (m, 2H), 3.93 - 3.73 (m, 2H), 3.71 - 3.61 (m, 2H), 3.46 - 3.36 (m, 1H), 2.59 - 2.53 (m, 1H), 2.40 - 2.22 (m, 9H), 2.07 - 1.96 (m, 1H), 1.85 - 1.70 (m, 4H), 1.67 - 1.52 (m, 3H), 1.51 - 1.38 (m, 2H), 0.66 - 0.59 (m, 2H), 0.42 - 0.36 (m, 2H) |

### Example 11-1

### 4-{4-[(1S,6R)-3,9-diazabicyclo[4.2.1]nonan-3-yl]-2-({1-[(dimethylamino)methyl]cyclopropyl}methoxy)-8-fluoroquinazolin-7-yl}-5-ethynyl-6-fluoronaphthalen-2-ol

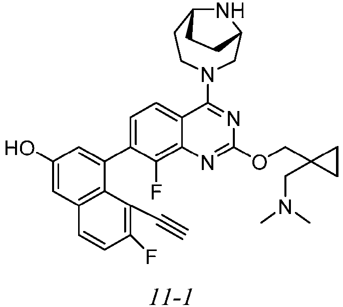

### Preparation of Intermediate 11A: tert-butyl (1S,6R)-3-[7-bromo-2-({1-[(dimethylamino)methyl|cyclopropyl}methoxy)-8-fluoroquinazolin-4-yl]-3,9-diazabicyclo[4.2.1]nonane-9-carboxylate

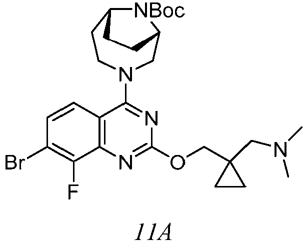

To a solution of (1-((dimethylamino)methyl)cyclopropyl)methanol (186 mg, 1.441 mmol) in THF (2 mL) was added NaH (57.6 mg, 1.441 mmol) slowly at 0 °C and the reaction mixture was stirred at the same temperature for 30 minutes. Then, tert-butyl (1S,6R)-3-(7-bromo-2-chloro-8-fluoroquinazolin-4-yl)-3,9-diazabicyc10[4.2.1]nonane-9-carboxylate (350 mg, 0.720 mmol) was added and the mixture was slowly warmed to room temperature and stirred for 2 h. The reaction mixture was then quenched with saturated ammonium chloride solution and extracted with ethyl acetate. The combined organic layers were washed with water and brine, dried over Na₂SO₄ filtered and concentrated to provide the crude product tert-butyl (1S,6R)-3-(7-bromo-2-((1-((dimethylamino)methyl)cyclopropyl)methoxy)-8-fluoroquinazolin-4-yl)-3,9-diazabicyclo[4.2.1]nonane-9-carboxylate (350 mg, 0.605 mmol, 84 % yield). MS(ESI) m/z: 580.1 (M+H+2).

### Preparation of Intermediate 11B: tert-butyl (1S,6R)-3-[2-({1-[(dimethylamino)methyl]cyclopropyl}methoxy)-8-fluoro-7-[7-fluoro-3-(methoxymethoxy)-8-{2-[tris(propan-2-yl)silyl]ethynyl}naphthalen-1-yl]quinazolin-4-yl]-3,9-diazabicyclo[4.2.1]nonane-9-carboxylate.

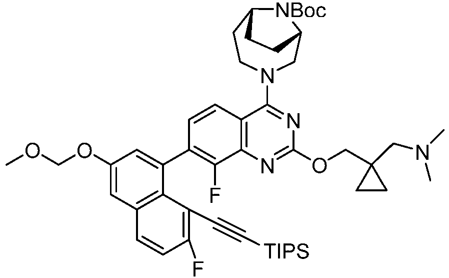

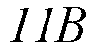

To a degassed solution of tert-butyl (1S,6R)-3-(7-bromo-2-((1-((dimethylamino)methyl)cyclopropyl)methoxy)-8-fluoroquinazolin-4-yl)-3,9-diazabicyclo[4.2.1]nonane-9-carboxylate (300 mg, 0.519 mmol), ((2-fluoro-6-(methoxymethoxy)-8-(4,4,5,5-tetramethyl-1,3,2-dioxaborolan-2-yl)naphthalen-1-yl)ethynyl)triisopropylsilane (319 mg, 0.622 mmol) and Na₂CO₃ (0.778 mL, 1.556 mmol) in 1,4-dioxane (5 mL) was added bis(triphenylphosphine)palladium(II) chloride (36.4 mg, 0.052 mmol) and the reaction mixture was heated at 100 °C for 2h. The reaction was then diluted with water and extracted with ethyl acetate. The combined organic layers were washed with water and brine, dried over Na₂SO₄ filtered and concentrated to provide the crude residue which was purified by silicagel column chromatography to afford tert-butyl (1S,6R)-3-(2-((1-((dimethylamino)methyl)cyclopropyl)methoxy)-8-fluoro-7-(7-fluoro-3-(methoxymethoxy)-8-((triisopropylsilyl)ethynyl)naphthalen-1-yl)quinazolin-4-yl)-3,9-diazabicyclo[4.2.1]nonane-9-carboxylate (130 mg, 0.147 mmol, 28.4 % yield). MS (ESI) m/z: 884.5 (M+1).

### Example 11-1

### 4-{4-[(1S,6R)-3,9-diazabicyclo[4.2.1]nonan-3-yl]-2-({1-[(dimethylamino)methy]cyclopropyl}methoxy)-8-fluoroquinazolin-7-yl}-5-ethynyl-6-fluoronaphthalen-2-ol

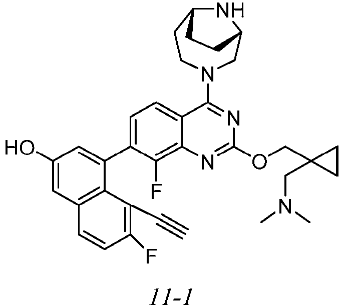

To a solution of 4-(4-((1S,6R)-3,9-diazabicyclo[4.2.1]nonan-3-yl)-2-((1-((dimethylamino)methyl)cyclopropyl)methoxy)-8-fluoroquinazolin-7-yl)-6-fluoro-5-((triisopropylsilyl)ethynyl)naphthalen-2-ol (120 mg, 0.162 mmol) in DMF (3 mL) was added CsF (123 mg, 0.811 mmol) at room temperature and the reaction mixture was stirred at 55 °C for 24 h. The reaction mixture was then concentrated to provide the crude residue. The crude material was purified by prep-HPLC (Conditions: Column - Waters XBridge C18 (19 x 150 mm, 5-µm particles); Mobile Phase A: 10-mM ammonium acetate; Mobile Phase B: acetonitrile; Gradient: 10-35% B over 20 minutes, then a 5-minute hold at 100% B; Flow: 20 mL/min) followed by SFC to afford 4-(4-((1S,6R)-3,9-diazabicyclo[4.2.1]nonan-3-yl)-2-((1-((dimethylamino)methyl)cyclopropyl)methoxy)-8-fluoroquinazolin-7-yl)-5-ethynyl-6-fluoronaphthalen-2-ol (0.9 mg, 1.388 µmol, 0.856 % yield). MS (ESI) m/z: 584.3 [M+H]⁺.

Examples in Table 7 were prepared according to procedures described for Example 11-1 from Intermediate 9A and the appropriate starting materials.

**Table 7**

| Example Number | Structure | Name | LCMS (M+H)⁺ | ¹H NMR |
|---|---|---|---|---|
| 11-2 | 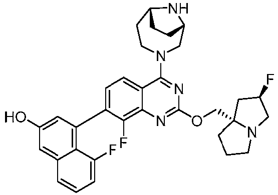 | 4-(2-{[(2R,7aS)-2-fluoro-hexahydro-1H-pyrrolizin-7a-yl]methoxy}-4-[(1S,6R)-3,9-diazabicyclo[4.2 .1]nonan-3-yl]-8-fluoroquinazolin -7-yl)-5-fluoronaphthale n-2-ol | 590.5 | ¹H NMR (400 MHz, DMSO-d₆) δ ppm: 10.15 (s, 1H), 7.87 (d, J = 8.8 Hz, 1H), 7.64 (d, J = 8.3 Hz, 1H), 7.45 - 7.37 (m, 1H), 7.31 (s, 1H), 7.28 - 7.23 (m, 1H), 7.02 (d, J = 2.4 Hz, 1H), 7.00 - 6.94 (m, 1H), 5.36 - 5.18 (m, 1H), 4.55 - 4.46 (m, 1H), 4.13 - 4.08 (m, 1H), 4.02 - 3.96 (m, 1H), 3.91 - 3.83 (m, 2H), 3.72 - 3.64 (m, 2H), 3.11 - 3.05 (m, 2H), 3.04 - 2.95 (m, 2H), 2.86 - 2.76 (m, 2H), 2.17-1.92 (m, 6H), 1.85 - 1.73 (m, 4H), 1.58 - 1.38 (m, 2H). |
| 11-3 | 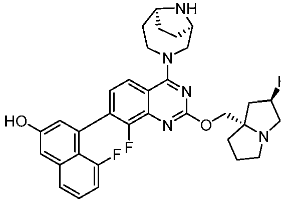 | 4-(2-{[(2R,7aS)-2-fluoro-hexahydro-1H-pyrrolizin-7a-yl]methoxy}-4-[(1R,6S)-3,9-diazabicyclo[4.2 .1]nonan-3-yl]-8-fluoroquinazolin -7-yl)-5-fluoronaphthale n-2-ol | 590.4 | ¹H NMR (400 MHz, DMSO-d₆) δ ppm: 7.87 (d, J = 8.9 Hz, 1H), 7.63 (d, J = 8.4 Hz, 1H), 7.44 - 7.36 (m, 1H), 7.31 - 7.23 (m, 2H), 7.02 - 6.94 (m, 2H), 5.36 - 5.18 (m, 1H), 4.55 - 4.46 (m, 1H), 4.09 - 4.05 (m, 1H), 4.03 - 3.98 (m, 1H), 3.89 - 3.82 (m, 2H), 3.68 - 3.64 (m, 2H), 3.10 - 3.06 (m, 3H), 3.00 (br s, 1H), 2.85 - 2.78 (m, 2H), 2.14 - 1.97 (m, 6H), 1.84 - 1.71 (m, 4H), 1.52 - 1.42 (m, 2H). |
| 11-3 | 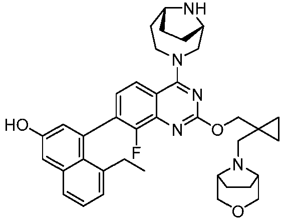 | 4-{4-[(1S,6R)-3,9-diazabicyclo[4.2 .1]nonan-3-yl]-8-fluoro-2-{[1-({3-oxa-8-azabicyclo[3.2.1 ]octan-8-yl}methyl)cyclo propyl]methoxy }quinazolin-7-yl}-5-ethylnaphthalen -2-ol | 638.3 | ¹H NMR (400 MHz, DMSO-d₆) δ ppm: 9.97 (br s, 1H), 7.87 (br d, J = 8.6 Hz, 1H), 7.66 (d, J = 8.3 Hz, 1H), 7.36 (t, J = 7.7 Hz, 1H), 7.28 - 7.18 (m, 2H), 7.11 (br d, J = 6.5 Hz, 1H), 6.90 (t, J = 2.3 Hz, 1H), 4.60 - 4.23 (m, 3H), 3.95 - 3.80 (m, 1H), 3.73 - 3.62 (m, 2H), 3.54 - 3.37 (m, 5H), 3.11 - 3.03 (m, 2H), 2.38 - 2.30 (m, 4H), 2.09 - 1.87 (m, 2H), 1.84 - 1.73 (m, 3H), 1.72 - 1.63 (m, 2H), 1.55 - 1.36 (m, 1H), 1.30 - 1.12 (m, 3H), 1.10 - 0.96 (m, 1H), 0.90 - 0.76 (m, 3H), 0.62 - 0.55 (m, 2H), 0.47 - 0.39 (m, 2H) |
| 11-4 | 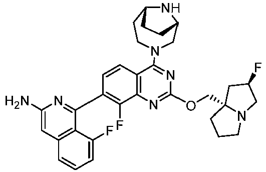 | 1-(2-{[(2R,7aS)-2-fluoro-hexahydro-1H-pyrrolizin-7a-yl]methoxy}-4-[(1S,6R)-3,9-diazabicyclo[4.2 .1]nonan-3-yl]-8-fluoroquinazolin -7-yl)-8-fluoroisoquinoli n-3-amine | 590.3 | ¹H NMR (400 MHz, DMSO-d₆) δ ppm: 7.97 - 7.88 (m, 1H), 7.50 - 7.41 (m, 2H), 7.30-7.28 (m, 1H), 6.86 - 6.73 (m, 2H), 6.30 (s, 2H), 5.38 - 5.20 (m, 2H), 5.14 - 5.06 (m, 1H), 4.11 - 4.09 (m, 1H), 4.00 - 3.94 (m, 1H), 3.12 - 2.95 (m, 7H), 2.87 - 2.80 (m, 2H), 2.38 - 2.26 (m, 2H), 2.18 - 1.93 (m, 6H), 1.87 - 1.56 (m, 4H) |
| 11-5 | 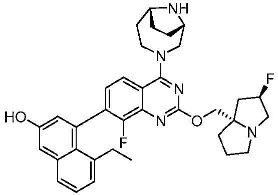 | 4-(2-{[(2R,7aS)-2-fluoro-hexahydro-1H-pyrrolizin-7a-yl]methoxy}-4-[(1S,6R)-3,9-diazabicyclo[4.2 .1]nonan-3-yl]-8-fluoroquinazolin -7-yl)-5-ethylnaphthalen -2-ol | 600.1 | ¹H NMR (400 MHz, DMSO-d₆) δ ppm: 7.88 (br d, J = 8.8 Hz, 1H), 7.66 (d, J = 8.0 Hz, 1H), 7.43 - 7.32 (m, 1H), 7.30 - 7.18 (m, 2H), 7.11 (dd, J = 1.1, 7.1 Hz, 1H), 6.90 (t, J = 2.4 Hz, 1H), 5.39 - 5.14 (m, 1H), 4.61 - 4.37 (m, 1H), 4.11 (dd, J = 1.3, 10.3 Hz, 1H), 4.00 (dd, J = 2.6, 10.4 Hz, 1H), 3.94 - 3.81 (m, 1H), 3.74 - 3.64 (m, 1H), 3.51 - 3.41 (m, 2H), 3.14 - 2.97 (m, 3H), 2.87 - 2.74 (m, 1H), 2.39 - 2.29 (m, 3H), 2.19 - 1.87 (m, 7H), 1.85 - 1.70 (m, 4H), 1.55 - 1.36 (m, 2H), 0.81 (t, J = 7.4 Hz, 3H) |
| 11-6 | 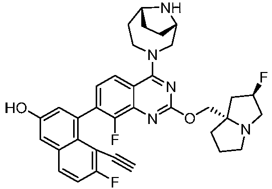 | 4-(2-{[(2R,7aS)-2-fluoro-hexahydro-1H-pyrrolizin-7a-yl]methoxy}-4-[(1S,6R)-3,9-diazabicyclo[4.2 .1]nonan-3-yl]-8-fluoroquinazolin -7-yl)-5-ethynyl-6-fluoronaphthale n-2-ol | 614.2 | ¹H NMR (400 MHz, DMSO-d₆) δ ppm: 7.93-7.91 (m, 1H), 7.89 - 7.77 (m, 1H), 7.44 (t, J = 8.9 Hz, 1H), 7.36 - 7.28 (m, 1H), 7.23 - 7.03 (m, 2H), 5.35 - 5.21 (m, 1H), 4.14 - 4.05 (m, 1H), 4.05 - 3.77 (m, 4H), 3.68-3.59 (m, 2H), 3.15 - 3.05 (m, 4H), 2.89 - 2.69 (m, 1H), 2.15 - 2.11 (m, 1H), 2.08 - 1.91 (m, 7H), 1.88 - 1.74 (m, 4H), 1.57 - 1.36 (m, 2H) |
| 11-7 | 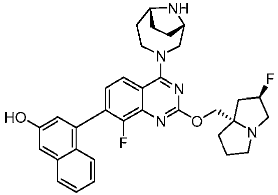 | 4-(2-{[(2R,7aS)-2-fluoro-hexahydro-1H-pyrrolizin-7a-yl]methoxy}-4-[(1S,6R)-3,9-diazabicyclo[4.2 .1]nonan-3-yl]-8-fluoroquinazolin -7-yl)naphthalen-2-ol | 572.0 | ¹H NMR (400 MHz, DMSO-d₆) δ ppm: 9.88 (s, 1H), 7.96 (s, 1H), 7.83 - 7.75 (m, 1H), 7.43 (br d, J = 8.0 Hz, 2H), 7.26 - 7.19 (m, 3H), 7.12 - 7.06 (m, 1H), 5.35 - 5.18 (m, 1H), 4.16 - 4.08 (m, 1H), 4.03 - 3.97 (m, 1H), 3.89 - 3.82 (m, 1H), 3.73 - 3.64 (m, 1H), 3.15 - 3.05 (m, 3H), 3.05 - 2.94 (m, 3H), 2.86 - 2.78 (m, 2H), 2.56 - 2.53 (m, 1H), 2.16 - 2.09 (m, 1H), 2.06 - 2.05 (m, 1H), 2.04 - 1.92 (m, 4H), 1.88 - 1.71 (m, 5H), 1.55 - 1.41 (m, 1H). |
| 11-8 | 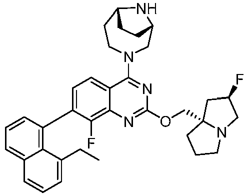 | 2-{[(2R,7aS)-2-fluoro-hexahydro-1H-pyrrolizin-7a-yl]methoxy}-4-[(1S,6R)-3,9-diazabicyclo[4.2 .1]nonan-3-yl]-7-(8-ethylnaphthalen -1-yl)-8-fluoroquinazolin e | 584.1 | ¹H NMR (400 MHz, DMSO-d₆) δ ppm: 8.07 - 8.01 (m, 1H), 7.92 - 7.88 (m, 2H), 7.59 - 7.49 (m, 2H), 7.41 - 7.31 (m, 2H), 7.29 - 7.20 (m, 1H), 5.37 - 5.19 (m, 1H), 4.42 (d, J = 9 Hz, 1H), 4.03 - 3.97 (m, 2H), 3.93 - 3.76 (m, 2H), 3.72 - 3.60 (m, 2H), 3.53 - 3.45 (m, 2H), 3.09 - 3.02 (m, 3H), 2.85 - 2.75 (m, 1H), 2.38 - 2.24 (m, 2H), 2.13 - 1.94 (m, 4H), 1.91 - 1.70 (m, 6H), 1.55 - 1.42 (m, 2H), 0.83 (t, J = 6 Hz, 3H). |

### Example 12-1

### 4-(2-{[(2R,7aS)-2-fluoro-hexahydro-1H-pyrrolizin-7a-yl]methoxy}-4-[(1S,6R)-3,9-diazabicyclo[4.2.1]nonan-3-yl]-8-fluoroquinazolin-7-yl)-5-fluoronaphthalen-2-ol

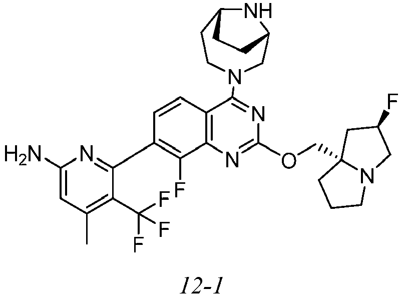

### Preparation of Intermediate 12A-1: tert-butyl (1S,6R)-3-(2-{[(2R,7aS)-2-fluoro-hexahydro-1H-pyrrolizin-7a-yl]methoxy}-7-bromo-8-fluoroquinazolin-4-yl)-3,9-diazabicyclo[4.2.1]nonane-9-carboxylate

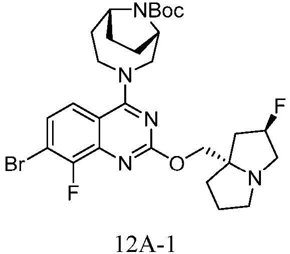

To a stirred solution of ((2*R*,7a*S*)-2-fluorotetrahydro-1H-pyrrolizin-7a(5H)-yl)methanol (246 mg, 1.544 mmol) in THF (20 mL) was added sodium hydride (49.4 mg, 2.059 mmol) and the mixture was stirred at 0 °C for one hour. To this reaction mixture, tert-butyl (1S,6R)-3-(7-bromo-2-chloro-8-fluoroquinazolin-4-yl)-3,9-diazabicyclo[4.2.1]nonane-9-carboxylate (500 mg, 1.029 mmol) was added and the reaction mixture was slowly warmed to room temperature and allowed to stir for 2h. The reaction mixture was quenched with water, extracted with ethyl acetate(50 mL x 2), dried over anhydrous sodium sulphate, filtered and concentrated under reduced pressure to afford tert-butyl (1*S*,6*R*)-3-(2-{[(2*R*,7a*S*)-2-fluoro-hexahydro-1H-pyrrolizin-7a-yl]methoxy}-7-bromo-8-fluoroquinazolin-4-yl)-3,9-diazabicyclo[4.2.1]nonane-9-carboxylate as a yellow solid. MS(ESI) m/z: 608.2 [M+H]⁺.

### Preparation of Intermediate 12A: tert-butyl (1S,6R)-3-(2-{[(2R,7aS)-2-fluoro-hexahydro-1H-pyrrolizin-7a-yl]methoxy}-8-fluoro-7-(4,4,5,5-tetramethyl-1,3,2-dioxaborolan-2-yljquinazolin-4-yl)-3,9-diazabicyclo[4.2.I]nonane-9-carboxylate

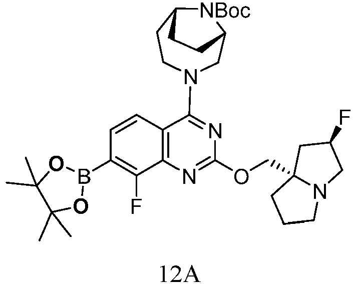

To a degassed solution of tert-butyl (1S,6R)-3-(2-{[(2R,7aS)-2-fluoro-hexahydro-1H-pyrrolizin-7a-yl]methoxy }-7-bromo-8-fluoroquinazolin-4-yl)-3,9-diazabicyclo[4.2.1]nonane-9-carboxylate (120 mg, 0.197 mmol), bis(pinacolato)diboron (75 mg, 0.296 mmol), potassium acetate (38.7 mg, 0.394 mmol) in 1,4-dioxane (3 mL) was added PdCl₂(dppf) (14.43 mg, 0.020 mmol) and the reaction mixture was heated at 120 °C for 1.5 h. The reaction mixture was then filtered through a Celite pad and the filtrate was taken forward in the next step without further work-up or purification.

### Preparation of Intermediate 12B: tert-butyl (1S,6R)-3-(2-{[(2R,7aS)-2-fluoro-hexahydro-1H-pyrrolizin-7a-yl]methoxy}-7-(6-{bis[(4-methoxyphenyl)methyl]amino}-4-methyl-3-(trifluoromethyl)pyridin-2-yl)-8-fluoroquinazolin-4-yl)-3,9-diazabicyclo[4.2.1]nonane-9-carboxylate

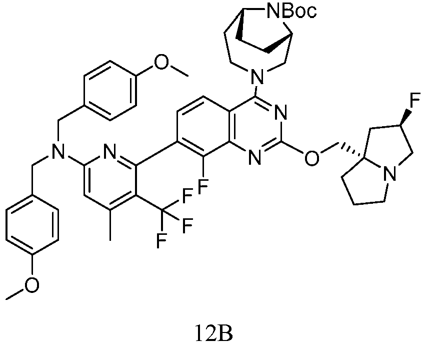

To a degassed solution of tert- butyl (1S,6R)-3-(2-{[(2R,7aS)-2-fluoro-hexahydro-1H-pyrrolizin-7a-yl]methoxy}-8-fluoro-7-(4,4,5,5-tetramethyl-1,3,2-dioxaborolan-2-yl)quinazolin-4-yl)-3,9-diazabicyclo[4.2.1]nonane-9-carboxylate (100 mg, 0.153 mmol) in 1,4-dioxane (2 mL) (crude product), 6-chloro-N,N-bis(4-methoxybenzyl)-4-methyl-5-(trifluoromethyl)pyridin-2-amine (CAS: 2411793-22-9) (83 mg, 0.183 mmol) and 1.5M aqueous solution of tripotassium phosphate (0.305 mL, 0.458 mmol) was added PdCl₂(dppf) (11.16 mg, 0.015 mmol) and the reaction mixture was stirred at 100 °C for 6 h. The reaction mixture was then diluted with water and extracted with ethyl acetate. The combined organic layers were washed with water and brine, dried over Na₂SO₄ filtered and concentrated to provide the crude residue. The crude residue was purified by silica gel column chromatography (24 g RediSep^{®} column, eluting with a gradient from 60-100% EtOAc in Petroleum Ether). Fractions containing the desired product were evaporated to afford tert-butyl (1S,6R)-3-(2-{[(2R,7aS)-2-fluoro-hexahydro-1H-pyrrolizin-7a-yl]methoxy}-7-(6-{bis[(4-methoxyphenyl)methyl]amino}-4-methyl-3-(trifluoromethyl)pyridin-2-yl)-8-fluoroquinazolin-4-yl)-3,9-diazabicyclo[4.2.1]nonane-9-carboxylate (70 mg, 0.074 mmol, 48.6 % yield) LCMS (ESI) m/z: 944.3 [M+H]⁺.

### Example 12-1

### 4-(2-{[(2R,7aS)-2-fluoro-hexahydro-1H-pyrrolizin-7a-yl]methoxy}-4-[(1S,6R)-3,9-diazabicyclo[4.2.1]nonan-3-yl]-8-fluoroquinazolin-7-yl)-5-fluoronaphthalen-2-ol

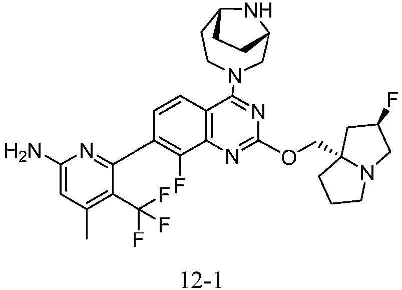

A stirred solution of TFA (1.5 mL, 19.47 mmol) and triethylsilane (0.5 mL, 3.13 mmol) was added to tert-butyl (1S,6R)-3-(2-{[(2R,7aS)-2-fluoro-hexahydro-1H-pyrrolizin-7a-yl]methoxy}-7-(6-{bis[(4-methoxyphenyl)methyl]amino}-4-methyl-3-(trifluoromethyl)pyridin-2-yl)-8-fluoroquinazolin-4-yl)-3,9-diazabicyclo[4.2.1]nonane-9-carboxylate (70 mg, 0.074 mmol) at room temperature and the resulting reaction mixture was heated at 40 °C for 24 h. Then, the reaction mixture was concentrated under reduced pressure, co-distilled with toluene (twice), neutralized with DIPEA and concentrated under reduced pressure to provide a crude residue, which was purified by preparative HPLC [HPLC Conditions: Column/dimensions: Column Name: Kinetex EVO (250 mm x 21 mm ID, 5µ); Mobile phase A= 10 mM Ammonium Bicarbonate in water pH 9.5, Mobile phase B= Acetonitrile:MeOH(1:1), Flow Rate: 19 mL/min; Retention time = 11.72 min.] to afford 6-(2-{[(2R,7aS)-2-fluoro-hexahydro-1H-pyrrolizin-7a-yl]methoxy}-4-[(1S,6R)-3,9- diazabicyclo[4.2.1]nonan-3-yl]-8-fluoroquinazolin-7-yl)-4-methyl-5-(trifluoromethyl)pyridin-2-amine (18 mg, 0.030 mmol, 40.2 % yield). LCMS (ESI) m/z: 604.5 [M+H]⁺. ¹H NMR (400 MHz, DMSO-d₆) δ ppm 7.86 - 7.79 (m, 1H), 7.18 - 7.07 (m, 1H), 6.82 - 6.71 (m, 2H), 6.47 - 6.44 (m, 1H), 5.39 - 5.15 (m, 1H), 4.53 - 4.40 (m, 1H), 4.13 - 4.05 (m, 1H), 4.02 - 3.92 (m, 1H), 3.88 - 3.77 (m, 2H), 3.70 - 3.60 (m, 2H), 3.46 - 3.38 (m, 2H), 3.12 - 3.05 (m, 2H), 3.04 - 2.98 (m, 1H), 2.86 - 2.77 (m, 1H), 2.37 - 2.35 (m, 3H), 2.16 - 2.09 (m, 1H), 2.01 - 1.92 (m, 3H), 1.84-1.80 (s, 3H), 1.79 - 1.68 (m, 3H), 1.50 - 1.36 (m, 2H).

Examples in Table 8 were prepared according to procedures described for Example 12-1 from the appropriate starting materials.

**Table 8**

| Example Number | Structure | Name | LCMS (M+H)⁺ | ¹H NMR |
|---|---|---|---|---|
| 12-2 | 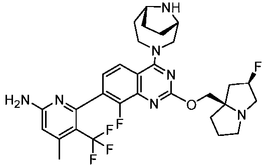 | 6-(2-{[(2R,7aR)-2-fluoro-hexahydro-1H-pyrrolizin-7a-yl]methoxy}-4-[(1S,6R)-3,9-diazabicyclo[4.2.1] nonan-3-yl]-8-fluoroquinazolin-7-yl)-4-methyl-5-(trifluoromethyl)py ridin-2-amine | 603.8 | ¹H NMR (400 MHz, DMSO-d₆) δ ppm: 7.93 - 7.77 (m, 1H), 7.18 - 7.06 (m, 1H), 6.77 (s, 2H), 6.47 (s, 1H), 5.46 - 5.27 (m, 1H), 4.52 - 4.40 (m, 1H), 4.16 - 3.98 (m, 2H), 3.92 - 3.77 (m, 2H), 3.70 - 3.62 (m, 2H), 3.00 - 2.93 (m, 2H), 2.89 - 2.73 (m, 1H), 2.39 - 2.35 (m, 3H), 2.31 - 2.25 (m, 1H), 2.14 - 1.80 (m, 8H), 1.78 - 1.71 (m, 4H), 1.69 - 1.56 (m, 1H), 1.50 - 13.9 (m, 1H) |
| 12-3 | 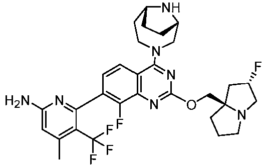 | 6-(2-{[(2S,7aR)-2-fluoro-hexahydro-1H-pyrrolizin-7a-yl]methoxy}-4-[(1S,6R)-3,9-diazabicyclo[4.2.1] nonan-3-yl]-8-fluoroquinazolin-7-yl)-4-methyl-5-(trifluoromethyl)py ridin-2-amine | 604.3 | ¹H NMR (400 MHz, DMSO-d₆) δ ppm: 7.88 (br d, J = 7.0 Hz, 1H), 7.14-7.08 (m, 1H), 6.76 (br s, 2H), 6.46 (s, 1H), 5.34-5.23 (m, 1H), 5.08 (br s, 1H), 4.19 - 3.92 (m, 2H), 3.30-3.10 (m, 8H), 2.92 - 2.68 (m, 3H), 2.36 (br s, 3H), 2.32 - 2.08 (m, 1H), 2.06 - 1.92 (m, 4H), 1.90 - 1.73 (m, 5H), 1.44 - 1.41 (m, 1H). |
| 12-4 | 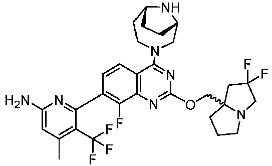 | 6-{4-[(1S,6R)-3,9-diazabicyclo[4.2.1] nonan-3-yl]-2-[(2,2-difluoro-hexahydro-1H-pyrrolizin-7a-yl)methoxy]-8-fluoroquinazolin-7-yl}-4-methyl-5-(trifluoromethyl)py ridin-2-amine | 622.3 | ¹H NMR (400 MHz, DMSO-d₆) δ ppm: 7.93 - 7.77 (m, 1H), 7.18 - 7.06 (m, 1H), 6.77 (s, 2H), 6.47 (s, 1H), 4.52 - 4.40 (m, 1H), 4.16 - 3.98 (m, 2H), 3.92 - 3.77 (m, 2H), 3.70 - 3.62 (m, 2H), 3.00 - 2.93 (m, 2H), 2.89 - 2.73 (m, 1H), 2.39 - 2.35 (m, 3H), 2.31 - 2.25 (m, 1H), 2.14 - 1.80 (m, 8H), 1.78 - 1.71 (m, 4H), 1.69 - 1.56 (m, 1H), 1.50 - 1.39 (m, 1H) |

### Example 13-1

### 6-(2-{[(6'R,7'aR)-6'-fluoro-hexahydrospiro[cyclopropane-1,2'-pyrrolizine]-7'a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amine

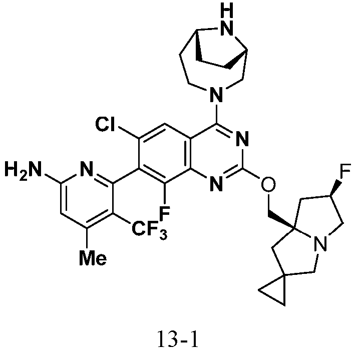

### Preparation of Intermediate 13A: 1-(tert-butyl) 2-methyl (2R,4R)-2-(2-(chloromethyl)allyl)-4-fluoropyrrolidine-1,2-dicarboxylate

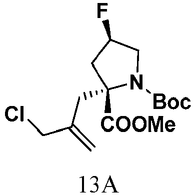

To a stirred solution of 1-(tert-butyl) 2-methyl (2S,4R)-4-fluoropyrrolidine-1,2-dicarboxylate (50 g, 202 mmolat -45 °C under a nitrogen atmosphere was added LiHMDS (1M THF solution, 303 mL, 303 mmol) dropwise over 30 min while maintaining the temperature at - 45 °C. After stirring at this temperature for 1h, a solution of 3-chloro-2-(chloromethyl)prop-1-ene (30.3 g, 243 mmol) in anhydrous THF (300.0 mL) was added dropwise. The reaction mixture was slowly warmed to room temperature over 2 h and stirred for 16 h. The reaction mixture was carefully quenched with saturated ammonium chloride solution (40 mL) and extracted with ethyl acetate (2x200 mL). The combined organic layers were washed with water and saturated brine solution, dried over anhydrous Na₂SO₄, filtered, and concentrated under reduced pressure to afford crude product. The crude residue was purified by column chromatography (Grace, 350 g snap, dry pack) over silica gel (230-400 mesh) by eluting with 10-30% ethyl acetate in petroleum ether. The desired fractions were pooled and concentrated under reduced pressure to obtain 1-(tert-butyl) 2-methyl (2R,4R)-2-(2-(chloromethyl)allyl)-4-fluoropyrrolidine-1,2-dicarboxylate (64 g, 91 mmol, 45% yield) as a colorless liquid. LCMS (ESI) m/z: 336.1 [M+H]⁺.

### Preparation of Intermediate 13B: methyl (2R,4R)-2-(2-(chloromethyl)allyl)-4-fluoropyrrolidine-2-carboxylate

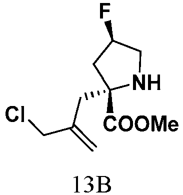

To a stirred solution of 1-(tert-butyl) 2-methyl (2R,4R)-2-(2-(chloromethyl)allyl)-4-fluoropyrrolidine-1,2-dicarboxylate (64 g, 191 mmol) in anhydrous DCM (600 mL) was added a HCl solution (4.0 M in dioxane, 119 mL, 476 mmol) at 0 °C under a nitrogen atmosphere. The reaction mixture was stirred at ambient temperature for 6 h followed by concentration under reduced pressure to obtain methyl (2R,4R)-2-(2-(chloromethyl)allyl)-4-fluoropyrrolidine-2-carboxylate, HCl (50 g, 180 mmol, 94% yield) as a pale brown solid. LCMS (ESI) m/z: 236.2 [M+H]⁺.

### Preparation of Intermediate 13C: methyl (2R,7aR)-2-fluoro-6-methylenetetrahydro-1H-pyrrolizine-7a(5H)-carboxylate

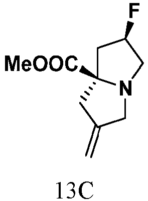

To a stirred solution of methyl (2R,4R)-2-(2-(chloromethyl)allyl)-4-fluoropyrrolidine-2-carboxylate (50 g, 212 mmol) in anhydrous acetonitrile (500 mL) was added TEA (44.4 mL, 318 mmol) The reaction mixture was stirred at ambient temperature for 16 h. The reaction mixture was then diluted with water (50 mL) and then treated with an aqueous bicarbonate solution (70 mL). The resulting solution was extracted with DCM (2 x 300 mL) and the combined organic layers were washed with saturated brine solution (50 mL), dried over anhydrous Na₂SO₄, filtered, and concentrated under reduced pressure to afford methyl (2R,7aR)-2-fluoro-6-methylenetetrahydro-1H-pyrrolizine-7a(5H)-carboxylate (35 g, 176 mmol, 83% yield) as a brown liquid. LCMS (ESI) m/z: 200.1 [M+H]⁺.

### Preparation of Intermediate 13D: methyl (6'R,7a'R)-6'fluorodihydro-1'H,3'H spiro[cyclopropane-1,2'-pyrrolizine]-7a'(5'H)-carboxylate

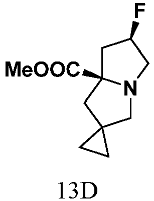

To a stirred solution of methyl (2R,7aR)-2-fluoro-6-methylenetetrahydro-1H-pyrrolizine-7a(5H)-carboxylate (2.0 g, 10.04 mmol) in anhydrous toluene (20 mL) under a nitrogen atmosphere, diiodomethane (3.64 mL, 45.2 mmol) was added at 0 °C. The reaction mixture was stirred at 0 °C for 30 min before the drop-wise addition of diethylzinc (50.2 mL, 50.2 mmol) (1M solution in Hexane). The reaction mixture was warmed to room temperature and stirred for 16 h before quenching with saturated aq. NH₄Cl (5 mL). The suspension was extracted with ethyl acetate (2x20 mL). The combined organic layers were dried over anhydrous Na₂SO₄, filtered and concentrated under reduced pressure to obtain the crude residue, which was purified by column chromatography (alumina- neutral) using 40-50% ethyl acetate in petroleum ether to obtain the methyl (6'R,7a'R)-6'-fluorodihydro-1'H,3'H-spiro[cyclopropane-1,2'-pyrrolizine]-7a'(5'H)-carboxylate (2.0 g, 8.05 mmol, 80 % yield) as a colourless liquid. LCMS-ELSD (ESI) m/z: 204.1 [M+H]⁺.

### Preparation of Intermediate 13E: ((6'R,7a'R)-6'-fluorodihydro-1'H,3'H-spiro[cyclopropane-1,2'-pyrrolizin]-7a'(5'H)-yl)methanol

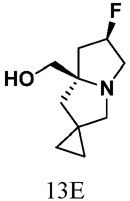

To a stirred solution of methyl (6'R,7a'R)-6'-fluorodihydro-1'H,3'H-spiro[cyclopropane-1,2'-pyrrolizine]-7a'(5'H)-carboxylate (2.0 g, 9.38 mmol) in THF (20 mL) was added LiAlH₄ (4.69 mL, 9.38 mmol, 2M in THF) at 0 °C. The reaction mixture was stirred at ambient temperature for 3 h followed by quenching with saturated aq. ammonium chloride at 0° C. Once the effervescence ceased, anhydrous sodium sulphate was added to the reaction mixture, followed by DCM (20 mL). The reaction mixture was stirred for 20 minutes before filtration. The filtrate was collected, dried over anhydrous sodium sulphate and concentrated under reduced pressure to obtain ((6'R,7a'R)-6'-fluorodihydro-1'H,3'H-spiro[cyclopropane-1,2'-pyrrolizin]-7a'(5'H)-yl)methanol (110 mg, 0.592 mmol, 6% yield) as a colourless liquid. LCMS-ELSD (ESI) m/z: 186.2 [M+H]⁺.

### Preparation of Intermediate 13F: tert-butyl 3-(7-(6-(bis(4-methoxybenzyl)amino)-4-methyl-3-(trifluoromethyl)pyridin-2-yl)-6-chloro-8-fluoro-2-(((6'R,7a''R)-6"-fluorodihydro1'H,3'H-spiro[cyclopropane-1,2'-pyrrolizin]-7a'(5'H)-yl)methoxy)quinazolin-4-yl)-3,8-diazabicyclo[3.2.1]octane-8-carboxylate

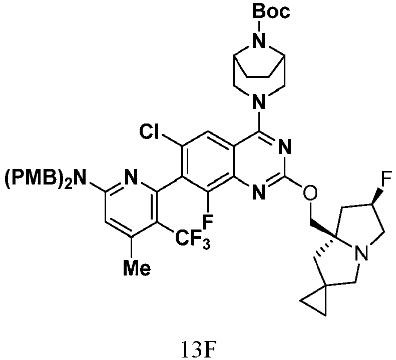

To a stirred solution of ((6'R,7a'R)-6'-fluorodihydro-1'H,3'H-spiro[cyclopropane-1,2'-pyrrolizin]-7a'(5'H)-yl)methanol (0.090 g, 0.485 mmol) in anhydrous THF (5.0 mL) at 0 °C was added sodium hydride (60% dispersion in mineral oil, 0.019 g, 0.485 mmol) and the reaction mixture was stirred at the same temperature for 30 min. A solution of Intermediate 3K (0.2g, 0.242 mmol) in THF (2 mL) was added dropwise while maintaining the temperature 0 °C. The reaction mixture was warmed to room temperature over 2 h. The reaction mixture was quenched with saturated aq. ammonium chloride solution (1 mL) and extracted with ethyl acetate (3x5 mL). The combined organic layers were dried over anhydrous sodium sulphate and concentrated under a reduced pressure to obtain the crude product. The crude product was purified by column chromatography (Grace, 50 g snap, dry pack) over neutral alumina using 50-100% ethyl acetate in petroleum ether. The desired fractions were pooled and concentrated under reduced pressure to obtain tert-butyl 3-(7-(6-(bis(4- methoxybenzyl)amino)-4-methyl-3-(trifluoromethyl)pyridin-2-yl)-6-chloro-8-fluoro-2-(((6'R,7a'R)-6'-fluorodihydro-1'H,3'H-spiro[cyclopropane-1,2'-pyrrolizin]-7a'(5'H)-yl)methoxy)quinazolin-4-yl)-3,8-diazabicyclo[3.2.1]octane-8-carboxylate (0.21g, 0.199 mmol, 82 % yield) as a pale yellow solid. LCMS (ESI) m/z: 990.4 [M+H]⁺.

### Preparation of Intermediate 13G: 7-(6-(bis(4-methoxybenzyl)amino)-4-methyl-3-(trifluoromethyl)pyridin-2-yl)-6-chloro-8-fluoro-2-(((6'R,7a'R)-6'-fluorodihydro-1'H,3'Hspiro[cyclopropane-1,2'-pyrrolizin]-7a'(5'H)-yl)methoxy)quinazolin-4-ol

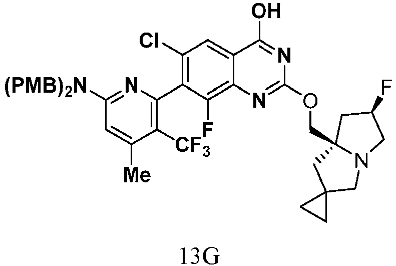

To a stirred solution of tert-butyl 3-(7-(6-(bis(4-methoxybenzyl)amino)-4-methyl-3-(trifluoromethyl)pyridin-2-yl)-6- chloro-8-fluoro-2-(((6'R,7a'R)-6'-fluorodihydro-1'H,3'H-spiro[cyclopropane-1,2'-pyrrolizin]- 7a'(5'H)-yl)methoxy)quinazolin-4-yl)-3,8-diazabicyclo[3.2.1]octane-8-carboxylate (240 mg, 0.242 mmol) in ethanol (5 mL) was added a 10% NaOH solution (2.42 mL, 2.423 mmol). The reaction was heated at 70 °C for 48 h. The reaction mixture was then concentrated under reduced pressure to obtain the crude residue which was purified by column chromatography (Biotage, Al₂O₃- neutral) using 30-40% ethyl acetate in petroleum ether to obtain 7-(6-(bis(4-methoxybenzyl)amino)-4-methyl-3-(trifluoromethyl)pyridin-2-yl)-6-chloro-8-fluoro-2-(((6'R,7a'R)-6'-fluorodihydro-1'H,3'H-spiro[cyclopropane-1,2'-pyrrolizin]-7a'(5'H)-yl)methoxy)quinazolin-4-ol (172 mg, 0.110 mmol, 46% yield) as a gummy liquid. LCMS (ESI) m/z: 796.2 [M+H]⁺.

### Preparation of intermediate 13H: tert-butyl (1S,6R)-3-(7-(6-(bis(4-methoxybenzyl)amino)-4-methyl-3- (trifluoromethyl)pyridin-2-yl)-6-chloro-8-fluoro-2-(((6'R,7a''R)-6-fluorodihydro1'H,3'H-spiro[cyclopropane-1,2'-pyrrolizin]-7a'(5'H)-yl)methoxy)quinazolin-4-yl)-3,9-diazabicyclo[4.2.1]nonane-9-carboxylate

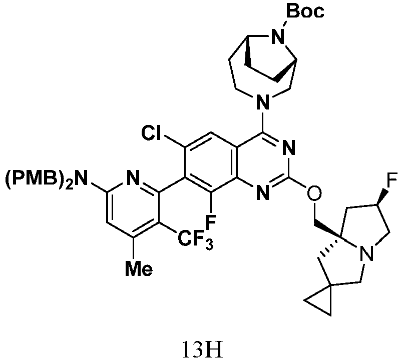

To a stirred solution of 7-(6-(bis(4-methoxybenzyl)amino)-4-methyl-3-(trifluoromethyl)pyridin-2-yl)-6-chloro-8-fluoro-2-(((6'R,7a'R)-6'-fluorodihydro-1'H,3'H-spiro[cyclopropane-1,2'-pyrrolizin]-7a'(5'H)-yl)methoxy)quinazolin-4-ol (0.17g, 0.214 mmol) in acetonitrile (2 mL) were added BOP (0.142 g, 0.320 mmol) and TEA (0.045 mL, 0.320 mmol). After stirring for 5 min, tert-butyl (1S,6R)-3,9-diazabicyclo[4.2.1]nonane-9-carboxylate (0.097 g, 0.427 mmol) was added and the mixture was stirred at 40 °C for 16 h. The reaction mixture was then quenched with water (10 mL) and extracted with ethyl acetate (2x15 mL). The organic layer was washed with brine (10 mL), dried over anhydrous sodium sulfate, filtered, and the filtrate was concentrated to afford 70 mg of crude product. The residue was purified by column chromatography (Biotage, Alumina-neutral) using 30-40% of ethyl acetate in petroleum ether to provide tert-butyl (1S,6R)-3-(7-(6-(bis(4-methoxybenzyl)amino)-4-methyl-3-(trifluoromethyl)pyridin-2-yl)-6-chloro-8-fluoro-2-(((6'R,7a'R)-6'-fluorodihydro-1'H,3'H-spiro[cyclopropane-1,2'-pyrrolizin]-7a'(5'H)-yl)methoxy)quinazolin-4-yl)-3,9-diazabicyclo[4.2.1]nonane-9-carboxylate (150 mg, 0.103 mmol, 48% yield). LCMS (ESI) m/z: 1004.4 [M+H]⁺.

### Example 13-1

### 6-(2-{[(6'R,7'aR)-6'-fluoro-hexahydrospiro[cyclopropane-1,2'-pyrrolizine]-7'a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amine

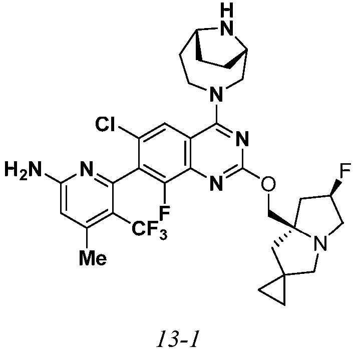

To a stirred solution of tert-butyl (1S,6R)-3-(7-(6-(bis(4-methoxybenzyl)amino)-4-methyl-3-(trifluoromethyl)pyridin-2- yl)-6-chloro-8-fluoro-2-(((6'R,7a'R)-6'-fluorodihydro-1'H,3'H-spiro[cyclopropane-1,2'-pyrrolizin]- 7a'(5'H)-yl)methoxy)quinazolin-4-yl)-3,9-diazabicyclo[4.2.1]nonane-9-carboxylate (0.15g, 0.149 mmol) in TFA (1 mL) was added water (0.01 mL) and triethylsilane (0.024 mL, 0.149 mmol). The reaction was allowed to stir at 40 °C for 16 h. The reaction mixture was then concentrated under reduced pressure to obtain a crude residue, which was purified by preparative HPLC [Column : Xbridge C18 (250 x 19)mm, 5micron Mobile phase A: 0.1% Ammonium formate in water Mobile phase B : Acetonitrile, Flow: 15mL/min, grade-30-70% mobile phase-B in mobile phase-A in 15 min] to obtain the desired compound (10 mg, 0.012 mmol, 8% yield) as an off white solid. LCMS (ESI) m/z: 664.4 [M+H]⁺. ¹H-NMR (400 MHz, DMSO-d6) δ: ¹H NMR (400 MHz, CD₃SOCD₃, 298 K) δ: 8.27 - 8.16 (m, 1H), 7.96 - 7.78 (m, 1H), 6.92 - 6.76 (m, 1H), 6.50 (s, 1H), 5.54 - 5.24 (m, 1H), 4.43 - 4.24 (m, 1H), 4.19 - 4.03 (m, 1H), 3.95 - 3.83 (m, 1H), 3.82 - 3.75 (m, 2H), 3.74 - 3.62 (m, 2H), 3.34-3.23 (m, 3H), 3.08 - 2.88 (m, 1H), 2.86 - 2.77 (m, 5H), 2.68 (td, J = 1.8, 3.5 Hz, 2H), 2.63 (d, J = 10.0 Hz, 1H), 2.41 - 2.29 (m, 3H), 2.13 - 1.71 (m, 5H), 1.46 (br s, 1H), 0.63 - 0.36 (m, 2H) ppm.

### Preparation of Intermediate 14-1: N-(5-bromonaphthalen-1-yl)-1,1,1-trimethyl-N-(trimethylsilyl)silanamine

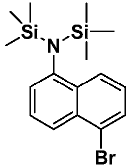

To a stirred solution of 5-bromonaphthalen-1-amine (40 g, 180 mmol) in anhydrous THF (650 mL) under N₂ atmosphere at -78 °C, LiHMDS (1M THF solution, 396 mL, 396 mmol) was added drop wise over 30 min. The reaction mixture was slowly warmed to 20 °C over 30 min and then again cooled back to -78 °C. A solution of TMSCl (48.3 mL, 378 mmol) in anhydrous THF was added drop wise to the reaction mixture at -78 °C and was slowly warmed to 20 °C over 1h. The reaction mixture was concentrated under reduced pressure to obtain the crude residue. The crude residue was dissolved in hexane (100 mL) and filtered through Celite, and the filtrate was concentrated under reduced pressure to obtain the crude product as a red oil, which was purified by flash column (Silica gel 100-200) chromatography using petroleum ether as an eluent. Pure fractions were concentrated under reduced pressure to provide N-(5-bromonaphthalen-1-yl)-1,1,1-trimethyl-N-(trimethylsilyl)silanamine (61 g, 166 mmol, 92 % yield) as a brown liquid. LCMS (ESI) m/z: 366.45 [M+H]⁺.

### Preparation of Intermediate 14-2: 5-fluoronaphthalen-1-amine

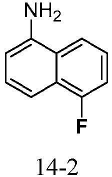

To a stirred solution of N-(5-bromonaphthalen-1-yl)-1,1,1-trimethyl-N-(trimethylsilyl)silanamine (100 g, 273 mmol) in anhydrous THF (1400 mL) under a N₂ atmosphere at -78 °C was added n-butyllithium (164 mL, 409 mmol) dropwise over 30 min. After complete addition, the reaction mixture was stirred for 10 min, and then a solution of N-fluorobenzenesulfonimide (138 g, 437 mmol) in anhydrous THF (400 mL) was added dropwise at -78 °C over 20 min. The resulting reaction mixture was slowly warmed to 20 °C for 1h, diluted with ice cold water (1000 mL) and extracted with ethyl acetate (3 x 800 mL). The extracted organic layers were combined, dried over anhydrous Na₂SO₄, filtered and concentrated under reduced pressure to obtain the crude residue. The residue was purified by flash column (Silica gel 100-200) chromatography using 10-20% ethyl acetate in petroleum ether as eluent. Pure fractions were concentrated under reduced pressure to obtain semi pure compound, which was further purified by reverse phase column chromatography using 80% acetonitrile in 0.01% ammonium formate in water. Pure fractions were concentrated under reduced pressure to obtain 5-fluoronaphthalen-1-amine (20 g, 115 mmol, 42.3 % yield) as a brown solid. LCMS (ESI) m/z: 162.19 [M+H]⁺.

### Preparation of Intermediate 14-3: 2,4-dibromo-5-fluoronaphthalen-1-amine

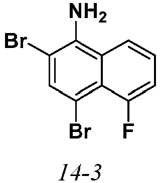

To a stirred solution of 5-fluoronaphthalen-1-amine (40 g, 228 mmol) in acetic acid (800 mL) at 0 °C was carefully added bromine (25.9 mL, 502 mmol) over 30 min and the resultant reaction mixture was stirred at 70 °C for 1 h. The reaction mixture was filtered, and the filter cake was washed with acetic acid (2x200 mL). The residue was suspended in 10% NaOH solution (600 mL) and filtered. The filter cake was washed with water (200 mL) and dried under reduced pressure to obtain the crude 2,4-dibromo-5-fluoronaphthalen-1-amine (66 g, 170 mmol, 74.3 % yield) as a pale-yellow solid. The crude compound was used in the next step without any further purification. LCMS (ESI) m/z: 319.98 [M+H]⁺.

### Preparation of Intermediate 14-4: 5-bromo-6-fluoronaphtho[1,2-d][1,2,3]oxadiazole

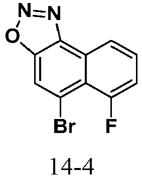

To a stirred solution of 2,4-dibromo-5-fluoronaphthalen-1-amine (66 g, 170 mmol) in acetic acid (1000 mL) was added propionic acid (136 mL, 1815 mmol) at 0 °C. After 10 min, sodium nitrite (17.56 g, 255 mmol) was added portion-wise to the reaction mixture at 0 °C. The reaction mixture was stirred for 30 min at 0 °C and warmed to 25 °C over 1 h. The reaction mixture was diluted with cold water (2000 mL), stirred for 10 min, and filtered. The solid was washed with water (2 X 500 mL), and dried under reduced pressure to obtain crude 5-bromo-6-fluoronaphtho[1,2-d][1,2,3]oxadiazole (40 g, 118 mmol, 69% yield) as a brown solid, which was used in the next step without any purification. LCMS (ESI) m/z: 265.9 [M+H]⁺.

### Preparation of Intermediate 14-5: 4-bromo-5-fluoronaphthalen-2-ol

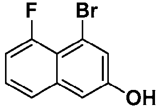

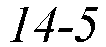

To a stirred solution of 5-bromo-6-fluoronaphtho[1,2-d][1,2,3]oxadiazole (40 g, 118 mmol)) in ethanol (500 mL) and tetrahydrofuran (250 mL) at 0 °C under nitrogen was added NaBH₄ (8.91 g, 235 mmol) portion-wise over 30 min. The reaction was stirred at the same temperature for 30 min and then warmed to 25 °C. The reaction mixture was carefully quenched with aq. ammonium chloride (20 mL) and concentrated under reduced pressure to remove the EtOH. The suspension was extracted with ethyl acetate (3 X 500 mL), combined organics were dried over anhydrous Na₂SO₄ filtered and concentrated under reduced pressure to obtain 4-bromo-5-fluoronaphthalen-2-ol (30 g, 86 mmol, 73% yield) which was used in the next step without further purification. LCMS (ESI) m/z: 241.08 [M+H]⁺.

### Preparation of Intermediate 14-6: 1-bromo-8-fluoro-3-(methoxymethoxy)naphthalene

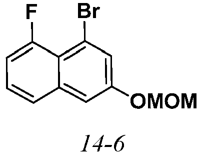

To a stirred solution of 4-bromo-5-fluoronaphthalen-2-ol (30 g, 86 mmol) and DIPEA (22.50 mL, 129 mmol) in anhydrous dichloromethane (300 mL) at 0 °C under a nitrogen atmosphere was dropwise added MOM-Cl (7.83 mL, 103 mmol) over 10 min. The resultant reaction mixture was allowed to warm to room temperature and stirred for 1h. The reaction mixture was diluted with cold water (500 mL) and extracted with DCM (2 X 500 mL). The combined organic layers were washed with brine (50 mL), dried over anhydrous Na₂SO₄ filtered and concentrated under reduced pressure to obtain a crude residue. The crude compound was purified by flash column (Silica gel 100-200) chromatography using 5-10% ethyl acetate in petroleum ether as eluent. Pure fractions were concentrated under reduced pressure to afford 1-bromo-8-fluoro-3-(methoxymethoxy)naphthalene (20.5 g, 68.3 mmol, 80% yield) as a brown solid. LCMS (ESI) m/z: 285.01 [M+H]⁺.

### Preparation of Intermediate 14-7: 1-bromo-8-fluoro-3-(methoxymethoxy)naphthalene

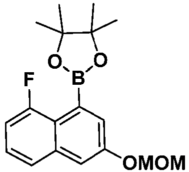

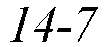

To a degassed solution of 1-bromo-8-fluoro-3-(methoxymethoxy)naphthalene (15 g, 50.0 mmol), bis-pinacolatodiboron (25.4 g, 100 mmol) and potassium acetate (14.72 g, 150 mmol) in anhydrous toluene (300 mL) was added PdCl₂(dppf) (3.66 g, 5.00 mmol) at room temperature under an inert atmosphere. The resultant reaction mixture was stirred at 110 °C for 3h. The reaction mixture was diluted with water (50 mL) and extracted with ethyl acetate (2 x 250 mL). The combined organic layers were washed with brine (50 mL), dried over anhydrous Na₂SO₄ filtered and concentrated under reduced pressure to obtain a crude residue. The crude material was purified by flash column (silica 100-200 mesh) chromatography by using 2-4% ethyl acetate in petroleum ether as eluent. Pure fractions were concentrated under reduced pressure to afford a brown gum which was further purified by reverse phase column chromatography using 80% acetonitrile in 0.01% of ammonium formate in water as eluent. Pure fractions were concentrated under reduced pressure to obtain 2-(8-fluoro-3-(methoxymethoxy)naphthalen-1-yl)-4,4,5,5-tetramethyl-1,3,2-dioxaborolane (9 g, 26.6 mmol, 53.2 % yield) as an off white solid. LCMS (ESI) m/z: 332.1 [M+H]⁺. 1H-NMR (400 MHz, DMSO-d₆): δ 7.52 (d, J = 0.80 Hz, 1H), 7.42-7.44 (m, 1H), 7.36-7.38 (m, 1H), 7.33-7.35 (m, 1H), 7.03 (dd, J = 1.20, 7.60 Hz, 1H), 5.31 (s, 2H), 3.52 (s, 3H), 1.46 (s, 12H) ppm.

### Preparation of Intermediate 15-1: 8-((triisopropylsilyl)ethynyl)naphthalene-1,3-diol

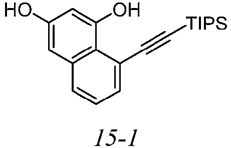

To a stirred solution of naphthalene-1,3-diol (20 g, 125 mmol), bromoethynyl)triisopropylsilane (34.3 g, 131 mmol), and potassium acetate (24.51 g, 250 mmol) in anhydrous dioxane (200 mL) was added dichloro(pcymene)ruthenium(II) dimer (7.65 g, 12.49 mmol) under a nitrogen atmosphere. The resulting mixture was stirred for 12h at 110°C. The reaction mixture was cooled to ambient temperature and was filtered through celite. The celite bed was washed with EtOAc (2x100 mL), the filtrate was combined and concentrated under vacuum to obtain a crude residue. The crude was purified by flash column (silica 100-200 mesh) chromatography using 12-15% ethyl acetate in petroleum ether as eluent. Pure fractions were concentrated under reduced pressure to obtain 8-((triisopropylsilyl)ethynyl)naphthalene-1,3-diol (30 g, 85 mmol, 67.7 %yield). LCMS (ESI) m/z: 341.55 [M+H]⁺.

### Preparation of Intermediate 15-2: 3-(methoxymethoxy)-8-((triisopropylsilyl)ethynyl)naphthalen-1-ol

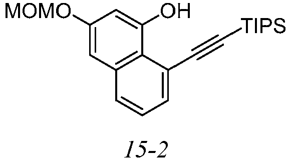

To a stirred solution of 8-((triisopropylsilyl)ethynyl)naphthalene-1,3-diol (25 g, 73.4 mmol) in anhydrous DCM (300 mL) under nitrogen was added DIPEA (38.5 mL, 220 mmol) at -10°C. After 10 min, MOM-Cl (6.13 mL, 81 mmol) was added to the reaction mixture dropwise over 20 min under a nitrogen atmosphere. The resulting mixture was stirred for 2h at the same temperature. The reaction mixture was diluted with 100 mL of DCM and washed with 200 mL of brine. The combined organics were dried over anhydrous Na₂SO₄, filtered and concentrated under reduced pressure to yield a crude residue. The crude material was purified by flash column (silica 100-200 mesh) chromatography using 5-10% ethyl acetate in petroleum ether as eluent. Pure fractions were concentrated under reduced pressure to obtain 3-(methoxymethoxy)-8-((triisopropylsilyl)ethynyl)naphthalen-1-ol (21 g, 53.5 mmol, 72.9 % yield) as a pale yellow oil. LCMS (ESI) m/z: 385.60 [M+H]⁺.

### Preparation of Intermediate 15-3: 3-(methoxymethoxy)-8-((triisopropylsilyl)ethynyl)naphthalen-1-yl pivalate

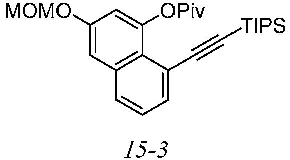

To a stirred solution of 3-(methoxymethoxy)-8-((triisopropylsilyl)ethynyl)naphthalen-1-ol (20 g, 52.0mmol), TEA (21.75 mL, 156 mmol), DMAP (1.271 g, 10.40 mmol) in anhydrous DCM (200 mL) at -10°C under a nitrogen atmosphere was added pivaloyl chloride (12.80 mL, 104 mmol) dropwise for 10 min. The resulting mixture was stirred for 2 h at room temperature under nitrogen. The reaction mixture was then diluted with 200 mL of DCM and washed with brine (200 mL). The organics were dried over anhydrous Na₂SO₄, filtered and concentrated under reduced pressure to yield a crude residue. The crude was purified by flash column (silica 100-200 mesh) chromatography using 10-15% ethyl acetate in petroleum ether as eluent. Pure fractions were concentrated under reduced pressure to obtain 3-(methoxymethoxy)-8-((triisopropylsilyl)ethynyl)naphthalen-1-yl pivalate (23 g, 49.1 mmol, 94 % yield) as a pale yellow oil. LCMS (ESI) m/z: 469.2 [M+H]⁺.

### Preparation of Intermediate 15-4: 8-ethynyl-3-(methoxymethoxy)naphthalen-1-yl pivalate

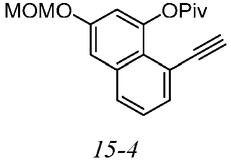

To a stirred solution of 3-(methoxymethoxy)-8-((triisopropylsilyl)ethynyl)naphthalen-1-yl pivalate (12 g, 25.6 mmol) in anhydrous DMF (130 mL) at room temperature under a nitrogen atmosphere was added anhydrous CsF (27.2 g, 179 mmol). The reaction mixture was stirred for 2 h. The reaction mixture was diluted with DCM (200 mL) and washed with brine (200 mL). The organics were dried over anhydrous Na₂SO₄, filtered and concentrated under reduced pressure to yield a crude residue. The crude was purified by flash column (silica 100-200 mesh) chromatography using 5-8% ethyl acetate in petroleum ether as eluent. Pure fractions were concentrated under reduced pressure to obtain 8-ethynyl-3-(methoxymethoxy)naphthalen-1-yl pivalate(7 g, 20.62 mmol, 81 % yield). LCMS (ESI) m/z: 313.3 [M+H]⁺.

### Preparation of Intermediate 15-5: 8-ethyl-3-methoxymethoxy)naphthalen-1-ylpivalate

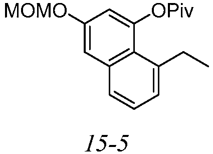

To a stirred solution of 8-ethynyl-3-(methoxymethoxy)naphthalen-1-yl pivalate (7 g, 22.41 mmol) in anhydrous methanol (70 mL) was added Pd/C (1.4 g, 13.16 mmol) at 25 °C. The suspension was degassed under reduced pressure and purged with H₂ several times. The mixture was stirred under H₂ atmosphere (1 atm) at 25 °C for 5 hours. The reaction mixture was filtered and concentrated under reduced pressure to obtain a crude residue. The crude material was purified by flash column (silica 100-200 mesh) chromatography using 15-20% ethyl acetate in petroleum ether as eluent. Pure fractions were concentrated under reduced pressure to provide 8-ethyl-3-methoxymethoxy)naphthalen-1-ylpivalate (6.56 g, 17.83 mmol, 80 % yield) as pale yellow oil. LCMS (ESI) m/z: 316.1 [M+H]⁺.

### Preparation of Intermediate 15-6: 8-ethyl-3-methoxymethoxy)naphthalen-1-ol

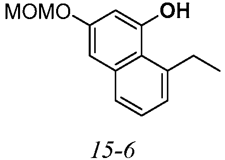

To a stirred solution of 8-ethyl-3-(methoxymethoxy)naphthalen-1-yl pivalate (10 g, 31.6 mmol) in THF:Water:MeOH (5:1:5) was added anhydrous LiOH (1.135 g, 47.4 mmol) at room temperature under nitrogen. The resulting mixture was stirred for 3 h at the same temperature. The reaction mixture was concentrated under reduced pressure to remove the MeOH. The reaction mixture was diluted with EtOAc (150 mL) and washed with brine (200 mL). The organics were dried over anhydrous Na₂SO₄, filtered, and concentrated under reduced pressure to yield a crude residue. The crude material was purified by flash column (silica 100-200 mesh) chromatography using 25-30% ethyl acetate in petroleum ether as eluent. Pure fractions were concentrated under reduced pressure to obtain 8-ethyl-3-methoxymethoxy)naphthalen-1-ol (6 g, 25.8 mmol, 82 % yield). LCMS (ESI) m/z: 233.2 [M+H]⁺.

### Preparation of Intermediate 15-7: 8-ethyl-3-(methoxymethoxy)naphthalen-1-yl trifluoromethanesulfonate

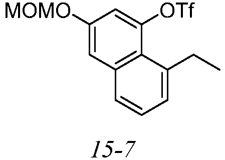

To a stirred solution of 8-ethyl-3-(methoxymethoxy)naphthalen-1-ol (3 g, 12.92 mmol) and DIPEA (22.50 mL, 129 mmol) in anhydrous dichloromethane (50 mL) at -40 °C under nitrogen was added Tf₂O (2.182 mL, 12.92 mmol) dropwise. The resultant reaction mixture was allowed to warm to room temperature and stirred for 1h. The reaction mixture was diluted with cold water (500mL) and extracted with DCM (2 X 500 mL). The combined organic layers were washed with brine (50 mL), dried over anhydrous Na₂SO₄, and concentrated under reduced pressure to obtain a crude residue. The crude compound was purified by flash column (Silicagel 100-200) chromatography using 5-10% ethyl acetate in petroleum ether as eluent. Pure fractions were concentrated under reduced pressure to afford 8-ethyl-3-(methoxymethoxy)naphthalen-1-yl trifluoromethanesulfonate (3.5 g, 9.03 mmol, 69.9 % yield) as a pale yellow oil. LCMS (ESI) m/z: 365.3 [M+H]⁺.

### Preparation of Intermediate 15-8: 2-(8-ethyl-3-(methoxymethoxy)naphthalen-1-yl)-4,4,5,5-tetramethyl-1,3,2-dioxaborolane

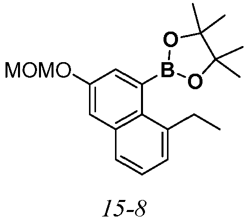

To a stirred solution of 8-ethyl-3-(methoxymethoxy)naphthalen-1-yl trifluoromethanesulfonate (7.4 g, 20.31 mmol) in 1,4-dioxane (80 mL) was added bispinacalatoborane (12.89 g, 50.8 mmol) and potassium acetate (5.98 g, 60.9 mmol). The mixture was degassed and purged with nitrogen for 5 min and PdCl₂(dppf) (1.659 g, 2.031 mmol) was added. The resulting mixture was stirred for 3 h at 100 °C temperature under nitrogen. The reaction mixture was diluted with water (50 mL) and extracted with ethyl acetate (250 mL). The combined organic layers were washed with brine (50 mL) dried over anhydrous Na₂SO₄ and concentrated under reduced pressure to obtain a crude residue. The crude was purified by flash column (silica 100-200 mesh) chromatography by using 2-4% ethyl acetate in petroleum ether as eluent. Pure fractions were concentrated under reduced pressure to afford a brown gum which was again purified by reverse phase column chromatography using 80% Acetonitrile in 0.01% of Ammonium formate in water as eluent. Pure fractions were concentrated under reduced pressure to obtain 2-(8-ethyl-3-(methoxymethoxy)naphthalen-1-yl)-4,4,5,5-tetramethyl-1,3,2-dioxaborolane (4.5 g, 13.10 mmol, 64.5% yield). LCMS (ESI) m/z: 344.2 [M+H]⁺. ¹H-NMR (400 MHz, CDCl₃): δ 7.62 (dd, J = 0.80, 8.00 Hz, 1H), 7.36-7.44 (m, 3H), 7.27 (t, J = 0.40 Hz, 1H), 5.31 (s, 2H), 3.53 (s, 3H), 3.21 (q, J = 7.20 Hz, 2H), 1.46 (s, 12H), 1.38 (t, J = 7.60 Hz, 3H) ppm.

### Preparation of Intermediate 16-1: 7-fluoro-8-((triisopropylsilyl)ethynyl)naphthalene-1,3-diol

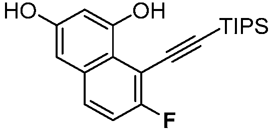

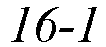

To a stirred solution of 7-fluoronaphthalene-1,3-diol (20 g, 112 mol), (bromoethynyl)triisopropylsilane (30.8 g, 118 mmol), and potassium acetate (22.03 g, 225 mmol) in anhydrous dioxane (200 mL) was added dichloro(p-cymene)ruthenium(II) dimer (6.87 g, 11.23 mmol) under a nitrogen atmosphere at room temperature. The resulting mixture was stirred for 12 h at 110°C under a nitrogen atmosphere. The resulting reaction mixture was filtered through Celite and the Celite bed was washed with EtOAc (2 x 50 mL). The filtrate was collected and concentrated under a vacuum to obtain the crude residue. The crude material was purified by flash column (silica 100-200 mesh) chromatography using 5-10% ethyl acetate in petroleum ether as eluent. Pure fractions were concentrated under reduced pressure to obtain 7-fluoro-8-((triisopropylsilyl)ethynyl)naphthalene-1,3-diol (32g, 88 mmol, 79% yield) as a pale yellow oil. LCMS (ESI) m/z: 359.1 [M+H]⁺.

### Preparation of Intermediate 16-2: 7-fluoro-3-(methoxymethoxy)-8-((triisopropylsilyl)ethynyl)naphthalen-1-ol.

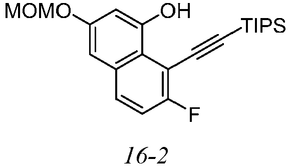

To a stirred solution of 7-fluoro-8-((triisopropylsilyl)ethynyl)naphthalene-1,3-diol (10 g, 27.9 mmol) in anhydrous DCM (100 mL) under nitrogen was added DIEA (14.61 mL, 84 mmol) at 0 °C. The reaction mixture was stirred at the same temperature for 10 min before the dropwise addition of MOM-Cl (2.54 mL, 33.5 mmol) to the reaction mixture. The resulting mixture was stirred for 1 h at room temperature under nitrogen. The reaction mixture was diluted with cold water (50 mL) and extracted with DCM (2 x 100 mL). The combined organic layers were washed with brine (50 mL), dried over anhydrous Na₂SO₄ and concentrated under reduced pressure to obtain a crude residue. The crude material was purified by flash column (silica 100-200 mesh) chromatography using 5-10% ethyl acetate in petroleum ether as eluent. Pure fractions were concentrated under reduced pressure to obtain 7-fluoro-3-(methoxymethoxy)-8-((triisopropylsilyl)ethynyl)naphthalen-1-ol (10 g, 24.84 mmol, 89% yield). LCMS (ESI) m/z: 403.1 [M+H]⁺.

### Preparation of Intermediate 16-3: 7-fluoro-3-(methoxymethoxy)-8-((triisopropylsilyl)ethynyl)naphthalen-1-yl pivalate

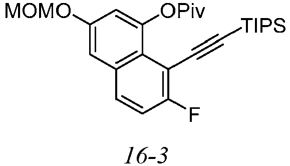

To a stirred solution of 7-fluoro-3-(methoxymethoxy)-8-((triisopropylsilyl)ethynyl)naphthalen-1-ol (17 g, 42.2 mmol) in anhydrous DCM (170 mL) at 0 °C under a nitrogen atmosphere were added TEA (17.66 mL, 127 mmol) and DMAP (1.032 g, 8.45 mmol). The reaction mixture was stirred at the same temperature for 10 min before the dropwise addition of pivaloyl chloride (6.23 mL, 50.7 mmol). The resulting reaction mixture was stirred at room temperature for 2 h. The reaction mixture was diluted with cold water (50 mL) and extracted with DCM (2 X 100 mL). The combined organic layers were washed with brine (200 mL), dried over anhydrous Na₂SO₄ and concentrated under reduced pressure to obtain a crude residue. The crude material was purified by flash column (silica 100-200 mesh) chromatography using 20-25% ethyl acetate in petroleum ether as eluent. Pure fractions were concentrated under reduced pressure to obtain 7-fluoro-3-(methoxymethoxy)-8-((triisopropylsilyl)ethynyl)naphthalen-1-yl pivalate (19 g, 35.1 mmol, 83 % yield). LCMS (ESI) m/z: 487.2 [M+H]⁺.

### Preparation of Intermediate 16-4: 7-fluoro-3-(methoxymethoxy)-8-((triisopropylsilyl)ethynyl)naphthalen-1-yl pivalate

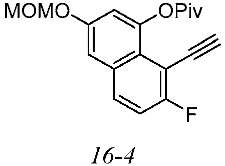

To a stirred solution of 7-fluoro-3-(methoxymethoxy)-8-((triisopropylsilyl)ethynyl)naphthalen-1-yl pivalate (20 g, 41.1 mmol) in anhydrous DMF (150 mL) under a nitrogen atmosphere was added CsF (43.7 g, 288 mmol) at room temperature. The resulting mixture was stirred for 5 h at room temperature. The reaction mixture was diluted with DCM (200 mL) and washed with brine (200 mL). Organics were dried over anhydrous Na₂SO₄, filtered and concentrated under reduced pressure to yield a crude residue. The crude material was purified by flash column (silica 100-200 mesh) chromatography using 5-8% ethyl acetate in petroleum ether as eluent. Pure fractions were concentrated under reduced pressure to obtain 8-ethynyl-7-fluoro-3-(methoxymethoxy)naphthalen-1-yl pivalate (12 g, 32.7 mmol, 80 % yield) as a pale yellow oil. LCMS (ESI) m/z: 331.2 [M+H]⁺.

### Preparation of Intermediate 16-5: 8-ethyl-7-fluoro-3-(methoxymethoxy)naphthalen-1-yl pivalate.

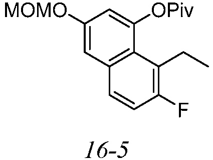

To a stirred solution of 8-ethynyl-7-fluoro-3-(methoxymethoxy)naphthalen-1-yl pivalate (15 g, 45.4 mmol) in ethanol (150 mL) was added 10% Pd/C (3 g, 28.2 mmol) at 25 °C. The suspension was degassed under reduced pressure and purged with H₂ several times. The mixture was stirred under H₂ (1 Atm) at 25 °C for 5 hours. The reaction mixture was filtered and concentrated under reduced pressure to obtain a crude residue. The crude was purified by flash column (silica 100-200 mesh) chromatography using 15-20% ethyl acetate in petroleum ether as eluent. Pure fractions were concentrated under reduced pressure to provide 8-ethyl-7-fluoro-3-(methoxymethoxy)naphthalen-1-yl pivalate (14 g, 34.3 mmol, 76 % yield) as a pale yellow oil. LCMS (ESI) m/z: 335.1 [M+H]⁺.

### Preparation of Intermediate 16-6: 8-ethyl-7-fluoro-3-(methoxymethoxy)naphthalen-1-ol

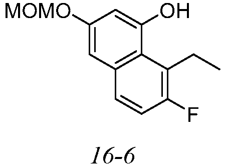

To a stirred solution of 8-ethyl-7-fluoro-3-(methoxymethoxy)naphthalen-1-yl pivalate (5 g, 14.95 mmol) in MeOH (50 mL) was added anhydrous LiOH (0.537 g, 22.43 mmol) at room temperature under nitrogen. The resulting mixture was stirred at the same temperature for 1 h. The reaction mixture was concentrated under reduced pressure to remove the MeOH. The reaction mixture was diluted with EtOAc (150 mL) and washed with brine (200 mL). The organics were dried over anhydrous Na₂SO₄, filtered and concentrated under reduced pressure to yield a crude residue. The crude material was purified by flash column (silica 100-200 mesh) chromatography using 25-30% ethyl acetate in petroleum ether as eluent. Pure fractions were concentrated under reduced pressure to obtain 8-ethyl-7-fluoro-3-(methoxymethoxy)naphthalen-1-ol (3.5g, 12.59 mmol, 84 % yield). LCMS (ESI) m/z: 251.2 [M+H]⁺.

### Preparation of Intermediate 16-7: 8-ethyl-7-fluoro-3-(methoxymethoxy)naphthalen-1-yl trifluorome thanesulfonate

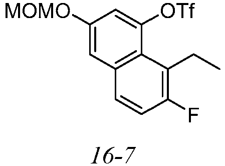

To a stirred solution of 8-ethyl-7-fluoro-3-(methoxymethoxy)naphthalen-1-ol (1.5 g, 5.99 mmol) and DIEA (3.66 mL, 20.98 mmol) in anhydrous dichloromethane (50 mL) at -10 °C was added Tf₂O (1.215 mL, 7.19 mmol) dropwise. The resultant reaction mixture was allowed to warm to room temperature and stirred for 1 h. The reaction mixture was diluted with cold water (500 mL) and then extracted with DCM (2 x 500 mL). The combined organic layers were washed with brine (50 mL), dried over anhydrous Na₂SO₄, filtered and concentrated under reduced pressure to obtain a crude residue. The crude compound was purified by flash column (Silicagel 100-200) chromatography using 5-10% ethyl acetate in petroleum ether as eluent. Pure fractions were concentrated under reduced pressure to afford 8-ethyl-7-fluoro-3-(methoxymethoxy)naphthalen-1-yl-trifluoromethanesulfonate (1.8 g, 4.66 mmol, 78 % yield) as a pale yellow oil. LCMS (ESI) m/z: 383.0 [M+H]⁺.

### Preparation of Intermediate 16-8: 2-(8- ethyl-7-fluoro-3-(methoxymethoxy)naphthalen-1-yl)-4,4,5,5-tetramethyl-1, 3, 2-dioxaborolane

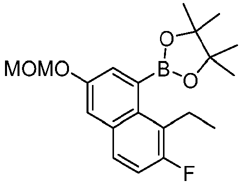

To a stirred solution of 8-ethyl-7-fluoro-3-(methoxymethoxy)naphthalen-1-yl trifluoromethanesulfonate (2.5 g, 6.54 mmol) in anhydrous 1,4-dioxane (80 mL) were added bispinacolatoborane (4.15 g, 16.35 mmol) and potassium acetate (1.925 g, 19.62 mmol) and the resulting mixture was degassed and purged with nitrogen for 5 min. Then, PdCl₂(dppf) (0.534 g, 0.654 mmol) was added to the reaction mixture. The resulting mixture was stirred for 2 h at 100 °C under a nitrogen atmosphere. The reaction mixture was then diluted with water (50 mL) and extracted with ethyl acetate (250 mL). The combined organic layers were washed with brine (50 mL) dried over anhydrous Na₂SO₄, filtered and concentrated under reduced pressure to obtain a crude residue. The crude maerial was purified by flash column (silica 100-200 mesh) chromatography using 2-4% ethyl acetate in petroleum ether as eluent. Pure fractions were concentrated under reduced pressure to afford a brown gum which was again purified by reverse phase column chromatography using 80% acetonitrile in 0.01% of ammonium formate in water as eluent. Pure fractions were concentrated under reduced pressure to obtain 22-(8-ethyl-7-fluoro-3-(methoxymethoxy)naphthalen-1-yl)-4,4,5,5-tetramethyl-1,3,2-dioxaborolane (1.8 g, 4.99 mmol, 76 % yield). LCMS (ESI) m/z: 329 [M-OCH₃]⁺. ¹H-NMR (400 MHz, CDCl3): δ 7.77 (t, J = 8.40 Hz, 1H), 7.54 (s, 1H), 7.37 (t, J = 9.60 Hz, 1H), 7.28 (s, 1H), 5.31 (s, 2H), 3.42 (s, 3H), 3.03 (d, J = 6.80 Hz, 2H), 1.39 (s, 12H), 1.19 (t, J = 7.20 Hz, 3H) ppm.

### Preparation of Intermediate 17-1: 1-(tert-butyl) 2-methyl-(S)-4,4-difluoropyrrolidine-1,2-dicarboxylate

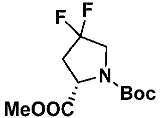

To a stirred solution of 1-(tert-butyl) 2-methyl (S)-4-oxopyrrolidine-1,2-dicarboxylate (30.0 g, 123 mmol) in DCM (20.0 mL) was added dropwise DAST (81 mL, 617 mmol) at 0 °C. The reaction mixture was stirred at ambient temperature for 16 h. The reaction mixture was quenched with water and extracted with dichloromethane. The combined organic layers were washed with water and saturated brine solution, dried over anhydrous Na₂SO₄, filtered, and concentrated under reduced pressure to afford the crude product. The crude product obtained was purified by column chromatography (Grace, 340g snap, dry pack) over silica (230-400 mesh) eluting with 20-50% ethyl acetate in petroleum ether. The desired fractions pooled and concentrated under reduced pressure to obtain 1-(tert-butyl) 2-methyl (S)-4,4-difluoropyrrolidine-1,2-dicarboxylate (28 g, 90 mmol, 73.4 % yield) as a pale brown liquid. LCMS-ELSD (ESI) m/z: 166.2 [M+H-Boc]⁺.

### Preparation of Intermediate 17-2: 1-(tert-butyl) 2-methyl-2-(3-((tertbutyldimethylsilyl)oxy)propyl)-4,4-difluoropyrrolidine-1,2-dicarboxylate

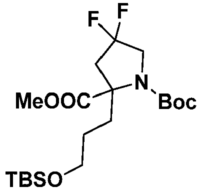

To a stirred solution of 1-(tert-butyl)-2-methyl (S)-4,4-difluoropyrrolidine-1,2-dicarboxylate (28 g, 106 mmol) in THF (300.0 mL) was added LiHMDS (158 mL, 158 mmol) at -45 °C dropwise. The reaction mixture was stirred at the same temperature for 30 min prior to the dropwise addition of 3-bromopropoxy)(tert-butyl)dimethylsilane (40.1 g, 158 mmol). The reaction mixture was stirred at the same temperature for 30 min and then slowly warmed to room temperature. The reaction mixture was quenched with saturated aq. ammonium chloride solution (15 mL) and then diluted with water (50 mL). The mixture was extracted with ethyl acetate (3x100 mL). The combined organic layers were dried over anhydrous Na₂SO₄, filtered and concentrated under vacuum. The residue was purified by silica gel column chromatography with 20-30% of ethyl acetate in petroleum ether to obtain the 1-(tert-butyl) 2-methyl-2-(3-((tertbutyldimethylsilyl)oxy)propyl)-4,4-difluoropyrrolidine-1,2-dicarboxylate (35 g, 80 mmol, 76% yield) as a pale brown liquid. LCMS-ELSD (ESI) m/z: 388.2 [M+H-Boc]⁺.

### Preparation of Intermediate 17-3: 1- (tert-butyl) 2-methyl-4,4-difluoro-2-(3-hydroxypropyl)pyrrolidine-1,2-dicarboxylate

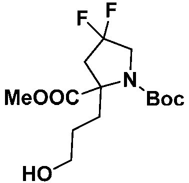

To a stirred solution of 1-(tert-butyl) 2-methyl-2-(3-((tertbutyldimethylsilyl)oxy)propyl)-4,4- difluoropyrrolidine-1,2-dicarboxylate (35.0 g, 80 mmol) in THF (50 mL) at 25 °C was added TBAF (1M in THF, 80 mL, 80 mmol) dropwise, and stirred at the same temperature for 4 h. The reaction mixture was quenched with saturated aq. ammonium chloride solution (90 mL) and diluted with ethyl acetate (100 mL). The mixture was extracted with ethyl acetate (3x150 mL). The combined organic layers were dried over anhydrous sodium sulphate and concentrated under reduced pressure to obtain the crude residue as a colorless oil. The crude was purified by silica gel column chromatography using 50% ethyl acetate in petroleum ether to obtain 1- (tert-butyl) 2-methyl-4,4-difluoro-2-(3-hydroxypropyl)pyrrolidine-1,2-dicarboxylate (20 g, 57.9 mmol, 72.4 % yield) as a pale brown liquid.. LCMS-ELSD (ESI) m/z: 224.2 [M+H-Boc]⁺.

### Preparation of Intermediate 17-4: 1-(tert-butyl) 2-methyl-4,4-difluoro-2-(3-iodopropyl)pyrrolidine-1, 2-dicarboxylate

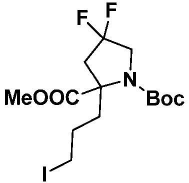

To a stirred solution of 1-(tert-butyl) 2-methyl-4,4-difluoro-2-(3-hydroxypropyl)pyrrolidine-1,2- dicarboxylate (20 g, 61.9 mmol) in DCM (100 mL) at 0 °C was added triphenylphosphine (48.7 g, 186 mmol), imidazole (8.42 g, 124 mmol) and reaction mixture was stirred for 10 min before the addition of iodine (62.8 g, 247 mmol). The reaction mixture was stirred at room temperature for 12 h and then quenched with sat. aq. sodium thiosulfate solution (30 mL). The suspension was extracted with dichloromethane (2x200 mL). The combined organic layers were dried over anhydrous sodium sulphate and concentrated under reduced pressure to obtain the crude residue. This crude was purified by silica gel column chromatography using 10-20% ethyl acetate in petroleum ether to obtain 1-(tert-butyl) 2-methyl-4,4-difluoro-2-(3-iodopropyl)pyrrolidine-1,2-dicarboxylate (20 g, 46.1 mmol, 75 % yield) as colourless oil. LCMS-ELSD (ESI) m/z: 333.0 [M+H-Boc]⁺.

### Preparation of Intermediate 17-5: methyl-4,4-difluoro-2-(3-iodopropyl)pyrrolidine-2- carboxylate

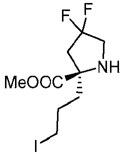

To a stirred solution of 1-(tert-butyl) 2-methyl-4,4-difluoro-2-(3-iodopropyl)pyrrolidine-1,2- dicarboxylate (15 g, 34.6 mmol) in DCM (100 mL) was added HCl in 1,4-dioxane (4M, 8.66 mL, 34.6 mmol) at 0 °C. The reaction mixture was stirred at ambient temperature for 16 h. The reaction mixture was then concentrated under reduced pressure at room temperature to obtain the crude residue of methyl (S)-4,4-difluoro-2-(3-iodopropyl)pyrrolidine-2-carboxylate.HCl (11.5 g, 31.1 mmol, 90% yield), which was used in the next step without any further purification. LCMS-ELSD (ESI) m/z: 334.1 [M+H]⁺.

### Preparation of Intermediate 17-6: methyl 2,2-difluorotetrahydro-1H-pyrrolizine-7a(5H)-carboxylate

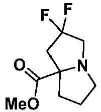

To a stirred solution of methyl-4,4-difluoro-2-(3-hydroxypropyl)pyrrolidine-2-carboxylate.HCl (11.5 g, 51.5 mmol) in THF (120.0 mL) was added TEA (35.9 mL, 258 mmol) at 0 °C followed by heating at 45 °C for 16 h. The reaction mixture was concentrated under reduced pressure to obtain the crude residue which was purified by column chromatography (alumina-neutral) using 40-50% ethyl acetate in petroleum ether to obtain the methyl 2,2-difluorotetrahydro-1H-pyrrolizine-7a(5H)-carboxylate (6 g, 43.5 mmol, 81% yield) as a brown liquid.

### Preparation of Intermediate 17-7: (2,2-difluorotetrahydro-1H-pyrrolizin-7a(5H)-yl)methanol

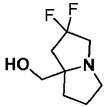

To a stirred solution of methyl 2,2-difluorotetrahydro-1H-pyrrolizine-7a(5H)-carboxylate (12.0 g, 58.5 mmol) in anhydrous THF (20.0 mL) under nitrogen at 0 °C was added LiAlH₄ (117 mL, 117 mmol) dropwise over 10 min. The mixture was stirred at this temperature for 1 h before quenching with saturated aq. ammonium chloride (5 mL) at 0 °C. Once the effervescence had ceased, anhydrous sodium sulphate was added to the reaction mixture, followed by dichloromethane (20 mL). The reaction mixture was stirred for 20 min and filtered. The filtrate was dried over anhydrous sodium sulphate and concentrated under reduced pressure to obtain the crude residue of (2,2-difluorotetrahydro-1H-pyrrolizin-7a(5H)- yl)methanol (4.1 g, 23.02 mmol, 39.4 % yield) as a pale yellow liquid. LCMS-ELSD (ESI) m/z: 178.1 [M+H]⁺.

### Preparation of Intermediate 18-1: 7-fluoro-3-(methoxymethoxy)-8-{2-[tris(propan-2-yl)silyl]ethynyl}naphthalen-1-yl trifluoromethanesulfonate

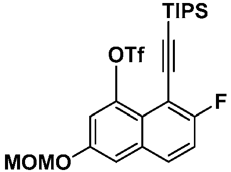

To a solution of 7-fluoro-3-(methoxymethoxy)-8-{2-[tris(propan-2-yl)silyl]ethynyl}naphthalen-1-ol (Intermediate 16-2 ) (8.6g, 22.25 mmol) and DIPEA (11.66 mL, 66.7 mmol) in DCM (35 mL) was added triflic anhydride (5.64 mL, 33.4 mmol) dropwise at -40 °C and the reaction mixture was stirred at the same temperature for 30 min. The reaction mixture was diluted with water and extracted with DCM. The combined organic layers were washed with water, brine, dried over anhydrous Na₂SO₄ and concentrated under reduced pressure to provide the crude residue, which was purified by silica gel column chromatography using CombiFlash instrument (80 g RediSep^{®} column, 5 to 10% EtOAc in petroleum ether) to afford 7-fluoro-3-(methoxymethoxy)-8-{2-[tris(propan-2-yl)silyl]ethynyl}naphthalen-1-yl trifluoromethanesulfonate (8.5 g, 16.39 mmol, 73.7 % yield) as a yellow oil. ¹H NMR (400 MHz, DMSO-d₆) δ ppm 8.12 (dd, J = 9.3, 5.8 Hz, 1H), 7.78 (d, J = 2.5 Hz, 1H), 7.65 (t, J = 9.0 Hz, 1H), 7.51 (d, J = 2.0 Hz, 1H), 5.37 (s, 2H), 3.43 (s, 3H), 1.31 - 1.08 (m, 21H).

### Preparation of Intermediate 18-2: {2-[2-fluoro-6-(methoxymethoxy)-8-(4,4,5,5-tetramethyl-1,3,2-dioxaborolan-2-yl)naphthalen-1-yl]ethynyl}tris(propan-2-yl)silane

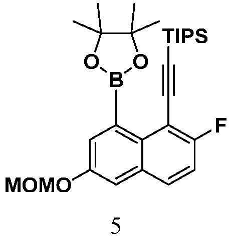

To a degassed solution of 7-fluoro-3-(methoxymethoxy)-8-{2-[tris(propan-2-yl)silyl]ethynyl}naphthalen-1-yl trifluoromethanesulfonate (4.0 g, 7.48 mmol), potassium acetate (2.203 g, 22.45 mmol) and bis(pinacolato)diboron (3.80 g, 14.96 mmol) in toluene (40 mL) was added 1,1-bis(diphenylphosphino)ferrocene]dichloropalladium(II) (0.547 g, 0.748 mmol) and the reaction mixture was heated at 120 °C for 3 h. The reaction mixture was cooled, filtered through a bed of Celite and washed with ethyl acetate. The filtrate was washed with water, brine, dried over anhydrous Na₂SO₄, filtered and concentrated under reduced pressure to provide the crude residue, which was purified by silica gel column chromatography using a CombiFlash instrument (40 g RediSep^{®} column, 10% EtOAc in petroleum ether) to afford {2-[2-fluoro-6-(methoxymethoxy)-8-(4,4,5,5-tetramethyl-1,3,2-dioxaborolan-2-yl)naphthalen-1-yl]ethynyl}tris(propan-2-yl)silane (2.4 g, 4.68 mmol, 62.6 % yield) as pale yellow solid. MS (ESI) m/z 513.4 [M+1]⁺.

### BIOLOGICAL ACTIVITY

### KRAS^{G12D} RAF Disruption Assay

Recombinant GMPPNP-loaded KRAS G12D (5 nM) was treated with compound at room temperature for 20 minutes in assay buffer (50mM Tris pH 7.5, 100mM NaCl, 1mM MgCl2, 1mM DTT, 100ug/mL BSA). Recombinant GST-RAF1 RBD (9 nM) was added, followed by the addition of SA-Tb (0.25 nM), and the reaction mixture was incubated for 3 hours. HTRF signal was measured (PerkinElmer Envision), the signal ratio (λₑₘ 520/ λₑₘ 495) was calculated, and IC₅₀ values were calculated from the dose-response curve.

### KRAS^{G12D} Nucleotide Exchange Assay

Recombinant GDP-loaded KRAS G12D (20 nM) was treated with compound at room temperature for 20 minutes in assay buffer (10 mM Hepes pH 7.4, 150 mM NaCl, 5 mM MgCl₂, 0.0025% Igepal-CA630, 0.05% BSA, 1 mM DTT, 0.5 nM SA-Tb). BIODIPY-labeled GDP (400 nM) and recombinant SOS (10 nM) were added, and the reaction was incubated for 30 minutes. HTRF signal was measured (PerkinElmer Envision), the signal ratio (λₑₘ 520/ λₑₘ 495) was calculated, and IC₅₀ values were calculated from the dose-response curve.

Table 9 shows IC50 values of compounds described herein.

**Table 9**

| *Example #* | *KRASG12D RAF Disruption Assay (IC₅₀,* □*M)* | *KRASG12D Nucleotide Exchange Assay (IC₅₀,* □*M)* |
|---|---|---|
| 1-1 | 41.99 | 33.33 |
| 1-2 | >100 | 13.66 |
| 2-1 | 6.19 | 0.04 |
| 2-2 | 10.88 | 0.14 |
| 3 | 0.05 | <0.002 |
| 4-1 | 0.06 | 0.005 |
| 4-2 | 0.43 | 0.006 |
| 4-3 | 0.52 | 0.02 |
| 4-4 | 1.81 | 0.010 |
| 5 | 2.87 | <0.002 |
| 6 | 3.68 | 0.01 |
| 7 | 0.48 | <0.002 |
| 8-1 | 5.7 | 0.04 |
| 8-2 | 19.6 | 1.3 |
| 8-3 | 17.3 | 0.07 |
| 8-4 | 37.8 | 7.7 |
| 8-5 | 0.07 | NA |
| 8-6 | 3.6 | 0.07 |
| 8-7 | 0.51 | 0.006 |
| 8-8 | 15.5 | 0.3 |
| 8-9 | 0.7 | 0.005 |
| 8-10 | 14.2 | 0.50 |
| 9-1 | 5.1 | 0.04 |
| 9-2 | 2.0 | 0.03 |
| 9-3 | 3.0 | 0.05 |
| 9-4 | 0.2 | 0.02 |
| 10-1 | 1.0 | 0.007 |
| 10-2 | 1.0 | 0.006 |
| 10-3 | 2.7 | 0.01 |
| 10-4 | 41.5 | 0.11 |
| 10-5 | 81 | 0.9 |
| 10-6 | >100 | 0.2 |
| 10-7 | 12.1 | 0.06 |
| 10-8 | 2.6 | 0.02 |
| 10-9 | 51.0 | 0.41 |
| 10-10 | 2.4 | 0.02 |
| 11-1 | 2.7 | 0.06 |
| 11-2 | 1.0 | 0.01 |
| 11-3 | 2.0 | 0.30 |
| 11-4 | 43.1 | 2.2 |
| 11-5 | 3.2 | 0.03 |
| 11-6 | 0.17 | 0.02 |
| 11-7 | 5.1 | 0.06 |
| 11-8 | 54.4 | 2.0 |
| 12-1 | 6.3 | 0.03 |
| 12-2 | 80.0 | 0.33 |
| 12-4 | >100 | 1.1 |
| 13-1 | 0.22 | 0.01 |

## Claims

1. A compound of formula (I): 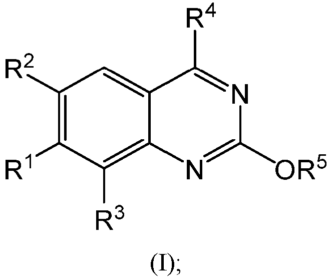 or a pharmaceutically acceptable salt thereof; wherein:
R¹ is aryl or heteroaryl, wherein the aryl and the heteroaryl are optionally substituted with one, two, three, four, or five substituents independently selected from C₁-C₃alkyl, C₂-C₄alkenyl, C₂-C₄alkynyl, amino, aminoC₁-C₃alkyl, C₃-C₄cycloalkyl, halo, haloC₁-C₃alkoxy, haloC₁-C₃alkyl, hydroxy, and hydroxyC₁-C₃alkyl;
R² and R³ are independently selected from hydrogen, C₁-C₃alkoxy, C₁-C₃alkyl, cyano, halo, haloC₁-C₃alkyl, and hydroxy;
R⁴ is selected from: 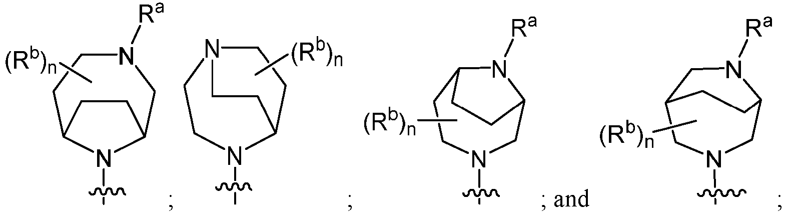 wherein
R^{a} is hydrogen or C₁-C₃alkyl;
n is 0, 1, 2, 3, or 4;
each R^{b} is independently selected from C₁-C₃alkyl, C₃-C₆cycloalkyl, halo, and hydroxy; or, alternatively, two geminal R^{b} groups, together with the carbon atom to which they are attached, can form a 3- to 6-membered cycloalkyl ring; and 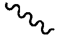
denotes the point of attachment to the parent molecular moiety;
R⁵ is -(C₁-C₃alkyl)-R⁶ or -(C₁-C₆alkyl)NR^{c}R^{d}, wherein R⁶ is selected from:
a C₃-C₆cycloalkyl substituted with NR^{c}R^{d}(C₁-C₃alkyl); and
a five- to ten-membered monocyclic, bicyclic, or tricyclic ring containing one nitrogen atom and optionally a second heteroatom selected from oxygen or nitrogen, wherein the ring contains zero to three double bonds and wherein the ring is optionally substituted with one, two, or three groups independently selected from C₁-C₃alkoxy, C₁-C₃alkyl, halo, haloC₁-C₃alkoxy, haloC₁-C₃alkyl, and hydroxy; wherein
R^{c} and R^{d}, together with the nitrogen atom to which they are attached, form a five-to ten-membered ring monocyclic or bicyclic ring optionally containing one additional heteroatom selected from nitrogen, oxygen, and sulfur, wherein the ring is optionally substituted with one, two, or three groups independently selected from C₁-C₃alkoxy, C₁-C₃alkoxyC₁-C₃alkyl, C₁-C₃alkyl, benzyl, halo, haloC₁-C₃alkyl, hydroxy, hydroxyC₁-C₃alkyl, and oxo; or
one of R^{c} and R^{d} is selected from hydrogen and C₁-C₃alkyl and the other is selected from hydrogen, C₁-C₃alkyl, C₁-C₃alkoxycarbonyl, and C₁-C₃alkylcarbonyl.

2. The compound of claim 1, or a pharmaceutically acceptable salt thereof; wherein R⁴ is selected from:

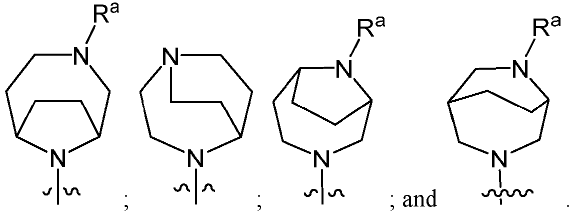

3. The compound of claim 1 or 2, or a pharmaceutically acceptable salt thereof, wherein R² and R³ are each halo.

4. The compound of any one of claims 1 to 3, or a pharmaceutically acceptable salt thereof, wherein R⁵ is -(C₁-C₃alkyl)-R⁶.

5. The compound of any one of claims 1 to 4, or a pharmaceutically acceptable salt thereof, wherein R⁵ is selected from: 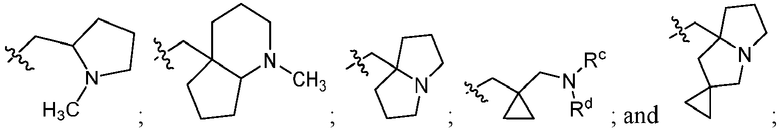 wherein each ring is optionally substituted with 1 or 2 groups independently selected from C₁-C₃alkoxy, C₁-C₃alkyl, halo, haloC₁-C₃alkyl, and hydroxy.

6. The compound of claim any one of claims 1 to 4, or a pharmaceutically acceptable salt thereof, wherein
(i) R⁵ is 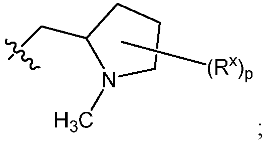 wherein p is 0, 1, or 2; and wherein each R^{x} is independently selected from C₁-C₃alkoxy, C₁-C₃alkyl, halo, haloC₁-C₃alkyl, and hydroxy; or
(ii) R⁵ is 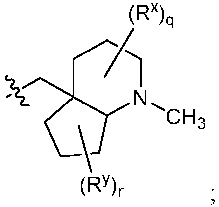 wherein q and r are each independenty 0 or 1; and wherein R^{x} and R^{y} are independently selected from C₁-C₃alkoxy, C₁-C₃alkyl, halo, haloC₁-C₃alkyl, and hydroxy; or
(iii) R⁵ is 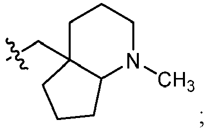 or
(iv) R⁵ is 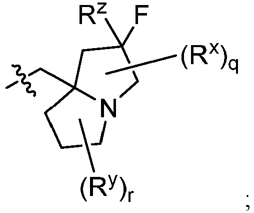 wherein q and r are each independenty 0 or 1; wherein R^{x} and R^{y} are independently selected from C₁-C₃alkoxy, C₁-C₃alkyl, halo, haloC₁-C₃alkyl, and hydroxy; and wherein R^{z} is hydrogen or fluoro; or
(v) R⁵ is 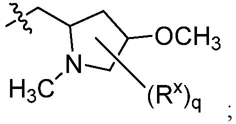 wherein q is 0 or 1; and wherein R^{x} is selected from C₁-C₃alkoxy, C₁-C₃alkyl, halo, haloC₁-C₃alkyl, and hydroxy; or
(vi) R⁵ is 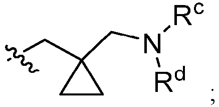 or
(vii) R⁵ is 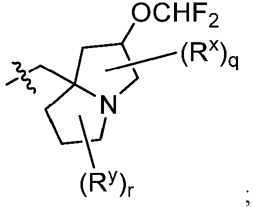 wherein q and r are each independenty 0 or 1; and wherein R^{x} and R^{y} are independently selected from C₁-C₃alkoxy, C₁-C₃alkyl, halo, haloC₁-C₃alkyl, and hydroxy; or
(viii) R⁵ is 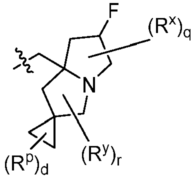 wherein q, r, and d are each independenty 0 or 1; and wherein R^{x}, R^{y}, and R^{p} are independently selected from C₁-C₃alkoxy, C₁-C₃alkyl, halo, haloC₁-C₃alkyl, and hydroxy; or
(ix) R⁵ is

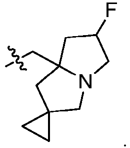

7. The compound of any one of claims 1 to 6, or a pharmaceutically acceptable salt thereof, wherein
(i) R¹ is naphthyl, wherein the naphthyl is substituted with a hydroxy group and is optionally further substituted with one or two additional groups selected from C₁-C₃alkyl, C₂-C₄alkynyl, and halo; or
(ii) R¹ is 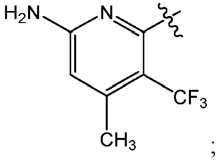 or
(iii) R¹ is 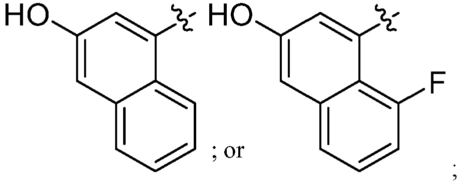 or
(iv) R¹ is 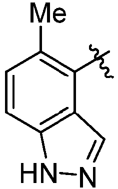 or
(v) R¹ is

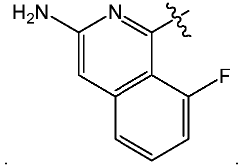

8. The compound of claim 1, or a pharmaceutically acceptable salt thereof, wherein
R² is hydrogen;
R³ is fluoro;
R¹ is selected from 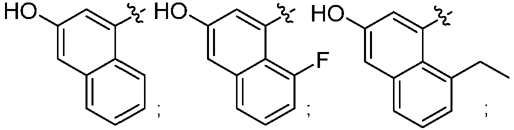 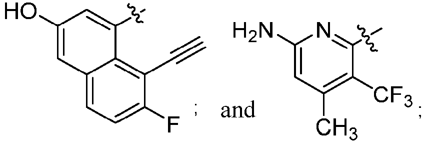 and
R⁵ is selected from 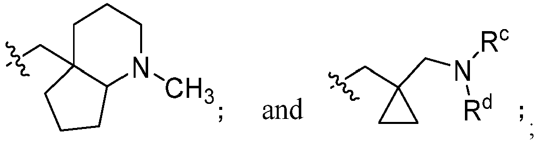 wherein 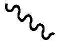
denotes the point of attachment to the parent molecular moiety.

9. The compound of claim 1, or a pharmaceutically acceptable salt thereof, wherein
R² is chloro;
R³ is fluoro;
R¹ is selected from 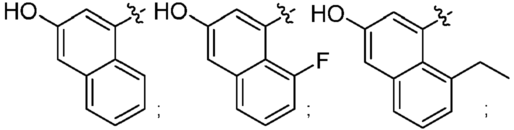 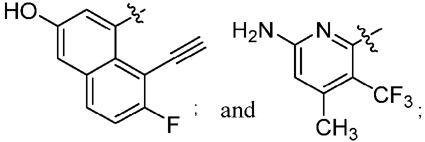 and
R⁵ is selected from 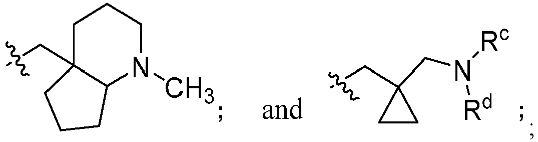 wherein 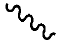
denotes the point of attachment to the parent molecular moiety.

10. The compound of claim 1 of formula (II): 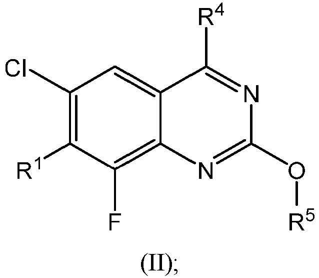 or a pharmaceutically acceptable salt thereof, wherein:
R¹ is selected from 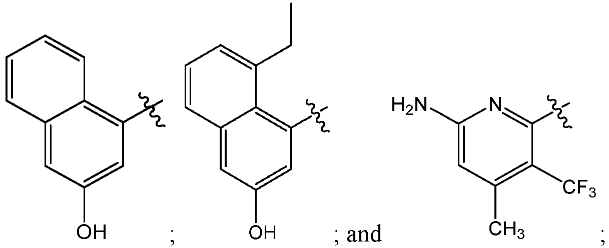 wherein 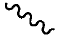 denotes the point of attachment to the parent molecular moiety;
R⁴ is selected from: 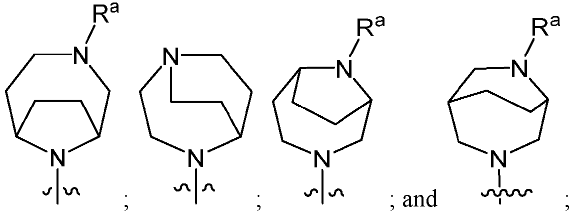 wherein
R^{a} is hydrogen or C₁-C₃alkyl; and 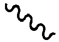
denotes the point of attachment to the parent molecular moiety; and
R⁵ is selected from: 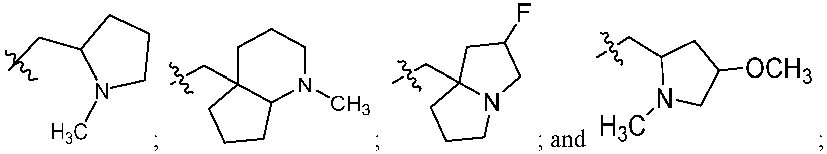 wherein 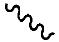
denotes the point of attachment to the parent molecular moiety.

11. A compound of claim 1 selected from: 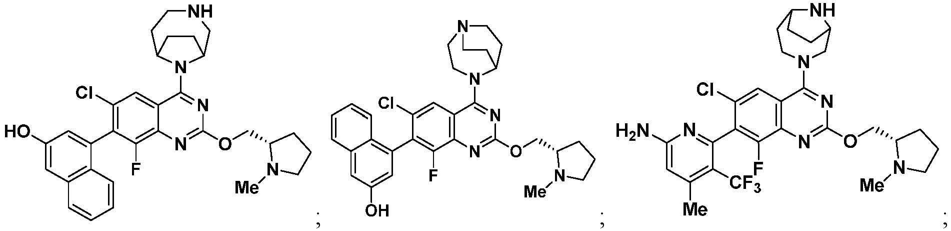 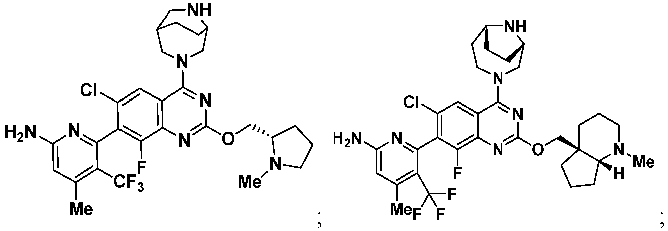 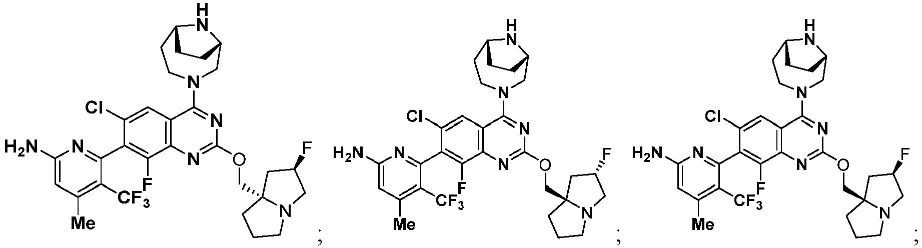 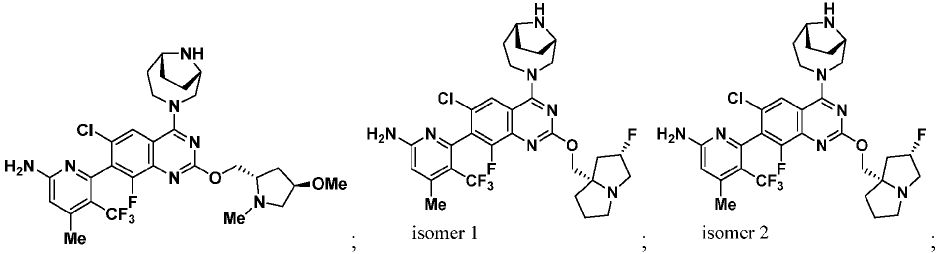 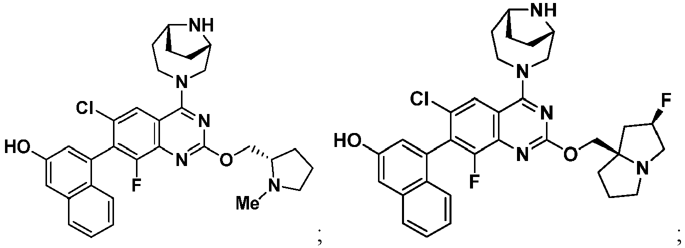 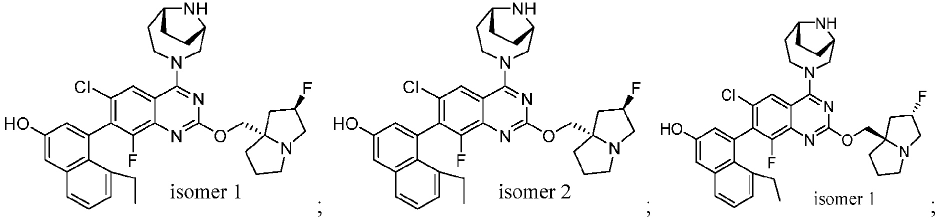 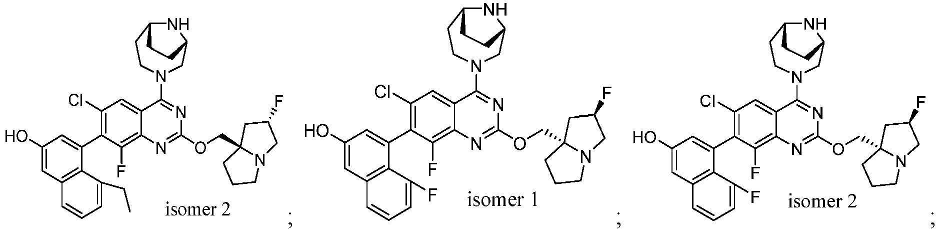 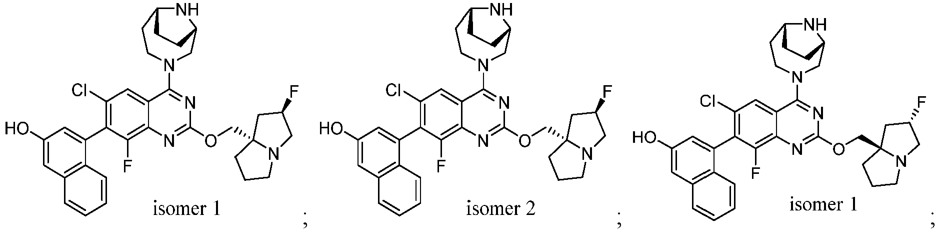 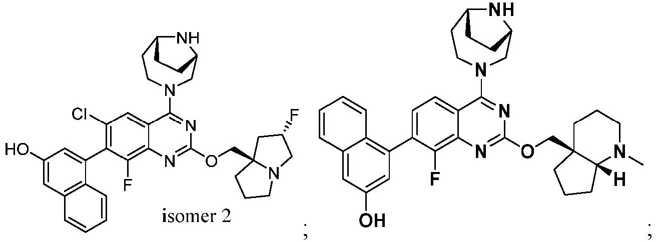 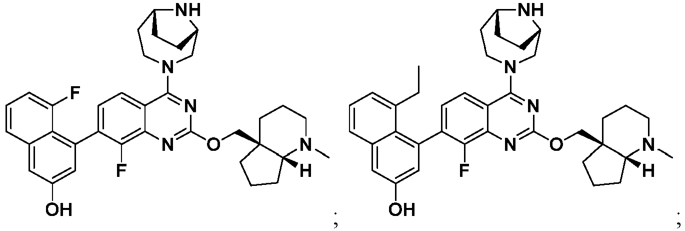 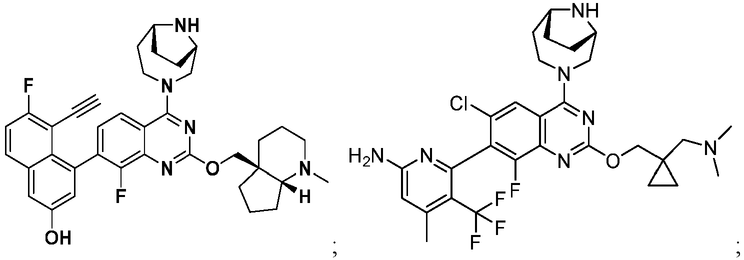 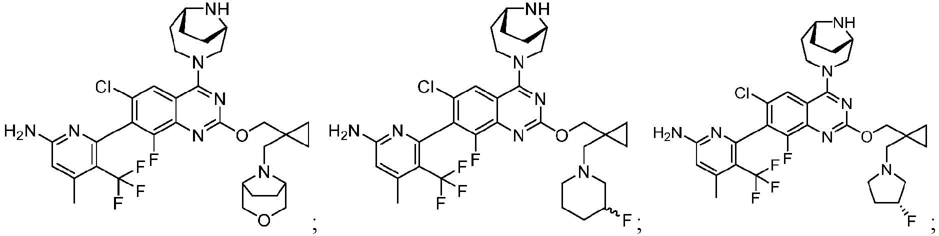 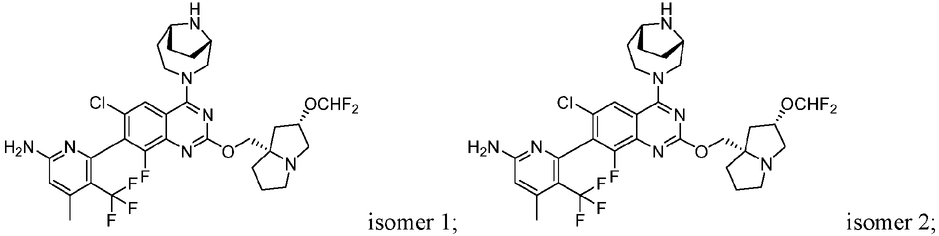 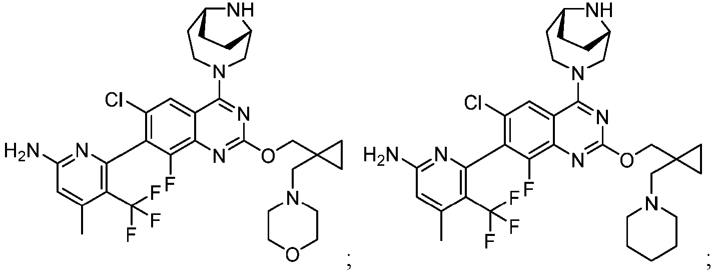 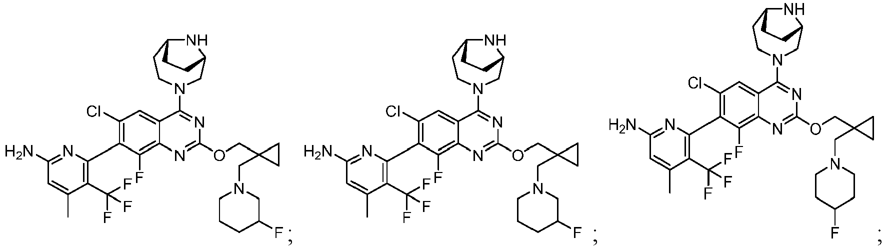 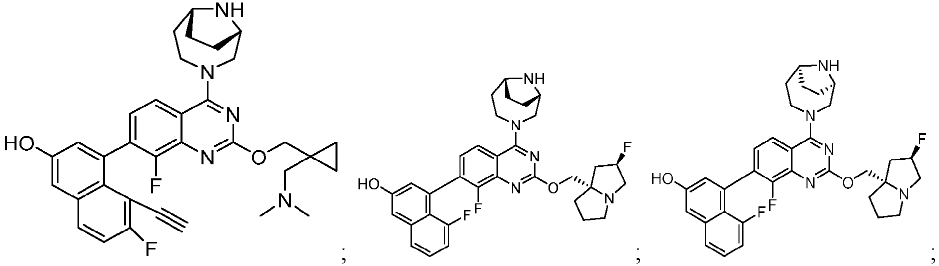 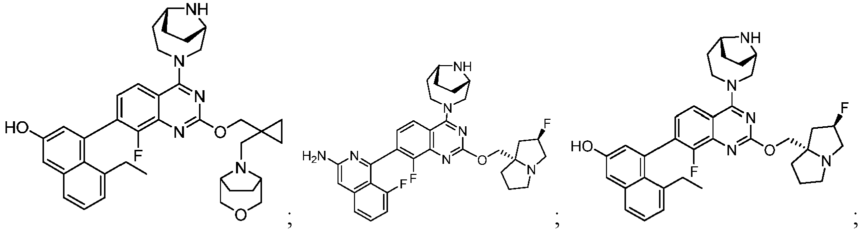 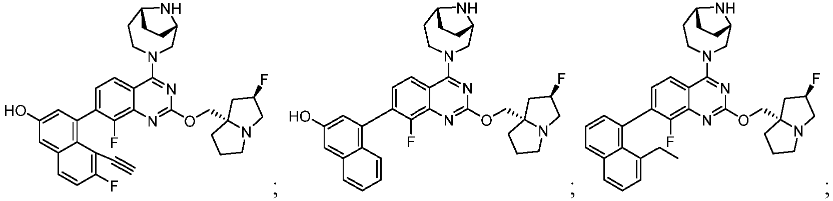 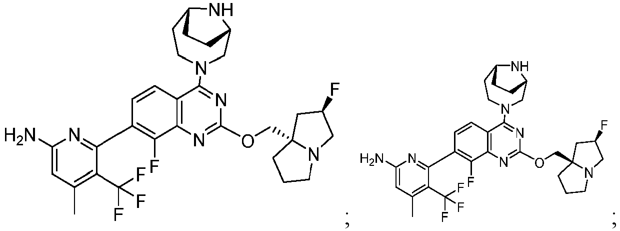 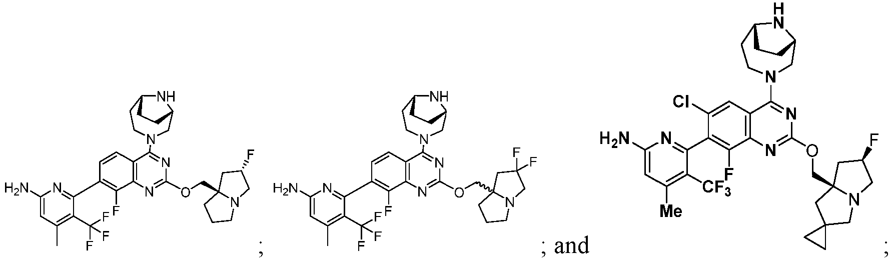 or a pharmaceutically acceptable salt thereof.

12. A compound of claim 1 selected from:
4-(6-chloro-4-{3,9-diazabicyclo[4.2.1]nonan-9-yl}-8-fluoro-2-{[(2S)-1-methylpyrrolidin-2-yl]methoxy}quinazolin-7-yl)naphthalen-2-ol;
4-(6-chloro-4-{1,4-diazabicyclo[3.2.2]nonan-4-yl}-8-fluoro-2-{[(2S)-1-methylpyrrolidin-2-yl]methoxy}quinazolin-7-yl)naphthalen-2-ol;
6-(6-chloro-4-{3,9-diazabicyclo[4.2.1]nonan-3-yl}-8-fluoro-2-{[(2S)-1-methylpyrrolidin-2-yl]methoxy}quinazolin-7-yl)-4-methyl-5-(trifluoromethyl)pyridin-2-amine;
6-(6-chloro-4-{3,6-diazabicyclo[3.2.2]nonan-3-yl}-8-fluoro-2-{[(2S)-1-methylpyrrolidin-2-yl]methoxy}quinazolin-7-yl)-4-methyl-5-(trifluoromethyl)pyridin-2-amine;
6-(2- f [(4aS,7aR)-1-methyl-octahydro-1H-cyclopenta[b]pyridin-4a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amine;
6-(2-f [(2R,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amine;
6-(2-{[(2S,7aR)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amine;
6-(2-{[(2R,7aR)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amine;
6-{6-chloro-4-[(1S,6R)-3,9-diazabicyclo[4.2.1]nonan-3-yl]-8-fluoro-2-{[(2S,4R)-4-methoxy-1-methylpyrrolidin-2-yl]methoxy}quinazolin-7-yl}-4-methyl-5-(trifluoromethyl)pyridin-2-amine;
6-(2-{[(2S,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amine;
6-(2-{[(2S,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amine, Isomer 1;
6-(2-{[(2S,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amine, Isomer 2;
4-{6-chloro-4-[(1S,6R)-3,9-diazabicyclo[4.2.1]nonan-3-yl]-8-fluoro-2-{[(2S)-1-methylpyrrolidin-2-ylmethoxy}quinazolin-7-yl}naphthalen-2-ol;
4-(2-{[(2R,7aR)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)naphthalen-2-ol;
4-(2-{[(2R,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-5-ethylnaphthalen-2-ol;
4-(2-{[(2R,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-5-ethylnaphthalen-2-ol, Isomer1;
4-(2-{[(2R,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-5-ethylnaphthalen-2-ol, Isomer2;
4-(2-f [(2S,7aR)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-5-ethylnaphthalen-2-ol;
4-(2-{[(2S,7aR)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-5-ethylnaphthalen-2-ol, Isomer 1;
4-(2-{[(2S,7aR)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-5-ethylnaphthalen-2-ol, Isomer 2;
4-(2-{[(2R,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-5-fluoronaphthalen-2-ol;
4-(2-{[(2R,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-5-fluoronaphthalen-2-ol, Isomer 1;
4-(2-{[(2R,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-5-fluoronaphthalen-2-ol, Isomer 2;
4-(2-{[(2R,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)naphthalen-2-ol;
4-(2-{[(2R,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)naphthalen-2-ol, Isomer 1;
4-(2-{[(2R,7aS)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)naphthalen-2-ol, Isomer 2;
4-(2-{[(2S,7aR)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)naphthalen-2-ol;
4-(2-{[(2S,7aR)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)naphthalen-2-ol, Isomer 1;
4-(2-{[(2S,7aR)-2-fluoro-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)naphthalen-2-ol, Isomer 2;
4-(2-{[(4aS,7aR)-1-methyl-octahydro-1H-cyclopenta[b]pyridin-4a-yl]methoxy}-4-[(1S,6R)-3,9-diazabicyclo[4.2.1]nonan-3-yl]-8-fluoroquinazolin-7-yl)naphthalen-2-ol;
4-(2-{[(4aS,7aR)-1-methyl-octahydro-1H-cyclopenta[b]pyridin-4a-yl]methoxy}-4-[(1S,6R)-3,9-diazabicyclo[4.2.1]nonan-3-yl]-8-fluoroquinazolin-7-yl)-5-fluoronaphthalen-2-ol;
4-(2-f [(4aS,7aR)-1-methyl-octahydro-1H-cyclopenta[b]pyridin-4a-yl]methoxy}-4-[(1S,6R)-3,9-diazabicyclo[4.2.1]nonan-3-yl]-8-fluoroquinazolin-7-yl)-5-ethylnaphthalen-2-ol;
4-(2-{[(4aS,7aR)-1-methyl-octahydro-1H-cyclopenta[b]pyridin-4a-yl]methoxy}-4-[(1S,6R)-3,9-diazabicyclo[4.2.1]nonan-3-yl]-8-fluoroquinazolin-7-yl)-5-ethynyl-6-fluoronaphthalen-2-ol;
6-{6-chloro-4-[(1S,6R)-3,9-diazabicyclo[4.2.1]nonan-3-yl]-2-({1-[(dimethylamino)methyl]cyclopropyl}methoxy)-8-fluoroquinazolin-7-yl}-4-methyl-5-(trifluoromethyl)pyridin-2-amine;
6-{6-chloro-4-[(1S,6R)-3,9-diazabicyclo[4.2.1]nonan-3-yl]-8-fluoro-2-{[1-({3-oxa-8-azabicyclo[3.2.1]octan-8-yl}methyl)cyclopropyl]methoxy}quinazolin-7-yl}-4-methyl-5-(trifluoromethyl)pyridin-2-amine;
6-{6-chloro-4-[(1S,6R)-3,9-diazabicyclo[4.2.1]nonan-3-yl]-8-fluoro-2-({1-[(3-fluoropiperidin-1-yl)methyl]cyclopropyl}methoxy)quinazolin-7-yl}-4-methyl-5-(trifluoromethyl)pyridin-2-amine;
6-{6-chloro-4-[(1S,6R)-3,9-diazabicyclo[4.2.1]nonan-3-yl]-8-fluoro-2-[(1-{[(3R)-3-fluoropyrrolidin-1-yl]methyl}cyclopropyl)methoxy]quinazolin-7-yl}-4-methyl-5-(trifluoromethyl)pyridin-2-amine;
6-(2-{[(2S,7aS)-2-(difluoromethoxy)-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amineisomer1;
6-(2-{[(2S,7aS)-2-(difluoromethoxy)-hexahydro-1H-pyrrolizin-7a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amineisomer2;
6-{6-chloro-4-[(1S,6R)-3,9-diazabicyclo[4.2.1]nonan-3-yl]-8-fluoro-2-({1-[(morpholin-4-yl)methyl]cyclopropyl}methoxy)quinazolin-7-yl}-4-methyl-5-(trifluoromethyl)pyridin-2-amine;
6-{6-chloro-4-[(1S,6R)-3,9-diazabicyclo[4.2.1]nonan-3-yl]-8-fluoro-2-({1-[(piperidin-1-yl)methyl]cyclopropyl}methoxy)quinazolin-7-yl}-4-methyl-5-(trifluoromethyl)pyridin-2-amine;
6-{6-chloro-4-[(1S,6R)-3,9-diazabicyclo[4.2.1]nonan-3-yl]-8-fluoro-2-({1-[(3-fluoropiperidin-1-yl)methyl]cyclopropyl}methoxy)quinazolin-7-yl}-4-methyl-5-(trifluoromethyl)pyridin-2-amine;
6-{6-chloro-4-[(1S,6R)-3,9-diazabicyclo[4.2.1]nonan-3-yl]-8-fluoro-2-({1-[(3-fluoropiperidin-1-yl)methyl]cyclopropyl}methoxy)quinazolin-7-yl}-4-methyl-5-(trifluoromethyl)pyridin-2-amine;
6-{6-chloro-4-[(1S,6R)-3,9-diazabicyclo[4.2.1]nonan-3-yl]-8-fluoro-2-({1-[(4-fluoropiperidin-1-yl)methyl]cyclopropyl}methoxy)quinazolin-7-yl}-4-methyl-5-(trifluoromethyl)pyridin-2-amine;
4-{4-[(1S,6R)-3,9-diazabicyclo[4.2.1]nonan-3-yl]-2-({1-[(dimethylamino)methyl]cyclopropyl}methoxy)-8-fluoroquinazolin-7-yl}-5-ethynyl-6-fluoronaphthalen-2-ol;
4-(2-{[(2R,7aS)-2-fluoro-hexahydro-1H-pyrrolizin-7a-yl]methoxy}-4-[(1S,6R)-3,9-diazabicyclo[4.2.1]nonan-3-yl]-8-fluoroquinazolin-7-yl)-5-fluoronaphthalen-2-ol;
4-(2-{[(2R,7aS)-2-fluoro-hexahydro-1H-pyrrolizin-7a-yl]methoxy}-4-[(1R,6S)-3,9-diazabicyclo[4.2.1]nonan-3-yl]-8-fluoroquinazolin-7-yl)-5-fluoronaphthalen-2-ol;
4-{4-[(1S,6R)-3,9-diazabicyclo[4.2.1]nonan-3-yl]-8-fluoro-2-{[1-({3-oxa-8-azabicyclo[3.2.1]octan-8-yl}methyl)cyclopropyl]methoxy}quinazolin-7-yl}-5-ethylnaphthalen-2-ol;
1-(2-{[(2R,7aS)-2-fluoro-hexahydro-1H-pyrrolizin-7a-yl]methoxy}-4-[(1S,6R)-3,9-diazabicyclo[4.2.1]nonan-3-yl]-8-fluoroquinazolin-7-yl)-8-fluoroisoquinolin-3-amine;
4-(2-{[(2R,7aS)-2-fluoro-hexahydro-1H-pyrrolizin-7a-yl]methoxy}-4-[(1S,6R)-3,9-diazabicyclo[4.2.1]nonan-3-yl]-8-fluoroquinazolin-7-yl)-5-ethylnaphthalen-2-ol;
4-(2-{[(2R,7aS)-2-fluoro-hexahydro-1H-pyrrolizin-7a-yl]methoxy}-4-[(1S,6R)-3,9-diazabicyclo[4.2.1]nonan-3-yl]-8-fluoroquinazolin-7-yl)-5-ethynyl-6-fluoronaphthalen-2-ol;
4-(2-{[(2R,7aS)-2-fluoro-hexahydro-1H-pyrrolizin-7a-yl]methoxy}-4-[(1S,6R)-3,9-diazabicyclo[4.2.1]nonan-3-yl]-8-fluoroquinazolin-7-yl)naphthalen-2-ol;
2-{[(2R,7aS)-2-fluoro-hexahydro-1H-pyrrolizin-7a-yl]methoxy}-4-[(1S,6R)-3,9-diazabicyclo[4.2.1]nonan-3-yl]-7-(8-ethylnaphthalen-1-yl)-8-fluoroquinazoline;
4-(2-{[(2R,7aS)-2-fluoro-hexahydro-1H-pyrrolizin-7a-yl]methoxy}-4-[(1S,6R)-3,9-diazabicyclo[4.2.1]nonan-3-yl]-8-fluoroquinazolin-7-yl)-5-fluoronaphthalen-2-ol;
6-(2-{[(2R,7aR)-2-fluoro-hexahydro-1H-pyrrolizin-7a-yl]methoxy}-4-[(1S,6R)-3,9-diazabicyclo[4.2.1]nonan-3-yl]-8-fluoroquinazolin-7-yl)-4-methyl-5-(trifluoromethyl)pyridin-2-amine;
6-(2-{[(2S,7aR)-2-fluoro-hexahydro-1H-pyrrolizin-7a-yl]methoxy}-4-[(1S,6R)-3,9-diazabicyclo[4.2.1]nonan-3-yl]-8-fluoroquinazolin-7-yl)-4-methyl-5-(trifluoromethyl)pyridin-2-amine;
6-{4-[(1S,6R)-3,9-diazabicyclo[4.2.1]nonan-3-yl]-2-[(2,2-difluoro-hexahydro-1H-pyrrolizin-7a-yl)methoxy]-8-fluoroquinazolin-7-yl}-4-methyl-5-(trifluoromethyl)pyridin-2-amine; and
6-(2-f[(6'R,7'aR)-6'-fluoro-hexahydrospiro[cyclopropane-1,2'-pyrrolizine]-7'a-yl]methoxy}-6-chloro-4-[(1S,6R)-3,9-diazabicyclo[4.2.1]nonan-3-yl]-8-fluoroquinazolin-7-yl)-4-methyl-5-(trifluoromethyl)pyridin-2-amine;
or a pharmaceutically acceptable salt thereof.

13. A pharmaceutical composition comprising a compound of any one of claims 1 to 12, or a pharmaceutically acceptable salt thereof, and one or more pharmaceutically acceptable excipients.

14. A compound of any one of claims 1 to 12, or a pharmaceutically acceptable salt thereof, for use in a method for treating cancer, wherein
(i) the cancer is pancreatic cancer, colorectal cancer, lung cancer and/or gastric cancer;
(ii) the cancer is susceptible to KRAS G12D inhibition; or
(iii) cancer expresses KRAS G12D mutation.

## Patentansprüche

1. Verbindung der Formel (I): 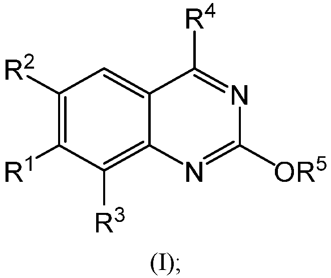 oder pharmazeutisch akzeptables Salz davon; wobei:
R¹ Aryl oder Heteroaryl ist, wobei das Aryl und das Heteroaryl wahlweise mit einem, zwei, drei, vier oder fünf Substituenten substituiert sind, die unabhängig ausgewählt sind unter C₁-C₃-Alkyl, C₂-C₄-Alkenyl, C₂-C₄-Alkynyl, Amino, Amino-C₁-C₃-alkyl, C₃-C₄-Cycloalkyl, Halo, Halo-C₁-C₃-alkoxy, Halo-C₁-C₃-alkyl, Hydroxy und Hydroxy-C₁-C₃-alkyl;
R² und R³ unabhängig unter Wasserstoff, C₁-C₃-Alkoxy, C₁-C₃-Alkyl, Cyano, Halo, Halo-C₁-C₃-alkyl und Hydroxy ausgewählt sind;
R⁴ ausgewählt ist unter: 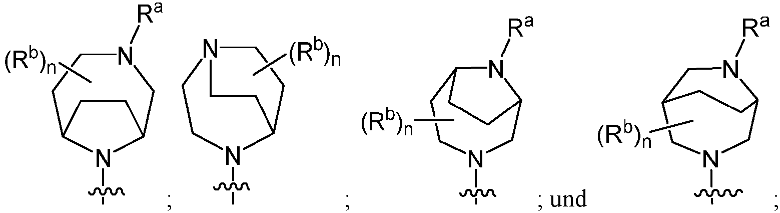 wobei
R^{a} Wasserstoff oder C₁-C₃-Alkyl ist;
n 0, 1, 2, 3 oder 4 beträgt;
jedes R^{b} unabhängig ausgewählt ist unter C₁-C₃-Alkyl, C₃-C₆-Cycloalkyl, Halo und Hydroxy; oder, alternativ, zwei geminale R^{b}-Gruppen, zusammen mit dem Kohlenstoffatom, an das sie angelagert sind, einen 3- bis 6-gliedrigen Cycloalkylring bilden können; und 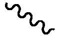
den Anlagerungspunkt an den ursprünglichen molekularen Anteil angibt;
R⁵ -(C₁-C₃-Alkyl)-R⁶ oder -(C₁-C₆-Alkyl)NR^{c}R^{d} ist, wobei R⁶ ausgewählt ist unter
einem C₃-C₆-Cycloalkyl, das mit NR^{c}R^{d}(C₁-C₃-Alkyl) substituiert ist; und
einem fünf- bis zehngliedrigen monocyclischen, bicyclischen oder tricyclischen Ring, der ein Stickstoffatom und wahlweise ein zweites Heteroatom enthält ausgewählt unter Sauerstoff oder Stickstoff,
wobei der Ring null bis drei Doppelbindungen enthält und wobei der Ring wahlweise mit einer, zwei oder drei Gruppen substituiert ist, die unabhängig ausgewählt sind unter C₁-C₃-Alkoxy, C₁-C₃-Alkyl, Halo, Halo-C₁-C₃-alkoxy, Halo-C₁-C₃-Alkyl und Hydroxy; wobei
R^{c} und R^{d}, zusammen mit dem Stickstoffatom,an das sie angelagert sind, einen fünfbis zehngliedrigen monocyclischen oder bicyclischen Ring bilden, der wahlweise ein zusätzliches Heteroatom enthält ausgewählt unter Stickstoff, Sauerstoff und Schwefel, wobei der Ring wahlweise mit einer, zwei oder drei Gruppen substituiert ist, die unabhängig ausgewählt sind unter C₁-C₃-Alkoxy, C₁-C₃-Alkoxy-C₁-C₃-alkyl, C₁-C₃-Alkyl, Benzyl, Halo, Halo-C₁-C₃-alkyl, Hydroxy, Hydroxy-C₁-C₃-alkyl und Oxo; oder
eines von R^{c} und R^{d} ausgewählt ist unter Wasserstoff und C₁-C₃-Alkyl und das andere ausgewählt ist unter Wasserstoff, C₁-C₃-Alkyl, C₁-C₃-Alkoxycarbonyl und C₁-C₃-Alkylcarbonyl.

2. Verbindung nach Anspruch 1 oder pharmazeutisch akzeptables Salz davon, wobei R⁴ ausgewählt ist unter:

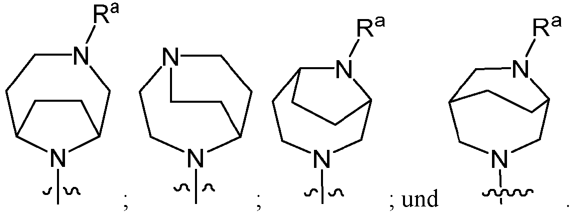

3. Verbindung nach Anspruch 1 oder 2 oder pharmazeutisch akzeptables Salz davon, wobei R² und R³ jedes Halo ist.

4. Verbindung nach einem der Ansprüche 1 bis 3 oder pharmazeutisch akzeptables Salz davon, wobei R⁵ -(C₁-C₃-Alkyl)-R⁶ ist.

5. Verbindung nach einem der Ansprüche 1 bis 4 oder pharmazeutisch akzeptables Salz davon, wobei R⁵ ausgewählt ist unter: 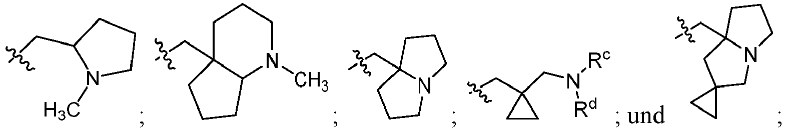 wobei jeder Ring wahlweise mit 1 oder 2 Gruppen substituiert ist, die unabhängig ausgewählt sind unter C₁-C₃-Alkoxy, C₁-C₃-Alkyl, Halo, Halo-C₁-C₃-alkyl und Hydroxy.

6. Verbindung nach einem der Ansprüche 1 bis 4 oder pharmazeutisch akzeptables Salz davon, wobei
(i) R⁵ 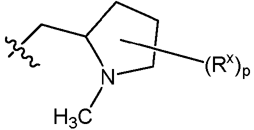 ist;
wobei p 0, 1 oder 2 beträgt; und wobei jedes R^{x} unabhängig ausgewählt ist unter C₁-C₃-Alkoxy, C₁-C₃-Alkyl, Halo, Halo-C₁-C₃-alkyl und Hydroxy; oder
(ii) R⁵ 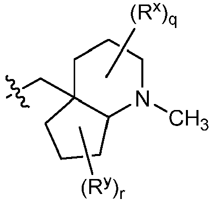 ist;
wobei q und r jedes unabhängig 0 oder 1 beträgt; und wobei R^{x} und R^{y} unabhängig ausgewählt sind unter C₁-C₃-Alkoxy, C₁-C₃-Alkyl, Halo, Halo-C₁-C₃-alkyl und Hydroxy; oder
(iii) R⁵ 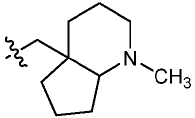 ist; oder
(iv) R⁵ 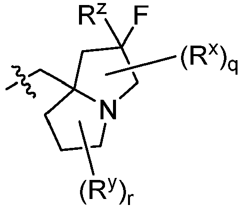 ist;
wobei q und r jedes unabhängig 0 oder 1 beträgt; wobei R^{x} und R^{y} unabhängig ausgewählt sind unter C₁-C₃-Alkoxy, C₁-C₃-Alkyl, Halo, Halo-C₁-C₃-alkyl und Hydroxy; und wobei R^{z} Wasserstoff oder Fluor ist; oder
(v) R⁵ 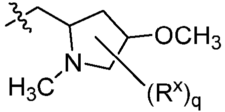 ist;
wobei q 0 oder 1 beträgt; und wobei R^{x} ausgewählt ist unter C₁-C₃-Alkoxy, C₁-C₃-Alkyl, Halo, Halo-C₁-C₃-alkyl und Hydroxy; oder
(vi) R⁵ 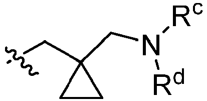 ist; oder
(vii) R⁵ 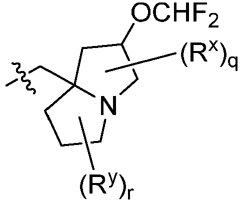 ist;
wobei q und r jedes unabhängig 0 oder 1 beträgt; und wobei R^{x} und R^{y} unabhängig ausgewählt sind unter C₁-C₃-Alkoxy, C₁-C₃-Alkyl, Halo, Halo-C₁-C₃-alkyl und Hydroxy; oder
(viii) R⁵ 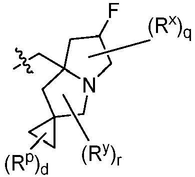 ist, wobei q, r und d jedes unabhängig 0 oder 1 beträgt; und wobei R^{x}, R^{y} und R^{p} unabhängig ausgewählt sind unter C₁-C₃-Alkoxy, C₁-C₃-Alkyl, Halo, Halo-C₁-C₃-alkyl und Hydroxy; oder
(ix) R⁵ 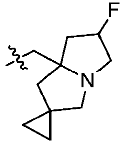 ist.

7. Verbindung nach einem der Ansprüche 1 bis 6 oder pharmazeutisch akzeptables Salz davon, wobei
(i) R¹ Naphthyl ist, wobei das Naphthyl mit einer Hydroxygruppe substituiert ist und wahlweise ferner mit einer oder zwei zusätzlichen Gruppen substituiert ist, die ausgewählt sind unter C₁-C₃-Alkyl, C₂-C₄-Alkynyl und Halo; oder
(ii) R¹ 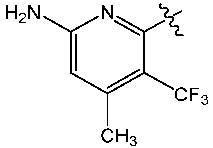 ist; oder
(iii) R¹ 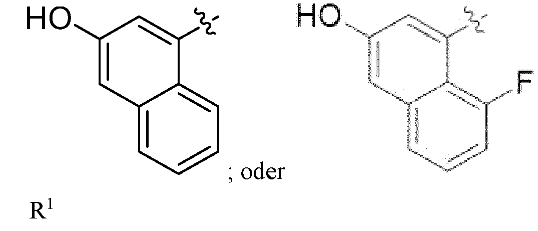 ist; oder
(iv) 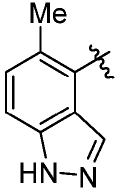 ist oder
(v) R¹ 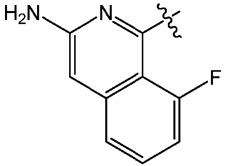 ist.

8. Verbindung nach Anspruch 1 oder pharmazeutisch akzeptables Salz davon, wobei
R² Wasserstoff ist;
R³ Fluor ist;
R¹ ausgewählt ist unter 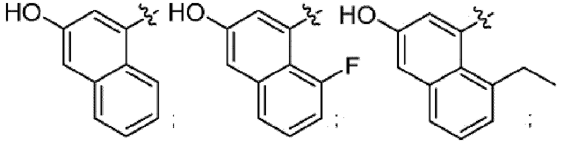 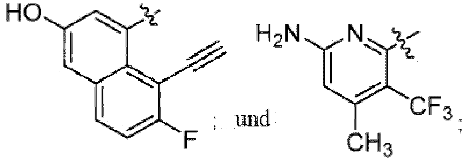 und
R⁵ ausgewählt ist unter 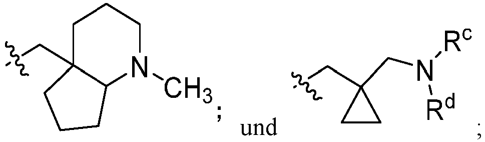 wobei 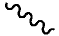
den Anlagerungspunkt an den ursprünglichen molekularen Anteil angibt

9. Verbindung nach Anspruch 1 oder pharmazeutisch akzeptables Salz davon, wobei
R² Chlor ist;
R³ Fluor ist;
R¹ ausgewählt ist unter 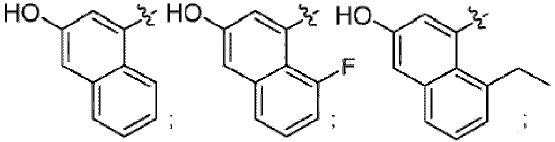 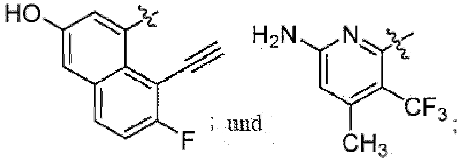 und
R⁵ ausgewählt ist unter 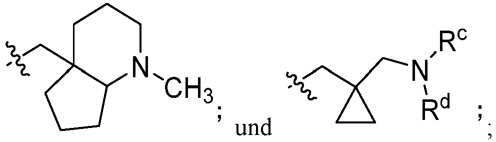 wobei 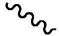
den Anlagerungspunkt an den ursprünglichen molekularen Anteil angibt.

10. Verbindung nach Anspruch 1 der Formel (II): 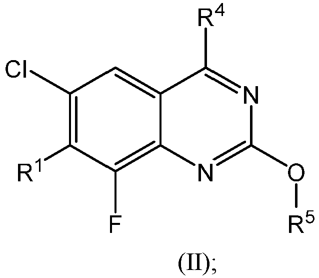 oder pharmazeutisch akzeptables Salz davon, wobei:
R¹ ausgewählt ist unter 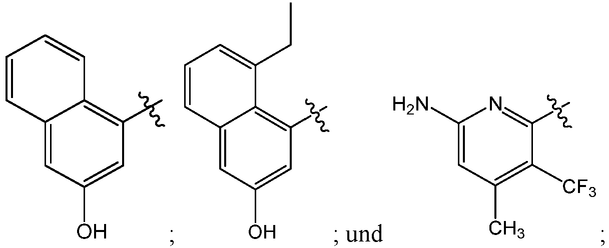
wobei 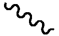 den Anlagerungspunkt an den ursprünglichen molekularen Anteil angibt;
R⁴ ausgewählt ist unter: 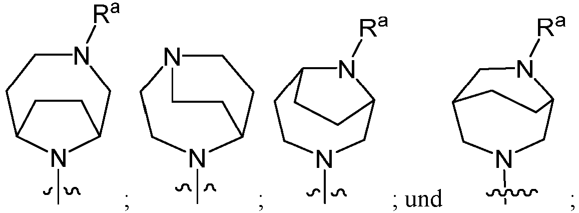 wobei
R^{a} Wasserstoff oder C₁-C₃-Alkyl ist; und 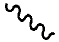
den Anlagerungspunkt an den ursprünglichen molekularen Anteil angibt; und
R⁵ ausgewählt ist unter: 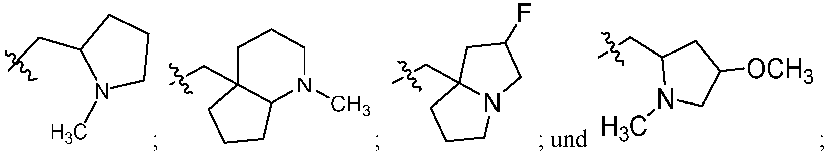 wobei 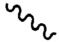
den Anlagerungspunkt an den ursprünglichen molekularen Anteil angibt.

11. Verbindung nach Anspruch 1 ausgewählt unter: 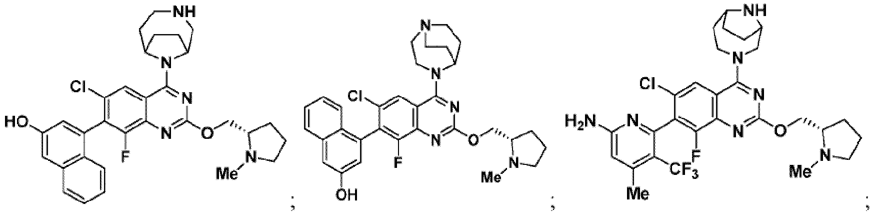 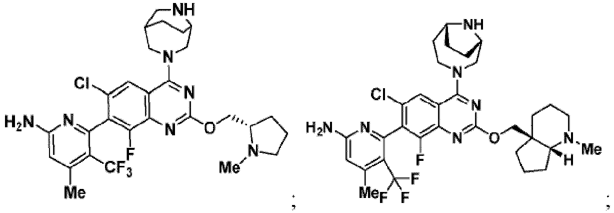 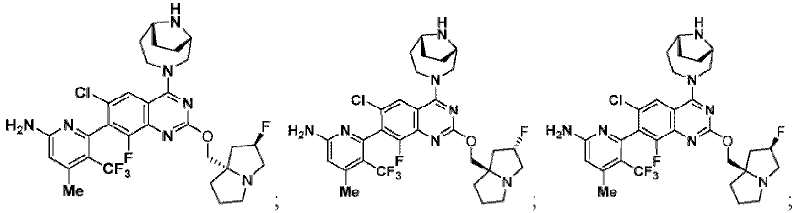 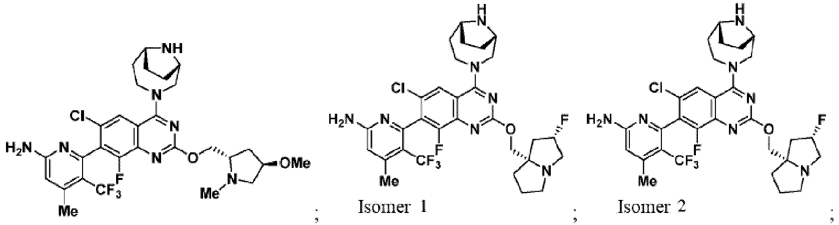 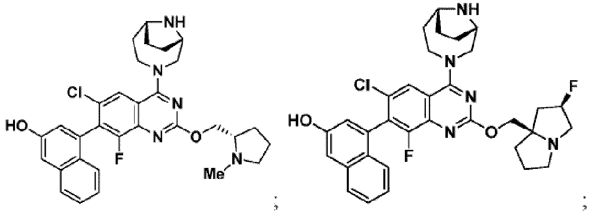 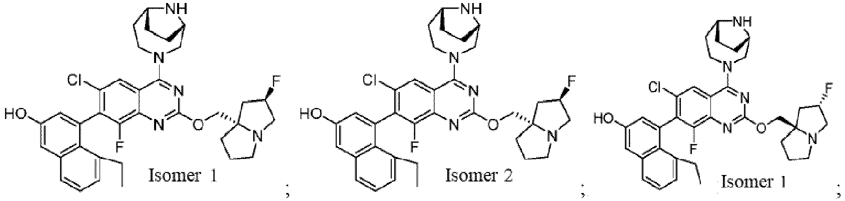 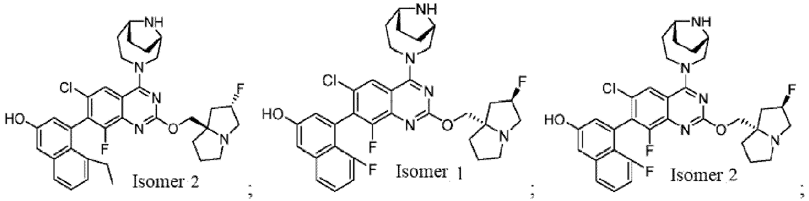 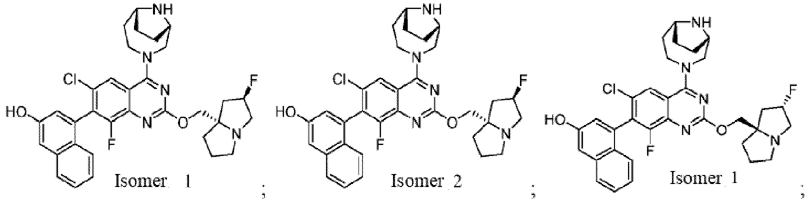 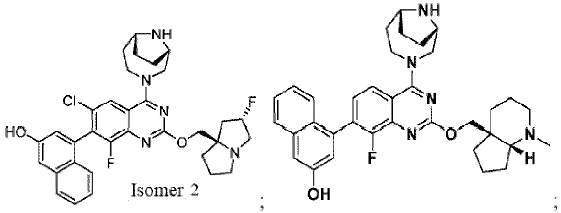 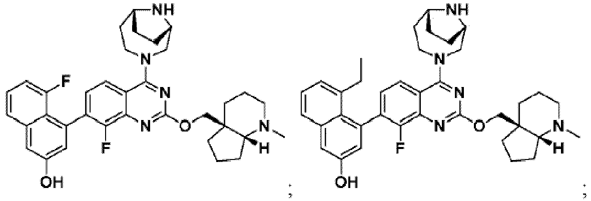 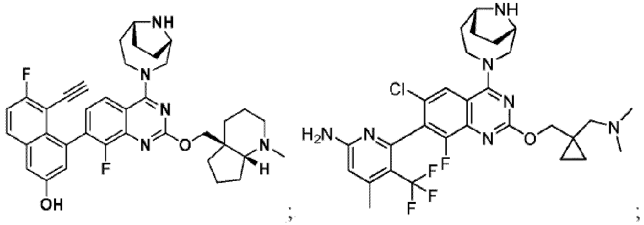 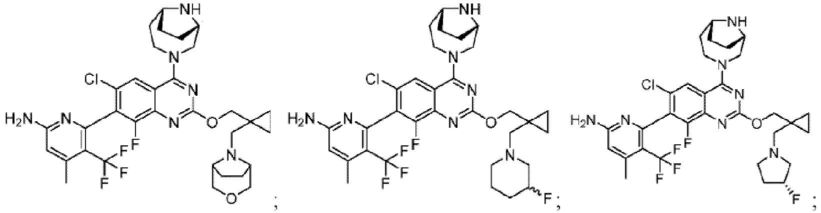 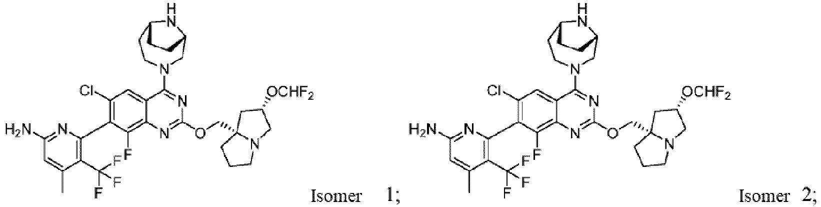 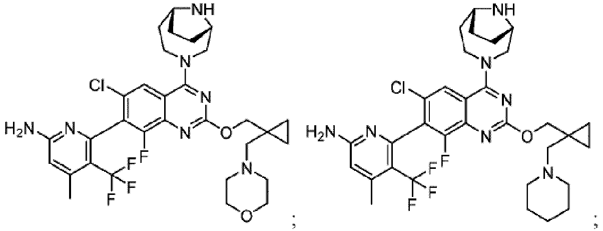 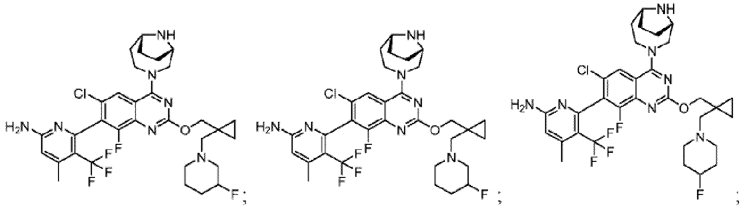 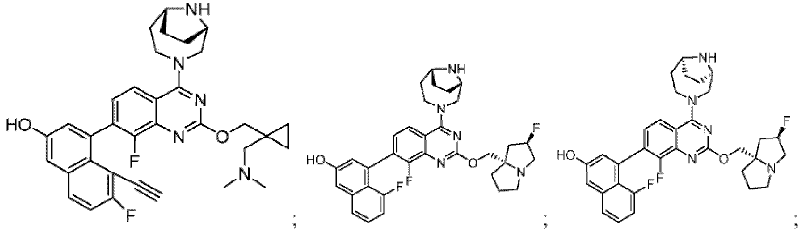 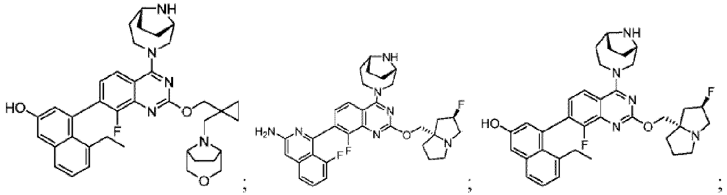 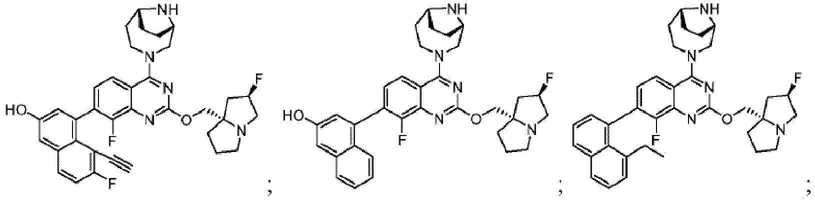 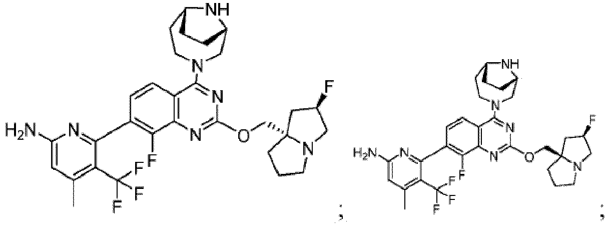 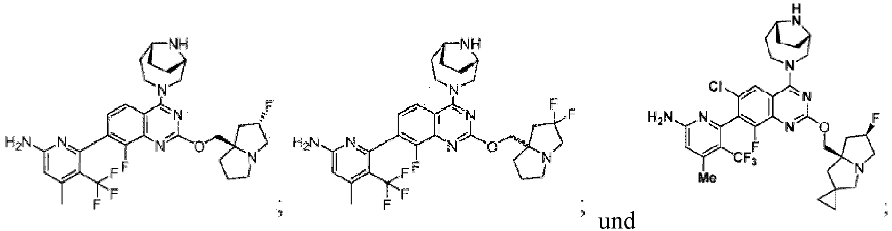 oder pharmazeutisch akzeptables Salz davon.

12. Verbindung nach Anspruch 1 ausgewählt unter:
4-(6-Chlor-4-{3,9-diazabicyclo[4.2.1]nonan-9-yl}-8-fluor-2-{[(2S)-1-methylpyrrolidin-2-yl]methoxy}chinazolin-7-yl)naphthalin-2-ol;
4-(6-Chlor-4-{1,4-diazabicyclo[3.2.2]nonan-4-yl}-8-fluor-2-{[(2S)-1-methylpyrrolidin-2-yl]methoxy}chinazolin-7-yl)naphthalin-2-ol;
6-(6-Chlor-4-{3,9-diazabicyclo[4.2.1]nonan-3-yl}-8-fluor-2-{[(2S)-1-methylpyrrolidin-2-yl]methoxy}chinazolin-7-yl)-4-methyl-5-(trifluormethyl)pyridin-2-amin;
6-(6-Chlor-4-{3,6-diazabicyclo[3.2.2]nonan-3-yl}-8-fluor-2-{[(2S)-1-methylpyrrolidin-2-yl]methoxy}chinazolin-7-yl)-4-methyl-5-(trifluormethyl)pyridin-2-amin;
6-(2-{[(4aS,7aR)-1-Methyl-octahydro-1H-cyclopenta[b]pyridin-4a-yl]methoxy}-6-chlor-4-[(1S,6R)-3,9-diazabicyclo[4.2.1]nonan-3-yl]-8-fluorchinazolin-7-yl)-4-methyl-5-(trifluormethyl)pyridin-2-amin;
6-(2-{[(2R,7aS)-2-Fluorhexahydro-1H-pyrrolizin-7a-yl]methoxy}-6-chlor-4-[(1S,6R)-3,9-diazabicyclo[4.2.1]nonan-3-yl]-8-fluorchinazolin-7-yl)-4-methyl-5-(trifluormethyl)pyridin-2-amin;
6-(2-{[(2S,7aR)-2-Fluorhexahydro-1H-pyrrolizin-7a-yl]methoxy}-6-chlor-4-[(1S,6R)-3,9-diazabicyclo[4.2.1]nonan-3-yl]-8-fluorchinazolin-7-yl)-4-methyl-5-(trifluormethyl)pyridin-2-amin;
6-(2-{[(2R,7aR)-2-Fluorhexahydro-1H-pyrrolizin-7a-yl]methoxy}-6-chlor-4-[(1S,6R)-3,9-diazabicyclo[4.2.1]nonan-3-yl]-8-fluorchinazolin-7-yl)-4-methyl-5-(trifluormethyl)pyridin-2-amin;
6-{6-Chlor-4-[(1S,6R)-3,9-diazabicyclo[4.2.1]nonan-3-yl]-8-fluor-2-{[(2S,4R)-4-methoxy-1-methylpyrrolidin-2-yl]methoxy}chinazolin-7-yl}-4-methyl-5-(trifluormethyl)pyridin-2-amin;
6-(2-{[(2S,7aS)-2-Fluorhexahydro-1H-pyrrolizin-7a-yl]methoxy}-6-chlor-4-[(1S,6R)-3,9-diazabicyclo[4.2.1]nonan-3-yl]-8-fluorchinazolin-7-yl)-4-methyl-5-(trifluormethyl)pyridin-2-amin;
6-(2-{[(2S,7aS)-2-Fluorhexahydro-1H-pyrrolizin-7a-yl]methoxy}-6-chlor-4-[(1S,6R)-3,9-diazabicyclo[4.2.1]nonan-3-yl]-8-fluorchinazolin-7-yl)-4-methyl-5-(trifluormethyl)pyridin-2-amin, Isomer1;
6-(2-{[(2S,7aS)-2-Fluorhexahydro-1H-pyrrolizin-7a-yl]methoxy}-6-chlor-4-[(1S,6R)-3,9-diazabicyclo[4.2.1]nonan-3-yl]-8-fluorchinazolin-7-yl)-4-methyl-5-(trifluormethyl)pyridin-2-amin, Isomer2;
4-{6-Chlor-4-[(1S,6R)-3,9-diazabicyclo[4.2.1]nonan-3-yl]-8-fluor-2-{[(2S)-1-methylpyrrolidin-2-yl]methoxy}chinazolin-7-yl}naphthalin-2-ol;
4-(2-{[(2R,7aR)-2-Fluorhexahydro-1H-pyrrolizin-7a-yl]methoxy}-6-chlor-4-[(1S,6R)-3,9-diazabicyclo[4.2.1]nonan-3-yl]-8-fluorchinazolin-7-yl)naphthalin-2-ol;
4-(2-{[(2R,7aS)-2-Fluorhexahydro-1H-pyrrolizin-7a-yl]methoxy}-6-chlor-4-[(1S,6R)-3,9-diazabicyclo[4.2.1]nonan-3-yl]-8-fluorchinazolin-7-yl)-5-ethylnaphthalin-2-ol;
4-(2-{[(2R,7aS)-2-Fluorhexahydro-1H-pyrrolizin-7a-yl]methoxy}-6-chlor-4-[(1S,6R)-3,9-diazabicyclo[4.2.1]nonan-3-yl]-8-fluorchinazolin-7-yl)-5-ethylnaphthalin-2-ol, Isomer 1;
4-(2-{[(2R,7aS)-2-Fluorhexahydro-1H-pyrrolizin-7a-yl]methoxy}-6-chlor-4-[(1S,6R)-3,9-diazabicyclo[4.2.1]nonan-3-yl]-8-fluorchinazolin-7-yl)-5-ethylnaphthalin-2-ol, Isomer 2;
4-(2-{[(2S,7aR)-2-Fluorhexahydro-1H-pyrrolizin-7a-yl]methoxy}-6-chlor-4-[(1S,6R)-3,9-diazabicyclo[4.2.1]nonan-3-yl]-8-fluorchinazolin-7-yl)-5-ethylnaphthalin-2-ol;
4-(2-{[(2S,7aR)-2-Fluorhexahydro-1H-pyrrolizin-7a-yl]methoxy}-6-chlor-4-[(1S,6R)-3,9-diazabicyclo[4.2.1]nonan-3-yl]-8-fluorchinazolin-7-yl)-5-ethylnaphthalin-2-ol, Isomer 1;
4-(2-{[(2S,7aR)-2-Fluorhexahydro-1H-pyrrolizin-7a-yl]methoxy}-6-chlor-4-[(1S,6R)-3,9-diazabicyclo[4.2.1]nonan-3-yl]-8-fluorchinazolin-7-yl)-5-ethylnaphthalin-2-ol, Isomer 2;
4-(2-{[(2R,7aS)-2-Fluorhexahydro-1H-pyrrolizin-7a-yl]methoxy}-6-chlor-4-[(1S,6R)-3,9-diazabicyclo[4.2.1]nonan-3-yl]-8-fluorchinazolin-7-yl)-5-fluornaphthalin-2-ol;
4-(2-{[(2R,7aS)-2-Fluorhexahydro-1H-pyrrolizin-7a-yl]methoxy}-6-chlor-4-[(1S,6R)-3,9-diazabicyclo[4.2.1]nonan-3-yl]-8-fluorchinazolin-7-yl)-5-fluornaphthalin-2-ol, Isomer 1;
4-(2-{[(2R,7aS)-2-Fluorhexahydro-1H-pyrrolizin-7a-yl]methoxy}-6-chlor-4-[(1S,6R)-3,9-diazabicyclo[4.2.1]nonan-3-yl]-8-fluorchinazolin-7-yl)-5-fluornaphthalin-2-ol, Isomer 2;
4-(2-{[(2R,7aS)-2-Fluorhexahydro-1H-pyrrolizin-7a-yl]methoxy}-6-chlor-4-[(1S,6R)-3,9-diazabicyclo[4.2.1]nonan-3-yl]-8-fluorchinazolin-7-yl)naphthalin-2-ol;
4-(2-{[(2R,7aS)-2-Fluorhexahydro-1H-pyrrolizin-7a-yl]methoxy}-6-chlor-4-[(1S,6R)-3,9-diazabicyclo[4.2.1]nonan-3-yl]-8-fluorchinazolin-7-yl)naphthalin-2-ol, Isomer 1;
4-(2-{[(2R,7aS)-2-Fluorhexahydro-1H-pyrrolizin-7a-yl]methoxy}-6-chlor-4-[(1S,6R)-3,9-diazabicyclo[4.2.1]nonan-3-yl]-8-fluorchinazolin-7-yl)naphthalin-2-ol, Isomer 2;
4-(2-{[(2S,7aR)-2-Fluorhexahydro-1H-pyrrolizin-7a-yl]methoxy}-6-chlor-4-[(1S,6R)-3,9-diazabicyclo[4.2.1]nonan-3-yl]-8-fluorchinazolin-7-yl)naphthalin-2-ol;
4-(2-{[(2S,7aR)-2-Fluorhexahydro-1H-pyrrolizin-7a-yl]methoxy}-6-chlor-4-[(1S,6R)-3,9-diazabicyclo[4.2.1]nonan-3-yl]-8-fluorchinazolin-7-yl)naphthalin-2-ol, Isomer 1;
4-(2-{[(2S,7aR)-2-Fluorhexahydro-1H-pyrrolizin-7a-yl]methoxy}-6-chlor-4-[(1S,6R)-3,9-diazabicyclo[4.2.1]nonan-3-yl]-8-fluorchinazolin-7-yl)naphthalin-2-ol, Isomer 2;
4-(2-{[(4aS,7aR)-1-Methyl-octahydro-1H-cyclopenta[b]pyridin-4a-yl]methoxy}-4-[(1S,6R)-3,9-diazabicyclo[4.2.1]nonan-3-yl]-8-fluorchinazolin-7-yl)naphthalin-2-ol;
4-(2-{[(4aS,7aR)-1-Methyl-octahydro-1H-cyclopenta[b]pyridin-4a-yl]methoxy}-4-[(1S,6R)-3,9-diazabicyclo[4.2.1]nonan-3-yl]-8-fluorchinazolin-7-yl)-5-fluornaphthalin-2-ol;
4-(2-{[(4aS,7aR)-1-Methyl-octahydro-1H-cyclopenta[b]pyridin-4a-yl]methoxy}-4-[(1S,6R)-3,9-diazabicyclo[4.2.1]nonan-3-yl]-8-fluorchinazolin-7-yl)-5-ethylnaphthalin-2-ol;
4-(2-{[(4aS,7aR)-1-Methyl-octahydro-1H-cyclopenta[b]pyridin-4a-yl]methoxy}-4-[(1S,6R)-3,9-diazabicyclo[4.2.1]nonan-3-yl]-8-fluorchinazolin-7-yl)-5-ethynyl-6-Fluornaphthalin-2-ol;
6-{6-Chlor-4-[(1S,6R)-3,9-diazabicyclo[4.2.1]nonan-3-yl]-2-({1-[(dimethylamino)methyl]cyclopropyl}methoxy)-8-fluorchinazolin-7-yl}-4-methyl-5-(trifluormethyl)pyridin-2-amin;
6-{6-Chlor-4-[(1S,6R)-3,9-diazabicyclo[4.2.1]nonan-3-yl]-8-fluor-2-{[1-({3-oxa-8-azabicyclo[3.2.1]octan-8-yl}methyl)cyclopropyl]methoxy}chinazolin-7-yl}-4-methyl-5-(trifluormethyl)pyridin-2-amin;
6-{6-Chlor-4-[(1S,6R)-3,9-diazabicyclo[4.2.1]nonan-3-yl]-8-Fluor-2-({1-[(3-fluorpiperidin-1-yl)methyl]cyclopropyl}methoxy)chinazolin-7-yl}-4-methyl-5-(trifluormethyl)pyridin-2-amin;
6-{6-Chlor-4-[(1S,6R)-3,9-diazabicyclo[4.2.1]nonan-3-yl]-8-fluor-2-[(1-{[(3R)-3-fluorpyrrolidin-1-yl]methyl}cyclopropyl)methoxy]chinazolin-7-yl}-4-methyl-5-(trifluormethyl)pyridin-2-amin;
6-(2-{[(2S,7aS)-2-(Difluormethoxy)-hexahydro-1H-pyrrolizin-7a-yl]methoxy}-6-chlor-4-[(1S,6R)-3,9-diazabicyclo[4.2.1]nonan-3-yl]-8-fluorchinazolin-7-yl)-4-methyl-5-(trifluormethyl)pyridin-2-amin Isomer 1;
6-(2-{[(2S,7aS)-2-(Difluormethoxy)-hexahydro-1H-pyrrolizin-7a-yl]methoxy}-6-chlor-4-[(1S,6R)-3,9-diazabicyclo[4.2.1]nonan-3-yl]-8-fluorchinazolin-7-yl)-4-methyl-5-(trifluormethyl)pyridin-2-amin Isomer 2;
6-{6-Chlor-4-[(1S,6R)-3,9-diazabicyclo[4.2.1]nonan-3-yl]-8-fluor-2-({1-[(morpholin-4-yl)methyl]cyclopropyl}methoxy)chinazolin-7-yl}-4-methyl-5-(trifluormethyl)pyridin-2-amin;
6-{6-Chlor-4-[(1S,6R)-3,9-diazabicyclo[4.2.1]nonan-3-yl]-8-fluor-2-({1-[(piperidin-1-yl)methyl]cyclopropyl}methoxy)chinazolin-7-yl}-4-methyl-5-(trifluormethyl)pyridin-2-amin;
6-{6-Chlor-4-[(1S,6R)-3,9-diazabicyclo[4.2.1]nonan-3-yl]-8-fluor-2-({1-[(3-fluorpiperidin-1-yl)methyl]cyclopropyl}methoxy)chinazolin-7-yl}-4-methyl-5-(trifluormethyl)pyridin-2-amin;
6-{6-Chlor-4-[(1S,6R)-3,9-diazabicyclo[4.2.1]nonan-3-yl]-8-fluor-2-({1-[(3-fluorpiperidin-1-yl)methyl]cyclopropyl}methoxy)chinazolin-7-yl}-4-methyl-5-(trifluormethyl)pyridin-2-amin;
6-{6-Chlor-4-[(1S,6R)-3,9-diazabicyclo[4.2.1]nonan-3-yl]-8-fluor-2-({1-[(4-fluorpiperidin-1-yl)methyl]cyclopropyl}methoxy)chinazolin-7-yl}-4-methyl-5-(trifluormethyl)pyridin-2-amin;
4-{4-[(1S,6R)-3,9-Diazabicyclo[4.2.1]nonan-3-yl]-2-({1-[(dimethylamino)methyl]cyclopropyl}methoxy)-8-fluorchinazolin-7-yl}-5-ethynyl-6-fluornaphthalin-2-ol;
4-(2-{[(2R,7aS)-2-Fluorhexahydro-1H-pyrrolizin-7a-yl]methoxy}-4-[(1S,6R)-3,9-diazabicyclo[4.2.1]nonan-3-yl]-8-fluorchinazolin-7-yl)-5-fluornaphthalin-2-ol;
4-(2-{[(2R,7aS)-2-Fluorhexahydro-1H-pyrrolizin-7a-yl]methoxy}-4-[(1R,6S)-3,9-diazabicyclo[4.2.1]nonan-3-yl]-8-fluorchinazolin-7-yl)-5-fluornaphthalin-2-ol;
4-{4-[(1S,6R)-3,9-Diazabicyclo[4.2.1]nonan-3-yl]-8-fluor-2-{[1-({3-oxa-8-azabicyclo[3.2.1]octan-8-yl}methyl)cyclopropyl]methoxy}chinazolin-7-yl}-5-ethylnaphthalin-2-ol;
1-(2-{ [(2R,7aS)-2-Fluorhexahydro-1H-pyrrolizin-7a-yl]methoxy}-4-[(1S,6R)-3,9-diazabicyclo[4.2.1]nonan-3-yl]-8-fluorchinazolin-7-yl)-8-fluorisoquinolin-3-amin;
4-(2-{[(2R,7aS)-2-Fluorhexahydro-1H-pyrrolizin-7a-yl]methoxy}-4-[(1S,6R)-3,9-diazabicyclo[4.2.1]nonan-3-yl]-8-fluorchinazolin-7-yl)-5-ethylnaphthalin-2-ol;
4-(2-{[(2R,7aS)-2-Fluorhexahydro-1H-pyrrolizin-7a-yl]methoxy}-4-[(1S,6R)-3,9-diazabicyclo[4.2.1]nonan-3-yl]-8-fluorchinazolin-7-yl)-5-ethynyl-6-fluornaphthalin-2-ol;
4-(2-{[(2R,7aS)-2-Fluorhexahydro-1H-pyrrolizin-7a-yl]methoxy}-4-[(1S,6R)-3,9-diazabicyclo[4.2.1]nonan-3-yl]-8-fluorchinazolin-7-yl)naphthalin-2-ol;
2-{[(2R,7aS)-2-Fluorhexahydro-1H-pyrrolizin-7a-yl]methoxy}-4-[(1S,6R)-3,9-diazabicyclo[4.2.1]nonan-3-yl]-7-(8-ethylnaphthalin-1-yl)-8-fluorchinazoline;
4-(2-{[(2R,7aS)-2-Fluorhexahydro-1H-pyrrolizin-7a-yl]methoxy}-4-[(1S,6R)-3,9-diazabicyclo[4.2.1]nonan-3-yl]-8-fluorchinazolin-7-yl)-5-fluornaphthalin-2-ol;
6-(2-{[(2R,7aR)-2-Fluorhexahydro-1H-pyrrolizin-7a-yl]methoxy}-4-[(1S,6R)-3,9-diazabicyclo[4.2.1]nonan-3-yl]-8-fluorchinazolin-7-yl)-4-methyl-5-(trifluormethyl)pyridin-2-amin;
6-(2-{[(2S,7aR)-2-Fluorhexahydro-1H-pyrrolizin-7a-yl]methoxy}-4-[(1S,6R)-3,9-diazabicyclo[4.2.1]nonan-3-yl]-8-fluorchinazolin-7-yl)-4-methyl-5-(trifluormethyl)pyridin-2-amin;
6-{4-[(1S,6R)-3,9-Diazabicyclo[4.2.1]nonan-3-yl]-2-[(2,2-difluor-hexahydro-1H-pyrrolizin-7a-yl)methoxy]-8-fluorchinazolin-7-yl}-4-methyl-5-(trifluormethyl)pyridin-2-amin; und
6-(2-{[(6'R,7'aR)-6'-Fluorhexahydrospiro[cyclopropane-1,2'-pyrrolizine]-7'a-yl]methoxy}-6-chlor-4-[(1S,6R)-3,9-diazabicyclo[4.2.1]nonan-3-yl]-8-fluorchinazolin-7-yl)-4-methyl-5-(trifluormethyl)pyridin-2-amin;
oder ein pharmazeutisch akzeptables Salz davon.

13. Pharmazeutische Zusammensetzung umfassend eine Verbindung nach einem der Ansprüche 1 bis 12 oder pharmazeutisch akzeptables Salz davon und einen oder mehrere pharmazeutisch akzeptable Trägerstoffe.

14. Verbindung nach einem der Ansprüche 1 bis 12 oder pharmazeutisch akzeptables Salz davon zur Verwendung bei einem Verfahren zum Behandeln von Krebs, wobei
(i) der Krebs Bauchspeicheldrüsenkrebs, Kolorektalkrebs, Lungenkrebs und/oder Magenkrebs ist;
(ii) der Krebs für KRAS G12D-Hemmung empfindlich ist; oder
(iii) der Krebs KRAS G12D-Mutation exprimiert.

## Revendications

1. Composé de formule (I) : 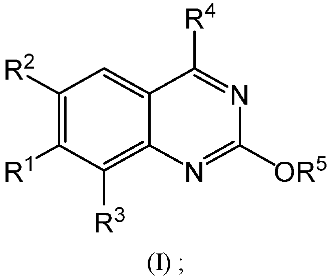 ou sel pharmaceutiquement acceptable de celui-ci ; où :
R¹ est un aryle ou un hétéroaryle, dans lequel l'aryle et l'hétéroaryle sont optionnellement substitués par un, deux, trois, quatre ou cinq substituants indépendamment choisis parmi : C₁-C₃ alkyle, C₂-C₄ alcényle, C₂-C₄ alcynyle, amino, amino-C₁-C₃ alkyle, C₃-C₄ cycloalkyle, halo, halo-C₁-C₃ alcoxy, halo-C₁-C₃ alkyle, hydroxy et hydroxy-C₁-C₃ alkyle ;
R² et R³ sont indépendamment choisis parmi : hydrogène, C₁-C₃ alcoxy, C₁-C₃ alkyle, cyano, halo, halo-C₁-C₃ alkyle et hydroxy ;
R⁴ est choisi parmi : 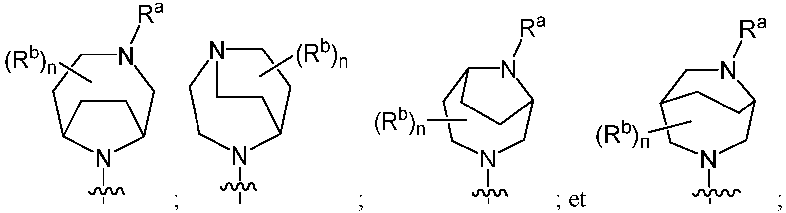 où :
R^{a} est un hydrogène ou un C₁-C₃ alkyle ;
n est 0, 1, 2, 3 ou 4 ;
chaque R^{b} est indépendamment choisi parmi : C₁-C₃ alkyle, C₃-C₆ cycloalkyle, halo et hydroxy ; ou, selon une variante, deux groupes R^{b} géminaux, accompagnés de l'atome de carbone auquel ils sont liés, peuvent former un cycle cycloalkyle à 3 à 6 chaînons ; et 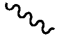
indique le point de liaison à la fraction moléculaire parente ;
R⁵ est un -(C₁-C₃ alkyl)-R⁶ ou un -(C₁-C₆ alkyl)NR^{c}R^{d}, dans lequel R⁶ est choisi parmi :
un C₃-C₆ cycloalkyle substitué par un NR^{c}R^{d}(C₁-C₃ alkyle) ; et
un cycle à cinq à dix chaînons monocyclique, bicyclique ou tricyclique contenant un atome d'azote et optionnellement un deuxième hétéroatome choisi parmi : oxygène ou azote,
dans lequel le cycle contient zéro à trois liaisons doubles et dans lequel le cycle est optionnellement substitué par un, deux ou trois groupés indépendamment choisis parmi : C₁-C₃ alcoxy, C₁-C₃ alkyle, halo, halo-C₁-C₃ alcoxy, halo-C₁-C₃ alkyle et hydroxy ; dans lequel :
R^{c} et R^{d}, accompagnés de l'atome d'azote auquel ils sont liés, forment un cycle à cinq à dix chaînons monocyclique ou bicyclique contenant optionnellement un hétéroatome supplémentaire choisi parmi : azote, oxygène et soufre, dans lequel le cycle est optionnellement substitué par un, deux ou trois groupes indépendamment choisis parmi : C₁-C₃ alcoxy, C₁-C₃ alcoxy-C₁-C₃ alkyle, C₁-C₃ alkyle, benzyle, halo, halo-C₁-C₃ alkyle, hydroxy, hydroxy-C₁-C₃ alkyle et oxo ; ou
un de R^{c} et R^{d} est choisi parmi : hydrogène et C₁-C₃ alkyle et l'autre est choisi parmi : hydrogène, C₁-C₃ alkyle, C₁-C₃ alcoxycarbonyle et C₁-C₃ alkylcarbonyle.

2. Composé selon la revendication 1, ou sel pharmaceutiquement acceptable de celui-ci ; dans lequel R⁴ est choisi parmi :

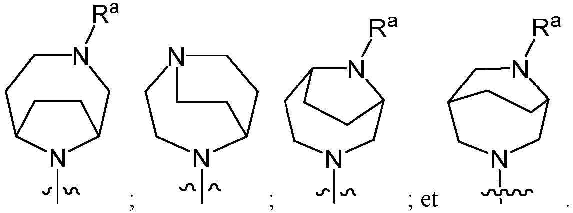

3. Composé selon la revendication 1 ou 2, sel pharmaceutiquement acceptable de celui-ci, dans lequel R² et R³ sont chacun un halo.

4. Composé selon l'une quelconque des revendications 1 à 3, ou sel pharmaceutiquement acceptable de celui-ci, dans lequel R⁵ est un -(C₁-C₃ alkyl)-R⁶.

5. Composé selon l'une quelconque des revendications 1 à 4, ou sel pharmaceutiquement acceptable de celui-ci, dans lequel R⁵ est choisi parmi : 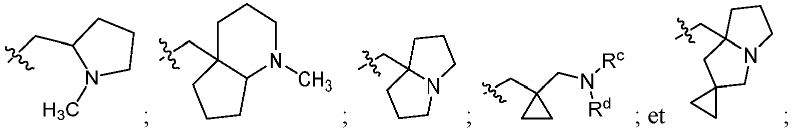 où chaque cycle est optionnellement substitué par 1 ou 2 groupes indépendamment choisis parmi : C₁-C₃ alcoxy, C₁-C₃ alkyle, halo, halo-C₁-C₃ alkyle et hydroxy.

6. Composé selon l'une quelconque des revendications 1 à 4, ou sel pharmaceutiquement acceptable de celui-ci, dans lequel :
(i) R⁵ est : 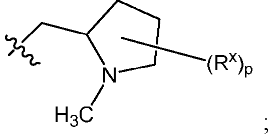 où p est 0, 1 ou 2 ; et où chaque R^{x} est indépendamment choisi parmi : C₁-C₃ alcoxy, C₁-C₃ alkyle, halo, halo-C₁-C₃ alkyle et hydroxy ; ou
(ii) R⁵ est : 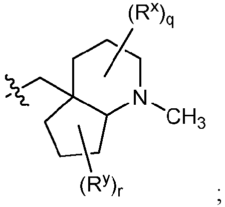 où q et r sont chacun indépendamment 0 ou 1; et où R^{x} et R^{y} sont indépendamment choisis parmi : C₁-C₃ alcoxy, C₁-C₃ alkyle, halo, halo-C₁-C₃ alkyle et hydroxy ; ou
(iii) R⁵ est : 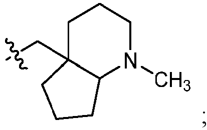 ou
(iv) R⁵ est : 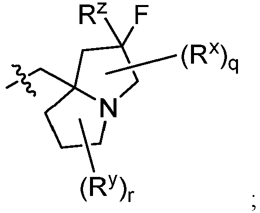 où q et r sont chacun indépendamment 0 ou 1 ; dans lequel R^{x} et R^{y} sont indépendamment choisis parmi : C₁-C₃ alcoxy, C₁-C₃ alkyle, halo, halo-C₁-C₃ alkyle et hydroxy ; et dans lequel R^{z} est un hydrogène ou un fluoro ; ou
(v) R⁵ est : 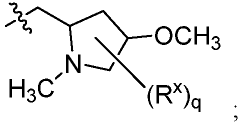 où q est 0 ou 1 ; et dans lequel R^{x} est choisi parmi : C₁-C₃ alcoxy, C₁-C₃ alkyle, halo, halo-C₁-C₃ alkyle et hydroxy ; ou
(vi) R⁵ est : 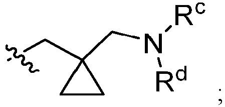 ou
(vii) R⁵ est : 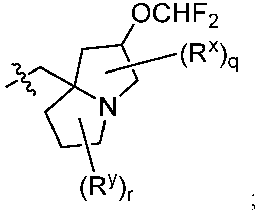 où q et r sont chacun indépendamment 0 ou 1 ; et dans lequel R^{x} et R^{y} sont indépendamment choisis parmi : C₁-C₃ alcoxy, C₁-C₃ alkyle, halo, halo-C₁-C₃ alkyle et hydroxy ; ou
(viii) R⁵ est: 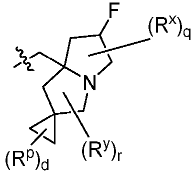 où q, r et d sont chacun indépendamment 0 ou 1 ; et dans lequel R^{x}, R^{y} et R^{p} sont indépendamment choisis parmi : C₁-C₃ alcoxy, C₁-C₃ alkyle, halo, halo-C₁-C₃ alkyle et hydroxy ; ou
(ix) R⁵ est :

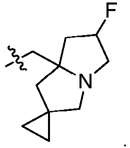

7. Composé selon l'une quelconque des revendications 1 à 6, ou sel pharmaceutiquement acceptable de celui-ci, dans lequel :
(i) R¹ est un naphtyle, dans lequel le naphtyle est substitué par un groupe hydroxy et est en outre optionnellement substitué par un ou deux groupes supplémentaires choisis parmi : C₁-C₃ alkyle, C₂-C₄ alcynyle et halo ; ou
(ii) R¹ est : 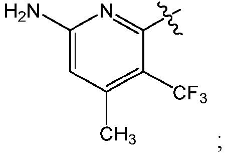 ou
(iii) R¹ est : 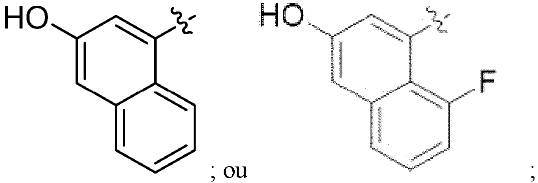 ou
(iv) R¹ est : 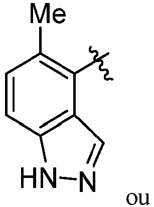
(v) R¹ est :

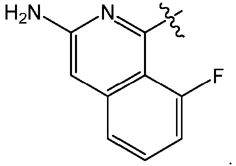

8. Composé selon la revendication 1, ou sel pharmaceutiquement acceptable de celui-ci, dans lequel :
R² est un hydrogène ;
R³ est un fluoro ;
R¹ est choisi parmi : 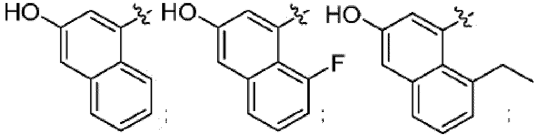 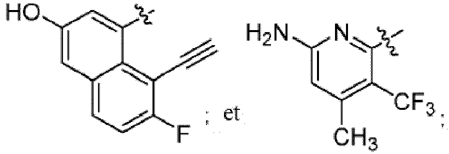 et
R⁵ est choisi parmi : 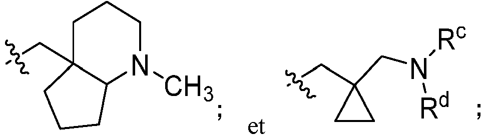 où 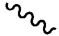
indique le point de liaison à la fraction moléculaire parente.

9. Composé selon la revendication 1, ou sel pharmaceutiquement acceptable de celui-ci, dans lequel :
R² est un chloro ;
R³ est un fluoro ;
R¹ est choisi parmi : 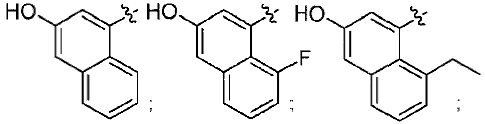 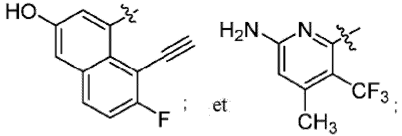 et
R⁵ est choisi parmi : 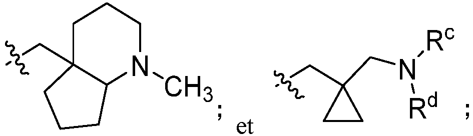 où 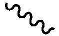
indique le point de liaison à la fraction moléculaire parente.

10. Composé selon la revendication 1 de formule (II) : 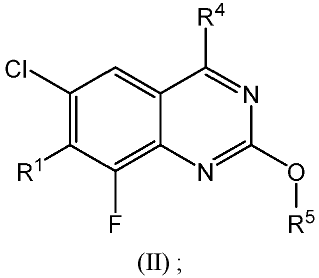 ou sel pharmaceutiquement acceptable de celui-ci, où :
R¹ est choisi parmi : 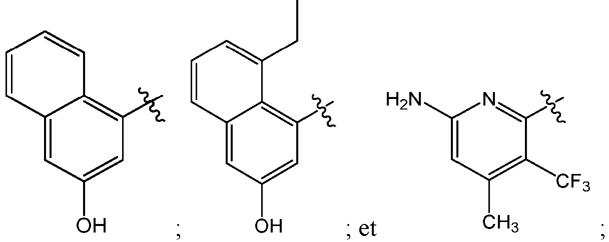
où 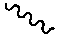 indique le point de liaison à la fraction moléculaire parente ;
R⁴ est choisi parmi : 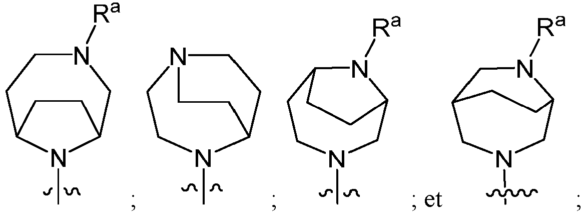 où
R^{a} est un hydrogène ou un C₁-C₃ alkyle ; et 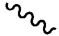
indique le point de liaison à la fraction moléculaire parente ; et
R⁵ est choisi parmi : 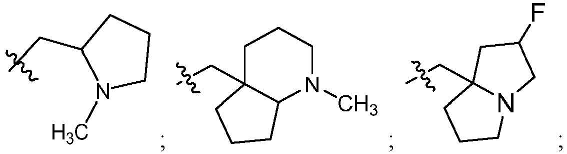 et 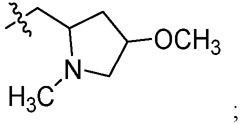 dans lequel : 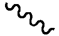 indique le point de liaison à la fraction moléculaire parente.

11. Composé selon la revendication 1 choisi parmi : 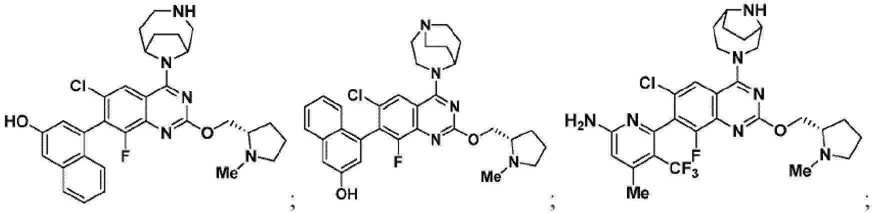 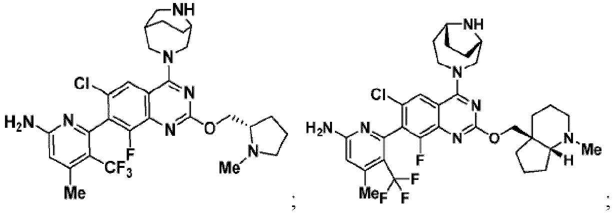 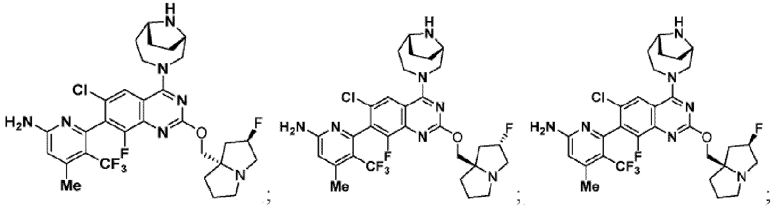 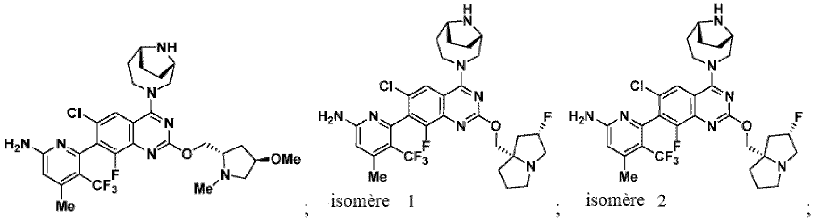 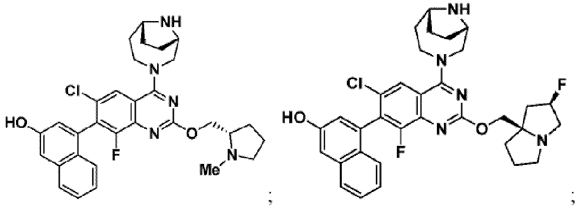 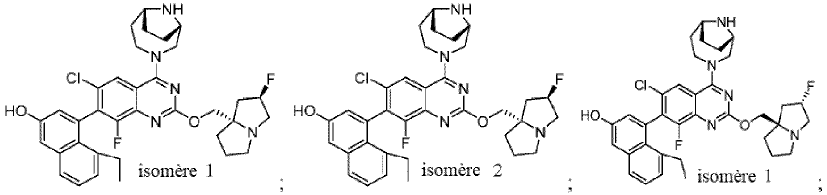 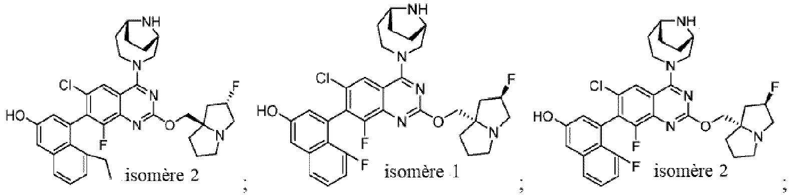 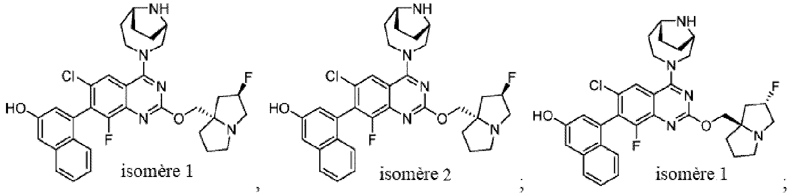 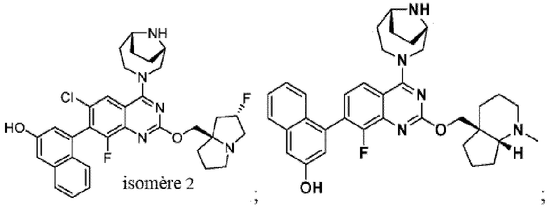 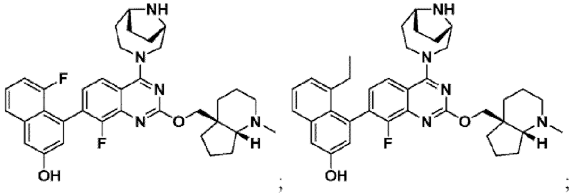 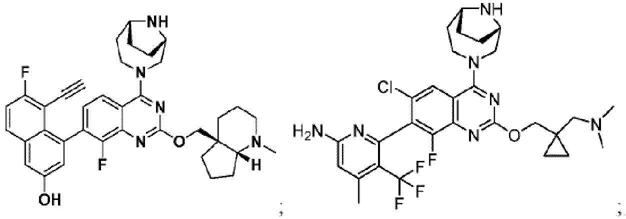 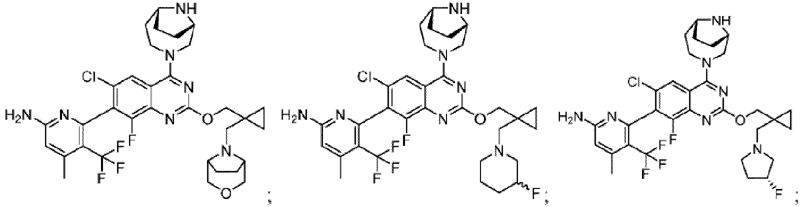 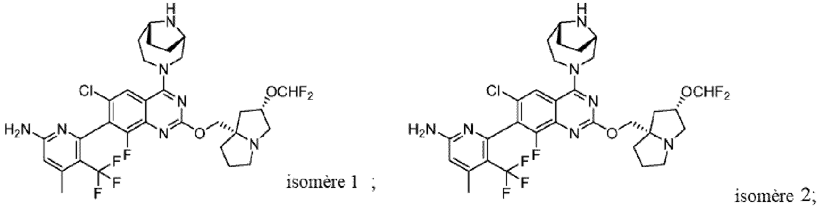 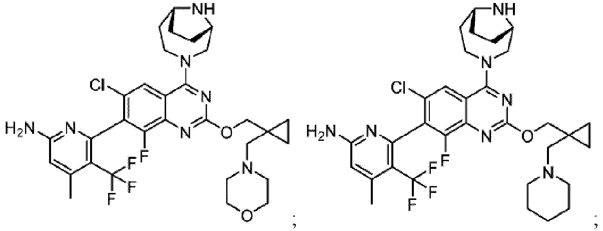 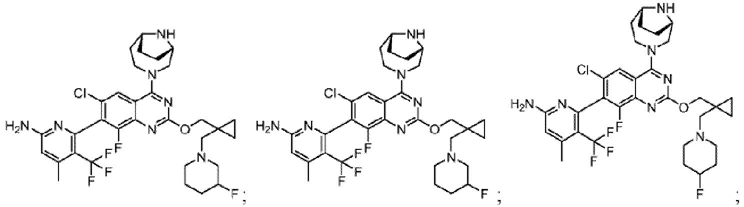 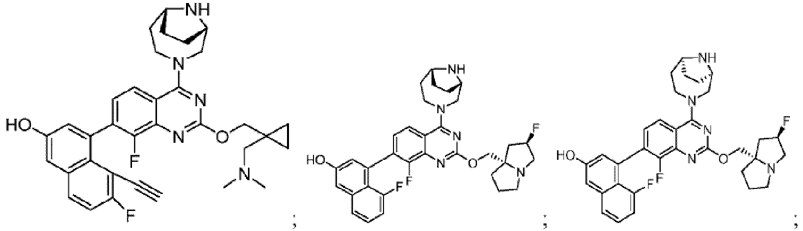 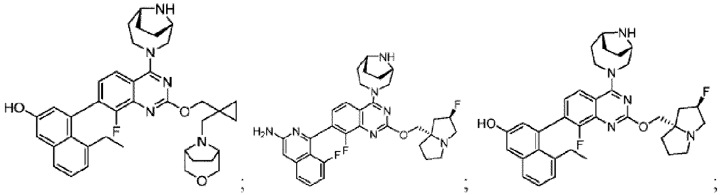 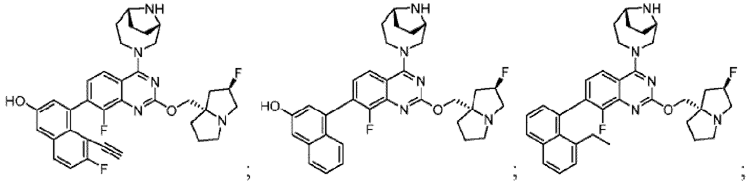 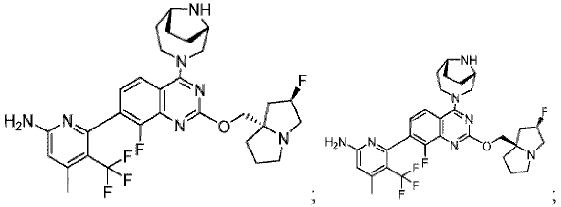 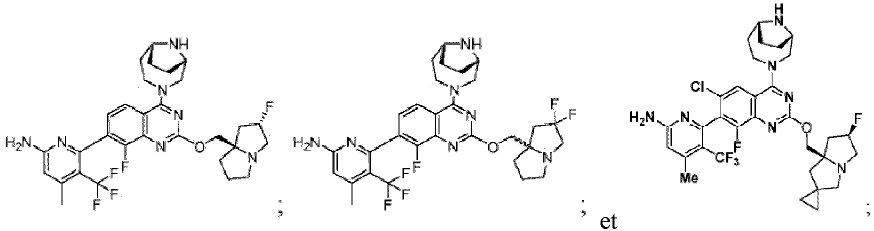 ou un sel pharmaceutiquement acceptable de ceux-ci.

12. Composé selon la revendication 1 choisi parmi :
4-(6-chloro-4-{3,9-diazabicyclo[4.2.1]nonan-9-yl}-8-fluoro-2-{[(2S)-1-méthylpyrrolidin-2-yl]méthoxy}quinazolin-7-yl)naphtalén-2-ol ;
4-(6-chloro-4-{1,4-diazabicyclo[3.2.2]nonan-4-yl}-8-fluoro-2-{[(2S)-1-méthylpyrrolidin-2-yl]méthoxy}quinazolin-7-yl)naphtalén-2-ol ;
6-(6-chloro-4-{3,9-diazabicyclo[4.2.1]nonan-3-yl}-8-fluoro-2-{[(2S)-1-méthylpyrrolidin-2-yl]méthoxy}quinazolin-7-yll)-4-méthyl-5-(trifluorométhyl)pyridin-2-amine ;
6-(6-chloro-4-{3,6-diazabicyclo[3.2.2]nonan-3-yl}-8-fluoro-2-{[(2S)-1-méthylpyrrolidin-2-yl]méthoxy}quinazolin-7-yl)-4-méthyl-5-(trifluorométhyl)pyridin-2-amine ;
6-(2-{[(4aS,7aR)-1-méthyl-octahydro-1H-cyclopenta[b]pyridin-4a-yl]méthoxy}-6-chloro-4-[(1S,6R)-3,9-diazabicyclo[4.2.1]nonan-3-yl]-8-fluoroquinazolin-7-yl)-4-méthyl-5-(trifluorométhyl)pyridin-2-amine ;
6-(2-{[(2R,7aS)-2-fluoro-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)-4-méthyl-5-(trifluorométhyl)pyridin-2-amine ;
6-(2-{[(2S,7aR)-2-fluoro-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)-4-méthyl-5-(trifluorométhyl)pyridin-2-amine ;
6-(2-{[(2R,7aR)-2-fluoro-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)-4-méthyl-5-(trifluorométhyl)pyridin-2-amine ;
6-{6-chloro-4-[(1S,6R)-3,9-diazabicyclo[4.2.1]nonan-3-yl]-8-fluoro-2-{[(2S,4R)-4-méthoxy-1-méthylpyrrolidin-2-yl]méthoxy}quinazolin-7-yl}-4-méthyl-5-(trifluorométhyl)pyridin-2-amine ;
6-(2-{[(2S,7aS)-2-fluoro-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)-4-méthyl-5-(trifluorométhyl)pyridin-2-amine ;
6-(2-{[(2S,7aS)-2-fluoro-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)-4-méthyl-5-(trifluorométhyl)pyridin-2-amine, Isomère 1 ;
6-(2-{[(2S,7aS)-2-fluoro-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)-4-méthyl-5-(trifluorométhyl)pyridin-2-amine, Isomère 2 ;
4-{6-chloro-4-[(1S,6R)-3,9-diazabicyclo[4.2.1]nonan-3-yl]-8-fluoro-2-{[(2S)-1-méthylpyrrolidin-2-yl]méthoxy}quinazolin-7-yl}naphtalén-2-ol ;
4-(2-{[(2R,7aR)-2-fluoro-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)naphtalén-2-ol ;
4-(2-{[(2R,7aS)-2-fluoro-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)-5-éthylnaphtalén-2-ol ;
4-(2-{[(2R,7aS)-2-fluoro-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)-5-éthylnaphtalén-2-ol, Isomère 1 ;
4-(2-{[(2R,7aS)-2-fluoro-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)-5-éthylnaphtalén-2-ol, Isomère 2 ;
4-(2-{[(2S,7aR)-2-fluoro-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)-5-éthylnaphtalén-2-ol ;
4-(2-{[(2S,7aR)-2-fluoro-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)-5-éthylnaphtalén-2-ol, Isomère 1 ;
4-(2-{[(2S,7aR)-2-fluoro-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)-5-éthylnaphtalén-2-ol, Isomère 2 ;
4-(2-{[(2R,7aS)-2-fluoro-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)-5-fluoronaphtalén-2-ol ;
4-(2-{[(2R,7aS)-2-fluoro-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)-5-fluoronaphtalén-2-ol, Isomère 1 ;
4-(2-{[(2R,7aS)-2-fluoro-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)-5-fluoronaphtalén-2-ol, Isomère 2 ;
4-(2-{[(2R,7aS)-2-fluoro-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)naphtalén-2-ol ;
4-(2-{[(2R,7aS)-2-fluoro-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)naphtalén-2-ol, Isomère 1 ;
4-(2-{[(2R,7aS)-2-fluoro-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)naphtalén-2-ol, Isomère 2 ;
4-(2-{[(2S,7aR)-2-fluoro-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)naphtalén-2-ol ;
4-(2-{[(2S,7aR)-2-fluoro-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)naphtalén-2-ol, Isomère 1 ;
4-(2-{[(2S,7aR)-2-fluoro-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)naphtalén-2-ol, Isomère 2 ;
4-(2-{[(4aS,7aR)-1-méthyl-octahydro-1H-cyclopenta[b]pyridin-4a-yl]méthoxy}-4-[(1S,6R)-3,9-diazabicyclo[4.2.1]nonan-3-yl]-8-fluoroquinazolin-7-yl)naphtalén-2-ol ;
4-(2-{[(4aS,7aR)-1-méthyl-octahydro-1H-cyclopenta[b]pyridin-4a-yl]méthoxy}-4-[(1S,6R)-3,9-diazabicyclo[4.2.1]nonan-3-yl]-8-fluoroquinazolin-7-yl)-5-fluoronaphtalén-2-ol ;
4-(2-{[(4aS,7aR)-1-méthyl-octahydro-1H-cyclopenta[b]pyridin-4a-yl]méthoxy}-4-[(1S,6R)-3,9-diazabicyclo[4.2.1]nonan-3-yl]-8-fluoroquinazolin-7-yl)-5-éthylnaphtalén-2-ol ;
4-(2-{[(4aS,7aR)-1-méthyl-octahydro-1H-cyclopenta[b]pyridin-4a-yl]méthoxy}-4-[(1S,6R)-3,9-diazabicyclo[4.2.1]nonan-3-yl]-8-fluoroquinazolin-7-yl)-5-éthynyl-6-fluoronaphtalén-2-ol ;
6-{6-chloro-4-[(1S,6R)-3,9-diazabicyclo[4.2.1]nonan-3-yl]-2-({1-[(diméthylamino)méthyl]cyclopropyl}méthoxy)-8-fluoroquinazolin-7-yl}-4-méthyl-5-(trifluorométhyl)pyridin-2-amine ;
6-{6-chloro-4-[(1S,6R)-3,9-diazabicyclo[4.2.1]nonan-3-yl]-8-fluoro-2-{[1-({3-oxa-8-azabicyclo[3.2.1]octan-8-yl}méthyl)cyclopropyl]méthoxy}quinazolin-7-yl}-4-méthyl-5-(trifluorométhyl)pyridin-2-amine ;
6-{6-chloro-4-[(1S,6R)-3,9-diazabicyclo[4.2.1]nonan-3-yl]-8-fluoro-2-({1-[(3-fluoropipéridin-1-yl)méthyl]cyclopropyl}méthoxy)quinazolin-7-yl}-4-méthyl-5-(trifluorométhyl)pyridin-2-amine ;
6-{6-chloro-4-[(1S,6R)-3,9-diazabicyclo[4.2.1]nonan-3-yl]-8-fluoro-2-[(1-{[(3R)-3-fluoropyrrolidin-1-yl]méthyl}cyclopropyl)méthoxy]quinazolin-7-yl}-4-méthyl-5-(trifluorométhyl)pyridin-2-amine ;
6-(2-{[(2S,7aS)-2-(difluorométhoxy)-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)-4-méthyl-5-(trifluorométhyl)pyridin-2-amine isomère 1 ;
6-(2-{[(2S,7aS)-2-(difluorométhoxy)-hexahydro-1H-pyrrolizin-7a-yl]méthoxy}-6-chloro-4-[(1S,6R)-3,9-diazabicyclo[4.2.1]nonan-3-yl]-8-fluoroquinazolin-7-yl)-4-méthyl-5-(trifluorométhyl)pyridin-2-amine isomère 2 ;
6-{6-chloro-4-[(1S,6R)-3,9-diazabicyclo[4.2.1]nonan-3-yl]-8-fluoro-2-({1-[(morpholin-4-yl)méthyl]cyclopropyl}méthoxy)quinazolin-7-yl}-4-méthyl-5-(trifluorométhyl)pyridin-2-amine ;
6-{6-chloro-4-[(1S,6R)-3,9-diazabicyclo[4.2.1]nonan-3-yl]-8-fluoro-2-({1-[(pipéridin-1-yl)méthyl]cyclopropyl}méthoxy)quinazolin-7-yl}-4-méthyl-5-(trifluorométhyl)pyridin-2-amine ;
6-{6-chloro-4-[(1S,6R)-3,9-diazabicyclo[4.2.1]nonan-3-yl]-8-fluoro-2-({1-[(3-fluoropipéridin-1-yl)méthyl]cyclopropyl}méthoxy)quinazolin-7-yl}-4-méthyl-5-(trifluorométhyl)pyridin-2-amine ;
6-{6-chloro-4-[(1S,6R)-3,9-diazabicyclo[4.2.1]nonan-3-yl]-8-fluoro-2-({1-[(3-fluoropipéridin-1-yl)méthyl]cyclopropyl}méthoxy)quinazolin-7-yl}-4-méthyl-5-(trifluorométhyl)pyridin-2-amine ;
6-{6-chloro-4-[(1S,6R)-3,9-diazabicyclo[4.2.1]nonan-3-yl]-8-fluoro-2-({1-[(4-fluoropipéridin-1-yl)méthyl]cyclopropyl}méthoxy)quinazolin-7-yl}-4-méthyl-5-(trifluorométhyl)pyridin-2-amine ;
4-{4-[(1S,6R)-3,9-diazabicyclo[4.2.1]nonan-3-yl]-2-({1-[(diméthylamino)méthyl]cyclopropyl}méthoxy)-8-fluoroquinazolin-7-yl}-5-éthynyl-6-fluoronaphtalén-2-ol ;
4-(2-{[(2R,7aS)-2-fluoro-hexahydro-1H-pyrrolizin-7a-yl]méthoxy}-4-[(1S,6R)-3,9-diazabicyclo[4.2.1]nonan-3-yl]-8-fluoroquinazolin-7-yl)-5-fluoronaphtalén-2-ol ;
4-(2-{[(2R,7aS)-2-fluoro-hexahydro-1H-pyrrolizin-7a-yl]méthoxy}-4-[(1R,6S)-3,9-diazabicyclo[4.2.1]nonan-3-yl]-8-fluoroquinazolin-7-yl)-5-fluoronaphtalén-2-ol ;
4-{4-[(1S,6R)-3,9-diazabicyclo[4.2.1]nonan-3-yl]-8-fluoro-2-{[1-({3-oxa-8-azabicyclo[3.2.1]octan-8-yl}méthyl)cyclopropyl]méthoxy}quinazolin-7-yl}-5-éthylnaphtalén-2-ol ;
1-(2-{[(2R,7aS)-2-fluoro-hexahydro-1H-pyrrolizin-7a-yl]méthoxy}-4-[(1S,6R)-3,9-diazabicyclo[4.2.1]nonan-3-yl]-8-fluoroquinazolin-7-yl)-8-fluoroisoquinoléin-3-amine ;
4-(2-{[(2R,7aS)-2-fluoro-hexahydro-1H-pyrrolizin-7a-yl]méthoxy}-4-[(1S,6R)-3,9-diazabicyclo[4.2.1]nonan-3-yl]-8-fluoroquinazolin-7-yl)-5-éthylnaphtalén-2-ol ;
4-(2-{[(2R,7aS)-2-fluoro-hexahydro-1H-pyrrolizin-7a-yl]méthoxy}-4-[(1S,6R)-3,9-diazabicyclo[4.2.1]nonan-3-yl]-8-fluoroquinazolin-7-yl)-5-éthynyl-6-fluoronaphtalén-2-ol ;
4-(2-{[(2R,7aS)-2-fluoro-hexahydro-1H-pyrrolizin-7a-yl]méthoxy}-4-[(1S,6R)-3,9-diazabicyclo[4.2.1]nonan-3-yl]-8-fluoroquinazolin-7-yl)naphtalén-2-ol ;
2-{[(2R,7aS)-2-fluoro-hexahydro-1H-pyrrolizin-7a-yl]méthoxy}-4-[(1S,6R)-3,9-diazabicyclo[4.2.1]nonan-3-yl]-7-(8-éthylnaphtalén-1-yl)-8-fluoroquinazoline ;
4-(2-{[(2R,7aS)-2-fluoro-hexahydro-1H-pyrrolizin-7a-yl]méthoxy}-4-[(1S,6R)-3,9-diazabicyclo[4.2.1]nonan-3-yl]-8-fluoroquinazolin-7-yl)-5-fluoronaphtalén-2-ol ;
6-(2-{[(2R,7aR)-2-fluoro-hexahydro-1H-pyrrolizin-7a-yl]méthoxy}-4-[(1S,6R)-3,9-diazabicyclo[4.2.1]nonan-3-yl]-8-fluoroquinazolin-7-yl)-4-méthyl-5-(trifluorométhyl)pyridin-2-amine ;
6-(2-{[(2S,7aR)-2-fluoro-hexahydro-1H-pyrrolizin-7a-yl]méthoxy}-4-[(1S,6R)-3,9-diazabicyclo[4.2.1]nonan-3-yl]-8-fluoroquinazolin-7-yl)-4-méthyl-5-(trifluorométhyl)pyridin-2-amine ;
6-{4-[(1S,6R)-3,9-diazabicyclo[4.2.1]nonan-3-yl]-2-[(2,2-difluoro-hexahydro-1H-pyrrolizin-7a-yl)méthoxy]-8-fluoroquinazolin-7-yl}-4-méthyl-5-(trifluorométhyl)pyridin-2-amine ; et
6-(2-{[(6'R,7'aR)-6'-fluoro-hexahydrospiro[cyclopropane-1,2'-pyrrolizine]-7'a-yl]méthoxy}-6-chloro-4-[(1S,6R)-3,9-diazabicyclo[4.2.1]nonan-3-yl]-8-fluoroquinazolin-7-yl)-4-méthyl-5-(trifluorométhyl)pyridin-2-amine ;
ou un sel pharmaceutiquement acceptable de ceux-ci.

13. Composition pharmaceutique comprenant un composé selon l'une quelconque des revendications 1 à 12, ou un sel pharmaceutiquement acceptable de celui-ci, et un ou plusieurs excipients pharmaceutiquement acceptables.

14. Composé selon l'une quelconque des revendications 1 à 12, ou sel pharmaceutiquement acceptable de celui-ci, pour utilisation dans une méthode de traitement d'un cancer, où :
(i) le cancer est un cancer pancréatique, un cancer colorectal, un cancer du poumon et/ou un cancer gastrique ;
(ii) le cancer est sensible à l'inhibition de KRAS G12D ; ou
(iii) le cancer exprime une mutation de KRAS G12D.
